(19) Europäisches Patentamt
European Patent Office
Office européen des brevets

(11) EP 1 656 181 B1

(12) EUROPEAN PATENT SPECIFICATION

(45) Date of publication and mention of the grant of the patent:
**04.06.2008 Bulletin 2008/23**

(21) Application number: **04781543.6**

(22) Date of filing: **18.08.2004**

(51) Int Cl.:
***A61N 1/362*** *(2006.01)*

(86) International application number:
**PCT/US2004/026883**

(87) International publication number:
**WO 2005/018737 (03.03.2005 Gazette 2005/09)**

# (54) DISORDERED BREATHING MANAGEMENT SYSTEM

STEUERUNGSGERÄT FÜR UNREGELMÄSSIGE ATMUNG

SYSTEME DE GESTION DES TROUBLES RESPIRATOIRES

(84) Designated Contracting States:
**AT BE BG CH CY CZ DE DK EE ES FI FR GB GR HU IE IT LI LU MC NL PL PT RO SE SI SK TR**

(30) Priority:
**18.08.2003 US 642998**
**18.08.2003 US 643203**
**18.08.2003 US 643006**
**18.08.2003 US 643016**
**18.08.2003 US 643154**
**18.09.2003 US 504229 P**
**11.03.2004 US 798794**
**15.04.2004 US 824776**
**17.08.2004 US 920675**
**17.08.2004 US 920568**
**17.08.2004 US 920569**
**17.08.2004 US 920549**

(43) Date of publication of application:
**17.05.2006 Bulletin 2006/20**

(60) Divisional application:
**08006191.4**

(73) Proprietor: **CARDIAC PACEMAKERS, INC.**
**St. Paul, Minnesota 55112-5798 (US)**

(72) Inventors:
- **STAHMANN, Jeffrey, E.**
**Ramsey, MN 55303 (US)**
- **LEE, Kent**
**Fridley,MN 55432 (US)**
- **HARTLEY, Jesse, W.**
**Lino lakes, MN 55014 (US)**
- **NI, Quan**
**Shoreview, MN 55126 (US)**
- **HATLESTAD, John, D.**
**Maplewood, MN 55117 (US)**
- **LOVETT, Eric, G.**
**Mendota Heights, Minnesota 55120 (US)**
- **KENKNIGHT, Bruce, H.**
**Maple Grove, Minnesota 55311 (US)**

(74) Representative: **Charig, Raymond Julian**
**Potter Clarkson LLP**
**Park View House**
**58 The Ropewalk**
**Nottingham NG1 5DD (GB)**

(56) References cited:
**EP-A- 1 172 125**
**US-A- 5 466 245**
**US-A- 5 645 570**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 656 181 B1

## Description

### FIELD OF THE INVENTION

The present invention relates generally to diagnosis and therapy for disordered breathing.

### BACKGROUND OF THE INVENTION

Disordered breathing may be caused by a wide spectrum of respiratory conditions involving the disruption of the normal respiratory cycle. Although disordered breathing often occurs during sleep, the condition may also occur while the patient is awake. Respiratory disruption can be particularly serious for patients concurrently suffering from cardiovascular deficiencies, such as congestive heart failure. Unfortunately, disordered breathing is often undiagnosed. If left untreated, the effects of disordered breathing may result in serious health consequences for the patient.

Various types of disordered respiration have been identified, including, for example, apnea, hypopnea, dyspnea, hyperpnea, tachypnea, and periodic breathing, including Cheyne-Stokes respiration (CSR). Apnea is a fairly common disorder characterized by periods of interrupted breathing. Apnea is typically classified based on its etiology. One type of apnea, denoted obstructive apnea, occurs when the patient's airway is obstructed by the collapse of soft tissue in the rear of the throat. Central apnea is caused by a derangement of the central nervous system control of respiration. The patient ceases to breathe when control signals from the brain to the respiratory muscles are absent or interrupted. Mixed apnea is a combination of the central and obstructive apnea types. Regardless of the type of apnea, people experiencing an apnea event stop breathing for a period of time. The cessation of breathing may occur repeatedly during sleep, sometimes hundreds of times a night and sometimes for a minute or longer.

In addition to apnea, other types of disordered respiration cycles have been identified, including hypopnea (shallow breathing), tachypnea (rapid breathing), hyperpnea (heavy breathing), and dyspnea (labored breathing). Combinations of the respiratory cycles described above may be observed, including, for example, periodic breathing and Cheyne-Stokes respiration (CSR). Periodic breathing is characterized by cyclic respiratory patterns that may exhibit rhythmic rises and falls in tidal volume. Cheyne-Stokes respiration is a specific form of periodic breathing wherein the tidal volume decreases to zero resulting in apneic intervals. The breathing interruptions of periodic breathing and CSR may be associated with central apnea, or may be obstructive in nature. CSR is frequently observed in patients with congestive heart failure (CHF) and is associated with an increased risk of accelerated CHF progression. Because of the cardiovascular implications, therapy for respiration-related sleep disorders is of particular interest.

US 5645570 discloses an implantable device able to measure sympatho-vagal activity in a patient to allow the possible piloting of a pharmacological and/or electrical therapeutic action effected by means of a separate implantable device, the implantable devices forming a single implantable assembly.

### SUMMARY OF THE INVENTION

The invention provides a medical device according to claim 1.

Disclosed herein are methods and systems for providing an adaptive therapy for disordered breathing. Systems and methods disclosed herein are further directed to adapting a cardiac electrical therapy to treat disordered breathing. Systems and methods disclosed herein are also directed to delivery of cardiac electrical stimulation therapy adapted to treat disordered breathing.

According to one aspect of the disclosure, a medical therapy device includes a sensing system configured to sense conditions affecting a patient, and a therapy control system coupled to the sensing system and configured to adapt a cardiac electrical therapy to treat disordered breathing. The device further includes a cardiac electrical stimulation generator coupled to the therapy control system and configured to deliver the adapted therapy to the patient.

According to another aspect, an automated therapy method involves receiving sensory information indicative of one or more physiological or non-physiological conditions, and adapting a cardiac electrical therapy to treat disordered breathing based on the sensory information. The method further involves generating an output signal deliverable to a heart based on the adapted cardiac electrical therapy. Preferably, at least two of receiving the sensory information, adapting the cardiac electrical therapy, and generating the output signal are performed using an implantable component.

The above summary of the present invention is not intended to describe each embodiment or every implementation of the present invention. Advantages and attainments, together with a more complete understanding of the invention, will become apparent and appreciated by referring to the following detailed description and claims taken in conjunction with the accompanying drawings.

## BRIEF DESCRIPTION OF THE DRAWINGS

Figure 1 is a flowchart illustrating a method for providing disordered breathing therapy in accordance with embodiments of the invention;

Figure 2 is a block diagram of a system configured to provide cardiac electrical therapy to treat disordered breathing in accordance with embodiments of the invention;

Figure 3 is a partial view of an implantable device that may include circuitry for implementing cardiac stimulation therapy for disordered breathing in accordance with embodiments of the invention;

Figure 4A is a view of a transthoracic cardiac sensing and/or stimulation device as implanted in a patient in accordance with an embodiment of the present invention;

Figure 4B is a block diagram illustrating various components of a transthoracic cardiac sensing and/or stimulation device in accordance with an embodiment of the present invention;

Figures 4C-4E are diagrams illustrating various components of a transthoracic cardiac sensing and/or stimulation device located in accordance with embodiments of the invention;

Figures 4F-4H are diagrams illustrating electrode subsystem placement relative to a heart in accordance with embodiments of the invention;

Figure 5A illustrates a normal respiration pattern as represented by a transthoracic impedance sensor signal;

Figure 5B illustrates a flowchart of a methodology for adapting the disordered breathing therapy in accordance with embodiments of the invention;

Figure 6 illustrates respiration intervals used for disordered breathing detection according to embodiments of the invention;

Figure 7 illustrates respiration intervals used in detection of sleep apnea and severe sleep apnea according to embodiments of the invention;

Figures 8A-B are graphs of respiration patterns that may be detected according to embodiments of the invention;

Figure 9 is a flow chart illustrating a method of apnea and hypopnea detection according to embodiments of the invention;

Figure 10 is a graph illustrating a breathing interval according to embodiments of the invention;

Figure 11 is a graph illustrating a hypopnea detection approach in accordance with embodiments of the invention;

Figures 12-13 are charts illustrating nomenclature for individual disordered breathing events and combinations of disordered breathing events that can be addressed in accordance with embodiments of the invention, respectively;

Figures 14A-14E are graphs illustrating disordered breathing events comprising a mixture of apnea and hypopnea respiration cycles;

Figure 14F is a graph illustrating a periodic breathing respiration pattern that may be detected according to embodiments of the invention;

Figure 14G is a graph illustrating a Cheyne-Stokes respiration pattern that may be detected according to embodiments of the invention;

Figure 15 is a flowchart of a method for detecting disordered breathing in accordance with an embodiment of the invention;

Figure 16A is a flowchart of a method of classifying a disordered breathing event in accordance with embodiments of the invention;

Figure 16B is a block diagram of disordered breathing classification circuitry that may be implemented in accordance with embodiments of the invention;

Figures 16C and 16D provide graphs of accelerometer signals representing chest wall motion for central and obstructive disordered breathing, respectively in accordance with embodiments of the invention;

Figures 16E and 16F are flowcharts of a method for classifying disordered breathing events as central, obstructive or mixed events in accordance with embodiments of the invention;

Figure 16G is a flowchart illustrating a method for triggering disordered breathing therapy based on a prediction of disordered breathing according to embodiments of the invention;

Figure 17 is a block diagram of a disordered breathing therapy system including disordered breathing prediction functionality in accordance with embodiments of the invention;

Figure 18 is a diagram conceptually illustrating how conditions affecting the patient may be used in predicting disordered breathing in accordance with embodiments of the invention;

Figure 19 is a diagram conceptually illustrating the operation of a disordered breathing prediction engine in accordance with embodiments of the invention;

Figure 20 is a flowchart illustrating a method for establishing and updating the prediction criteria library according to embodiments of the invention;

Figure 21 is a flowchart illustrating a method of adapting a disordered breathing therapy according to embodiments of the invention;

Figure 22 is a flowchart illustrating a method of adapting a disordered breathing therapy taking into account both therapy effectiveness and therapy impact in accordance with embodiments of the invention;

Figure 23 illustrates a patient instrumented with a sleep quality monitor according to embodiments of the invention;

Figure 24 is a block diagram of a sleep detector that may be used in connection with a therapy system for disordered breathing in accordance with embodiments of the invention;

Figure 25 is a flowchart illustrating a method of detecting sleep according to embodiments of the invention;

Figure 26 is a flowchart illustrating a method for detecting sleep using accelerometer and minute ventilation signals according to embodiments of the invention;

Figures 27 and 28 are graphs illustrating a patient's activity and heart rate, respectively;

Figure 29 is a graph of the patient's minute ventilation signal;

Figure 30 illustrates adjustment of the activity sleep threshold in accordance with embodiments of the invention;

Figure 31 are graphs of autonomic arousal responses which may be utilized by the disordered breathing therapy system in accordance with embodiments of the invention;

Figure 32 depicts a flow diagram illustrating various optional processes that may be implemented in connection with arousal detection according to embodiments of the invention;

Figure 33 is a block diagram of an arousal detector that may be implemented in cooperation with a cardiac electrical therapy system in accordance with embodiments of the invention;

Figures 34 and 35 are block diagrams illustrating a controller configured to receive one or more inputs for modifying the rate at which cardiac pacing for disordered breathing is delivered in accordance with embodiments of the invention;

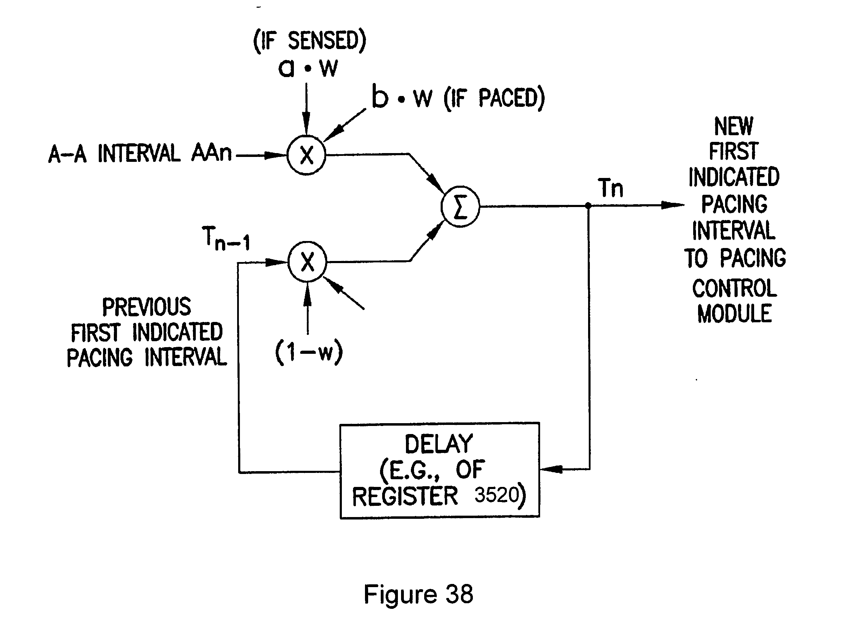

Figures 36-38 are signal flow diagrams illustrating pacing rate adjustment in accordance with embodiments of the invention;

Figure 39 is a block diagram illustrating a controller that includes several different inputs to modify the rate at which pacing or other therapy is delivered based on disordered breathing detection in accordance with embodiments of the invention;

Figures 40 and 41 are graphs illustrating modification of a pacing rate in accordance with embodiments of the invention;

Figure 42 is a graph illustrating a method of using at least one of coefficients a and b as a function of one or more previous cardiac intervals in accordance with embodiments of the invention;

Figure 43 is a flowchart illustrating a method involving collection of medical information associated with a respiratory event in accordance with embodiments of the invention;

Figure 44 is a block diagram of a respiratory logbook in accordance with embodiments of the invention;

Figure 45 illustrates an exemplary depiction of a respiratory logbook user interface display in accordance with embodiments of the invention;

Figures 46A and 46B are timing diagrams illustrating the acquisition of respiratory logbook information in accordance with embodiments of the invention;

Figure 47A and 47B illustrate marked respiration waveforms in accordance with embodiments of the invention;

Figure 48 is a block diagram of a sleep logbook in accordance with embodiments of the invention;

Figure 49 illustrates an exemplary depiction of a sleep logbook user interface display in accordance with embodiments of the invention;

Figures 50A and 50B are flowcharts depicting methods of classifying sleep stages according to embodiments of the invention;

Figure 51 illustrates a block diagram of a system suitable for implementing a sleep stage classification method according to embodiments of the invention;

Figure 52 presents a block diagram illustrating sleep stage discrimination circuitry configured according to embodiments of the invention;

Figures 53 and 54 are flowcharts illustrating methods of performing sleep stage classification in accordance with embodiments of the invention;

Figure 55 is a process flow diagram illustrating a process for using sleep stage classification in cooperation with therapy delivery and testing in accordance with embodiments of the invention;

Figure 56 illustrates a medical system that may be used to perform sleep stage informed therapy in accordance with embodiments of the invention;

Figures 57A-57D illustrate various configurations of a muscle atonia sensor mechanically coupled to an implanted medical device in accordance with embodiments of the invention;

Figure 58 is a block diagram of a sleep-disordered breathing therapy system 5800 arranged in accordance with embodiments of the invention; and

Figure 59 is a flowchart illustrating a method for providing cardiac electrical therapy to mitigate sleep-disordered breathing according to embodiments of the invention.

While the invention is amenable to various modifications and alternative forms, specifics thereof have been shown by way of example in the drawings and will be described in detail below. It is to be understood, however, that the intention is not to limit the invention to the particular embodiments described. On the contrary, the invention is intended to cover all modifications, equivalents, and alternatives falling within the scope of the invention as defined by the appended claims.

## DETAILED DESCRIPTION OF VARIOUS EMBODIMENTS

In the following description of the illustrated embodiments, references are made to the accompanying drawings which form a part hereof, and in which are shown by way of illustration, various embodiments by which the invention may be practiced. It is to be understood that other embodiments may be utilized, and structural and functional changes may be made without departing from the scope of the present invention.

Methods, devices, and systems implementing a disordered breathing therapy approach in accordance with embodiments of the present invention may incorporate one or more of the features, components, structures, methods, and/or combinations thereof described herein. For example, a medical system may be implemented to include one or more of the components, features and/or processes described below. It is intended that such a method, device, or system need not include all of the components, features, and/or functions described herein, but may be implemented to include one or more selected components, features and functions that provide useful structures and/or functionality.

A significant percentage of patients between the ages of 30 and 60 experience some symptoms of disordered breathing. Disordered breathing primarily occurs during sleep, and is associated with excessive daytime sleepiness, systemic hypertension, increased risk of stroke, angina, and myocardial infarction. Disordered breathing is particularly prevalent among congestive heart failure patients, and may contribute to the progression of heart failure.

Various therapies have been used to treat disordered breathing, including both central and obstructive types. Obstructive sleep apnea has been associated with prolapse of the tongue and its surrounding structure into the pharynx, thus occluding the respiratory pathway. A commonly prescribed treatment for obstructive apnea is continuous positive airway pressure (CPAP). A CPAP device delivers air pressure through a nasal mask worn by the patient. The application of continuous positive airway pressure keeps the patient’s throat open, reducing or eliminating the obstruction causing the apnea.

Prolapse of the tongue muscles has been attributed to diminishing neuromuscular activity of the upper airway. A treatment for obstructive sleep apnea involves compensating for the decreased muscle activity by electrical activation of the tongue muscles. The hypoglossal (HG) nerve innervates the protrusor and retractor tongue muscles. An appropriately applied electrical stimulation to the hypoglossal nerve, for example, may prevent backward movement of the tongue, thus preventing the tongue from obstructing the airway.

Cardiac pacing during periods of sleep or wakefulness may prevent or reduce incidents of disordered breathing. Various embodiments discussed herein relate to systems and methods for adapting an effective cardiac electrical therapy to mitigate disordered breathing. Such a therapy may be adapted, for example, to achieve an overall level of therapy efficacy. The therapy may be adapted to provide a tiered therapy capable of achieving a variety of therapeutic goals.

For example, the therapy may be adapted to mitigate a presently-occurring disordered breathing episode or to prevent the occurrence of a predicted disordered breathing episodes. The cardiac electrical therapy may be adapted to achieve a efficacy, e.g., a desired reduction in the overall frequency and/or severity of disordered breathing episodes. The cardiac electrical therapy may also be adapted to provide a therapy that balances therapeutic goals with conservation of device life, for example.

The therapy may be adapted to adjust the impact of the therapy on the patient, for example, to reduce the impact of the therapy on the patient. In adapting a reduced impact therapy, the system may take into account various conditions for evaluating the impact of the therapy on the patient. For example, conditions such as patient comfort, as indicated by patient feedback, undesirable side effects, stress on physiological systems involved in the disordered breathing therapy, interaction with cardiac pacing algorithms, e.g., bradycardia pacing, cardiac resynchronization pacing and/or anti-tachycardia pacing, as determined by interactive effects of the disordered breathing therapy with cardiac pacing, and/or sleep quality, as measured by one or more sleep quality indices, may be taken into account to adapt a therapy that reduces an impact of the therapy on the patient.

Impact to the patient may involve a decreased useful service life of an implantable therapeutic device used to deliver disordered breathing therapy and/or pacing therapy for cardiac dysfunction. For example, a level of disordered breathing therapy may be unacceptably high if the energy requirements of the therapy result in an excessively decreased device service life. In this situation, early device removal and replacement produces a negative impact to the patient. Therefore, cardiac electrical therapy to mitigate disordered breathing may be adapted based on a projected decrease in device lifetime.

In one implementation, therapy approaches described herein may be used within the structure of an advanced patient management system. In this implementation, an advanced patient management system having capability for adaptive disordered breathing therapy allows a physician to remotely and automatically monitor cardiac and/or respiratory functions, as well as other patient conditions, and to initiate or modify therapy, if desired. In one example, an implantable cardiac rhythm management system, such as a cardiac pacemaker, defibrillator, or resynchronization device, or other device may be equipped with various telecommunications and information technologies to enable real-time data collection, diagnosis, and treatment of the patient.

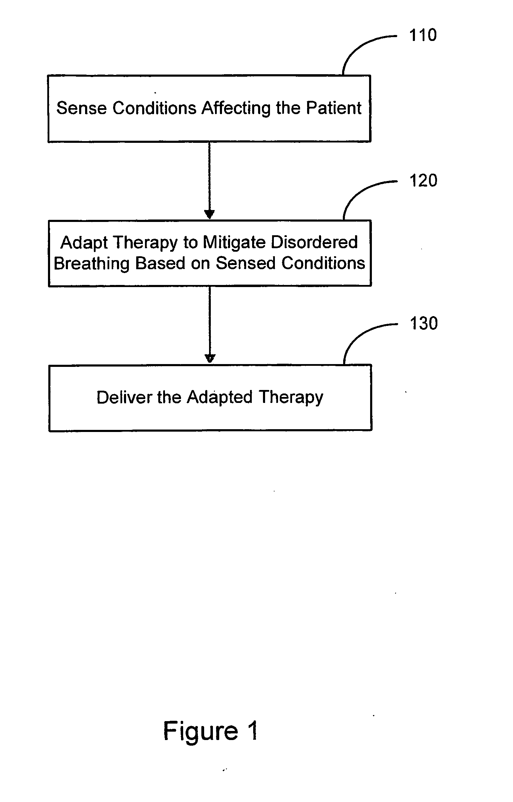

The flowchart of Figure 1 illustrates a method for providing cardiac electrical therapy to treat disordered breathing in accordance with embodiments of the invention. The method includes sensing 110 one or more conditions affecting the patient. A cardiac electrical therapy is adapted 120 to treat disordered breathing based on the sensed conditions. The therapy may be adapted, for example, to achieve a desired therapeutic goal, to reduce the impact of the therapy, and/or to balance therapy efficacy with therapy impact. Therapy impact involves situations that may result in patient stress, patient discomfort, reduction in sleep quality, interactions with other pacing algorithms, and/or decrease in the life of the therapy device. The adapted therapy is delivered 130 to the patient. At least one of sensing the conditions, adapting the therapy, and delivering the adapted therapy is performed at least in part implantably. Implantably performing an operation comprises performing the operation using a component, device, or system that is partially or fully implanted within the body.

Once initiated, the system may continue to sense conditions affecting the patient and therapy may be modified based on various factors, including, for example, detected occurrences of disordered breathing, predicted occurrences of disordered breathing, periodically updated assessments of therapy efficacy, patient comfort during therapy, sleep quality during therapy, pacing interactions, or other parameters.

Modification of the therapy may involve initiating the therapy, terminating the therapy or adjusting a therapy parameter. In various scenarios, modification of the cardiac electrical therapy may involve cardiac pacing using different pacing vectors, pacing in fewer or more heart chambers, increasing or decreasing the pacing energy, non-excitatory pacing, such as sub-threshold pacing and/or pacing during a refractory period.

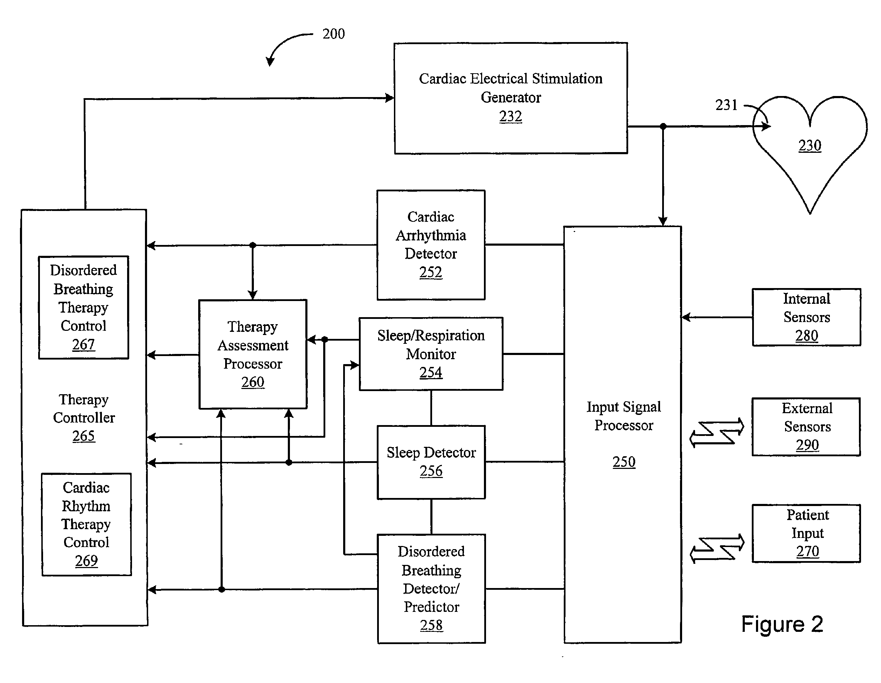

The block diagram of Figure 2 illustrates a therapy system 200 that may be used to implement a disordered breathing therapy methodology in accordance with embodiments of the invention. Figure 2 illustrates a system 200 that may be used to provide cardiac electrical therapy for disordered breathing as well as cardiac rhythm therapy. Various cardiac rhythm therapies, including single chamber cardiac pacing, dual chamber cardiac pacing, biventricular cardiac pacing, defibrillation, cardioversion, and/or cardiac resynchronization therapy, among other cardiac arrhythmia therapies may be provided by the therapy system 200. Delivery of one or more cardiac rhythm therapies may be operated separately or in cooperation with delivery of disordered breathing therapy. Although illustrative embodiments involve therapy systems having an implantable therapy control system, an implantable therapy delivery system, and implantable sensors, it is understood that the therapy system 200 may be configured so that all of the system 200 or portions of the system 200 are arranged externally to the patient. Sensors and other components of the sensing system may involve patient-external sensors or components, patient-internal sensors or components or a combination of patient-external and patient-internal sensors or other components.

The therapy system 200 may include circuitry for providing cardiac rhythm management therapy, in addition to and possibly in cooperation with, disordered breathing therapy. For example, the therapy controller 265 may control a cardiac electrical stimulation therapy for cardiac arrhythmia, including bradyarrhythmia and/or tachyarrhymia. The system may include an arrhythmia detector 252. In this embodiment, the cardiac electrical stimulation generator 232 is coupled to a lead system having implanted electrodes 231 to electrically couple the heart 230 to the electrical stimulation generator 232.

The cardiac therapy module 265 receives cardiac signals from the implanted cardiac electrodes 231 and analyzes the cardiac signals to determine an appropriate cardiac rhythm therapy. The cardiac rhythm therapy may include pacing therapy controlled by the cardiac rhythm therapy controller 269 to treat cardiac rhythms that are too slow. In this situation, the cardiac rhythm therapy controller 269 controls the cardiac stimulation circuit 232 to deliver of periodic low energy pacing pulses to one or more heart chambers to ensure that the periodic contractions of the heart are maintained at a hemodynamically sufficient rate.

The cardiac therapy may also include therapy to terminate tachyarrhythmia, wherein the heart rate is too fast. The cardiac arrhythmia detector 252 detects episodes of tachyarrhythmia, such as ventricular tachycardia and/or fibrillation. The arrhythmia detector 252 recognizes cardiac signals indicative of tachyarrhythmia. In response to the detection of a tachyarrhythmia, the cardiac rhythm therapy controller may initiate the delivery of high energy electrical pulses from the stimulation generator 232 to the heart 230 to terminate the arrhythmia.

Conditions affecting the patient may be detected using one or more patient-internal sensors 280, one or more patient-external sensors 290, and one or more patient input devices 270. One or more of the sensors 280, 290, and/or input devices 270 may be network-based. The sensors and/or other components of the therapy system 200 may be coupled using wired or wireless communications links. In one example, some or all of the patient-internal sensors 280, patient-external sensors 290, and patient input devices 270 may use remote communication capabilities, such as a wireless Bluetooth communications link or a proprietary wireless communications protocol. In one implementation, a wireless communications link couples the sensors 280, 290, input devices 270, to other components of the therapy system 200.

The therapy system 200 includes signal processing circuitry 250 for processing the signals received from the patient-internal sensors 280, patient-external sensors 290, and/or other input devices 270 used for detecting physiological or non-physiological conditions. The patient-internal sensors may include one or more implanted cardiac electrodes 231. The cardiac electrodes 231, patient-internal sensors 280, patient-external sensors 290, and/or other input device 270 may be used to sense or detect one or more of the conditions listed in Table 1.

In one implementation, heart rate and tidal volume may be determined using cardiac and respiration signals derived from an intracardiac electrocardiogram (EGM) sensor and transthoracic impedance sensor, respectively. The EGM and transthoracic impedance signals may be used in connection with cardiac rhythm management, as well as disordered breathing therapy. The therapy system 200 may derive additional physiological and non-physiological patient conditions using additional sensors and input devices. For example, a patient's activity may be detected using an implantable accelerometer, the patient's perceptions of restful sleep may be input using an external patient input device, and the patient's proximity to bed may be detected using a proximity to bed sensor involving both patient-internal and patient-external components.

In one embodiment, the therapy system 200 may include disordered breathing predictor/detector 258 that receives inputs from the sensors and input devices 231, 280, 290, 270 through the input signal processing circuitry 250. The disordered breathing predictor/detector 258 uses the sensed conditions to detect, predict, and/or classify episodes of disordered breathing. In various embodiments, the disordered breathing detector/predictor may include circuitry for implementing a respiration monitor, including a user-accessible respiratory logbook as described in more detail herein.

The disordered breathing predictor/detector 258 is coupled to the therapy controller 265 that includes circuitry for controlling cardiac electrical stimulation therapy for disordered breathing 267. Patient respiration, including disordered breathing events, may be monitored by a respiration monitor 254. Respiration monitoring may involve event-based collection of medical information associated with respiratory events.

Therapy to treat disordered breathing may be initiated, modified, or terminated by the disordered breathing therapy control circuitry 267, for example, based upon detection or prediction of disordered breathing. Additionally or alternatively, the disordered breathing therapy control circuitry 267 may adapt the disordered breathing therapy using medical information collected by the respiration monitor 254.

In accordance with an embodiment of the invention, the therapy controller 265 may receive information related to sleep from a sleep detector 256. The sleep processor may use the sensed physiological or non-physiological conditions to detect sleep onset and offset, sleep stages, and/or arousal events. The disordered breathing therapy circuitry, as well as the cardiac rhythm therapy circuitry may utilize the sleep onset, offset, arousal, and/or sleep stage information to enhance therapy delivered to the patient. In one example, the therapy controller 265 may adjust the cardiac rhythm pacing therapy and/or disordered breathing therapy delivered to the patient during sleep.

As previously discussed, disordered breathing occurs most frequently during sleep and may cause the patient to arouse from sleep repeatedly throughout the night. Thus, sleep information, e.g., sleep onset/offset, sleep stage, and/or sleep arousal information, may be used by the disordered breathing predictor/detector 258 in connection with the detection and/or prediction of disordered breathing. For example, the disordered breathing predictor/detector 258 may sense for disordered breathing events only during sleep, or may verify an initial detection/prediction of disordered breathing by determining if the patient is asleep.

The sleep-related information may also be utilized by a sleep quality monitor 254. The sleep quality monitor may assess and/or quantify various aspects of sleep quality, including, for example, the number of sleep disruptions, e.g., disordered breathing events occurring during sleep, the number and/or depth of arousal events associated with the disordered breathing events, and or other aspects of sleep quality. The sleep quality monitor may further determine the number of disordered breathing events occurring during various sleep stages, e.g., rapid eye movement (REM) sleep stages, and non-REM sleep stages. The sleep quality monitor may include functionality for organizing and storing sleep-related information in a sleep logbook format, the sleep information accessible through an interactive user interface.

In one embodiment, therapy delivered by the system 200 may be adapted to achieve or maintain therapeutic goals, for example, based on an assessment of the effectiveness of the therapy, the impact of the therapy on the patient, and/or interactions between the disordered breathing therapy and other types of therapy. The system 200 may include, for example, a therapy assessment processor 260 configured to evaluate one or more of therapy efficacy, impact, and interaction. The therapy assessment processor 260 may utilize inputs from the cardiac arrhythmia detector 252, the sleep quality monitor 254, the sleep processor 256, the disordered breathing predictor/detector 258 as well as the cardiac sensors 231, other patient internal sensors 280, patient-external sensors 290, and/or other input devices 270.

Utilizing information from one or more of the components of the therapy system 200, the therapy controller 265 controls the delivery an appropriate cardiac electrical therapy to mitigate the disordered breathing. In various illustrative therapy regimens, pacing to mitigate disordered breathing may involve pacing at a rate exceeding an intrinsic rate, pacing at a rate above the patient's normal pacing rate, or above the patient's normal sleep rate, pacing according to selected modes, e.g., bi-ventricular or single chamber modes, or pacing at a predetermined energy level that is above or below a capture threshold. The pacing may involve any or all of the heart chambers, for example, right and left atria and right and left ventricles, and may further involve multi-site pacing within one heart chamber. In one example, the pacing pulses may be delivered to left and right ventricles substantially simultaneously, or according to other timing sequences. Cardiac electrical stimulation therapy for disordered breathing may involve non-excitatory pacing, such as sub-threshold pacing and/or pacing during a refractory period. The cardiac electrical therapy for disordered breathing may be coordinated with therapy provided to the patient to treat cardiac rhythm disorders, including, for example, bradycardia and/or cardiac resynchronization therapies.

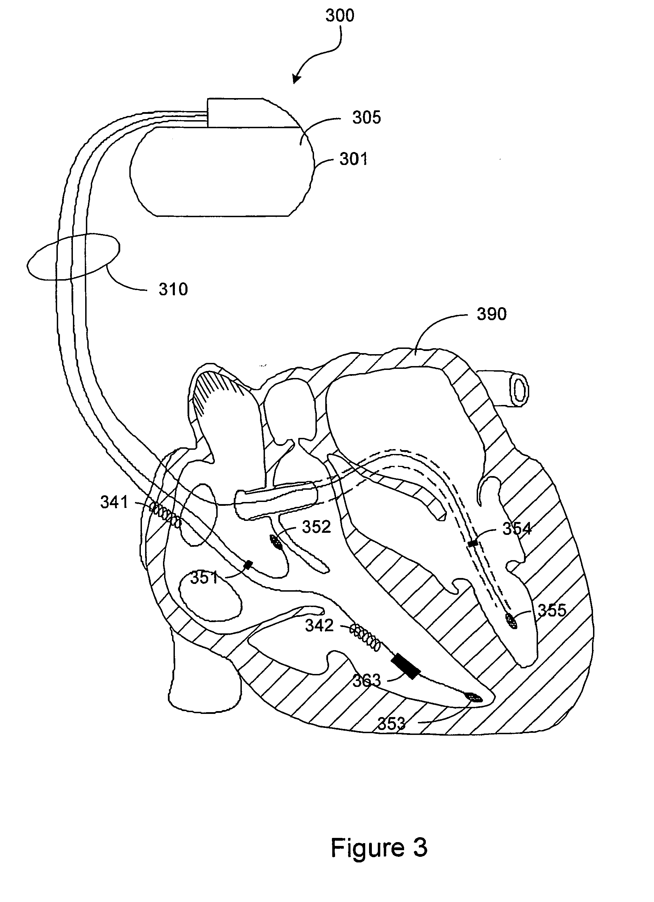

Figure 3 is a partial view of an implantable device that may include circuitry for implementing cardiac stimulation therapy for disordered breathing in accordance with embodiments of the invention. In this example, the implantable device comprises a cardiac rhythm management device (CRM) 300 including an implantable electrical stimulation generator 305 electrically and physically coupled to an intracardiac lead system 310. Portions of the intracardiac lead system 310 are inserted into the patient's heart 390. The intracardiac lead system 310 includes one or more electrodes configured to sense electrical cardiac activity of the heart, deliver electrical stimulation to the heart, sense the patient's transthoracic impedance, and/or sense other physiological parameters, e,g, cardiac chamber pressure or temperature. Portions of the housing 301 of the pulse generator 305 may optionally serve as a can electrode.

Communications circuitry is disposed within the housing 301 for facilitating communication between the electrical stimulation generator 305 and remote devices having wireless communication functionality, such as a portable or bed-side communication station, patient-carried/worn communication station, or external programmer, for example. The wireless communications circuitry can also facilitate unidirectional or bidirectional communication with one or more implanted, external, cutaneous, or subcutaneous physiologic or non-physiologic sensors, patient-input devices and/or other information systems.

The housing 301 of the electrical stimulation generator 305 may optionally incorporate various sensors, including, for example, a motion sensor that may be programmed to sense various conditions. For example, the motion sensor may be optionally configured to sense snoring, patient activity level, and/or chest wall movements associated with respiratory effort, for example. In one example implementation, the motion detector may be implemented as an accelerometer positioned in or on the housing 301 of the electrical stimulation generator 305. If the motion sensor is implemented as an accelerometer, the motion sensor may also provide respiratory information, e.g. rales, coughing, and cardiac information, e.g. S1-S4 heart sounds, murmurs, and/or other acoustic information.

The lead system 310 of the CRM 300 may incorporate one or more electrodes used to sense transthoracic impedance. Transthoracic impedance sensing may be used to acquire the patient's respiration waveform, or other respiration-related information. Transthoracic impedance may be sensed using one or more intracardiac electrodes 341, 342, 351-355, 363 positioned in one or more chambers of the heart 390. The intracardiac electrodes 341, 342, 351-355, 363 may be coupled to impedance drive/sense circuitry positioned within the housing of the electrical stimulation generator 305.

In one implementation, impedance drive/sense circuitry disposed within the housing 301 generates a current that flows through the tissue between an impedance drive electrode 351 and a can electrode on the housing 301 of the electrical stimulation generator 305. The voltage at an impedance sense electrode 352 relative to the can electrode changes as the patient's transthoracic impedance changes. The voltage signal developed between the impedance sense electrode 352 and the can electrode is detected by the impedance sense circuitry disposed within the housing 301 of the electrical stimulation generator 305. Other locations and/or combinations of impedance sense and drive electrodes are also possible.

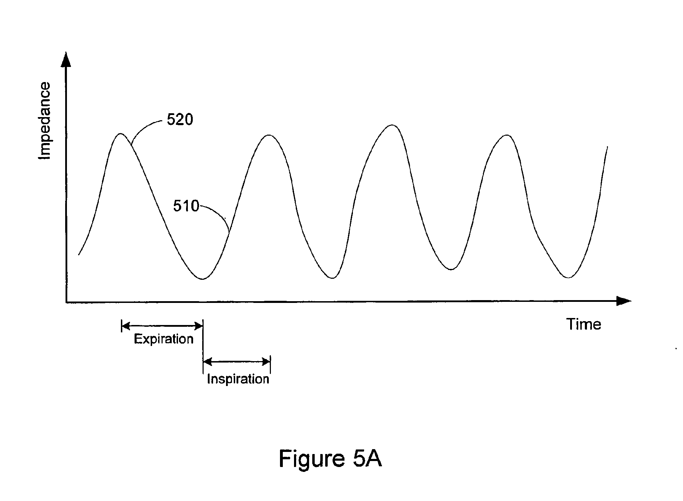

The voltage signal developed at the impedance sense electrode 352, illustrated in Figure 5A, is proportional to the patient's transthoracic impedance and represents the patient's respiration waveform. The transthoracic impedance increases during respiratory inspiration 510 and decreases during respiratory expiration 520. The peak-to-peak transition of the transthoracic impedance is proportional to the amount of air moved in one breath, denoted the tidal volume. The amount of air moved per minute is denoted the minute ventilation. A normal "at rest" respiration pattern, e.g., during non-REM sleep, includes regular, rhythmic inspiration - expiration cycles without substantial interruptions, as indicated in Figure 5A.

Returning to Figure 3, the lead system 310 may include one or more cardiac pace/sense electrodes 351-355 positioned in, on, or about one or more heart chambers for sensing electrical signals from the patient's heart 390 and/or delivering pacing pulses to the heart 390. The intracardiac sense/pace electrodes 351-355, such as those illustrated in Figure 3, may be used to sense and/or pace one or more chambers of the heart, including the left ventricle, the right ventricle, the left atrium and/or the right atrium. The lead system 310 may include one or more defibrillation electrodes 341, 342 for delivering defibrillation/cardioversion shocks to the heart 390.

As described above, the housing 301 of the electrical stimulation generator 305 may include circuitry for detecting cardiac arrhythmias and/or for controlling pacing and/or defibrillation therapy in the form of electrical stimulation pulses or shocks delivered to the heart through the lead system 310. Also disposed within the housing 301 may be circuitry for controlling cardiac electrical stimulation therapy for disordered breathing, including a sleep quality monitor 254, sleep processor 256, disordered breathing detector/predictor 258, therapy assessment processor 260, and/or therapy controller circuitry 265 as described in connection with Figure 2.

In accordance with another embodiment, an implantable transthoracic cardiac sensing and/or stimulation (ITCS) device may be implemented to detect/monitor normal and abnormal cardiac and/or respiratory activity, and may be configured to deliver an appropriate therapy in response to abnormal activity or conditions. An ITCS device 400 of the present invention may be configured for monitoring, diagnosing, and/or treating cardiac and disordered breathing events/ conditions. An ITCS device 400 is typically implemented to sense activity of both the cardiac system and the respiratory system. Using appropriate sensors, the ITCS device 400 may be implemented to detect and monitor a variety of disordered breathing conditions, including sleep and non-sleep related disordered breathing conditions. An ITCS device 400 may further be implemented to detect sleep, and may further be implemented to detect stages of patient sleep. An ITCS device 400 so implemented may be configured to perform a variety of sensing, monitoring, diagnosing, and/or therapy control/coordination functions, including those described herein and in the references respectively incorporated herein.

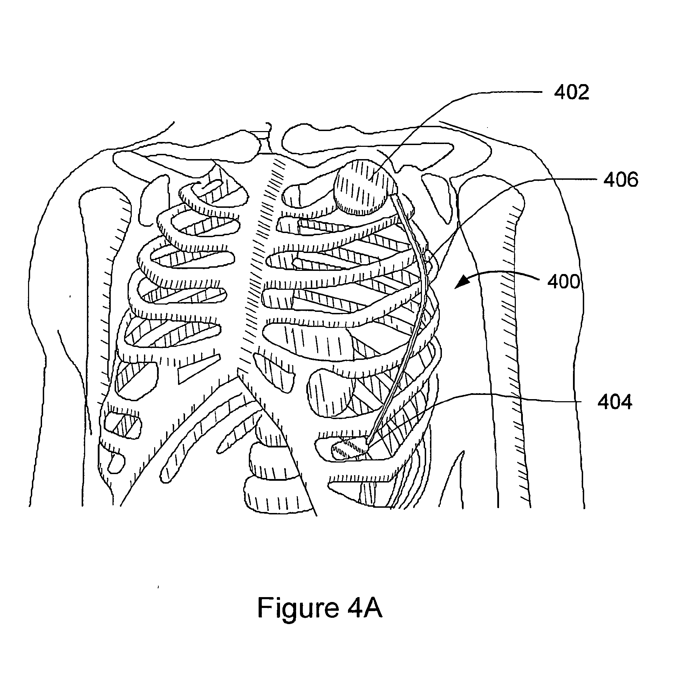

Figure 4A is a block diagram illustrating various components of an ITCS device 400 that provides for disordered breathing detection and/or treatment in accordance with embodiments of the present invention. In general terms, cardiac activity and disordered breathing (e.g., sleep disordered breathing and wakeful disordered breathing) may be detected, monitored, and/or treated with use of an ITCS device 400, such as the one shown in Figure 4A. An ITCS device 400 may be implanted under the skin in the chest region of a patient. The ITCS device 400 may, for example, be implanted subcutaneously such that all or selected elements of the device are positioned on the patient's front, back, side, or other body locations suitable for sensing cardiac activity and delivering cardiac stimulation therapy. It is understood that elements of the ITCS device 400 may be located at several different body locations, such as in the chest, abdominal, or subclavian region with electrode elements respectively positioned at different regions near, around, or on the heart.

The primary housing (e.g., the active or non-active can) 402 of the ITCS device 400, for example, may be configured for positioning outside of the rib cage at an intercostal or subcostal location, within the abdomen, or in the upper chest region (e.g., subclavian location, such as above the third rib). In one implementation, one or more electrodes may be located on the primary housing 402 and/or at other locations (e.g., electrode 404) about, but not in direct contact with the heart, great vessels or coronary vasculature. A pulse generator and a cardiac stimulation controller are disposed in the primary housing 402. The cardiac stimulator controller determines and coordinates appropriate cardiac and/or respiratory therapy to be delivered to a patient, and the pulse generator produces the appropriate energy waveforms associated with a selected therapy. Also disposed in the primary housing 402 is a cardiac activity detector configured to detect normal and abnormal (e.g., arrhythmic) cardiac activity.

In a further implementation, one or more subcutaneous electrode subsystems or electrode arrays 404 may be used to sense cardiac activity and deliver cardiac stimulation energy in an ITCS device 400 configuration employing an active can or a configuration employing a non-active can. Electrodes (e.g., electrode 404) may be situated at anterior and/or posterior locations relative to the heart.

The ITCS device 400 depicted in Figure 4A may be configured in a manner described hereinabove or may have other configurations. An ITCS device 400 of the present invention may be implemented to include one or more of cardiac and/or respiratory detection/monitoring circuitry (e.g., for cardiac activity, breathing patterns such as from transthoracic impedance signals, heart sounds, blood gas/chemistry such as oxygen saturation and/or pH), cardiac and respiratory diagnostics circuitry, and cardiac and respiratory therapy circuitry. An ITCS device 400 of the present invention may be implemented to provide for upgradeability in terms of functionality and/or configuration. For example, an ITCS device 400 may be implemented as an upgradeable or reconfigurable cardiac/respiratory monitor or stimulation device.

An ITCS device 400 in accordance with embodiments of the present invention provides for patient breathing monitoring and disordered breathing detection and/or prediction. Such embodiments may further provide treatment for detected or predicted disordered breathing events or conditions, as determined by a therapy controller or in response to an externally generated command signal (such as received from an advanced patient management system or programmer). Detection and treatment of disordered breathing and/or respiratory conditions may be facilitated by use of an ITCS device 400 having appropriate sensing/detection/therapy delivery capabilities, or by cooperative use of an ITCS device 400 and an external programmer or an advanced patient management system via a communications interface.

With continued reference to Figure 4A, the ITCS device 400 includes a housing 402 within which various cardiac and respiratory sensing, detection, processing, and energy delivery circuitry may be housed. Communications circuitry is disposed within the housing 402 for facilitating communication between the ITCS device 400 and an external communication device, such as a portable or bed-side communication station, patient-carried/worn communication station, or external programmer, for example. The communications circuitry may also facilitate unidirectional or bidirectional communication with one or more external, cutaneous, or subcutaneous physiologic or non-physiologic sensors. The housing 402 is typically configured to include one or more electrodes (e.g., can electrode and/or indifferent electrode).

Although the housing 402 is typically configured as an active can, it is appreciated that a non-active can configuration may be implemented, in which case at least two electrodes spaced apart from the housing 402 are employed.

In the configuration shown in Figure 4A, a subcutaneous electrode 404 may be positioned under the skin in the chest region and situated distal from the housing 402. The subcutaneous and, if applicable, housing electrode(s) may be positioned about the heart at various locations and orientations, such as at various anterior and/or posterior locations relative to the heart. The subcutaneous electrode 404 is coupled to circuitry within the housing 402 via a lead assembly 406. One or more conductors (e.g., coils or cables) are provided within the lead assembly 406 and electrically couple the subcutaneous electrode 404 with circuitry in the housing 402. One or more sense, sense/pace or defibrillation electrodes may be situated on the elongated structure of the electrode support, the housing 402, and/or the distal electrode assembly (shown as subcutaneous electrode 404 in the configuration shown in Figure 4A).

In one configuration, the lead assembly 406 is generally flexible and has a construction similar to conventional implantable, medical electrical leads (e.g., defibrillation leads or combined defibrillation/pacing leads). In another configuration, the lead assembly 406 is constructed to be somewhat flexible, yet has an elastic, spring, or mechanical memory that retains a desired configuration after being shaped or manipulated by a clinician. For example, the lead assembly 406 may incorporate a gooseneck or braid system that may be distorted under manual force to take on a desired shape. In this manner, the lead assembly 406 may be shape-fit to accommodate the unique anatomical configuration of a given patient, and generally retains a customized shape after implantation. Shaping of the lead assembly 406 according to this configuration may occur prior to, and during, ITCS device 400 implantation.

In accordance with a further configuration, the lead assembly 406 includes an electrode support assembly, such as an elongated structure that positionally stabilizes the subcutaneous electrode 404 with respect to the housing 402. In this configuration, the rigidity of the elongated structure maintains a desired spacing between the subcutaneous electrode 404 and the housing 402, and a desired orientation of the subcutaneous electrode 104/housing 402 relative to the patient's heart. The elongated structure may be formed from a structural plastic, composite or metallic material, and includes, or is covered by, a biocompatible material. Appropriate electrical isolation between the housing 402 and subcutaneous electrode 404 is provided in cases where the elongated structure is formed from an electrically conductive material, such as metal.

In one configuration, the electrode support assembly and the housing 402 define a unitary structure (e.g., a single housing/unit). The electronic components and electrode conductors/connectors are disposed within or on the unitary ITCS device 400 housing/electrode support assembly. At least two electrodes are supported on the unitary structure near opposing ends of the housing/electrode support assembly. The unitary structure may have an arcuate or angled shape, for example.

According to another configuration, the electrode support assembly defines a physically separable unit relative to the housing 402. The electrode support assembly includes mechanical and electrical couplings that facilitate mating engagement with corresponding mechanical and electrical couplings of the housing 402. For example, a header block arrangement may be configured to include both electrical and mechanical couplings that provide for mechanical and electrical connections between the electrode support assembly and housing 402. The header block arrangement may be provided on the housing 402 or the electrode support assembly. Alternatively, a mechanical/electrical coupler may be used to establish mechanical and electrical connections between the electrode support assembly and housing 402. In such a configuration, a variety of different electrode support assemblies of varying shapes, sizes, and electrode configurations may be made available for physically and electrically connecting to a standard ITCS device 400 housing 402.

It is noted that the electrodes and the lead assembly 406 may be configured to assume a variety of shapes. For example, the lead assembly 406 may have a wedge, chevron, flattened oval, or a ribbon shape, and the subcutaneous electrode 404 may include a number of spaced electrodes, such as an array or band of electrodes. Moreover, two or more subcutaneous electrodes 404 may be mounted to multiple electrode support assemblies 406 to achieve a desired spaced relationship amongst subcutaneous electrodes 404.

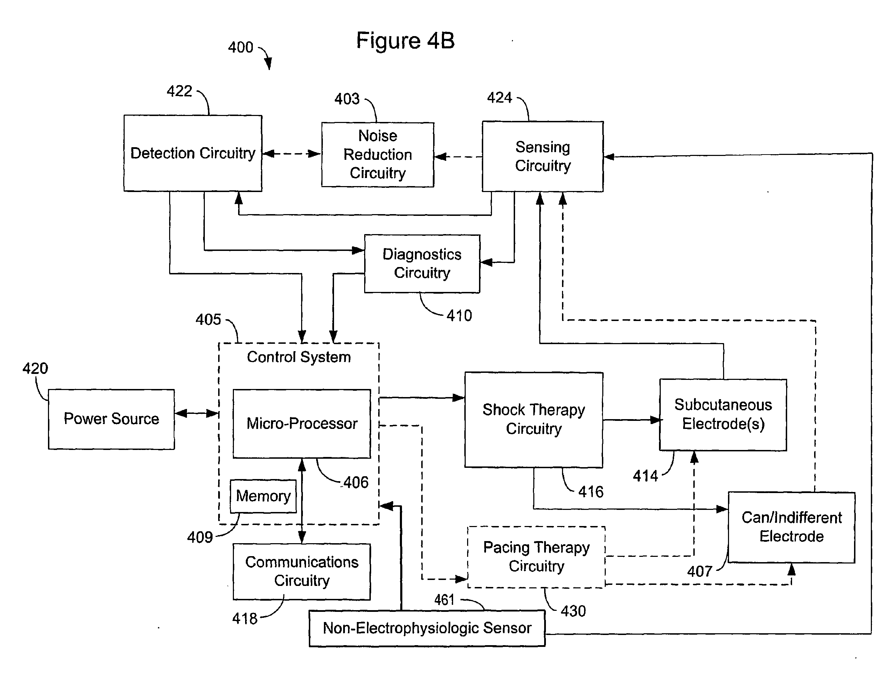

Figure 4B is a block diagram depicting various components of an ITCS device 400 in accordance with one configuration. According to this configuration, the ITCS device 400 incorporates a processor-based control system 405 which includes a micro-processor 426 coupled to appropriate memory (volatile and non-volatile) 409, it being understood that any logic-based control architecture may be used. The control system 405 is coupled to circuitry and components to sense, detect, and analyze electrical signals produced by the heart and deliver electrical stimulation energy to the heart under predetermined conditions to treat cardiac arrhythmias. In certain configurations, the control system 405 and associated components also provide pacing therapy to the heart. The electrical energy delivered by the ITCS device 400 may be in the form of low energy pacing pulses, non-excitatory energy (e.g., sub-threshold stimulation energy) or high-energy pulses for cardioversion or defibrillation.

Cardiac signals are sensed using the subcutaneous electrode(s) 414 and the can or indifferent electrode 407 provided on the ITCS device 400 housing. Cardiac signals may also be sensed using only the subcutaneous electrodes 414, such as in a non-active can configuration. As such, unipolar, bipolar, or combined unipolar/bipolar electrode configurations as well as multi-element electrodes and combinations of noise canceling and standard electrodes may be employed. The sensed cardiac signals are received by sensing circuitry 424, which includes sense amplification circuitry and may also include filtering circuitry and an analog-to-digital (A/D) converter. The sensed cardiac signals processed by the sensing circuitry 424 may be received by noise reduction circuitry 403, which may further reduce noise before signals are sent to the detection circuitry 422.

Noise reduction circuitry 403 may also be incorporated after sensing circuitry 422 in cases where high power or computationally intensive noise reduction algorithms are required. The noise reduction circuitry 403, by way of amplifiers used to perform operations with the electrode signals, may also perform the function of the sensing circuitry 424. Combining the functions of sensing circuitry 424 and noise reduction circuitry 403 may be useful to minimize the necessary componentry and lower the power requirements of the system.

In the illustrative configuration shown in Figure 4B, the detection circuitry 422 is coupled to, or otherwise incorporates, noise reduction circuitry 403. The noise reduction circuitry 403 operates to improve the signal-to-noise ratio (SNR) of sensed cardiac signals by removing noise content of the sensed cardiac signals introduced from various sources. Typical types of transthoracic cardiac signal noise includes electrical noise and noise produced from skeletal muscles, for example.

Detection circuitry 422 typically includes a signal processor that coordinates analysis of the sensed cardiac signals and/or other sensor inputs to detect cardiac arrhythmias, such as, in particular, tachyarrhythmia. Rate based and/or morphological discrimination algorithms may be implemented by the signal processor of the detection circuitry 422 to detect and verify the presence and severity of an arrhythmic episode.

The detection circuitry 422 communicates cardiac signal information to the control system 405. Memory circuitry 409 of the control system 405 contains parameters for operating in various sensing, defibrillation, and, if applicable, pacing modes, and stores data indicative of cardiac signals received by the detection circuitry 422. The memory circuitry 409 may also be configured to store historical ECG and therapy data, which may be used for various purposes and transmitted to an external receiving device as needed or desired.

In certain configurations, the ITCS device 400 may include diagnostics circuitry 410. The diagnostics circuitry 410 typically receives input signals from the detection circuitry 422 and the sensing circuitry 424. The diagnostics circuitry 410 provides diagnostics data to the control system 405, it being understood that the control system 405 may incorporate all or part of the diagnostics circuitry 410 or its functionality. The control system 405 may store and use information provided by the diagnostics circuitry 410 for a variety of diagnostics purposes. This diagnostic information may be stored, for example, subsequent to a triggering event or at predetermined intervals, and may include system diagnostics, such as power source status, therapy delivery history, and/or patient diagnostics. The diagnostic information may take the form of electrical signals or other sensor data acquired immediately prior to therapy delivery.

According to a configuration that provides cardioversion and defibrillation therapies, the control system 405 processes cardiac signal data received from the detection circuitry 422 and initiates appropriate tachyarrhythmia therapies to terminate cardiac arrhythmic episodes and return the heart to normal sinus rhythm. The control system 405 is coupled to shock therapy circuitry 416. The shock therapy circuitry 416 is coupled to the subcutaneous electrode(s) 414 and the can or indifferent electrode 407 of the ITCS device 400 housing. Upon command, the shock therapy circuitry 416 delivers cardioversion and defibrillation stimulation energy to the heart in accordance with a selected cardioversion or defibrillation therapy. In a less sophisticated configuration, the shock therapy circuitry 416 is controlled to deliver defibrillation therapies, in contrast to a configuration that provides for delivery of both cardioversion and defibrillation therapies.

In accordance with another configuration, an ITCS device 400 may incorporate a cardiac pacing capability in addition to cardioversion and/or defibrillation capabilities. As is shown in dotted lines in Figure 4B, the ITCS device 400 may include pacing therapy circuitry 430, which is coupled to the control system 405 and the subcutaneous and can/ indifferent electrodes 414, 407. Upon command, the pacing therapy circuitry delivers pacing pulses to the heart in accordance with a selected pacing therapy. Control signals, developed in accordance with a pacing regimen by pacemaker circuitry within the control system 405, are initiated and transmitted to the pacing therapy circuitry 430 where pacing pulses are generated. A pacing regimen may be modified by the control system 405.

A number of cardiac pacing therapies may be useful in a transthoracic cardiac monitoring and/or stimulation device. Such cardiac pacing therapies may be delivered via the pacing therapy circuitry 430 as shown in Figure 4B. Alternatively, cardiac pacing therapies may be delivered via the shock therapy circuitry 416, which effectively obviates the need for separate pacemaker circuitry.

The ITCS device 400 shown in Figure 4B is configured to receive signals from one or more physiologic and/or non-physiologic sensors. Depending on the type of sensor employed, signals generated by the sensors may be communicated to transducer circuitry coupled directly to the detection circuitry 422 or indirectly via the sensing circuitry 424. It is noted that certain sensors may transmit sense data to the control system 405 without processing by the detection circuitry 422.

Non-electrophysiological cardiac sensors 461 may be coupled directly to the detection circuitry 422 or indirectly via the sensing circuitry 424. Non-electrophysiological cardiac sensors 461 sense cardiac activity that is non-electrophysiological in nature. Examples of non-electrophysiological cardiac sensors 461 include blood oxygen sensors, transthoracic impedance sensors, blood volume sensors, acoustic sensors and/or pressure transducers, and accelerometers. Signals from these sensors are developed based on cardiac activity, but are not derived directly from electrophysiological sources (e.g., R-waves or P-waves). A non-electrophysiological cardiac sensor 461, as is illustrated in Figure 4B, may be connected to one or more of the sensing circuitry 424, detection circuitry 422 (connection not shown for clarity), and the control system 405.

Communications circuitry 418 is coupled to the microprocessor 426 of the control system 405. The communications circuitry 418 allows the ITCS device 400 to communicate with one or more receiving devices or systems situated external to the ITCS device 400. By way of example, the ITCS device 400 may communicate with a patient-worn, portable or bedside communication system via the communications circuitry 418. In one configuration, one or more physiologic or non-physiologic sensors (subcutaneous, cutaneous, or external of patient) may be equipped with a short-range wireless communication interface, such as an interface conforming to a known communications standard, such as Bluetooth or IEEE 802 standards. Data acquired by such sensors may be communicated to the ITCS device 400 via the communications circuitry 418. It is noted that physiologic or non-physiologic sensors equipped with wireless transmitters or transceivers may communicate with a receiving system external of the patient.

The communications circuitry 418 may allow the ITCS device 400 to communicate with an external programmer. In one configuration, the communications circuitry 418 and the programmer unit (not shown) use a wire loop antenna and a radio frequency telemetric link, as is known in the art, to receive and transmit signals and data between the programmer unit and communications circuitry 418. In this manner, programming commands and data are transferred between the ITCS device 400 and the programmer unit during and after implant. Using a programmer, a physician is able to set or modify various parameters used by the ITCS device 400. For example, a physician may set or modify parameters affecting sensing, detection, pacing, and defibrillation functions of the ITCS device 400, including pacing and cardioversion/defibrillation therapy modes.

Typically, the ITCS device 400 is encased and hermetically sealed in a housing suitable for implanting in a human body as is known in the art. Power to the ITCS device 400 is supplied by an electrochemical power source 420 housed within the ITCS device 400. In one configuration, the power source 420 includes a rechargeable battery. According to this configuration, charging circuitry is coupled to the power source 420 to facilitate repeated non-invasive charging of the power source 420. The communications circuitry 418, or separate receiver circuitry, is configured to receive RF energy transmitted by an external RF energy transmitter. The ITCS device 400 may, in addition to a rechargeable power source, include a non-rechargeable battery. It is understood that a rechargeable power source need not be used, in which case a long-life non-rechargeable battery is employed.

The components, functionality, and structural configurations depicted in Figures 4A-4E are intended to provide an understanding of various features and combination of features that may be incorporated in an ITCS device 400. It is understood that a wide variety of ITCS and other implantable cardiac monitoring and/or stimulation device configurations are contemplated, ranging from relatively sophisticated to relatively simple designs. As such, particular ITCS or cardiac monitoring and/or stimulation device configurations may include particular features as described herein, while other such device configurations may exclude particular features described herein.

In accordance with embodiments of the invention, an ITCS device 400 may be implemented to include a subcutaneous electrode system that provides for one or both of cardiac sensing and arrhythmia therapy delivery. According to one approach, an ITCS device 400 may be implemented as a chronically implantable system that performs monitoring, diagnostic and/or therapeutic functions. The ITCS device 400 may automatically detect and treat cardiac arrhythmias.

In one configuration, an ITCS device 400 includes a pulse generator and one or more electrodes that are implanted subcutaneously in the chest region of the body, such as in the anterior thoracic region of the body. The ITCS device 400 may be used to provide atrial and/or ventricular therapy for bradycardia and tachycardia arrhythmias. Tachyarrhythmia therapy may include cardioversion, defibrillation and anti-tachycardia pacing (ATP), for example, to treat atrial or ventricular tachycardia or fibrillation. Bradycardia therapy may include temporary post-shock pacing for bradycardia or asystole.

In one configuration, an ITCS device 400 according to one approach may utilize conventional pulse generator and subcutaneous electrode implant techniques. The pulse generator device and electrodes may be chronically implanted subcutaneously. Such an ITCS may be used to automatically detect and treat arrhythmias similarly to conventional implantable systems. In another configuration, the ITCS device 400 may include a unitary structure (e.g., a single housing/unit). The electronic components and electrode conductors/connectors are disposed within or on the unitary ITCS device 400 housing/electrode support assembly.

The ITCS device 400 contains the electronics and may be similar to a conventional implantable defibrillator. High voltage shock therapy may be delivered between two or more electrodes, one of which may be the pulse generator housing (e.g., can), placed subcutaneously in the thoracic region of the body.

Additionally or alternatively, the ITCS device 400 may also provide lower energy electrical stimulation for bradycardia therapy. The ITCS device 400 may provide brady pacing similarly to a conventional pacemaker. The ITCS device 400 may provide temporary post-shock pacing for bradycardia or asystole. Sensing and/or pacing may be accomplished using sense/pace electrodes positioned on an electrode subsystem also incorporating shock electrodes, or by separate electrodes implanted subcutaneously.

The ITCS device 400 may detect a variety of physiological signals that may be used in connection with various diagnostic, therapeutic or monitoring implementations in accordance with the present invention. For example, the ITCS device 400 may include sensors or circuitry for detecting pulse pressure signals, blood oxygen level, heart sounds, cardiac acceleration, and other non-electrophysiological signals related to cardiac activity. In one embodiment, the ITCS device 400 senses intrathoracic impedance, from which various respiratory parameters may be derived, including, for example, respiratory tidal volume and minute ventilation. Sensors and associated circuitry may be incorporated in connection with an ITCS device 400 for detecting one or more body movement or body position related signals. For example, accelerometers and GPS devices may be employed to detect patient activity, patient location, body orientation, or torso position.

The ITCS device 400 may be used within the structure of an APM system. APM systems may allow physicians to remotely and automatically monitor cardiac and respiratory functions, as well as other patient conditions. In one example, implantable cardiac rhythm management systems, such as cardiac pacemakers, defibrillators, and resynchronization devices, may be equipped with various telecommunications and information technologies that enable real-time data collection, diagnosis, and treatment of the patient. Various embodiments described herein may be used in connection with advanced patient management.

An ITCS device 400 according to one approach provides an easy to implant therapeutic, diagnostic or monitoring system. The ITCS system may be implanted without the need for intravenous or intrathoracic access, providing a simpler, less invasive implant procedure and minimizing lead and surgical complications. In addition, this system would have advantages for use in patients for whom transvenous lead systems cause complications. Such complications include, but are not limited to, surgical complications, infection, insufficient vessel patency, complications associated with the presence of artificial valves, and limitations in pediatric patients due to patient growth, among others. An ITCS system according to this approach is distinct from conventional approaches in that it may be configured to include a combination of two or more electrode subsystems that are implanted subcutaneously in the anterior thorax.

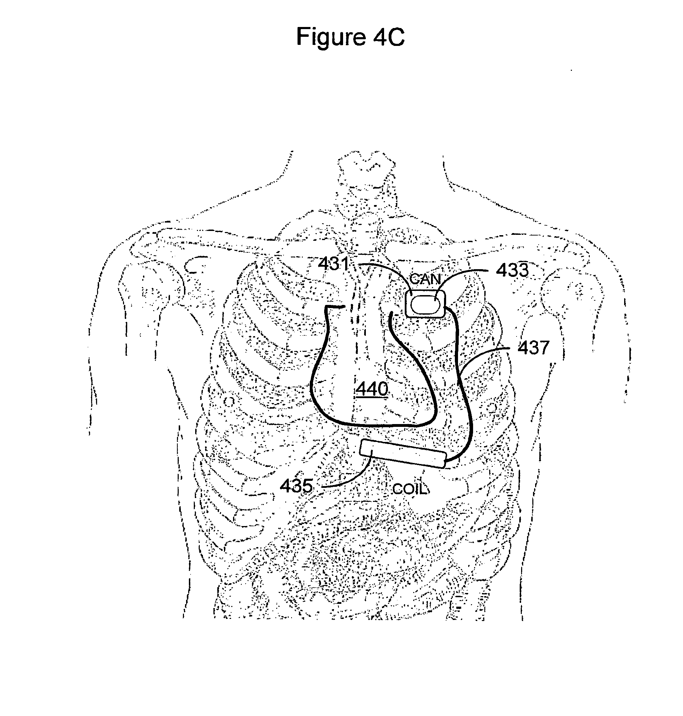

In one ITCS system configuration, as is illustrated in Figure 4C, electrode subsystems of the ITCS system include a first electrode subsystem, including a can electrode 433, and a second electrode subsystem 435 that may include at least one coil electrode, for example. The second electrode subsystem 435 may include a number of electrodes used for sensing and/or electrical stimulation. In various configurations, the second electrode subsystem 435 may include a single electrode or a combination of electrodes. The single electrode or combination of electrodes including the second electrode subsystem 435 may include coil electrodes, tip electrodes, ring electrodes, multi-element coils, spiral coils, spiral coils mounted on non-conductive backing, and screen patch electrodes, for example. A suitable non-conductive backing material is silicone rubber, for example.

The can electrode 433 is located on the housing 431 that encloses the ITCS device 400 electronics. In one embodiment, the can electrode 433 includes the entirety of the external surface of housing 431. In other embodiments, various portions of the housing 431 may be electrically isolated from the can electrode 433 or from tissue. For example, the active area of the can electrode 433 may include all or a portion of either the anterior or posterior surface of the housing 431 to direct current flow in a manner advantageous for cardiac sensing and/or stimulation.

The housing 431 may resemble that of a conventional implantable ICD, is approximately 20-100 cc in volume, with a thickness of 0.4 to 2 cm and with a surface area on each face of approximately 30 to 100 $cm^2$. As previously discussed, portions of the housing may be electrically isolated from tissue to optimally direct current flow. For example, portions of the housing 431 may be covered with a non-conductive, or otherwise electrically resistive, material to direct current flow. Suitable non-conductive material coatings include those formed from silicone rubber, polyurethane, or parylene, for example.

Figure 4CA illustrates the housing 431 and can electrode 433 placed subcutaneously, superior to the heart 440 in the left pectoral region, which is a location commonly used for conventional pacemaker and defibrillator implants. The second electrode subsystem 435 may include a coil electrode mounted on the distal end of a lead body 437, where the coil is approximately 3-15 French in diameter and 5-12 cm in length. The coil electrode may have a slight preformed curve along its length. The lead may be introduced through the lumen of a subcutaneous sheath, through a common tunneling implant technique, and the second electrode subsystem 435, e.g., including a coil electrode, may be placed subcutaneously, deep to any subcutaneous fat and adjacent to the underlying muscle layer.

In this configuration, the second electrode subsystem 435 is located approximately parallel with the inferior aspect of the right ventricle of the heart 440, just inferior to the right ventricular free wall, with one end extending just past the apex of the heart 440. For example, the tip of the electrode subsystem 435 may extend less than about 3 cm and may be about 1-2 cm left lateral to the apex of the heart 440. This electrode arrangement may be used to include a majority of ventricular tissue within a volume defined between the housing 431 and the second electrode subsystem 435. In one configuration, a majority of the ventricular tissue is included within a volume associated with an area bounded by lines drawn between the distal and proximal ends of the second electrode subsystem 435 and the medial and lateral edges of the left pectoral can electrode 433.

In one example arrangement, the volume including a majority of ventricular tissue may be associated with a cross sectional area bounded by lines drawn between the ends of the electrode subsystems 433, 435 or between active elements of the electrode subsystems 433, 435. In one implementation, the lines drawn between active elements of the electrode subsystems 433, 435 may include a medial edge and a lateral edge of the can electrode 433, and a proximal end and a distal end of a coil electrode utilized within the second electrode subsystem 435. Arranging the electrode subsystems so that a majority of ventricular tissue is contained within a volume defined between the active elements of the electrode subsystems 433, 435 provides an efficient position for defibrillation by increasing the voltage gradient in the ventricles of the heart 440 for a given applied voltage between electrode subsystems 433, 435.

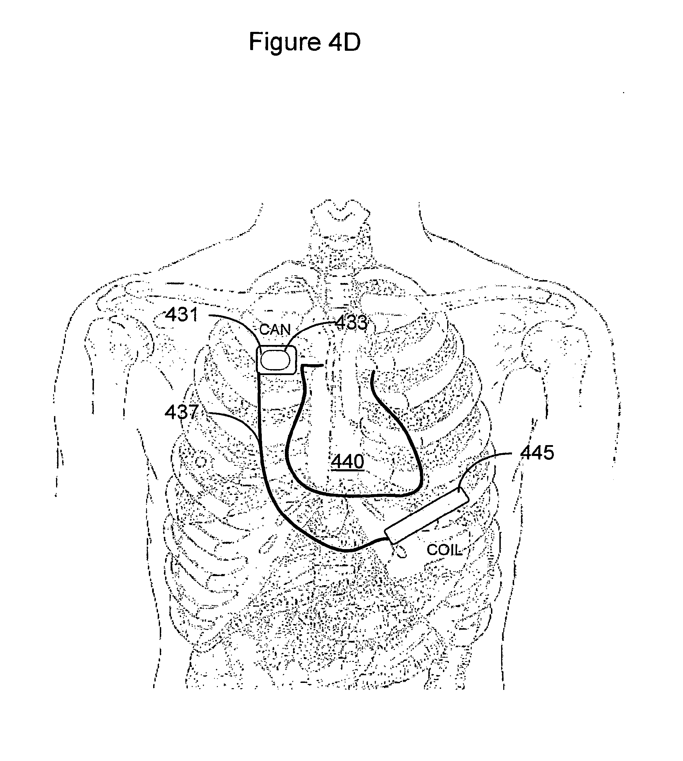

In a similar configuration, and as shown in Figure 4D, the housing 431 including the can electrode 433 is placed in the right pectoral region. The second electrode subsystem 435 is located more laterally, to again include a majority of the ventricular tissue in a volume defined between the can electrode 433 and the second electrode subsystem 435.

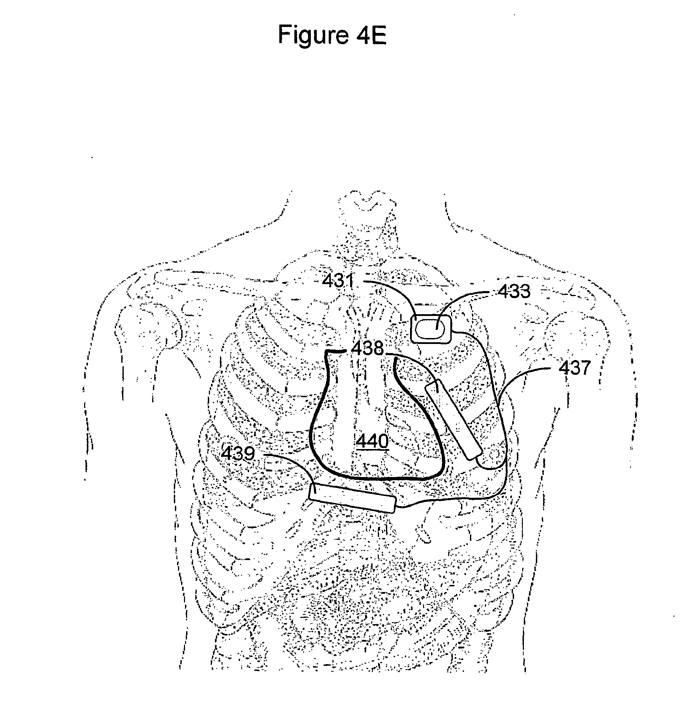

In a further configuration, and as shown in Figure 4E, the ITCS device housing 431 containing the electronics (i.e., the can) is not used as an electrode. In this case, an electrode system including two electrode subsystems 438, 439 coupled to the housing 431 may be implanted subcutaneously in the chest region of the body, such as in the anterior thorax. The first and the second electrode subsystems 438, 439 are placed in opposition with respect to the ventricles of the heart 440, with the majority of the ventricular tissue of the heart 440 included within a volume defined between the electrode subsystems 438, 439. As illustrated in Figure 4E, the first electrode system 438 is located superior to the heart 440 relative to a superior aspect of the heart 440, e.g., parallel to the left ventricular free wall. The second electrode system 439 is located inferior to the heart 440 and positioned in relation to an inferior aspect of the heart 440, e.g., parallel to the right ventricular free wall.

In this configuration, the first and the second electrode subsystems 438, 439 may include any combination of electrodes, including or excluding the can electrode, used for sensing and/or electrical stimulation. In various configurations, the electrode subsystems 438, 439 may each be a single electrode or a combination of electrodes. The electrode or electrodes including the first and second electrode subsystems 438, 439 may include any combination of one or more coil electrodes, tip electrodes, ring electrodes, multi-element coils, spiral coils, spiral coils mounted on non-conductive backing, and screen patch electrodes, for example.

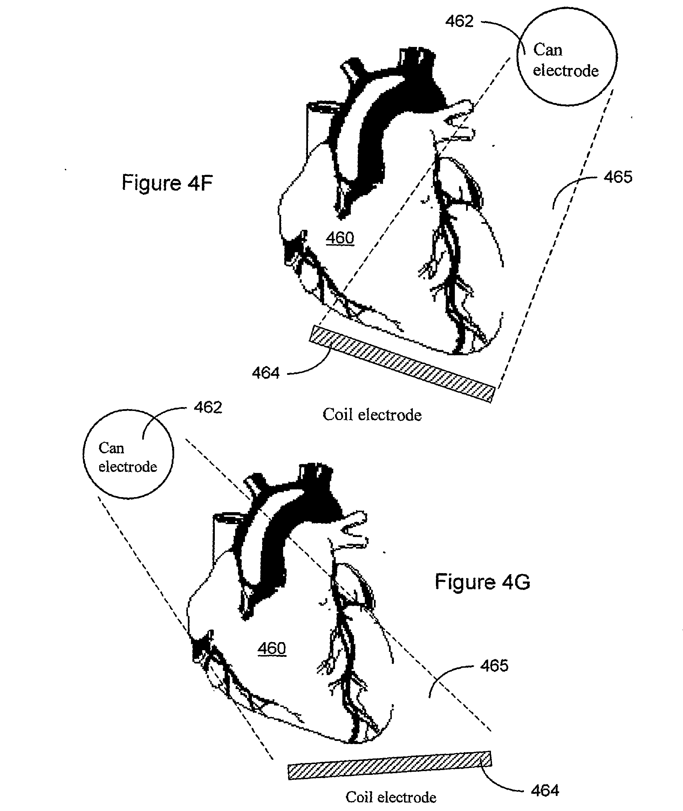

Figures 4F-4H provide additional detailed views of subcutaneous electrode subsystem placement considered particularly useful with ITCS devices incorporating disordered breathing detection in accordance with embodiments of the present invention. Figure 4F illustrates first and second electrode subsystems configured as a can electrode 462 and a coil electrode 464, respectively. Figure 4F illustrates the can electrode 462 located superior to the heart 460 in the left pectoral region and the coil electrode 464 located inferior to the heart 460, parallel to the right ventricular free wall of the heart 460.

The can electrode 462 and the coil electrode 464 are located so that the majority of ventricular tissue is included within a volume defined between the can electrode 462 and the coil electrode 464. Figure 4F illustrates a cross sectional area 465 formed by the lines drawn between active elements of the can electrode 462 and the coil electrode 464. Lines drawn between active areas of the electrodes 462, 464, may be defined by a medial edge and a lateral edge of the can electrode 462, and a proximal end and a distal end of a coil electrode utilized as the second electrode subsystem 464. The coil electrode 464 extends a predetermined distance beyond the apex of the heart 460, e.g. less than about 3 cm.

A similar configuration is illustrated in Figure 4G. In this embodiment, the can electrode 462 is placed superior to the heart 460 in the right pectoral region. The coil electrode 464 is located inferior to the heart. In one arrangement, the coil electrode is located relative to an inferior aspect of the heart 460, for example, the apex of the heart. The can electrode 462 and the coil electrode 464 are positioned so that the majority of ventricular tissue is included within a volume defined between the can electrode 462 and the coil electrode 464.

Figure 4G illustrates a cross sectional area 465 formed by the lines drawn between active elements of the can electrode 462 and the coil electrode 464. Lines drawn between active areas of the electrodes 462, 464, may be defined by a medial edge and a lateral edge of the can electrode 462, and a proximal end and a distal end of a coil electrode utilized as the second electrode subsystem 464. The coil electrode 464 extends a predetermined distance beyond the apex of the heart 460, e.g. less than about 3 cm.

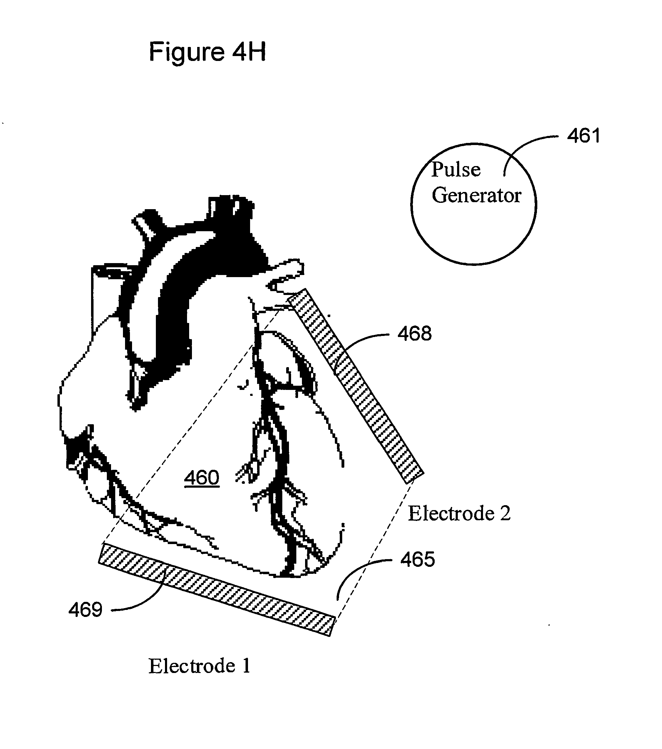

Figure 4H illustrates a configuration wherein the pulse generator housing 461 does not include an electrode. In this implementation two electrode subsystems are positioned about the heart so that a majority of ventricular tissue is included within a volume defined between the electrode subsystems. According to this embodiment, the first and second electrodes are configured as first and second coil electrodes 468, 469.

The first coil electrode 468 is located superior to the heart 460 and may be located relative to a superior aspect of the heart, e.g., the left ventricular free wall. The second coil electrode 469 is located inferior to the heart 460. The second electrode 469 may be located in relation to an inferior aspect of the heart 460. In one configuration, the second electrode 469 is positioned parallel to the right ventricular free wall with a tip of the electrode 469 extending less than about 3 cm beyond the apex of the heart 460. As illustrated in Figure 4H, the volume defined between the electrodes may be defined by the cross sectional area 465 bounded by lines drawn between active areas of the electrodes 468, 469.

Disordered Breathing Detection

According to various embodiments of the invention, detection of disordered breathing events may be used in connection with adapting a therapy to treat disordered breathing. In one embodiment, detection and assessment of disordered breathing is used to adapt (initiate, modify and/or terminate) therapy delivery. In another embodiment, disordered breathing events detected during and/or after therapy delivery may be used to assess the effectiveness of the disordered breathing therapy. In various implementations, some of which are described below, episodes of disordered breathing may be detected and classified by analyzing the patient's respiration patterns and/or other conditions associated with disordered breathing.

In accordance with one embodiment of the invention, the cardiac electrical therapy for disordered breathing may be adapted based on detected episodes of disordered breathing. In one scenario, one or more episodes of disordered breathing are detected and the cardiac electrical therapy is initiated or increased to treat the detected episodes. Adaptation of the therapy may continue, enabling the system to deliver a therapeutically appropriate therapy throughout the disordered breathing episode or episodes. If the system determines that the disordered breathing has mitigated or ceased, then the therapy may be reduced or terminated. Therapy may continue after the disordered breathing episode has stopped to prevent future occurrences of disordered breathing.

An exemplary set of conditions that may be used for detecting disordered breathing and/or adapting the cardiac electrical therapy are provided in Table 1. The list provided in Table 1 is not exhaustive and the use of other conditions or additional conditions to detect disordered breathing and/or adapt the therapy are also possible. The conditions may include both physiological and non-physiological conditions. The physiological conditions may encompass a broad category of conditions associated with the internal physiological conditions of the patient. Physiological conditions may be further subdivided, for example, into conditions of the cardiovascular, respiratory, and nervous systems, blood chemistry, body-related conditions, e.g., posture and activity, in addition to respiration quality, sleep quality, and patient.

Non-physiological conditions may include contextual conditions relating to patient-external or background conditions. Non-physiological conditions may be broadly defined to include, for example, conditions relating to the present environment of the patient, including patient location, ambient temperature, ambient humidity, air pollution index. Non-physiological/contextual conditions may also include historical/background conditions relating to the patient, including the patient's normal sleep time and the patient's medical history, for example.

Table 1 provides a representative set of conditions affecting the patient that may be used to detect disordered breathing and/or to adapt the disordered breathing therapy. Table 1 also provides example sensing methods that may be employed to sense the conditions.

Table 1

| Condition Category | | | Condition used to detect/predict disordered breathing and/or adapt therapy | Sensing method examples |
|---|---|---|---|---|
| Physiological | Sleep and Respiration Quality/ Patient Comfort | Sleep Fragmentation (arousal-based measures) | Sleep efficiency Arousals/hour Undisturbed sleep time Undisturbed sleep efficiency Sleep disturbance index Undisturbed sleep time Sleep staging | Electroencephlogram (EEG) Electromyogram (EMG) Activity sensor (accelerometer, Transthoracic impedance sensor) Posture sensor Sleep stage detector (muscle atonia sensor) |
| | | Disturbed Breathing-Based Measures | Percent time in periodic breathing Apnea/ hypopnea index | Transthoracic impedance sensor |
| | | Patient-reported | Restful sleep Patient comfort | Patient log |
| | Cardiovascular System | | Heart rate Heart rate variability (HRV) | EGM Electrocardiogram (ECG) |
| | | | Ventricular filling pressure | Intracardiac pressure sensor |
| | | | Blood pressure | Blood pressure sensor |
| | Respiratory System | | Snoring | Accelerometer Microphone |
| | | | Respiration pattern (Tidal volume Minute ventilation Respiratory rate) | Transthoracic pressure sensor (AC) |
| | | | Patency of upper airway | Intrathoracic impedance sensor |
| | | | Pulmonary congestion | Transthoracic impedance sensor (DC) |
| | Nervous System | | Sympathetic nerve activity | Muscle sympathetic nerve Activity sensor HRV via EGM or ECG |

(continued)

| Condition Category | | Condition used to detect/predict disordered breathing and/or adapt therapy | Sensing method examples |
|---|---|---|---|
| | Blood Chemistry | $CO_2$ saturation | $CO_2$ sensor |
| | | $O_2$ saturation | 02 sensor |
| | | Blood alcohol content | Breathalyzer |
| | | Adrenalin | Blood analysis |
| | | Brain Natriuretic Peptide (BNP) | Blood analysis |
| | | C-Reactive Protein | Blood analysis |
| | | Drug/Medication/ Tobacco use | Patient-reported |
| | Body-Related | Posture Activity | Posture sensor Accelerometer, MV, etc. |
| Contextual | Environmental | Ambient Temperature | Thermometer |
| | | Humidity | Hygrometer |
| | | Location | GPS, proximity sensor |
| | | Pollution | Air quality website |
| | | Time | Clock |
| | | Ambient light | Photodetector |
| | | Noise | Microphone |
| | | Barometric Pressure | Barometer |
| | | Altitude | Altimeter |
| | Historical/Background | Historical sleep time | Historical sensor data |
| | | History of disordered breathing | Patient log |
| | | Medical/psychological history | Medical records |

Detection of disordered breathing may involve detecting one or more conditions indicative of disordered breathing listed in Table 1. The patient conditions listed in Table 1 may be employed in a multi-sensor approach to detect and confirm episodes of disordered breathing. For example, the accuracy of a preliminary disordered breathing detection may be enhanced by verifying the patient is asleep, in bed, inactive, lying down, or that the present environmental conditions are associated with disordered breathing in the patient.

Table 2 provides examples of how a representative subset of the physiological and non-physiological conditions listed in Table 1 may be used in connection with disordered breathing detection.

Table 2

| Condition Type | Condition | Examples of how condition may be used in disordered breathing detection |
|---|---|---|
| Physiological | Heart rate | Decrease in heart rate may indicate disordered breathing episode. Decrease in heart rate may indicate the patient is asleep. |

(continued)

| Condition Type | Condition | Examples of how condition may be used in disordered breathing detection |
|---|---|---|
| | Heart rate variability | Disordered breathing causes heart rate variability to decrease. Changes in HRV associated with sleep disordered breathing may be observed while the patient is awake or asleep |
| | Ventricular filling pressure | May be used to identify/predict pulmonary congestion associated with respiratory disturbance. |
| | Blood pressure | Swings in on-line blood pressure measures are associated with apnea. Disordered breathing generally increases blood pressure variability - these changes may be observed while the patient is awake or asleep. |
| | Snoring | Snoring is associated with a higher incidence of obstructive sleep apnea and may be used to detect disordered breathing. |
| | Respiration pattern/rate | Respiration patterns including, e.g., respiration rate, may be used to detect disordered breathing episodes. Respiration patterns may be used to determine the type of disordered breathing. Respiration patterns may be used to detect that the patient is asleep. |
| | Patency of upper airway | Patency of upper airway is related to obstructive sleep apnea and may be used to detect episodes of obstructive sleep apnea. |
| | Pulmonary congestion | Pulmonary congestion is associated with respiratory disturbances. |
| | Sympathetic nerve activity | End of apnea associated with a spike in SNA. Changes in SNA observed while the patient is awake or asleep may be associated with sleep disordered breathing |
| | CO2 | Low CO2 levels initiate central apnea. |
| | O2 | O2 desaturation occurs during severe apnea/ hypopnea episodes. |
| | Blood alcohol content | Alcohol tends to increase incidence of snoring & obstructive apnea. |
| | Adrenalin | End of apnea associated with a spike in blood adrenaline. |
| | BNP | A marker of heart failure status, which is associated with Cheyne-Stokes Respiration |
| | C-Reactive Protein | A measure of inflammation that may be related to apnea. |
| | Drug/Medication/Tobacco use | These substances may affect the incidence of both central & obstructive apnea. |
| | Muscle atonia | Muscle atonia may be used to detect REM and non-REM sleep. |

(continued)

| Condition Type | Condition | Examples of how condition may be used in disordered breathing detection |
|---|---|---|
| | Eye movement | Eye movement may be used to detect REM and non-REM sleep. |
| Non-physiological/ Contextual | Temperature | Ambient temperature may be a condition predisposing the patient to episodes of disordered breathing and may be useful in disordered breathing detection. |
| | Humidity | Humidity may be a condition predisposing the patient to episodes of disordered breathing and may be useful in disordered breathing detection. |
| | Pollution | Pollution may be a condition predisposing the patient to episodes of disordered breathing and may be useful in disordered breathing detection. |
| | Posture | Posture may be used to confirm or determine the patient is asleep. |
| | Activity | Patient activity may be used in relation to sleep detection. |
| | Location | Patient location may used to determine if the patient is in bed as a part of sleep detection. |

Episodes of disordered breathing are associated with acute and chronic physiological effects. Acute responses to disordered breathing may include, for example, negative intrathoracic pressure, hypoxia, arousal from sleep, and increases in blood pressure and heart rate. During obstructive apnea episodes, negative intrathoracic pressure may arise from an increased effort to generate airflow. Attempted inspiration in the presence of an occluded airway results in an abrupt reduction in intrathoracic pressure. The repeated futile inspiratory efforts associated with obstructive sleep apnea may trigger a series of secondary responses, including mechanical, hemodynamic, chemical, neural, and inflammatory responses.

Obstructive sleep apneas may be terminated by arousal from sleep several seconds after the apneic peak, allowing the breathing to resume. Coincident with arousal from sleep, surges in sympathetic nerve activity, blood pressure, and heart rate may occur. The adverse effects of obstructive apnea are not confined to sleep. Waking conditions such as sympathetic nerve activity and systemic blood pressure are increased. There may also be a sustained reduction in vagal tone, causing reduction in total heart rate variability during periods of wakefulness.

Central sleep apnea is generally caused by a failure of respiratory control signals from the brain. Central sleep apnea is a component of Cheyne-Stokes respiration (CSR), a respiration pattern primarily observed in patients suffering from chronic heart failure (CHF). Cheyne-Stokes respiration is a form of periodic breathing in which central apneas and hypopneas alternate with periods of hyperventilation causing a waxing-waning pattern of tidal volume. In some CHF patients, obstructive sleep apnea and central sleep apnea may coexist. In these patients, there may be a gradual shift from predominantly obstructive apneas at the beginning of the night to predominantly central apneas at the end of the night.

Several mechanisms may be involved in central apneas observed in patients suffering from chronic heart failure. According to one mechanism, increased carbon dioxide sensitivity in CHF patients triggers hyperventilation initiating a sleep apnea episode. Breathing is regulated by a negative feedback system that maintains the arterial partial pressure of carbon dioxide ($PaCO_2$) within limits. Changes in $PaCO_2$ lead to changes in ventilation wherein the greater the sensitivity to carbon dioxide, the greater the ventilatory response.

In patients with cardiopulmonary disorders, an increase in carbon dioxide sensitivity may minimize perturbations in $PaCO_2$, thus protecting them against the long-term consequences of hypercapnia, an excess of carbon dioxide in the blood. This protective mechanism may be advantageous while the patient is awake, however, the increased sensitivity to carbon dioxide may disrupt breathing during sleep.

During sleep, ventilation decreases and $PaCO_2$ levels increase. If the $PaCO_2$ level decreases below level referred to as the apneic threshold, ventilation ceases, central sleep apnea ensues, and $PaCO_2$ rises to previous levels.

In patients with increased sensitivity to carbon dioxide, the negative-feedback system that controls breathing initiates a large ventilatory response when $PaCO_2$ rises. The resultant hyperventilation, by driving the $PaCO_2$ level below the apneic threshold, results in central sleep apnea. As a result of the apnea, the $PaCO_2$ level rises again, leading to an increase in ventilation. In this way, cycles of hyperventilation and central apnea may recur throughout sleep.

The posture of CHF patients during sleep may also be implicated in triggering apnea. When CHF patients lie down, the prone posture may create central fluid accumulation and pulmonary congestion causing the patient to reflexively hyperventilate. Hyperventilation may initiate the cyclical pattern of hyperventilation-apnea described above.

Arousals are not necessarily required in central sleep apneas for the resumption of breathing at the termination of the apneic event. In central apnea, the arousals follow the initiation of breathing and may facilitate the development of oscillations in ventilation by recurrently stimulating hyperventilation and reducing $PaCO_2$ below the apneic threshold. Once triggered, the pattern of alternating hyperventilation and apnea is sustained by the combination of increased respiratory drive, pulmonary congestion, arousals, and apnea-induced hypoxia causing $PaCO_2$ oscillations above and below the apneic threshold. Shifts in the patient's state of consciousness, particularly with repeated arousals, may further destabilize breathing.

With the transition from wakefulness to NREM sleep, the waking neural drive to breathe is lost, and the threshold for a ventilatory response to carbon dioxide is increased. Therefore, if the patient's $PaCO_2$ level during wakefulness is below this higher sleeping threshold, the transition to NREM sleep may be accompanied by a transient loss of respiratory drive resulting in a central apnea. During the apnea, the $PaCO_2$ rises until it reaches the new higher threshold level and initiates breathing. If sleep becomes firmly established, regular breathing resumes. However, if an arousal should occur, the increased $PaCO_2$ level associated with sleep is now relatively too high for a state of wakefulness and will stimulate hyperventilation. Thus, although arousals terminate obstructive sleep apneas, arousals trigger the respiratory oscillations associated with central apneas, particularly Cheyne-Stokes respiration.

In addition to the acute responses to sleep disordered breathing, such as those discussed above, sleep disordered breathing is also associated with a number of secondary or chronic responses, including, for example, chronic decrease in heart rate variability (HRV) and blood pressure changes. Patients with central sleep apnea may have higher urinary and circulating norepinephrine concentrations and lower $PaCO_2$ during both sleep and wakefulness.

Acute responses to disordered breathing are associated with physiological conditions that are modulated during an ongoing disordered breathing event. Sensing conditions modulated by the acute responses to disordered breathing may be used to detect a disordered breathing event contemporaneously with the occurrence of the disordered breathing event. Chronic responses to disordered breathing may be modulated by an aggregation of disordered breathing events that occur over time. Chronic responses to disordered breathing may be used to determine if disordered breathing events have occurred.

Both acute and chronic responses to disordered breathing may be used to asses the efficacy and impact of disordered breathing therapy. In one implementation, a first subset of patient conditions may be used to detect disordered breathing, including presently occurring events, and/or an aggregation of events occurring over time. A second subset of patient conditions, possibly overlapping the subset used for disordered breathing detection, may be used to assess the disordered breathing therapy. For example, according to one embodiment, the efficacy of the therapy may be assessed and the therapy may be adapted to enhance the efficacy based on the assessment. In another embodiment, the therapy may be assessed to determine an impact of the therapy on the patient. The therapy may be adapted to reduce therapy impact of the therapy on the patient based on the assessment. In yet a further embodiment, the therapy may be adapted both to enhance therapy effectiveness and to reduce an impact of the therapy on the patient. Other constraints may be utilized for therapy adaptation, including, for example, preservation of useable device life, and/or avoidance of interactions between disordered breathing therapy and other therapies delivered to the patient.

Conditions used to assess therapy effectiveness may be different from, or the same as, conditions used to assess an impact of the therapy on the patient. Table 3 provides a representative set of conditions that may be used for therapy assessment.

Table 3

| Condition | Therapy Impact | Therapy Efficacy |
|---|---|---|
| Arousal-Based Sleep Fragmentation Measures | May be used to assess therapy impact during sleep. | |
| Restful sleep (Patient reported) | May be used to assess therapy impact during sleep. | |
| Discomfort (Patient reported) | May be used to assess therapy impact. | |
| Pacing algorithm interaction | May be used to assess therapy impact. | |

(continued)

| Condition | Therapy Impact | Therapy Efficacy |
|---|---|---|
| Remaining useful life of therapy device | May be used to assess therapy impact. | |
| Disturbed Breathing-Based Measures | | May be used to analyze/assess efficacy of therapy to mitigate disordered breathing episodes. |
| Respiration quality (Patient reported) | | May be used to analyze/assess efficacy of therapy to mitigate disordered breathing episodes. |
| Heart rate variability (HRV) | | Disordered breathing causes heart rate variability to decrease. Therapy may be modified based on changes in HRV |
| Blood pressure | | Disordered breathing causes blood pressure increase |
| Sympathetic nerve activity (SNA) | | Changes in sympathetic nerve activity are caused by disordered breathing. Therapy may be adjusted based on the level of SNA |
| Blood chemistry | | A number of disordered breathing related changes may occur in a patient's blood chemistry, including, e.g., higher norepinephrine levels, and lower $PaCO_2$ |

It is understood that the patient conditions that may be used in connection with disordered breathing therapy, including detection of disordered breathing and/or therapy assessment, for example, are not limited to the representative sets listed in Tables 1-3 or those described herein. Further, although illustrative sensing methods for detecting the patient conditions listed above are provided, it is understood that the patient conditions may be detected using a wide variety of technologies. The invention is not limited to the particular conditions or the particular sensing technologies discussed herein in connection with the illustrative embodiments.

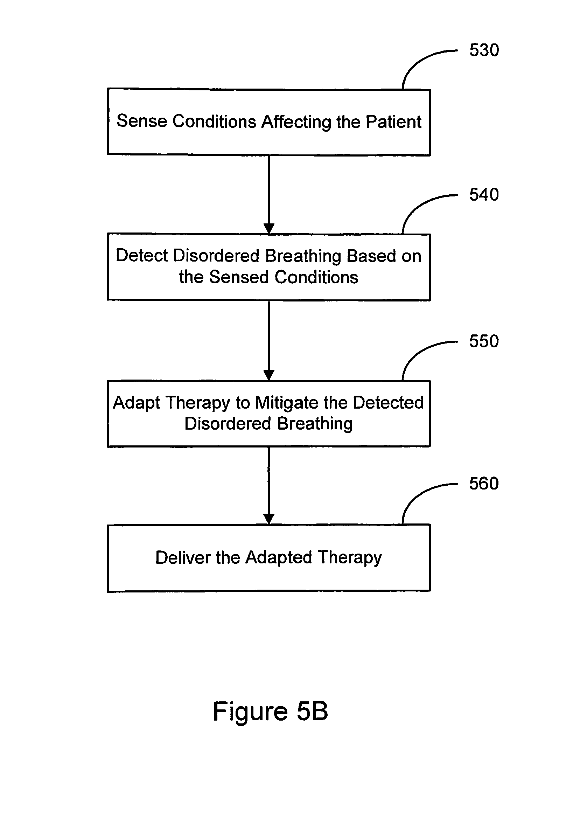

In accordance with embodiments of the invention, disordered breathing is detected, and therapy for treating disordered breathing is adapted based on the detection of disordered breathing. A methodology for adapting the disordered breathing therapy is illustrated by the flowchart of Figure 5B. One or more conditions affecting the patient are sensed 530 and disordered breathing is detected 540 based on the sensed conditions. A therapy is adapted 550 to treat the detected disordered breathing. The system delivers 560 the adapted therapy.

In one embodiment, the disordered breathing predictor/detector 258 (Figure 2) may detect episodes of disordered breathing by monitoring the respiratory waveform output of a transthoracic impedance sensor. When the tidal volume (TV) of the patient's respiration, as indicated by the transthoracic impedance signal, falls below a hypopnea threshold, then a hypopnea event is declared. For example, a hypopnea event may be declared if the patient's tidal volume falls below about 50% of a recent average tidal volume or other baseline tidal volume value. If the patient's tidal volume falls further to an apnea threshold, e.g., about 10% of the recent average tidal volume or other baseline value, an apnea event is declared.

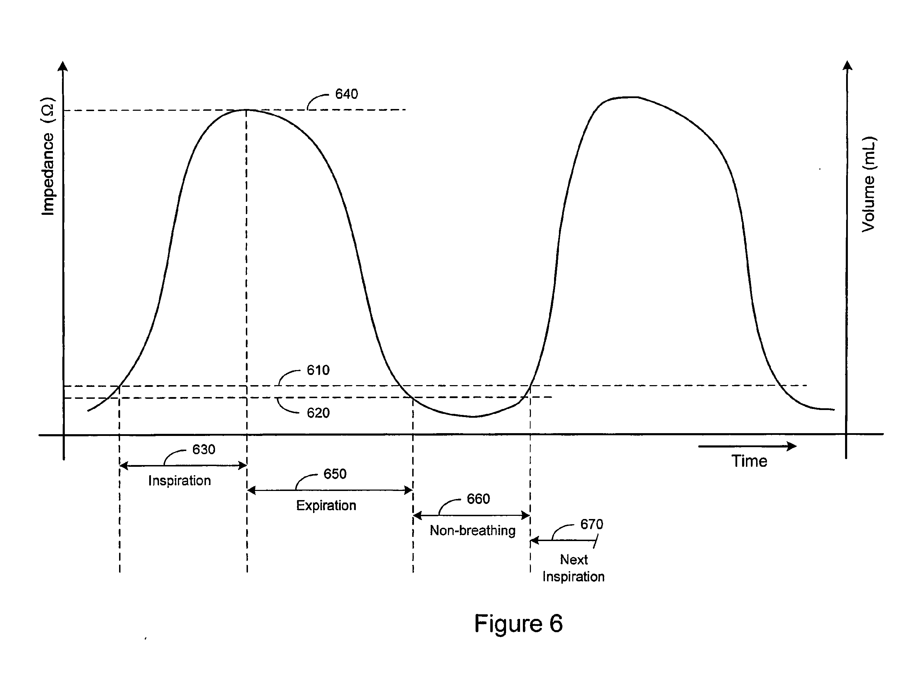

In another embodiment, detection of disordered breathing involves defining and examining a number of respiratory cycle intervals. Figure 6 illustrates respiration intervals used for disordered breathing detection according to an embodiment of the invention. A respiration cycle is divided into an inspiration period corresponding to the patient inhaling, an expiration period, corresponding to the patient exhaling, and a non-breathing period occurring between inhaling and exhaling. Respiration intervals are established using inspiration 610 and expiration 620 thresholds. The inspiration threshold 610 marks the beginning of an inspiration period 630 and is determined by the transthoracic impedance signal rising above the inspiration threshold 610. The inspiration period 630 ends when the transthoracic impedance signal is maximum 640. A maximum transthoracic impedance signal 640 corresponds to both the end of the inspiration interval 630 and the beginning of the expiration interval 650. The expiration interval 650 continues until the transthoracic impedance falls below an expiration threshold 620. A non-breathing interval 660 starts from the end of the expiration period 650 and continues until the beginning of the next inspiration period 670.

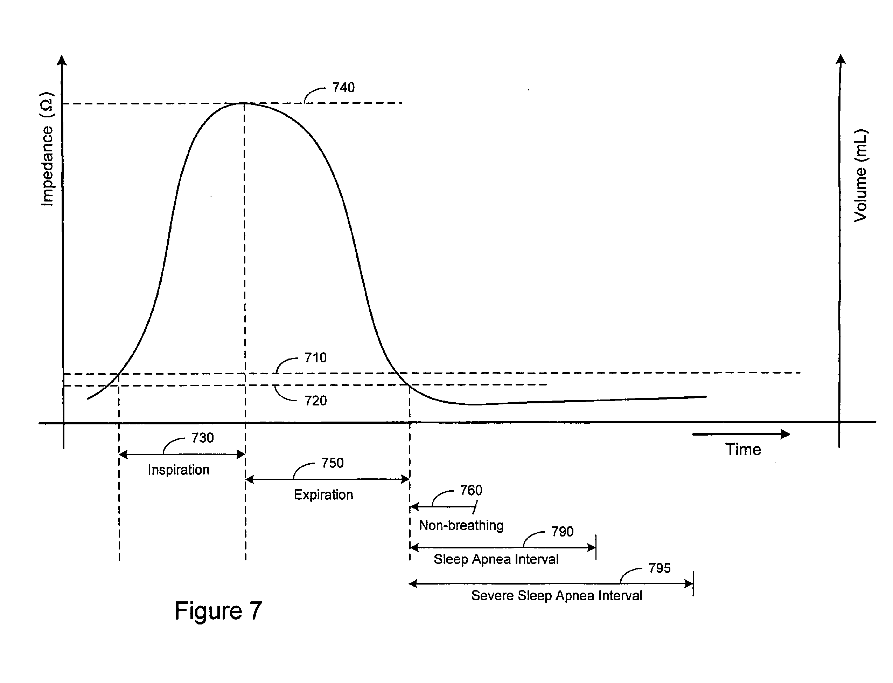

Detection of sleep apnea and severe sleep apnea according to embodiments of the invention is illustrated in Figure 7. The patient's respiration signals are monitored and the respiration cycles are defined according to inspiration 730, expiration 750, and non-breathing 760 intervals as described in connection with Figure 6. A condition of sleep apnea is detected when a non-breathing period 760 exceeds a first predetermined interval 790, denoted the sleep apnea interval. A condition of severe sleep apnea is detected when the non-breathing period 760 exceeds a second predetermined interval 795, denoted the severe sleep apnea interval. For example, sleep apnea may be detected when the non-breathing interval exceeds about 10 seconds, and severe sleep apnea may be detected when the non-breathing interval exceeds about 20 seconds.

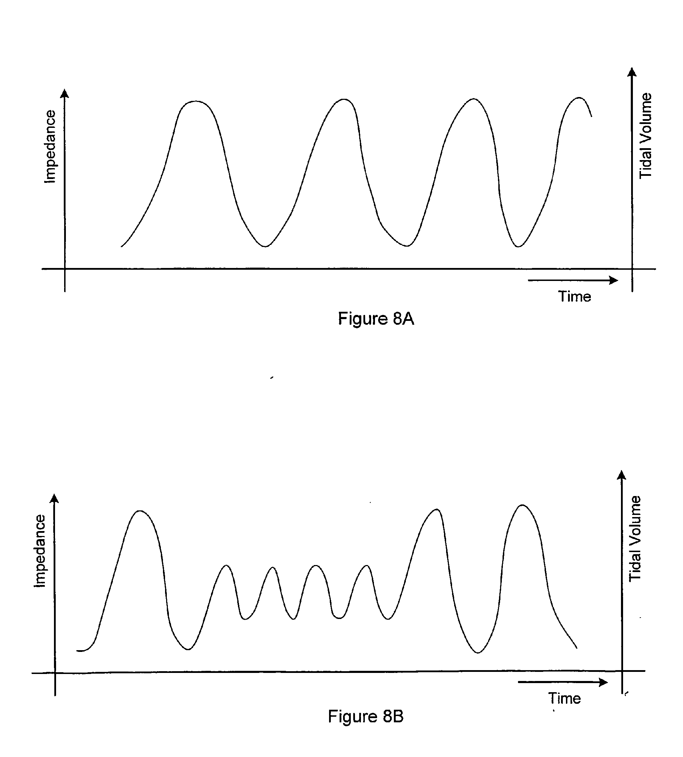

Hypopnea is a condition of disordered breathing characterized by abnormally shallow breathing. Figures 8A-B are graphs of tidal volume derived from transthoracic impedance measurements. The graphs compare the tidal volume of a normal breathing cycle to the tidal volume of a hypopnea episode. Figure 8A illustrates normal respiration tidal volume and rate. As shown in Figure 8B, hypopnea involves a period of abnormally shallow respiration.

According to an embodiment of the invention, hypopnea is detected by comparing a patient's respiratory tidal volume to a hypopnea tidal volume threshold. The tidal volume for each respiration cycle is derived from transthoracic impedance measurements acquired in the manner described above. The hypopnea tidal volume threshold may be established using clinical results providing a representative tidal volume and duration of hypopnea events. In one configuration, hypopnea is detected when an average of the patient's respiratory tidal volume taken over a selected time interval falls below the hypopnea tidal volume threshold. Furthermore, various combinations of hypopnea cycles, breath intervals, and non-breathing intervals may be used to detect hypopnea, where the non-breathing intervals are determined as described above.

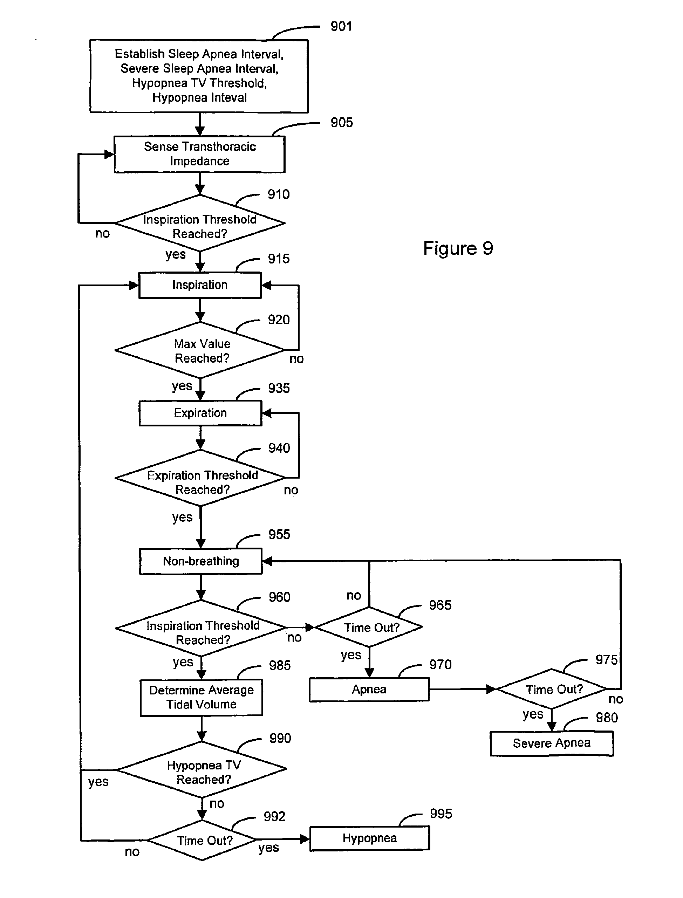

Figure 9 is a flowchart illustrating a method of apnea and/or hypopnea detection according to embodiments of the invention. Various parameters are established 901 before analyzing the patient's respiration for disordered breathing episodes, including, for example, inspiration and expiration thresholds, sleep apnea interval, severe sleep apnea interval, and hypopnea tidal volume threshold.

The patient's transthoracic impedance is measured 905 as described in more detail above. If the transthoracic impedance exceeds 910 the inspiration threshold, the beginning of an inspiration interval is detected 915. If the transthoracic impedance remains below 910 the inspiration threshold, then the impedance signal is checked 905 periodically until inspiration 915 occurs.

During the inspiration interval, the patient's transthoracic impedance is monitored until a maximum value of the transthoracic impedance is detected 920. Detection of the maximum value signals an end of the inspiration period and a beginning of an expiration period 935.

The expiration interval is characterized by decreasing transthoracic impedance. When the transthoracic impedance falls 940 below the expiration threshold, a non-breathing interval is detected 955.

If the transthoracic impedance does not exceed 960 the inspiration threshold within a first predetermined interval 965, denoted the sleep apnea interval, then a condition of sleep apnea is detected 970. Severe sleep apnea is detected 980 if the non-breathing period extends beyond a second predetermined interval 975, denoted the severe sleep apnea interval.

When the transthoracic impedance exceeds 960 the inspiration threshold, the tidal volume from the peak-to-peak transthoracic impedance is calculated, along with a moving average of past tidal volumes 985. The peak-to-peak transthoracic impedance provides a value proportional to the tidal volume of the respiration cycle. This value is compared 990 to a hypopnea tidal volume threshold. If the peak-to-peak transthoracic impedance is consistent with 990 the hypopnea tidal volume threshold for a predetermined time 992, then a hypopnea cycle is detected 995.

Additional sensors, such as motion sensors and/or posture sensors, may be used to confirm or verify the detection of a sleep apnea or hypopnea episode. The additional sensors may be employed to prevent false or missed detections of sleep apnea/hypopnea due to posture and/or motion related artifacts.

Another embodiment of the invention involves classifying respiration patterns as disordered breathing episodes based on the breath intervals and/or tidal volumes of one or more respiration cycles within the respiration patterns. According to this embodiment, the duration and tidal volumes associated with a respiration pattern are compared to duration and tidal volume thresholds. The respiration pattern is detected as a disordered breathing episode based on the comparison.

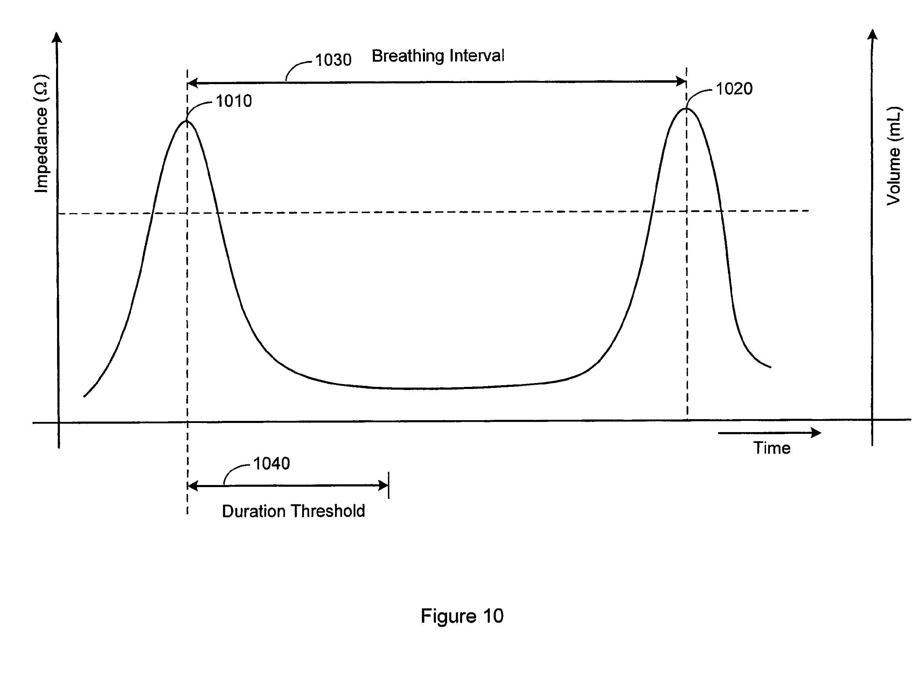

According to principles of the invention, a breath interval is established for each respiration cycle. A breath interval represents the interval of time between successive breaths, as illustrated in Figure 10. A breath interval 1030 may be defined in a variety of ways, for example, as the interval of time between successive maxima 1010, 1020 of the impedance signal waveform.

Detection of disordered breathing, in accordance with embodiments of the invention, involves the establishment of a duration threshold and a tidal volume threshold. If a breath interval exceeds the duration threshold, an apnea event is detected. Detection of sleep apnea, in accordance with this embodiment, is illustrated in the graph of Figure 10. Apnea represents a period of non-breathing. A breath interval 1030 exceeding a duration threshold 1040, comprises an apnea episode.

Hypopnea may be detected using the duration threshold and tidal volume threshold. A hypopnea event represents a period of shallow breathing. Each respiration cycle in a hypopnea event is characterized by a tidal volume less than the tidal volume threshold. Further, the hypopnea event involves a period of shallow breathing greater than the duration threshold.

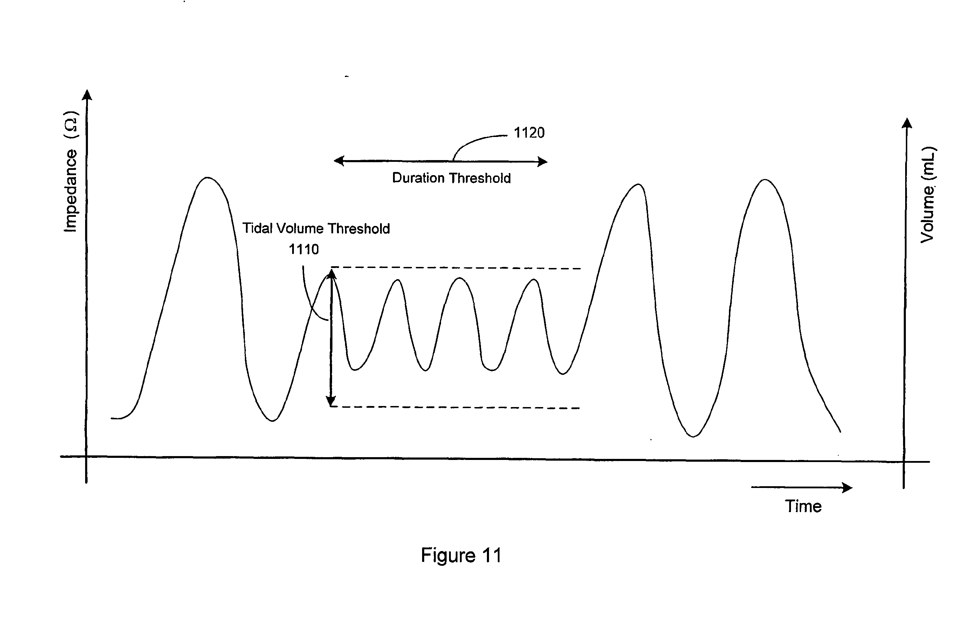

A hypopnea detection approach, in accordance with embodiments of the invention, is illustrated in Figure 11. Shallow breathing is detected when the tidal volume of one or more breaths is below a tidal volume threshold 1110. If the shallow breathing continues for an interval greater than a duration threshold 1120, then the breathing pattern represented by the sequence of shallow respiration cycles, is classified as a hypopnea event.

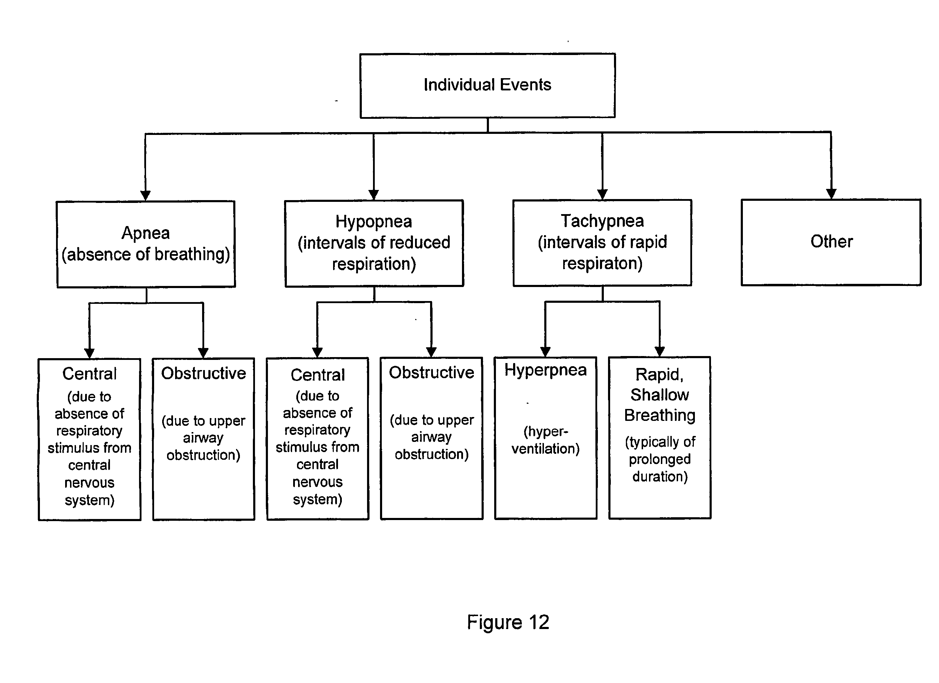

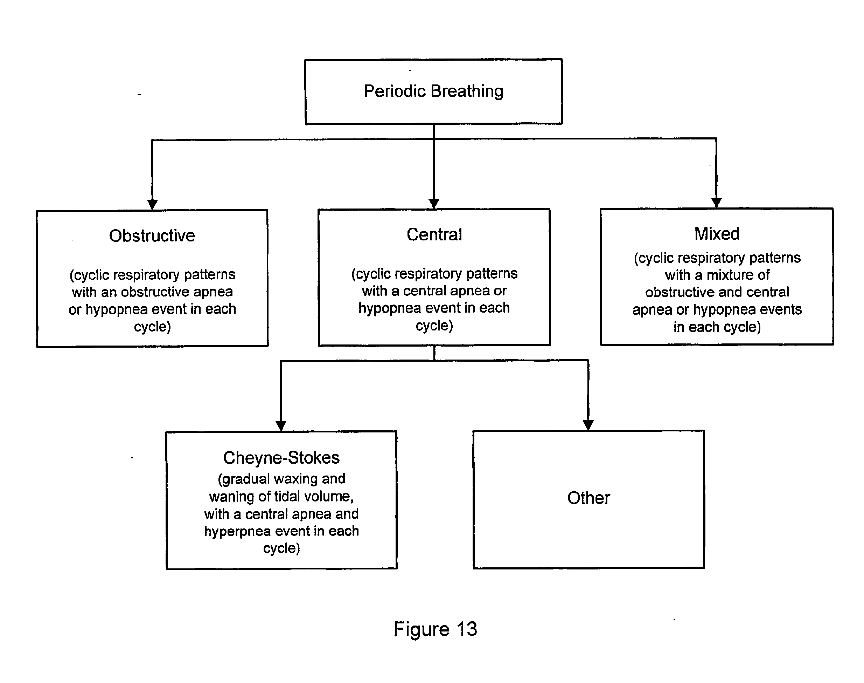

Figures 12 and 13 provide charts illustrating classification of individual disordered breathing events and series of periodically recurring disordered breathing events, respectively. As illustrated in Figure 12, individual disordered breathing events may be grouped into apnea, hypopnea, tachypnea and other disordered breathing events. Apnea events are characterized by an absence of breathing. Intervals of reduced respiration are classified as hypopnea events. Tachypnea events include intervals of rapid respiration characterized by an elevated respiration rate.

As illustrated in Figure 12, apnea and hypopnea events may be further subdivided as either central events, related to central nervous system dysfunction, or obstructive events, caused by upper airway obstruction. A tachypnea event may be further classified as a hyperpnea event, represented by hyperventilation, i.e., rapid deep breathing. A tachypnea event may alternatively be classified as rapid breathing, typically of prolonged duration.

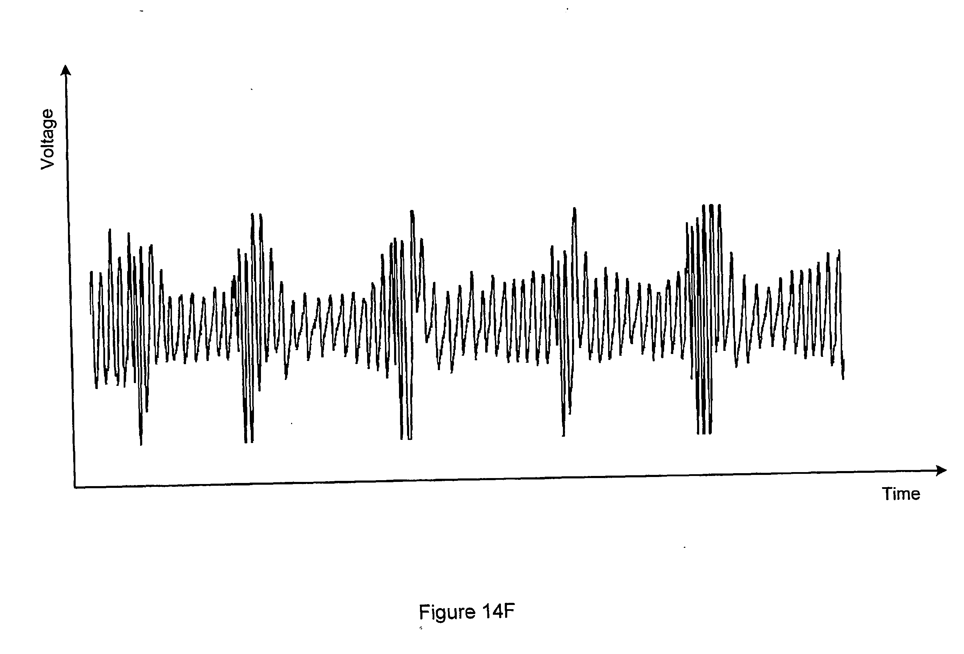

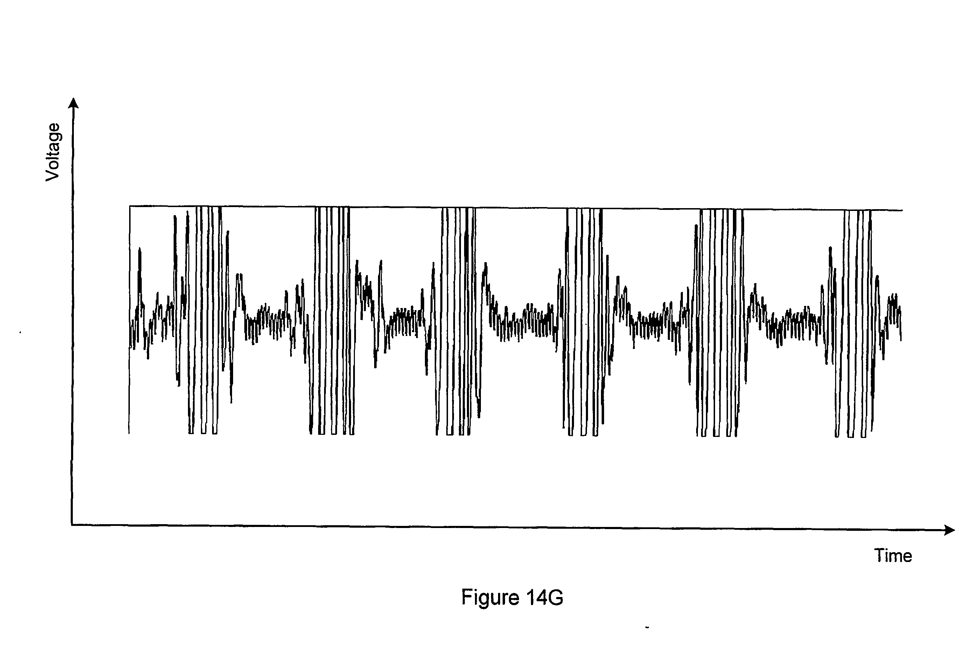

Figure 13 illustrates classification of combinations of periodically recurring disordered breathing events. Periodic breathing may be classified as obstructive, central or mixed. Obstructive periodic breathing is characterized by cyclic respiratory patterns with an obstructive apnea or hypopnea event in each cycle. Central periodic breathing involves cyclic respiratory patterns including a central apnea or hypopnea event in each cycle. Periodic breathing, illustrated in Figure 14F, may also be of mixed origin. Mixed origin periodic breathing is characterized by cyclic respiratory patterns having a mixture of obstructive and central apnea events in each cycle. Cheyne-Stokes respiration, illustrated in Figure 14G, is a particular type of periodic breathing involving a gradual waxing and waning of tidal volume and having a central apnea and hyperpnea event in each cycle. Other manifestations of periodic breathing are also possible. The various forms of disordered breathing may be determined based on the characteristic respiration patterns associated with particular types of disordered breathing.

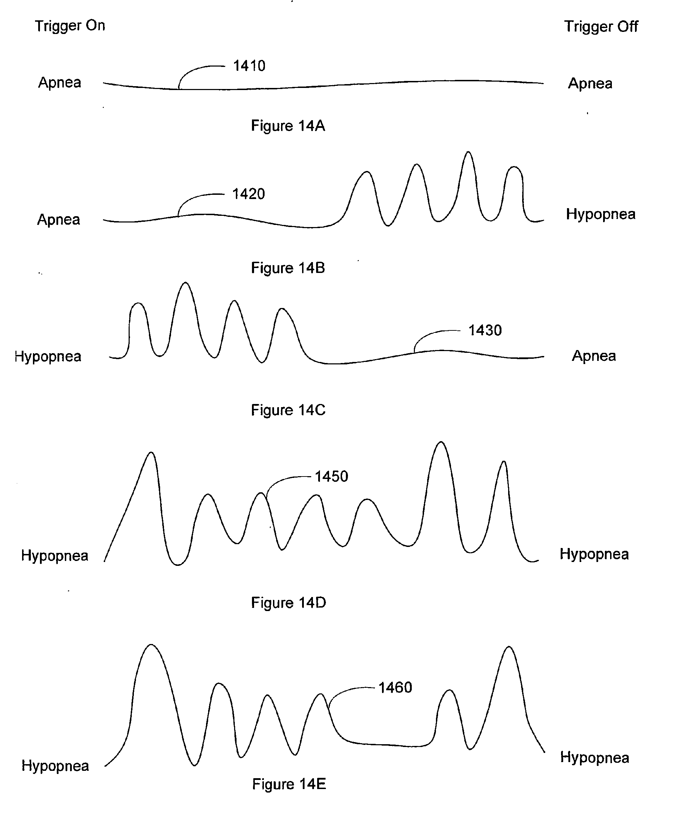

As illustrated in Figures 14A-E, a respiration pattern detected as a disordered breathing episode may include only an apnea respiration cycle 1410 (Figure 14A), only hypopnea respiration cycles 1450 (Figure 14D), or a mixture of hypopnea and apnea respiration cycles 1420 (Figure 14B), 1430 (Figure 14C), 1460 (Figure 14E). A disordered breathing event 1420 may begin with an apnea respiration cycle and end with one or more hypopnea cycles. In another pattern, the disordered breathing event 1430 may begin with hypopnea cycles and end with an apnea cycle. In yet another pattern, a disordered breathing event 1460 may begin and end with hypopnea cycles with an apnea cycle in between the hypopnea cycles.

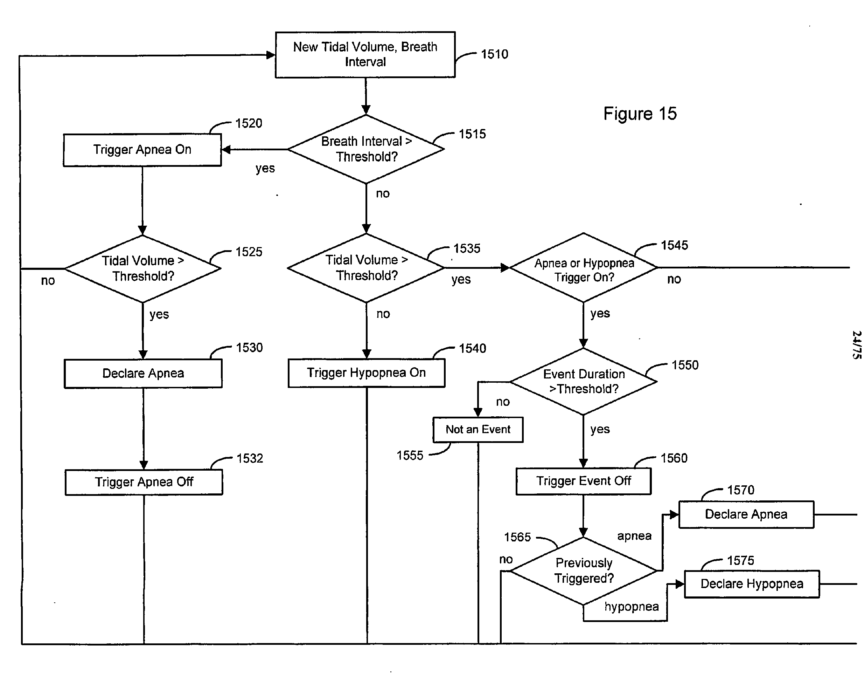

Figure 15 is a flow graph of a method for detecting disordered breathing in accordance with embodiments of the invention. The method illustrated in Figure 15 operates by classifying breathing patterns using breath intervals in conjunction with tidal volume and duration thresholds as previously described above. In this example, a duration threshold and a tidal volume threshold are established for determining both apnea and hypopnea breath intervals. An apnea episode is detected if the breath interval exceeds the duration threshold. A hypopnea episode is detected if the tidal volume of successive breaths remains less than the tidal volume threshold for a period in excess of the duration threshold. Mixed apnea/hypopnea episodes may also occur. In these cases, the period of disordered breathing is characterized by shallow breaths or non-breathing intervals. During the mixed apnea/hypopnea episodes, the tidal volume of each breath remains less than the tidal volume threshold for a period exceeding the duration threshold.

Transthoracic impedance is sensed and used to determine the patient's respiration cycles. Each breath 1510 may be characterized by a breath interval, the interval of time between two impedance signal maxima, and a tidal volume (TV).

If a breath interval exceeds 1515 the duration threshold, then the respiration pattern is consistent with an apnea event, and an apnea event trigger is turned on 1520. If the tidal volume of the breath interval exceeds 1525 the tidal volume threshold, then the breathing pattern is characterized by two respiration cycles of normal volume separated by a non-breathing interval. This pattern represents a purely apneic disordered breathing event, and apnea is detected 1530. Because the final breath of the breath interval was normal, the apnea event trigger is turned off 1532, signaling the end of the disordered breathing episode. However, if the tidal volume of the breath interval does not exceed 1525 the tidal volume threshold, the disordered breathing period is continuing and the next breath is checked 1510.

If the breath interval does not exceed 1515 the duration threshold, then the tidal volume of the breath is checked 1535. If the tidal volume does not exceed 1535 the tidal volume threshold, the breathing pattern is consistent with a hypopnea cycle and a hypopnea event trigger is set on 1540. If the tidal volume exceeds the tidal volume threshold, then the breath is normal.

If a period of disordered breathing is in progress, detection of a normal breath signals the end of the disordered breathing. If disordered breathing was previously detected 1545, and if the disordered breathing event duration has not exceeded 1550 the duration threshold, and the current breath is normal, then no disordered breathing event is detected 1555. If disordered breathing was previously detected 1545, and if the disordered breathing event duration has extended for a period of time exceeding 1550 the duration threshold, and the current breath is normal, then the disordered breathing trigger is turned off 1560. In this situation, the duration of the disordered breathing episode was of sufficient duration to be classified as a disordered breathing episode. If an apnea event was previously triggered 1565, then an apnea event is declared 1570. If a hypopnea was previously triggered 1565, then a hypopnea event is declared 1575.

Central/Obstructive Disordered Breathing Discrimination

In some embodiments of the invention, the disordered breathing predictor/detector 258 (Figure 2) may include circuitry for classifying the origin of disordered breathing events. The origin of disordered breathing events may be classified as central, obstructive, or a combination of central and obstructive origin types. Classification of disordered breathing origin may be used to more accurately diagnose breathing rhythm disorders. Further, therapy delivered to treat central disordered breathing may differ from therapy delivered to treat obstructive disordered breathing. Classification of disordered breathing origin allows the therapy controller 265 (Figure 2) to provide an appropriate therapy corresponding to the disordered breathing origin.

According to various implementations, disordered breathing events may be classified based on a patient's motion associated with respiratory effort during the disordered breathing event. For example, central apnea may be identified by insufficient respiration for at least about 10 seconds with insufficient respiratory effort. Obstructive apnea may be identified by insufficient respiratory inspiration for at least about 10 seconds accompanied by respiratory effort. Respiratory effort may be detected by sensing patient motion associated with respiratory effort during the disordered breathing event. The sensed motion may comprise motion of the patient's chest, abdomen, diaphragm, and/or other motion associated with respiratory effort.

Disordered breathing episodes may be classified as central disordered breathing, obstructive disordered breathing, or a combination of central and obstructive types. Various forms of disordered breathing that may be classified with respect to origin (central, obstructive, or mixed origin) may include, for example, apnea, hypopnea, hyperpnea, tachypnea, periodic breathing, Cheyne-Stokes respiration (CSR), and/or other forms of disordered breathing.

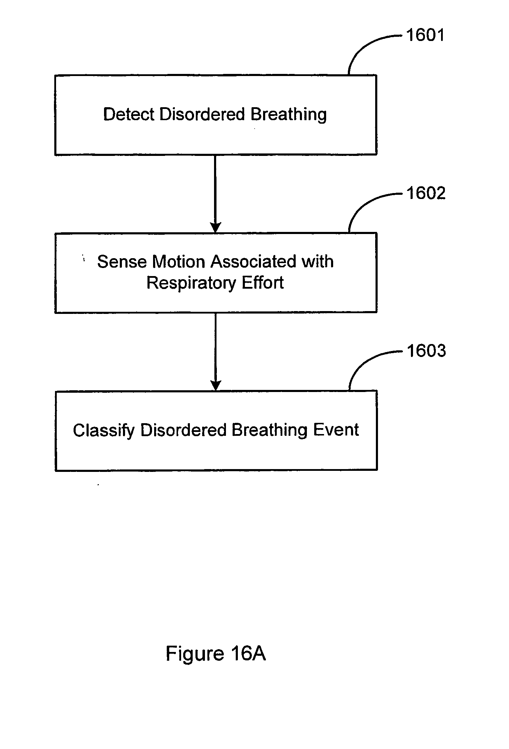

Figure 16A is a flowchart of a method of classifying a disordered breathing event in accordance with embodiments of the invention. The method involves detecting 1601 a disordered breathing event and sensing 1602 motion associated with respiratory effort during the disordered breathing event. Disordered breathing may be detected based on the patient's respiration patterns, or by other methods. Motion associated with respiratory effort may be involve chest wall motion, abdominal motion and/or other motions associated with respiratory effort. The disordered breathing event may be classified 1603 as central, obstructive, or a mixture of central and obstructive types based on the patient's movements associated with respiratory effort during the disordered breathing event.

In one scenario, the disordered breathing event may include both central and obstructive types. The disordered breathing event may be classified as a mixed central and obstructive disordered breathing event if central disordered breathing is classified during one portion of the disordered breathing event and obstructive disordered breathing is classified during another portion of the disordered breathing event.

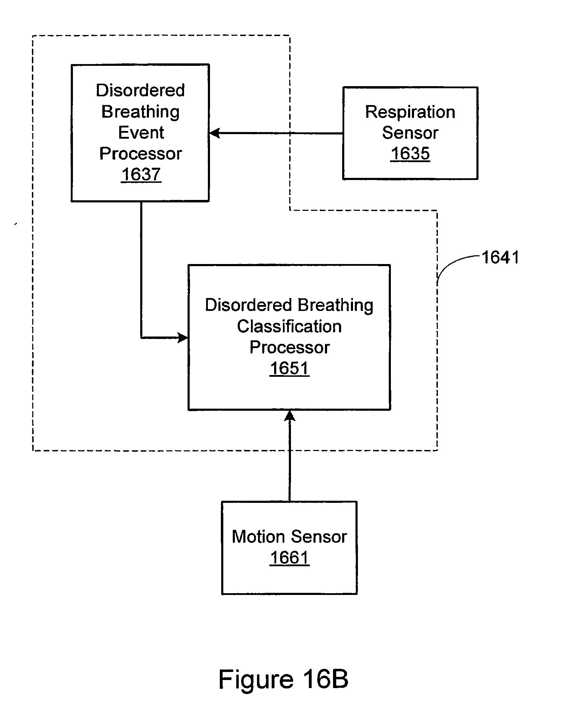

Figure 16B is a block diagram of disordered breathing classification circuitry 1600 that may be implemented in the disordered breathing predictor/detector 258 (Figure 2) for classifying disordered breathing in accordance with embodiments of the invention. The disordered breathing classification circuitry 1600 illustrated in Figure 16B includes a disordered breathing classification processor 1651 that receives signals from a disordered breathing event detector 1637 and a motion sensor 1661.

The disordered breathing event detector 1637 received signals from at least one sensor 1635, e.g., a respiration sensor, for detecting a physiological signal indicative of disordered breathing. The disordered breathing event processor 1637 analyzes the sensor signals and may determine that a disordered breathing event is in progress based on the analysis.

In one implementation, the sensor 1635 generates a signal modulated by patient respiration. Such a signal may be generated, for example, by a transthoracic impedance sensor, an airflow meter, or by other sensing methods. A disordered breathing event may be detected based on the patient's breath intervals and/or tidal volume as described more fully herein.

The motion sensor 1661 may be configured to sense chest wall motion, abdominal motion, and/or other patient movement indicative of respiratory effort. The motion sensor 1661 generates a signal indicative of respiratory effort that is communicated to the disordered breathing classification processor 1651.

The sensors 1635, 1661 may comprise any number of patient-internal and/or patient-external sensors coupled through leads or wirelessly to other components of the disordered breathing classification circuitry 1600. In various embodiments, a signal indicative of the patient's respiration may be acquired using an implantable or patient-external transthoracic impedance sensor, blood oxygen sensor, microphone, flow meter, or by other patient-internal and/or patient-external sensing methods.

Sensing chest, abdominal, or other motion associated with respiratory effort may be accomplished using a patient-internal or patient-external sensing device. In one example, patient motion associated with respiratory effort may be sensed using an implanted or patient-external accelerometer. The accelerometer may be incorporated as a component of an implanted medical device, such as an implantable cardiac rhythm management system having functionality for delivering cardiac electrical therapy for disordered breathing.

In another example, motion associated with respiratory effort may be detected based on changes in an electromyogram (EMG) sensor signal. An EMG sensor may be positioned internally or externally to detect electrical activity of a patient's intercostal, pectoral and/or diaphragmatic muscles indicative of motion.. In yet another example, motion associated with respiratory effort may be detected using a transthoracic impedance sensor. The patient's transthoracic impedance is modulated as the chest wall and/or abdomen moves during inspiratory attempts. Transthoracic impedance may be sensed using intracardiac electrodes, subcutaneous electrodes, or patient-external electrodes positioned at appropriate locations in, on, or about the patient's thorax.

A disordered breathing event may be classified as a central, obstructive or mixed type based on the based on the patient's respiratory efforts during disordered breathing episodes. The disordered breathing classification processor 1651 may discriminate between central and obstructive disordered breathing events using signals received from the motion sensor 1661 and the disordered breathing detector 1641. If patient motion associated with respiratory effort is of sufficient magnitude during disordered breathing, then the disordered breathing classification processor 1651 may determine that the disordered breathing event is obstructive in origin. If respiratory effort motion is insufficient during the disordered breathing event, then the disordered breathing classification processor 1651 may classify the disordered breathing event as central in origin. If the respiratory effort motion is sufficient during one portion of the disordered breathing episode, but is insufficient during another portion, then the disordered breathing classification processor 1651 may classify the episode as a mixture of central and obstructive types.

In one configuration, the disordered breathing classification circuitry 1600 may be fully patient-external. In another configuration, some functions of the disordered breathing classification circuitry may be implemented in an implantable device and other functions may be implemented as a patient external device. The implantable and the patient-external disordered breathing classification system components may be coupled through leads or a wireless communications link, such as through a Blue Tooth or a proprietary wireless communication link.

In yet another configuration, the disordered breathing classification circuitry 1600 may be fully implantable. Disordered breathing classification circuitry 1600 may be incorporated, for example, as a component of a cardiac device such as a pacemaker, defibrillator, cardiac resynchronizer, implantable cardiac monitor, or other implantable medical device.

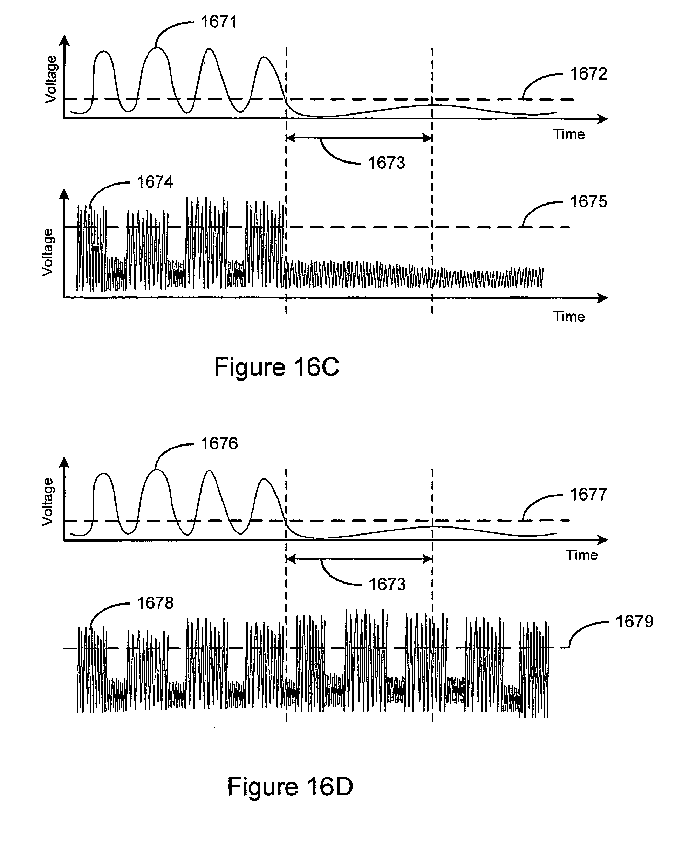

Classification of the disordered breathing event in accordance with the processes of the invention involves evaluating chest wall motion or other motion associated with respiratory effort. Figures 16C and 16D provide graphs of accelerometer signals representing chest wall motion for central and obstructive disordered breathing, respectively. As illustrated in Figure 16C, apnea is detected when the transthoracic impedance signal 1671 remains below an inspiration threshold 1672 for a period of time greater than an apnea interval 1673, e.g., 10 seconds. In this example, the apnea event is a central apnea event and the signal 1674 from an accelerometer sensing the patient's chest wall motion also falls below a motion threshold 1675 during the period of non-respiration. The lack of chest wall motion indicates that the patient's breathing reflex is not being triggered by the central nervous system, indicative of a central disordered breathing event.

Figure 16D illustrates the accelerometer signal and transthoracic impedance signal for an obstructive apnea event. Apnea is detected when the transthoracic impedance signal 1676 remains below an inspiration threshold 1677 for a period of time greater than an apnea interval 1673. In this example, the apnea event is an obstructive apnea event and the signal 1678 from an accelerometer sensing the patient's chest wall motion rises above a chest well motion threshold 1679 during the period of non-respiration. The chest wall motion indicates that the patient's breathing reflex is being triggered by the central nervous system, indicative of an obstructive disordered breathing event.

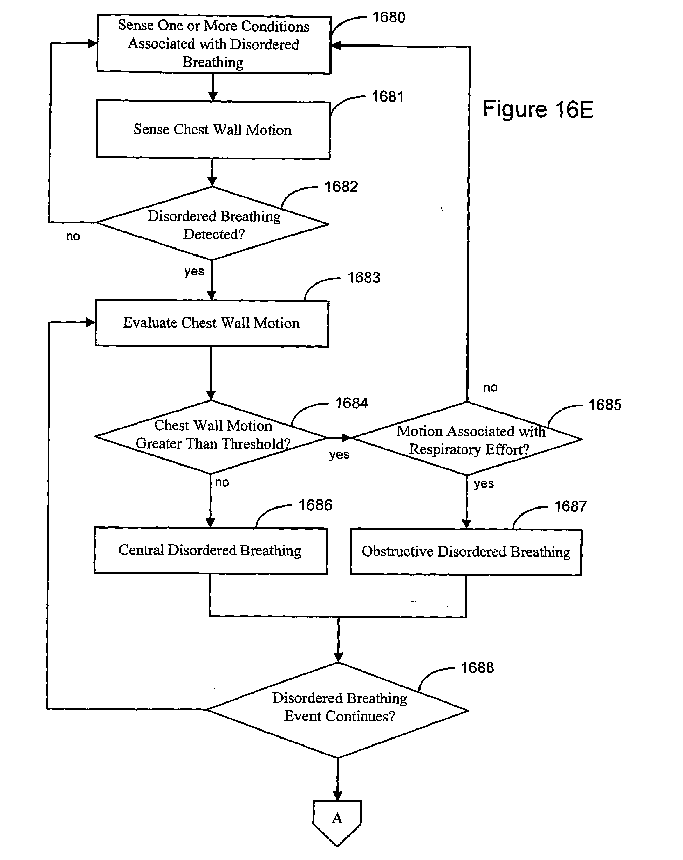

Figure 16E is a flowchart of a method for classifying disordered breathing events as central, obstructive or mixed events in accordance with embodiments of the invention. One or more conditions associated with disordered breathing are sensed 1680. For example, one or more of the conditions listed in Table 1 may be sensed to detect that a disordered breathing event is occurring. The patient's chest wall motion is sensed 1681 during the disordered breathing event.

If disordered breathing is detected 1682, then the chest wall motion signals are analyzed 1683 for obstructive/central origin discrimination. A parameter, e.g., average amplitude or frequency, of the signal produced by the motion sensor may be compared to a threshold. If the chest wall motion signal is not greater 1684 than a threshold, then the disordered breathing is classified 1686 as central disordered breathing. If the chest wall motion signal is greater than or equal to the threshold 1684 and the chest wall motion is associated with respiratory effort 1685, then the disordered breathing is classified 1687 as obstructive disordered breathing. For example, if chest wall motion from the accelerometer is synchronous with a reduced transthoracic impedance during a disordered breathing episode, then the concurrence of disordered breathing and chest wall motion indicates disordered breathing that is obstructive in origin.

If the disordered breathing event continues 1688, then chest wall motion continues to be sensed 1683. A second or subsequent portion of the disordered breathing event may have a different classification from the initial classification based on the presence or lack of motion associated with respiratory effort.

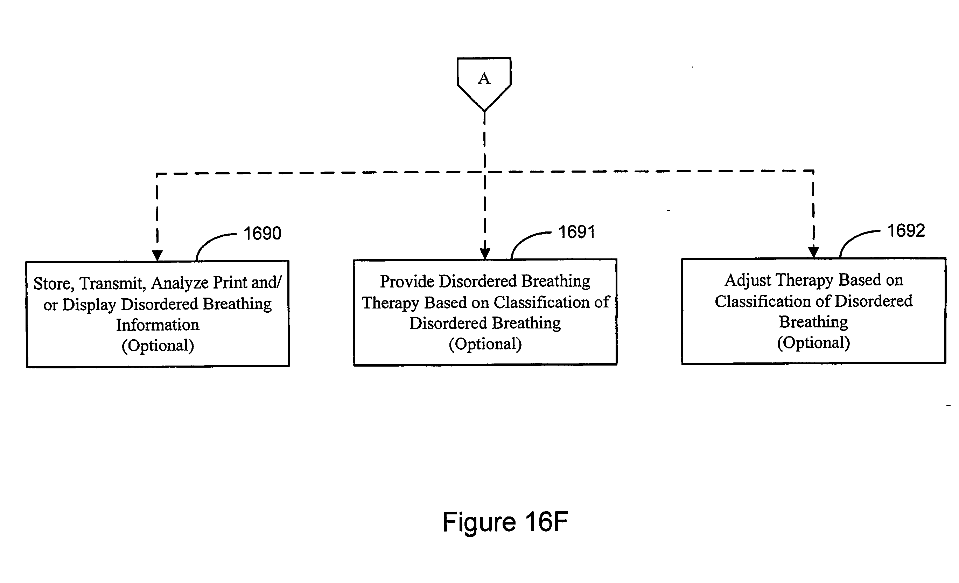

The flowchart of Figure 16F follows from Figure 16E and illustrates optional processes that may be implemented following classification of the disordered breathing event. Disordered breathing information may optionally be stored, transmitted, displayed, and/or printed 1690. For example, disordered breathing information may be stored over several weeks or months to enhance diagnosis of disordered breathing or other conditions, or to analyze disordered breathing trends and/or therapy effectiveness.

Additionally, or alternatively, classification of the origin of disordered breathing events may be used in connection with providing 1691 a therapy to treat the disordered breathing. Therapy for treating disordered breathing may involve cardiac electrical therapy, among other therapies. In one scenario, a first therapy regimen may be used to treat disordered breathing that is central in origin. A second therapy regimen may be used to treat disordered breathing that is obstructive in origin. The first and/or the second therapies may be initiated after the origin of the disordered breathing is determined.

Further, therapies other than disordered breathing therapy may be initiated, modified, or terminated 1692 based on the classification of disordered breathing. For example, as previously discussed, disordered breathing in the form of Cheyne-Stokes respiration is related to congestive heart failure and may be used to monitor the progression of CHF. As previously discussed, Cheyne-Stokes respiration is marked by periodic patterns of waxing and waning respiration interrupted by periods of central apnea. Characteristics of the disordered breathing experienced by the patient, e.g., origin, duration, and severity, may be used to initiate or adjust therapy, such as cardiac pacing therapy and/or cardiac resynchronization therapy, delivered to the patient.

In various embodiments of the invention described herein, discrimination between central and obstructive disordered breathing is based on sensing chest wall motion using an implanted motion sensor, e.g., an accelerometer. In other embodiments, a patient-external motion detector, such as a patient-external accelerometer, patient-external respiratory bands, transthoracic impedance sensor, or a mercury switch, may be used alone or in combination with other implanted or patient-external respiratory sensors and detection algorithms for central/obstructive disordered breathing classification.

In one example, a movement sensor, such as an accelerometer, is mounted inside an implantable CRM device to sense chest wall motions that are indicative of obstructive apnea. The output of the movement sensor may be used in combination with other sensors (such as trans-thoracic impedance) for classification of obstructive apnea. Multi-sensor pulse generators are products in a unique position to provide accurate long-term monitoring and prediction of the progression of disease in patients with disordered breathing. Discrimination between types of apnea events allows more accurate diagnosis, monitoring, and/or treatment of abnormal respiration patterns associated with CHF or sleep disordered breathing. Monitoring with discrimination between types of apnea may enable therapy improvements to counteract the effects of abnormal respiratory patterns.

## Disordered Breathing Prediction

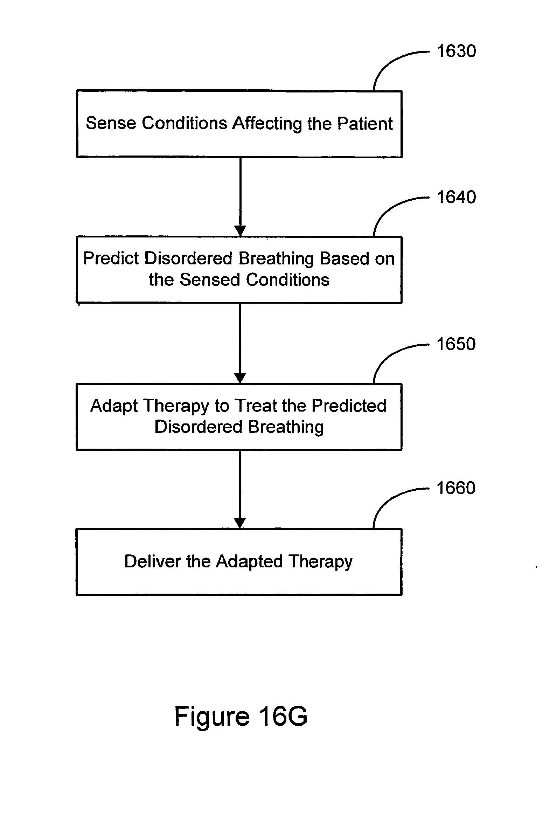

The flowchart of Figure 16G illustrates a method for triggering disordered breathing therapy based on a prediction of disordered breathing according to various embodiments of the invention. The method involves sensing 1630 one or more conditions predictive of disordered breathing and predicting disordered breathing 1640 based on the sensed conditions. Disordered breathing may be predicted, for example, by comparing the detected conditions to disordered breathing prediction criteria. A representative set of conditions that may be used to predict disordered breathing are listed in Table 1. The representative set of conditions listed in Table 1 is not exhaustive, and conditions other than those listed may be used to predict disordered breathing. If disordered breathing is predicted, therapy is adapted 1650 to treat the disordered breathing, e.g., reduce the severity of the disordered breathing or prevent the disordered breathing from occurring. The adapted therapy is delivered 1660 to the patient. One or more of sensing the conditions affecting the patient, predicting the disordered breathing based on the sensed conditions and delivering the therapy to treat the disordered breathing is performed as least in part implantably.

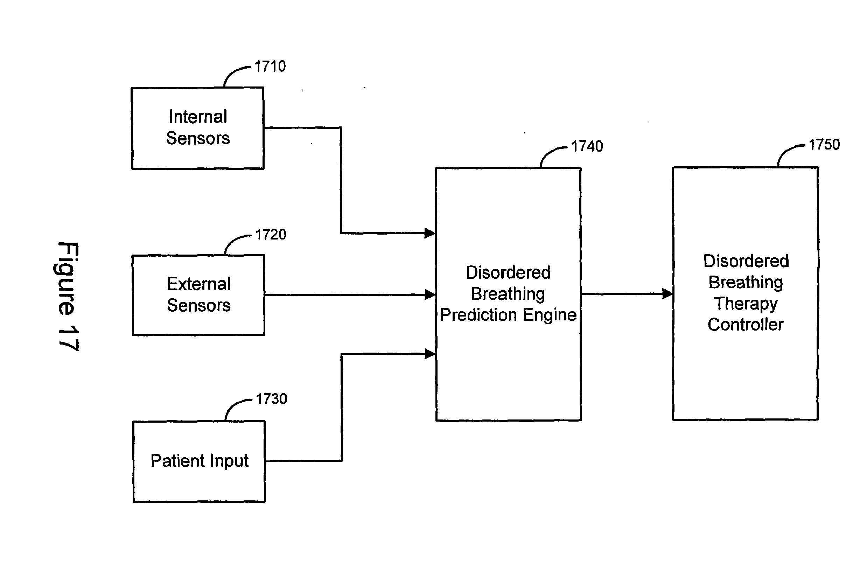

Figure 17 illustrates a block diagram of a disordered breathing therapy system configured in accordance with embodiments of the invention and including disordered breathing prediction functionality. The system may use patient-internal sensors 1710, implanted within the body of the patient, to detect physiological conditions. For example, the system may determine heart rate, heart rate variability, respiration cycles, tidal volume, and/or other physiological signals using an intracardiac electrocardiogram (EGM) signal detector and transthoracic impedance sensor that are part of an implanted cardiac rhythm management system such as a cardiac pacemaker or defibrillator.

The system may use patient-external sensors 1720 to detect physiological or non-physiological conditions. In one scenario, whether the patient is snoring may be useful in predicting disordered breathing. Snoring may be detected using an external microphone or an implanted accelerometer, for example. In another situation, temperature and humidity may be factors that exacerbate the patient's disordered breathing. Signals from temperature and humidity sensors may be used to aid in the prediction of disordered breathing.

Additionally, the system may use information input 1730 by the patient to inform the disordered breathing prediction system of one or more patient conditions. In various embodiments, the patient's medical history, self-described medication use, alcohol or tobacco use, day-time sleepiness, or perceptions of sleep quality over the past one or more sleep periods may be useful in connection with the disordered breathing prediction.

Signals from one or more of the patient-internal sensors 1710, patient-external sensors 1720, and patient input devices 1730 may be coupled to a disordered breathing prediction engine 1740 for prediction evaluation. In one implementation, the prediction engine 1740 may compare the patient conditions to one or more sets of disordered breathing criteria and predict disordered breathing based on the comparison. The prediction engine 1740 is coupled to a therapy controller 1750. If disordered breathing is predicted, the therapy controller 1750 delivers an appropriate therapy to the patient to mitigate the disordered breathing.

In one example, the patient conditions may be sensed and processed using implantable sensors 1710, and the prediction analysis and therapy delivery may be performed by a patient-external disordered breathing prediction engine 1740 and a patient-external therapy controller 1750. Some or all of the implantable sensors 1710 may have remote communication capabilities, such as a wireless proprietary or a wireless Bluetooth communications link. In this implementation, the wireless communications link couples the implantable sensor or sensors 1710 to the patient-external disordered breathing prediction engine 1740. Electrical signals representing patient conditions are produced by the implantable sensors 1710 and transmitted to the patient-external disordered breathing prediction engine 1740.

In another example, an implantable therapy device may incorporate a disordered breathing prediction engine 1740 and one or more patient-external sensors 1720. Signals representing the patient conditions may be transmitted from the patient-external sensors to the implanted prediction engine 1740 over a wireless communication link.

In a further example, the prediction engine may be a patient-external device coupled wirelessly to the therapy controller. Various combinations of wireless or wired connections between the patient-internal sensors 1710, patient-external sensors 1720, patient input devices 1730, the prediction engine 1740, and the therapy controller 1750 are possible.

The above examples provide a few of the many possible configurations that may be used to provide disordered breathing therapy based on the prediction of disordered breathing in accordance with various embodiments of the invention. It is understood that the components and functionality depicted in the figures and described herein can be implemented in hardware, software, or a combination of hardware and software. It is further understood that the components and functionality depicted as separate or discrete blocks/elements in the figures can be implemented in combination with other components and functionality, and that the depiction of such components and functionality in individual or integral form is for purposes of clarity of explanation, and not of limitation.

One subset of the detected patient conditions, such as the representative conditions listed in Table 1, may represent conditions that predispose the patient to disordered breathing. Predisposing conditions may be statistically associated with an onset of disordered breathing during the next few hours following the detection of the conditions leading to the disordered breathing prediction. Another subset of conditions may represent precursor conditions used to predict an imminent onset of disordered breathing that may occur within a time window measured in terms of a few minutes or seconds. Detection of patient conditions associated with disordered breathing and prediction of disordered breathing based on predisposing or precursor conditions is performed on real-time basis.

A subset of patient conditions may be used to verify or otherwise inform the disordered breathing prediction. In one example, information regarding sleep onset or sleep stage or state, e.g., REM or non-REM sleep, may be employed in prediction of sleep disordered breathing. A subset of the conditions listed in Table 1 may be used to detect whether the patient is asleep and to track the various stages of sleep. Another subset of the conditions may be employed to detect disordered breathing episodes, to classify disordered breathing episodes. Table 4 below provides examples of how some conditions listed in Table 1 may be used in disordered breathing prediction.

Table 4

| Condition | Examples of how condition is used in disordered breathing prediction |
|---|---|
| Heart rate | Decrease in heart rate may indicate disordered breathing episode.<br>Decrease in heart rate may indicate the patient is asleep. |

(continued)

| Condition | Examples of how condition is used in disordered breathing prediction |
|---|---|
| Heart rate variability | May be used to determine sleep state and reduction in heart rate variability is a chronic factor associated with disordered breathing. |
| Ventricular filling pressure | May be used to identify/predict pulmonary congestion associated with respiratory disturbance. |
| Blood pressure | Swings in on-line blood pressure measures are associated with apnea. |
| Snoring | Snoring is associated with a higher incidence of obstructive sleep apnea and may be used to detect disordered breathing. |
| Respiration signals/respiration patterns | Respiration patterns may be used to detect disordered breathing episodes. Respiration patterns may be used to determine the type of disordered breathing. Respiration patterns may be used to detect that the patient is asleep. Hyperventilation may be used to predict disordered breathing. Previous episodes of disordered breathing may be used to predict further episodes. One form of disordered breathing may be used to predict another form of disordered breathing |
| Patency of upper airway | Patency of upper airway is related to obstructive sleep apnea and may be used to detect episodes of obstructive sleep apnea. |
| Pulmonary congestion | Pulmonary congestion is associated with respiratory disturbances. |
| Sympathetic nerve activity | End of apnea associated with a spike in SNA |
| CO2 saturation | Low CO2 levels initiate central apnea. |
| O2 saturation | O2 desaturation occurs during severe apnea/hypopnea episodes. |
| Blood alcohol content | Alcohol tends to increase incidence of snoring & obstructive apnea. |
| Adrenalin | End of apnea associated with a spike in blood adrenaline. |
| Brain Natriuretic Peptide (BNP) | A marker of heart failure status, which is associated with Cheyne-Stokes Respiration |
| C-Reactive Protein | A measure of inflammation that may be related to apnea. |
| Drug/Medication/Tobacco use | These substances may affect the likelihood of both central & obstructive apnea. |
| Muscle atonia Eye movement | Muscle atonia may be used to detect REM and non-REM sleep. |
| | Eye movement may be used to detect REM and non-REM sleep. |
| Temperature | Ambient temperature may be a condition predisposing the patient to episodes of disordered breathing. |
| Humidity | Humidity may be a condition predisposing the patient to episodes of disordered breathing. |
| Pollution | Pollution may be a condition predisposing the patient to episodes of disordered breathing. |
| Posture | Posture may be used to determine if the patient is asleep and may predispose the patient to disordered breathing. Posture may be a condition predisposing the patient to episodes of disordered breathing. |
| Activity | Patient activity may be used in relation to sleep detection. |
| Sleep stage | NREM sleep may be associated with a higher probability of DB |
| Location | Patient location may used to determine if the patient is in bed as a part of sleep detection. |

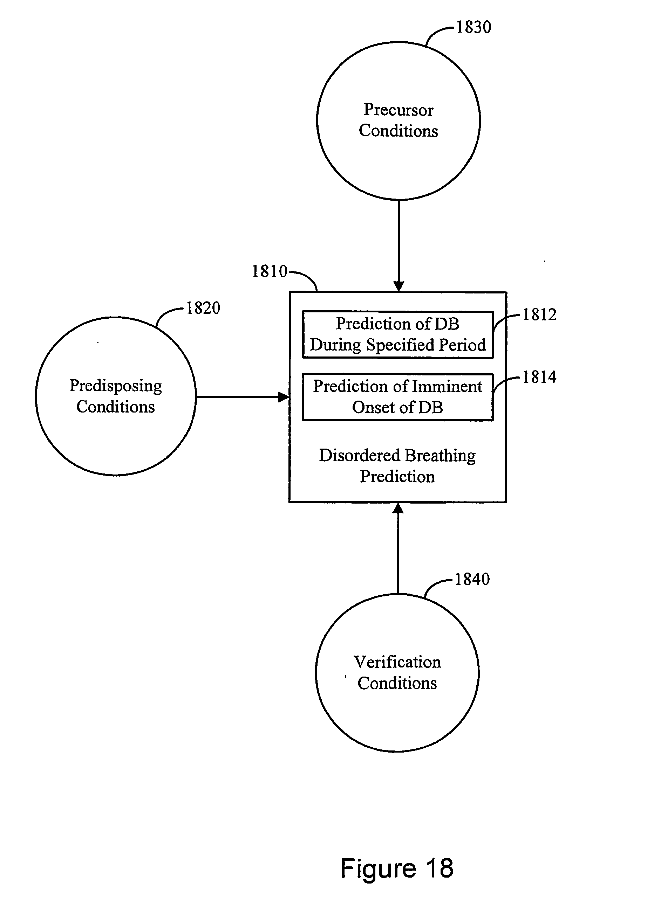

Figure 18 conceptually illustrates how patient conditions such as those listed in Table 1 and/or 4 may be used in predicting disordered breathing 1810 according to embodiments of the invention. In one embodiment, the system tracks one or more of the conditions listed in Table 1, Table 4, or both, to predict disordered breathing. For example, over the course of a period of time, e.g., at least a 16 hour window preceding and including the patient's historical sleep time, the system may track one or more conditions to determine the presence and/or level of each particular condition.

In one implementation, the system tracks conditions that have been determined to predispose 1820 the patient to an attack of disordered breathing. Predisposing conditions represent patient conditions statistically associated with an onset of disordered breathing. The presence of one or more predisposing conditions may indicate that disordered breathing is likely to occur within the next time period, such as an eight hour period following the disordered breathing prediction, or during the current sleep period. For example, the predisposing conditions may include the air pollution index of the patient's environment downloaded from an air quality website, recent tobacco use reported by the patient, the degree of the patient's pulmonary congestion detected by an implanted transthoracic impedance sensor, as well as other predisposing conditions detected patient-internally and/or patient-externally.

Additionally, or alternatively, the system may use previous episodes of disordered breathing to determine that the patient is predisposed to further episodes of disordered breathing within particular time period, such as during a sleep period. For example, previous episodes of disordered breathing during a first interval within the sleep period may be an indication that additional episodes are likely to occur in a second and subsequent interval within the same sleep period. In one example, the occurrence of a first type of disordered breathing may be used to predict a second type of disordered breathing. In another example, the periodicity of disordered breathing may be used to predict future episodes of disordered breathing.

The disordered breathing prediction engine may use the type, duration, frequency, and/or severity of the previous disordered breathing episodes to inform the disordered breathing prediction analysis. Quantification of the severity, frequency, and duration of disordered breathing may be accomplished using any of a number of disturbed breathing measures, including, for example, percent time in disordered breathing and the apnea/hypopnea index.

A further example of a condition predisposing a patient to hypopnea or apnea is body posture. A supine posture is more likely to result in obstruction of the upper airway and can be used to predict episodes of obstructive hypopnea and apnea. Posture and/or torso orientation sensing may be accomplished, for example, using an implantable multiaxis accelerometer.

As previously discussed, sleep disordered breathing is a prevalent form of disordered breathing. Thus, a patient may be more likely to experience episodes of disordered breathing when the patient is in bed sleeping. Thus, proximity to bed may be employed as a predisposing condition to disordered breathing. The disordered breathing therapy system may use a bed proximity sensor to detect that the patient is in bed. Bed proximity may be detected by placing a beacon transmitter on the patient's bed. Receiver circuitry on or in the patient, for example, incorporated in the patient's pacemaker, receives the beacon signal and determines that the patient is in bed.

Conditions that predispose the patient to disordered breathing 1820 are conditions that indicate the likelihood that one or more episodes of disordered breathing will occur during the next time period, such as over the course of the night or other sleep period. Based on predisposing conditions 1820, an onset of disordered breathing may be predicted 1812 to occur within a time window that may include several hours, for example, eight hours.

A second set of conditions, denoted herein as precursor conditions 1830, may be used to predict 1814 an impending onset of disordered breathing. Precursor conditions 1830 indicate that an episode of disordered breathing is imminent and will occur within a time window that may be measured in terms of minutes or seconds, for example. In one implementation, precursor conditions 1830 may be used to predict that an episode of disordered breathing will occur within the next 1800 seconds, for example.

In one embodiment, precursor conditions 1830 indicative of an impending onset of disordered breathing may include, for example, pre-apnea or pre-hypopnea conditions. In one implementation, changes in blood gas concentration, such as $CO_2$, may be causal to central apnea. Therefore, a precursor condition of pre-apnea in a particular patient may be detected when the patient's $CO_2$ level, as measured, for example, by a patient-external $CO_2$ sensor, falls below a selected level, indicating an impending onset of an apnea episode.

In another embodiment, a patient's heart rate variability may be significantly altered before, during, and after episodes of apnea. Heart rate variability may be used, for example, as a precursor condition to predict an impending episode of disordered breathing.

In yet another embodiment, a pre-apnea or pre-hypopnea condition may be detected by analyzing the patient's respiration patterns. Respiration cycles just prior to disordered breathing event, e.g., an apnea or hypopnea event, may exhibit a characteristic pattern. For example, an apnea event for many patients is preceded by a period of hyperventilation with a number of rapid, deep breaths. The pattern of hyperventilation may be detected by analyzing patient's transthoracic impedance signal to determine respiration rate and tidal volume.

Cheyne-Stokes respiration and some apnea/hypopnea episodes may exhibit a crescendo - decrescendo respiration pattern. The crescendo - decrescendo respiration pattern produces hyperventilation during the crescendo stage and hypoventilation during the decrescendo phase. Hyperventilation, secondary to pulmonary congestion, drives arterial partial pressure of carbon dioxide down. A decrease in arterial partial pressure of carbon dioxide below an apnea level may be a causal mechanism for central apnea. According to one embodiment of the invention, detection of an impending onset of disordered breathing may be implemented by detecting a series of increasing tidal volumes followed by a series of decreasing tidal volumes.

For some patients, disordered breathing occurs at regular intervals, allowing the periodicity of the disordered breathing episodes to be used as a precursor condition. If disordered breathing episodes of the patient occur at regular intervals, the next episode of disordered breathing may be predicted based on the time elapsed since the last episode was detected.

In addition, the occurrence of one form of disordered breathing may be used to predict another form of disordered breathing. For example, a patient may characteristically experience one or more episodes of obstructive sleep apnea during the first part of the night followed by central sleep apnea episodes during the later part of the night. In another example, one or more episodes of hypopnea may be used to predict future apnea episodes.

Snoring is an additional example of a pre-apnea or pre-hypopnea condition. In many, patient snoring, or more generally any abnormal airflow in the upper airway, which may be detectable via acoustic means, precedes more significant sleep disordered breathing conditions such as hypopnea or apnea. Precursor conditions 1830 may be analyzed individually, or in combination with one or more predisposing conditions 1820, to predict the impending onset of a disordered breathing episode.

The conditions and associated prediction criteria used for disordered breathing prediction may be highly patient specific. Conditions that are reliably predictors of disordered breathing in one patient may not be effective for another patient. Therefore, conditions used to predict disordered breathing and the respective prediction criteria are preferably based on patient-specific data.

A subset of patient conditions may be used to verify or confirm a prediction of disordered breathing. For example, before or after a prediction of disordered breathing is made, one or more verification conditions 1840 may be checked to confirm the prediction. The verification conditions, as well as the physiological and contextual conditions used to predict disordered breathing, may be highly patient specific.

In one example embodiment, a characteristic pattern of respiration is a reliable predictor of disordered breathing in a particular patient only when the patient is supine. If the prediction is made while the patient not supine, normal variations in respiration cycles in this particular patient may lead to an erroneous prediction of disordered breathing. Thus, before disordered breathing is predicted, a posture sensor signal is checked to verify that the patient is supine. If the patient is supine and the patient's respiration cycles are consistent with criteria indicating that disordered breathing is likely, the disordered breathing prediction is made.

In another example, the patient is known to suffer from episodes of apnea during sleep. The patient's sleep apnea may be predicted using a number of contextual and physiological conditions. The prediction of sleep apnea may be made after assessing that the patient's posture and location are consistent with sleep. Before a prediction of sleep apnea is made, the system confirms that the patient is lying down in bed by checking the signal from an implantable posture sensor and a bed proximity sensor.

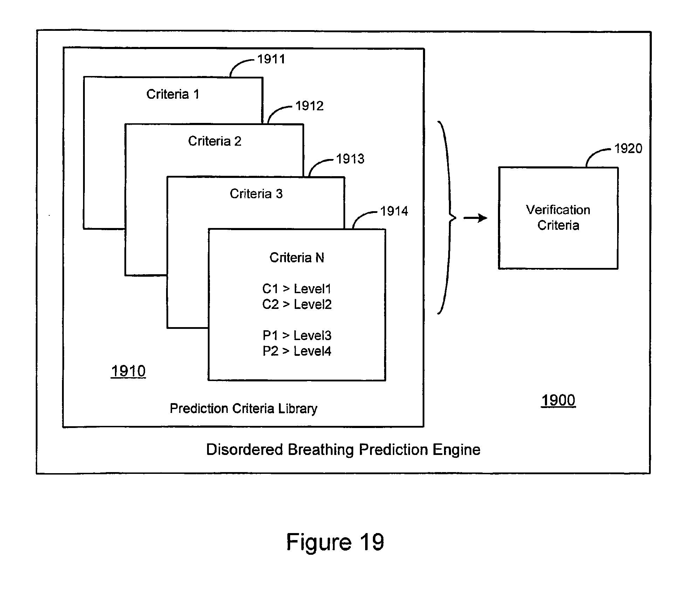

The operation of a disordered breathing prediction engine 1900, according various to embodiments, is conceptually illustrated in the block diagram of Figure 19. Periodically, one or more patient conditions are detected and compared to a library 1910 of prediction criteria. The prediction criteria library 1910 may incorporate one or more sets of prediction criteria 1911, 1912, 1913, 1914. Each of these sets of criteria may be compared to the detected patient conditions. If the criteria of a prediction criteria set 1911, 1912, 1913, 1914 are substantially consistent with the patient conditions, a preliminary disordered breathing prediction may be made.

In various embodiments, the prediction criteria sets 1911, 1912, 1913, 1914 represent one or more condition thresholds associated with an onset of disordered breathing. In one example embodiment, the level of one or more detected conditions may be compared to the prediction criteria sets 1911, 1912 1913, 1914. If the levels of the one or more conditions are substantially consistent with the thresholds specified in a prediction criteria set 1911, 1912, 1913, 1914, a preliminary prediction of disordered breathing may be made.

The examples that follow are described in terms of a condition being consistent with a prediction criteria when the condition exceeds a prediction criteria threshold. However, it will be understood that different threshold requirements may be defined for different conditions. For example, one condition may be defined to be consistent with a prediction criterion when the condition exceeds a prediction criterion threshold. Another condition may be defined to be consistent with a prediction criterion threshold when the condition falls below the threshold. In yet another example, a condition may be defined to be consistent with the prediction criterion when the condition falls within a specified range of values. Patient conditions may be compared to prediction criteria based on the timing, rate of change, or maximum or minimum value of the condition, for example.

In the example provided in Figure 19, the prediction criteria N 1914 involves two contextual conditions, C1 and C2, and two physiological conditions, P1 and P2. In this particular example, if conditions C1, C2, P1, and P2 exceed levels Level1, Level2, Level3, and Level4, respectively, the patient may be likely to experience disordered breathing during the night. Therefore, when conditions C1, C2, and P1, P2 reach the levels specified in criteria N 1914, preliminary prediction of disordered breathing is made. One or more additional verification criteria 1920 may be used to confirm the preliminary predication of disordered breathing.

In another embodiment of the invention, the relationships between the detected conditions are analyzed to predict disordered breathing. In this embodiment, the disordered breathing prediction may be based on the existence and relative values associated with two or more patient conditions. For example, if condition A is present at a level of x, then condition B must also be present at a level of f(x) before a disordered breathing prediction is made.

In yet another embodiment of the invention, the estimated probability, $P(C_n)$, that disordered breathing will occur if a particular condition level is detected may be expressed as a function of the ratio of the number of times disordered breathing occurred within a selected time interval following the detection of the particular condition level to the total number of observed occurrences of the condition level. The probability that disordered breathing will occur, $P(C_n)$, is compared to a threshold probability level to make the disordered breathing prediction. Other methods of calculating the estimated probability are also possible.

The prediction of disordered breathing may be based on the convergence or divergence of a number of conditions occurring within the same time period. In this situation, a composite probability score may be computed as a combination of the individual probabilities. In one embodiment, the probabilities are combined by adding the condition probabilities after multiplying each of the condition probabilities by a weighting factor. For example, if the disordered breathing prediction is based on four substantially simultaneous conditions, $C_1$, $C_2$, $C_3$, and $C_4$, the total probability score $PS_T$ may be calculated as:

$$PS_T = A \times P(C_1) + B \times P(C_2) + C \times P(C_3) + D \times P(C_4), \qquad [1]$$

where A, B, C, and D are scalar weighting factors that may be used to estimate the relative importance of each of the conditions $C_1$, $C_2$, $C_3$, and $C_4$. If the probability score exceeds a selected prediction criteria threshold, then disordered breathing is predicted.

Although the above process describes combining the estimated probabilities for each condition by adding each of the estimated probabilities, other methods are also possible. For example, a detected patient condition may operate against a prediction of disordered breathing. In this situation, the estimated probability, $P_n(C_n)$, that disordered breathing will not occur if a particular condition level is detected may be expressed as a function of the ratio of the number of times disordered breathing did not occur within a selected time interval following the detection of the particular condition level to the total number of observed occurrences of the condition level. This value may be subtracted from the total to determine the probability score. Non-linear methods of combining the estimated probabilities to arrive at a composite probability are also possible.

If the conditions affecting the patient are consistent with a prediction of disordered breathing, the prediction may be verified by comparing one or more verification conditions to verification criteria. If the verification conditions are consistent with the verification criteria, a prediction of disordered breathing is made.

In the embodiments described above, predictions of disordered breathing are based upon comparisons of one or more patient conditions to sets of prediction criteria. The initial data from which the initial prediction criteria sets are formed may be derived from past observations taken from population data, or from data collected from a particular patient. The initial prediction criteria sets may then be modified as additional data are collected from the patient.

In one embodiment, an estimated accuracy for the prediction criteria is updated for every prediction event. The estimated positive predictive value (PPV) for a prediction criteria set N may be expressed as:

$$PPV_N = \frac{TP}{TP + FP} \qquad [2]$$

where TP (true positive) is the number of times the prediction criteria set successfully predicted disordered breathing, and FP (false positive) is the number of times the prediction criteria erroneously predicted disordered breathing.

If the estimated accuracy of prediction criteria set N, $PPV_N$, falls below a predetermined level, for example, 0.7, the prediction criteria set N may be modified. In one embodiment, a possible prediction criteria set is formed, for example, by modifying the threshold level of one or more of the conditions represented by the original prediction criteria set N. In one embodiment, each threshold in the original prediction criteria set N is modified by an incremental value, to make the prediction criteria set more accurate.

In another embodiment, conditions represented in the original prediction criteria set N are compared to the conditions that are present just prior to a disordered breathing occurrence to determine how the modification for the possible prediction criteria set should be implemented. For example, if the level of a particular condition just prior to the occurrence shows a relatively large variation just prior to the disordered breathing episode, but the levels of other conditions remain constant, then only the changing level may be modified in the possible prediction criteria set.

Each time the possible prediction criteria set is satisfied, no prediction of disordered breathing is made, however, the accuracy of the possible prediction criteria set is updated, for example, using an equation similar in form to Equation 2. If the accuracy of the possible prediction criteria set reaches a selected level, for example, 0.7, and the accuracy original prediction criteria set N remains below 0.7, the possible prediction criteria set may replace the original prediction criteria set N in the prediction criteria library.

According to various embodiments, new prediction criteria sets may be added to the prediction criteria library. In accordance with these embodiments, if a disordered breathing episode occurs without prediction, the levels of the detected patient conditions prior to the disordered breathing episode are saved as a possible prediction criteria set. Each time the possible prediction criteria set is satisfied, no prediction of disordered breathing is made, however, the accuracy of the possible prediction criteria set is updated, for example, using an equation similar in form to Equation 2. If the accuracy of the possible prediction criteria set reaches a selected level, for example, 0.7, the possible prediction criteria set may be added to the prediction criteria library.

The system may also be adjusted to provide increasingly sensitive disordered breathing prediction criteria sets, according to various embodiments. The estimated sensitivity for a prediction criteria set N may be expressed as:

$$\text{Sensitivity}_N = \frac{TP}{TP + FN} \qquad [3]$$

where TP (true positive) is the number of times the prediction criteria successfully predicted disordered breathing, and FN (false negative) is the number of times the prediction criteria erroneously predicted that disordered breathing would not occur.

In one embodiment, if the prediction criteria accuracy for the prediction criteria set N becomes larger than a selected number, for example, 0.9, then the threshold levels of one or more of the conditions represented in the prediction criteria set N may be adjusted to provide enhanced sensitivity.

In one example, the threshold level of each condition represented in the prediction criteria set N is modified by an incremental value, thus making the prediction criteria set N more sensitive. In another embodiment, conditions represented in the prediction criteria set N are compared to the conditions that are present just prior to a disordered breathing occurrence to determine how the modification of the prediction criteria set N should be implemented. In yet another embodiment, a condition threshold level that is modified is based upon the relative importance of the condition in the overall prediction criteria. In another example, if the level of a particular condition is changing just prior to the occurrence of the disordered breathing episode, but the levels of other conditions remain constant, only the changing condition may be modified.

Following adjustment by any of the processes described above, the adjusted prediction criteria set may be designated as a possible prediction criteria set. Each time the possible prediction criteria set is satisfied, no prediction of disordered breathing is made, however, the accuracy of the possible prediction criteria set is updated, for example, using Equation 2 or 3. If the accuracy of a possible prediction criteria set reaches a selected level, for example, 0.7, the possible prediction criteria set may be added to the prediction criteria library.

The system may also be adjusted to provide improved specificity or a negative predictive value (NPV) of disordered breathing prediction criteria in a manner similar to the adaptive method described previously. Calculation of specificity and NPV for a prediction criteria N may be accomplished using equations 4 and 5 below.

$$\text{Specificity}_N = \frac{TN}{TN + FP} \qquad [4]$$

$$\text{NPV}_N = \frac{TN}{TN + FN} \qquad [5]$$

where TN (true negative) is the number of times the prediction criteria successfully predicted the absence of disordered breathing, FP (false positive) is the number of times the prediction criteria erroneously predicted disordered breathing and FN (false negative) is the number of times the prediction criteria erroneously predicted the absence of disordered breathing.

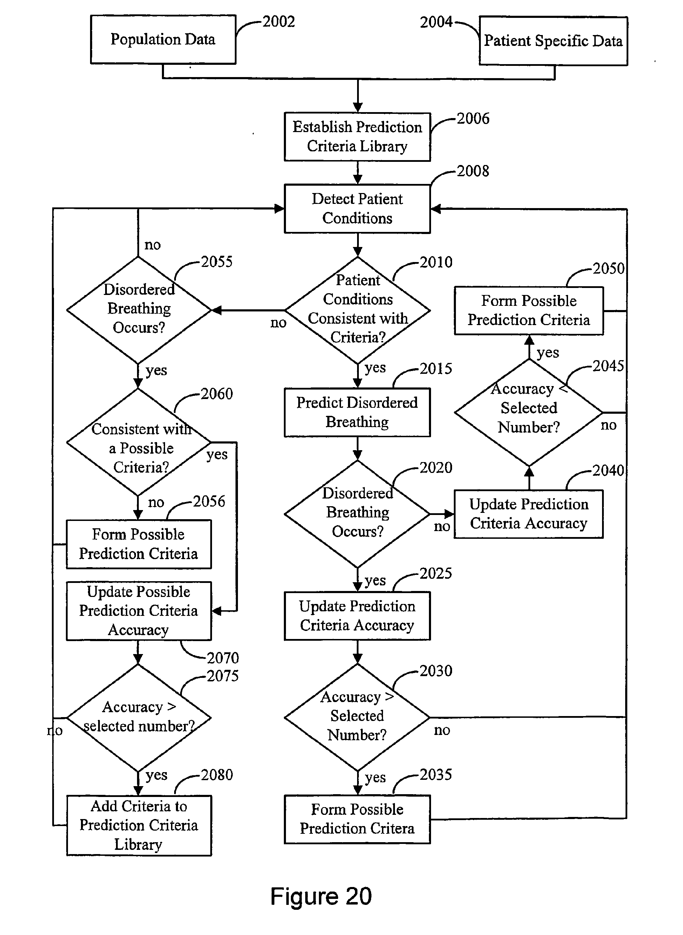

The flowchart of Figure 20 illustrates a method for establishing and updating the prediction criteria library according to embodiments of the invention. Previous observations of disordered breathing may be assimilated from population data 2002 or from past observation of the specific patient 2004. One or more prediction criteria sets are determined and organized in a prediction criteria library 2006.

Conditions associated with disordered breathing are periodically detected 2008 and compared to the prediction criteria sets in the prediction criteria library. If the conditions are consistent 2010 with any of the prediction criteria sets in the library, then disordered breathing is predicted 2015. Within a selected time window following the disordered breathing prediction, the system determines if disordered breathing occurs 2020.

One illustrative approach to detecting disordered breathing involves monitoring a respiratory waveform output, for example, using a transthoracic impedance sensor. When the tidal volume (TV) of the patient's respiration, as indicated by the transthoracic impedance signal, falls below a hypopnea threshold, then a hypopnea event is declared. For example, a hypopnea event may be declared if the patient's tidal volume fall below about 50% of the recent average tidal volume or other baseline tidal volume. When the patient's tidal volume falls further to an apnea threshold, e.g., about 10% of the recent average tidal volume, an apnea event is declared.

If disordered breathing occurs 2020, the prediction criteria accuracy of the prediction criteria set used for the disordered breathing prediction is updated 2025. If the updated prediction criteria accuracy is greater 2030 than a selected number, then a possible prediction criteria set is formed 2035. The possible prediction criteria set may be formed, for example, by substituting more sensitive condition levels when compared to the original prediction criteria set.

If disordered breathing is not detected 2020 following the prediction, then the prediction criteria set accuracy is updated 2040. If the prediction criteria set accuracy decreases 2045 below a selected number, then a possible prediction criteria set 2050 is formed. The possible prediction criteria set may be formed, for example, by substituting more stringent condition levels to produce a more accurate prediction.

If the detected patient conditions are not consistent 2010 with any of the prediction criteria sets in the prediction criteria library, disordered breathing is not predicted. Within a time window following the disordered breathing prediction, the system determines if disordered breathing occurs 2055. If disordered breathing occurs 2055, then the system checks to see if the patient conditions are consistent 2060 with any of the possible prediction criteria sets. If the patient conditions are not consistent 2060 with any of the possible prediction criteria sets, a possible prediction criteria set is formed 2056.

If the patient conditions are consistent 2060 with a possible criteria set, the possible prediction criteria set accuracy is updated 2070. If the possible prediction criteria accuracy increases beyond a selected number 2075, the possible prediction criteria set is added 2080 to the prediction criteria library.

<u>Adaptation of Therapy Based on Therapy Effectiveness</u>

As previously discussed in connection with Figure 2, a disordered breathing therapy system may include a therapy assessment processor 260 for assessing various parameters of the therapy. The therapy assessment processor may receive input from one or more of the patient-internal sensors 280, 231, patient-external sensors 290, and/or other input devices 270 capable of sensing conditions affecting the patient. The therapy assessment processor may also receive information from one or more of the cardiac arrhythmia detector 252, sleep quality monitor 254, sleep detector 256 and/or disordered breathing detector/predictor 258. The therapy assessment processor may use the information acquired from one or more of these sources to adapt the therapy to achieve a therapeutic goal, for example, to adapt the therapy to achieve a level of effectiveness.

In one implementation, a therapeutic goal may involve terminating detected disordered breathing episodes and the disordered breathing therapy may be adapted to achieve this goal. Additionally, or alternatively, a therapeutic goal may involve terminating a disordered breathing episode and preventing further disordered breathing. In this example situation, the therapy regimen may be adapted to provide a first therapy to terminate the disordered breathing episode and provide a second preventative therapy to reduce or eliminate further disordered breathing episodes. The second preventative therapy may be adapted to reduce episodes of disordered breathing below a predetermined disordered breathing episode threshold. A disordered breathing episode threshold may be expressed, for example, in terms of an apnea/hypopnea index (AHI) or percent time in periodic breathing (%PB).

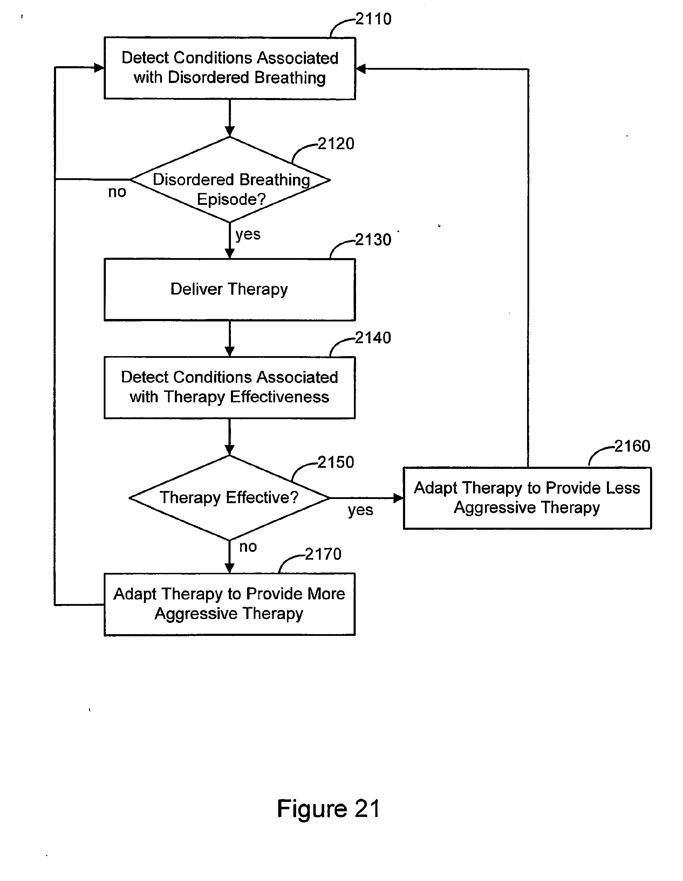

Figure 21 is a flowchart illustrating a method of adapting a disordered breathing therapy according to embodiments of the invention. The flowchart of Figure 21 illustrates a method of adapting disordered breathing therapy to achieve a desired level of therapy efficacy. In this embodiment, a first set of conditions associated with disordered breathing is detected 2110 and used to determine if a disordered breathing episode is occurring. If disordered breathing is detected 2120, disordered breathing therapy is delivered 2130 to the patient to mitigate the disordered breathing. In one embodiment, the therapy delivered to the patient may include, for example, cardiac pacing at a rate in excess of an intrinsic rate, or in excess of a normally programmed rate, such as a normally programmed sleep rate.

Adapting the cardiac electrical therapy may also involve modifying the electrical stimulation energy with or without an increase in the pacing rate. Increased stimulation energy has been shown to produce higher cardiac contractility, which may be particularly beneficial for patients suffering from chronic heart failure. Loss of cardiac contractility is thought to initiate and drive the progression of heart failure, a disorder that is intertwined with Cheyne-Stokes respiration.

Further, adapting a cardiac electrical therapy to mitigate disordered breathing may involve adapting a therapy involving non-excitatory electrical stimulation of one or more heart chambers, e.g., the left and/or right ventricles, or other cardiac sites. Non-excitatory electrical stimulation may be delivered during absolute refractory periods of the cardiac tissue, for example, to improve cardiac contractility. The non-excitatory stimulation therapy may be used alone or in combination with the pacing to provide a comprehensive therapy regimen for patients with CHF and disordered breathing such as Cheyne-Stokes respiration.

In other embodiments, adapting the cardiac electrical therapy to mitigate disordered breathing may involve initiating a particular pacing regimen or switching from one pacing mode to another pacing mode. In one example, the cardiac pacing regimen may be switched from a dual-chamber pacing mode to a bi-ventricular or other resynchronization mode. In other examples, the pacing mode may be switched to a pacing mode that promotes atrial pacing, or promotes consistent ventricular pacing. In yet another example, the cardiac electrical therapy may involve initiating multi-site electrical stimulation to the heart or changing from one electrical stimulation site to another. The pacing mode may be switched from single chamber to multiple chambers, or the reverse. For example, a bi-ventricular mode may be switched to a left ventricular mode only. Alternatively, a single chamber mode, e.g., LV or RV, may be switched to a bi-ventricular mode. Other therapy regimens, involving various pacing modes, pacing sites, or non-excitatory electrical stimulations, are possible in connection with providing cardiac electrical therapy for disordered breathing. The type of cardiac electrical therapy beneficial to a patient is highly patient specific and may be determined based on the responses of a particular patient.

A second set of conditions associated with therapy effectiveness is sensed 2140 and used to assess the effectiveness of the therapy. The detected conditions used to assess the efficacy of the therapy and adapt the therapy to mitigate disordered breathing may represent one or more of the acute conditions associated with disordered breathing, e.g., detected episodes of interrupted breathing, hypoxia, arousals, negative intrathoracic pressure, blood pressure, and heart rate or blood pressure surges.

Additionally, or alternatively, the conditions used to assess therapy efficacy and adapt the cardiac electrical therapy may include one or more chronic conditions associated with disordered breathing, including, for example, decreased heart rate variability, increased blood pressure, chronic changes in sympathetic nerve activity, and changes in blood chemistry, such as increased levels of $PaCO_2$ and norepinephrine levels, among others.

In general, a therapeutic goal in the treatment of disordered breathing is to provide the least aggressive therapy that effectively mitigates, terminates or prevents the patient's disordered breathing or achieves a particular therapeutic goal associated with disordered breathing therapy. The disordered breathing therapy regimen may be enhanced by increasing the intensity or level of therapy to more effectively mitigate the disordered breathing. Alternatively, the disordered breathing therapy regimen may be enhanced by reducing the intensity or level of therapy while maintaining a desired decrease in the severity or frequency of disordered breathing episodes, thus reducing undesirable side effects from the therapy and extending the device lifetime.

If the therapy effectiveness is acceptable 2150, e.g., terminates or reduces the patient's disordered breathing or meets some other desired goal, then the therapy may be adapted 2160 to provide a less aggressive therapy, e.g., decreased pacing rate, decreased pacing energy, or altered pacing mode, as described above. If the therapy is not effective 2150, then the therapy may be adapted 2170 to enhance therapy efficacy by providing a more aggressive therapy, e.g., increased pacing rate, increased pacing energy, or pacing mode switch.

In one embodiment, therapy may be determined to be ineffective if disordered breathing continues unmitigated following therapy delivery. In this situation, the therapy may be adapted to provide a more aggressive therapy, for example, cardiac pacing at a higher rate. In another embodiment, if the disordered breathing decreases sufficiently in severity, or is otherwise sufficiently mitigated, the therapy may be enhanced by adapting the therapy to provide a less aggressive therapy, e.g., pacing at a lower rate or a decreased energy level. As previously discussed, a less aggressive therapy is preferable to reduce the risk of arousal, to avoid unnecessary stress on the patient's heart, and to prolong battery life, for example.

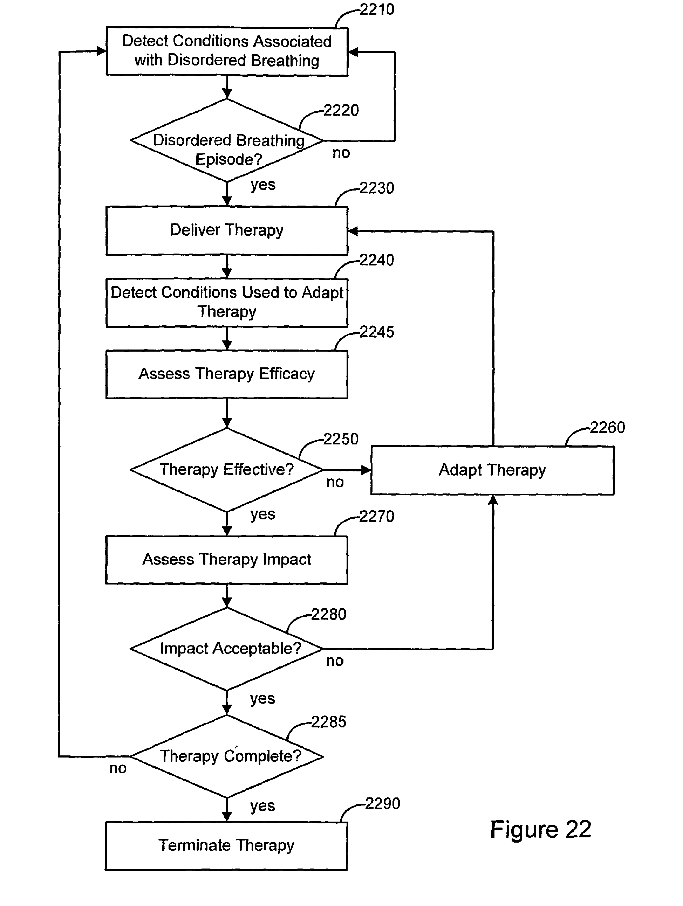

The flowchart of Figure 22 illustrates a method of adapting a disordered breathing therapy taking into account both therapy effectiveness and therapy impact in accordance with embodiments of the invention. In this example, a first set of conditions indicative of disordered breathing are sensed 2210 and used to determine if a disordered breathing episode is occurring. If disordered breathing is detected 2220, therapy is delivered 2230 to the patient to mitigate the disordered breathing.

A second set of conditions, possibly overlapping the first set, are sensed 2240 and used to adapt the therapy. Based on the second set of sensed conditions, therapy efficacy is assessed 2245. If the therapy efficacy is not acceptable 2250, then the therapy may be adapted 2260 to enhance therapy efficacy. If the therapy efficacy is acceptable 2250, then the impact of the therapy on the patient may be assessed 2270.

If the therapy impact on the patient is acceptable 2280, the system continues to deliver the therapy. When the therapy regimen is complete 2285, then therapy is terminated 2290.

If the therapy impact on the patient exceeds acceptable limits, the therapy impact is not acceptable 2280, and the therapy may be adapted 2260 to reduce the therapy impact. Various methods of assessing the impact of the therapy and determining if the therapy impact is acceptable are described herein.

The methods illustrated in the flow graphs of Figures 21 and 22 contemplate real-time monitoring of patient conditions allowing the therapy system to dynamically adjust the therapy regimen to accommodate the changing needs of the patient. In one configuration, the therapy may be adjusted during the period that therapy is delivered to the patient. In another configuration, the therapy may be adapted between disordered breathing episodes or from night-to-night based on assessment of the efficacy of therapy delivered in connection with one or more previously detected disordered breathing episodes.

Evaluation of the impact of disordered breathing therapy on the patient preferably takes into consideration the impact of disordered breathing therapy on the overall therapeutic goals for the patient, including cardiac pacing therapy goals and disordered breathing therapy goals. The disordered breathing therapy may involve a variety of therapy regimens implemented to achieve predetermined therapeutic goals. The effectiveness of the therapy, or the degree to which the therapy meets one or more therapeutic goals, may be assessed by detecting and analyzing episodes of disordered breathing that occur during therapy delivery, or during other periods, including periods of wakefulness.

For example, a therapeutic goal may involve terminating a disordered breathing episode and the disordered breathing therapy may be adapted to achieve this goal. Additionally, or alternatively, a therapeutic goal may involve terminating a disordered breathing episode and preventing further disordered breathing. In this example situation, the therapy regimen may be adapted to provide a first therapy to terminate the disordered breathing episode and provide a second preventative therapy to reduce or eliminate further disordered breathing episodes. The second preventative therapy may be adapted to reduce episodes of disordered breathing below a predetermined disordered breathing episode threshold. A disordered breathing episode threshold may be expressed, for example, in terms of an apnea/hypopnea index (AHI) or percent time in periodic breathing (%PB).

Cardiac Pacing for Disordered Breathing

In various implementations, disordered breathing therapy may comprise overdrive pacing. Overdrive pacing comprises pacing one or more heart chambers at a rate higher than an intrinsic rate. In accordance with embodiments of the invention, therapy to mitigate disordered breathing involves overdrive cardiac pacing of one or more atria and/or one or more ventricles as treatment for disordered breathing.

When operating in the overdrive pacing mode, a cardiac rhythm management device may deliver pacing pulses at a pacing preference (PP) rate that is a small amount above the intrinsic heart rate. If intrinsic beats are detected, the PP rate may be increased until it becomes slightly faster than the intrinsic heart rate of the sensed beat. The PP rate may then be gradually decreased to search for the intrinsic heart rate. After an intrinsic beat is sensed, the PP rate may be increased until the pacing rate is a small amount above the intrinsic heart rate.

In one implementation, a CRM device may be switched to operate in the overdrive pacing mode upon detection or prediction of disordered breathing. In another implementation, the CRM device may be switched to operate in the overdrive pacing mode following a determination that the patient is asleep. In yet another implementation, characteristics of the disordered breathing are used to develop an indicated pacing interval. The description that follows involves atrial overdrive pacing in the AAI(R) or DDD(R) modes. It will be appreciated that similar techniques may be implemented to effect ventricular overdrive pacing in the VVI(R) mode or overdrive pacing in a biventricular mode.

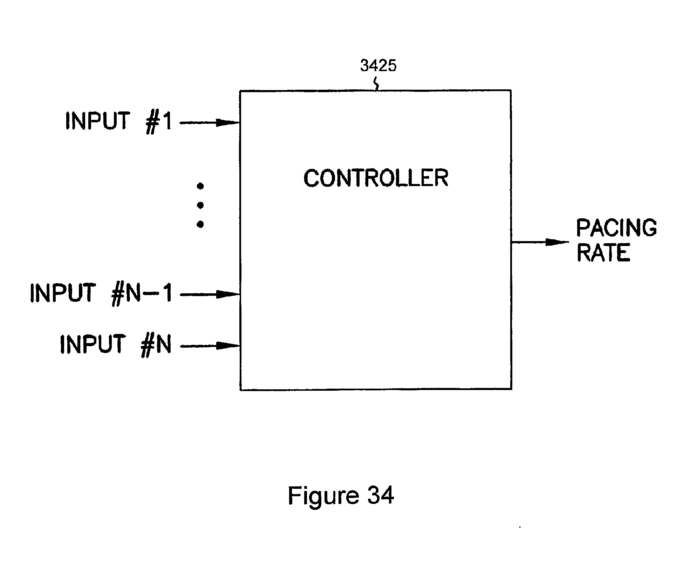

Figure 34 is a block diagram illustrating a pacemaker controller 3425 in accordance with embodiments of the invention. The pacemaker controller 3425 uses signals from several different inputs to modify the rate at which pacing or other therapy is delivered. For example, Input #1 may provide information about atrial heart rate, Input #2 may provide information about ventricular heart rate, Input #3 may provide an accelerometer-based indication of activity, and Input #4 may provide an impedance-based indication of respiration, such as minute ventilation. Based on at least one of these and/or other inputs, controller 3425 provides an output indication of pacing rate as a control signal delivered to a therapy delivery circuit, such as to one or more of an atrial therapy delivery circuit and a ventricular therapy delivery circuit.

Atrial and ventricular therapy delivery circuits issue pacing pulses based on one or more such control signals received from controller 3425. Control of the pacing rate may be performed by controller 3425, either alone or in combination with peripheral circuits or modules, using software, hardware, firmware, or any combination of the like. The software embodiments provide flexibility in how inputs are processed and may also provide the opportunity to remotely upgrade the device software while still implanted in the patient without having to perform surgery to remove and/or replace the device.

In various embodiments, a CRM device provides cardiac pacing therapy to treat disordered breathing. The CRM device obtains intervals between successive sensed or evoked atrial beats. The CRM device computes a new first indicated pacing interval based at least in part on the duration of a cardiac interval and a previous value of the first indicated pacing interval. In various implementations, the cardiac interval duration used to compute the new first indicated pacing interval may comprise a previous cardiac interval duration or a most recent cardiac interval duration. The CRM device provides pacing therapy delivered at a rate corresponding to the inverse of the duration of the first indicated pacing interval.

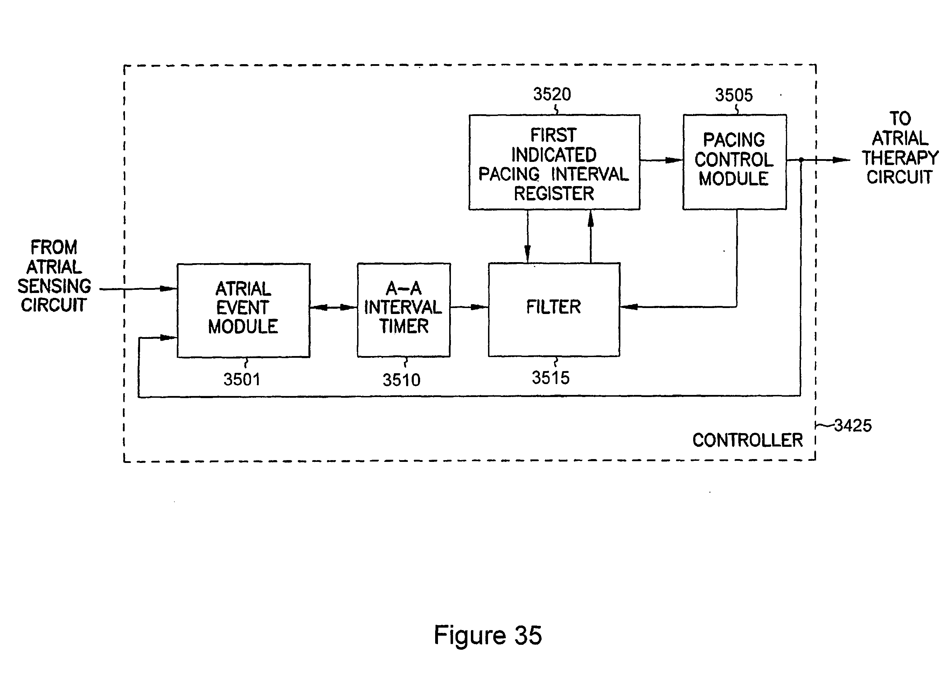

Figure 35 is a block diagram illustrating one conceptualization of portions of the controller 3425 used to effect overdrive pacing for disordered breathing therapy in accordance with embodiments of the invention. The description that follows involves atrial overdrive pacing in the AAI(R) or DDD(R) modes. It will be appreciated that similar techniques may be implemented to effect ventricular overdrive pacing in the VVI(R) mode or overdrive pacing in a biventricular mode.

At least one signal from an atrial sensing circuit is received by atrial event module 501, which recognizes the occurrence of atrial events included within the signal. Such events are also referred to as "beats," "activations," "depolarizations," "P-waves," or "contractions." Atrial event module 3501 may detect intrinsic events (also referred to as sensed events) from the signal obtained from atrial sensing circuit. Atrial event module 3501 may also detect evoked events (resulting from a pace) either from the signal obtained from atrial sensing circuit, or preferably from an atrial pacing control signal obtained from pacing control module 3505, which also triggers the delivery of a pacing stimulus by atrial therapy circuit. Thus, atrial events include both intrinsic/sensed events and evoked/paced events.

A time interval between successive atrial events, referred to as an A-A interval, is recorded by a first timer, such as A-A interval timer 3510. A filter 3515 computes a "first indicated pacing interval," i.e., one indication of a desired time interval between atrial events or, stated differently, a desired atrial heart rate. The first indicated pacing interval is also referred to as an atrial pacing preference (APP) indicated pacing interval. In various embodiments, filter 3515 includes an averager, a weighted averager, a median filter, an infinite impulse (IIR) filter, a finite impulse response (FIR) filter, or any other analog of digital signal processing circuit providing the desired signal processing described more particularly below.

In one embodiment, filter 3515 computes a new value of the first indicated pacing interval (also referred to as the APP-indicated pacing interval) based on the duration of the most recent A-A interval recorded by timer 3510 and on a previous value of the first indicated pacing interval stored in first indicated pacing interval register 3520. Register 3520 is then updated by storing the newly computed first indicated pacing interval in register 3520. Based on the first indicated pacing interval stored in register 3520, pacing control module 3505 delivers control signals to atrial therapy circuit for delivering therapy, such as pacing stimuli, at the APP-indicated atrial heart rate corresponding to the inverse of the duration of the first indicated pacing interval.

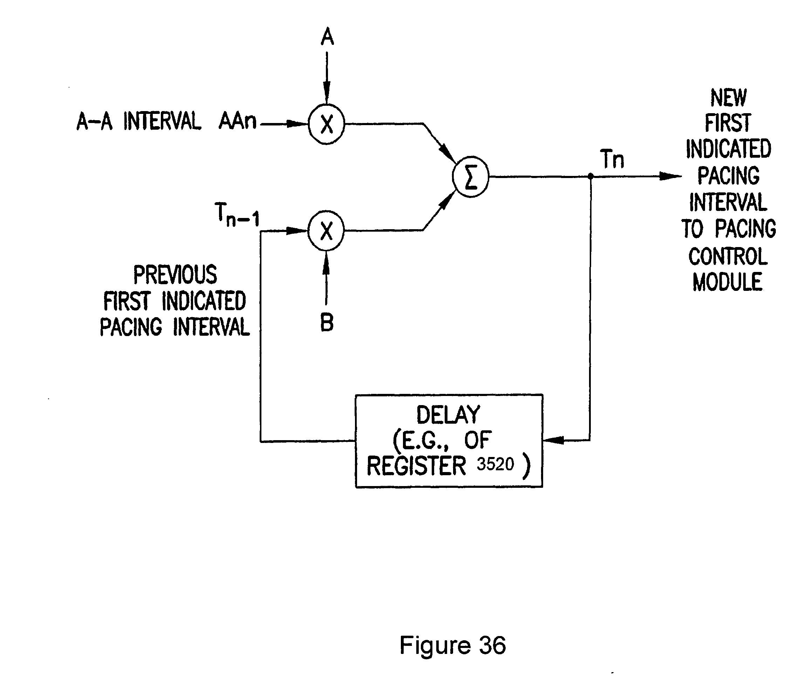

Figure 36 is a signal flow diagram illustrating one embodiment of operating filter 3515. Upon the occurrence of a sensed or evoked atrial beat, timer 3510 provides filter 3515 with the duration of the A-A interval concluded by that beat, which is referred to as the most recent A-A interval ($AA_n$). Filter 515 also receives the previous value of the first indicated pacing interval ($T_{n-1}$) stored in register 3520. The most recent A-A interval $AA_n$ and the previous value of the first indicated pacing interval $T_{n-1}$ are each scaled by respective constants A and B, and then summed to obtain a new value of the first indicated pacing interval ($T_n$), which is stored in register 3520 and provided to pacing control module 3505. In one embodiment, the coefficients A and B are different values, and are either programmable, variable, or constant.

If no atrial beat is sensed during the new first indicated pacing interval $T_n$, which is measured as the time from the occurrence of the atrial beat concluding the most recent A-A interval $AA_n$, then pacing control module 3505 instructs atrial therapy circuit to deliver an atrial pacing pulse upon the expiration of the new first indicated pacing interval $T_n$. In one embodiment, operation of the filter is described by $T_n = A \bullet AA_n + B \bullet T_{n-1}$, where A and B are coefficients (also referred to as "weights"), $AA_n$ is the most recent A-A interval duration, and $T_{n-1}$ is the previous value of the first indicated pacing interval.

From these examples, it can be seen that the first indicated pacing interval can be calculated using either a sensed or paced terminating event and using either a sensed or paced initiating event.

Initialization of filter 3515 includes seeding the filter by storing, in register 3520, an initial interval value. In one embodiment, register 3520 is initialized to an interval value corresponding to a lower rate limit (LRL), i.e., a minimum rate at which pacing pulses are delivered by device. Register 3520 could alternatively be initialized with any other suitable value.

In one embodiment, operation of filter 3515 is based on whether the beat concluding the most recent A-A interval $AA_n$ is a sensed/intrinsic beat or a paced/evoked beat. In this embodiment, the pacing control module 3505, which controls the timing and delivery of pacing pulses, provides an input to filter 3515 that indicates whether the most recent A-A interval $AA_n$ was concluded by an evoked beat initiated by a pacing stimulus delivered by CRM device, or was concluded by an intrinsic beat sensed by atrial sensing circuit.

In general terms, if the most recent A-A interval $AA_n$ is concluded by a sensed/intrinsic beat, then filter 3515 provides a new first indicated pacing interval $T_n$ that is adjusted from the value of the previous first indicated pacing interval $T_{n-1}$. For example, the new first indicated pacing interval $T_n$ may be decreased by an amount that is based at least partially on the duration of the most recent A-A interval $AA_n$ and on the duration of the previous value of the first indicated pacing interval $T_{n-1}$. If, however, the most recent A-A interval $AA_n$ is concluded by a paced/evoked beat, then filter 3515 may provide a new first indicated pacing interval $T_n$ that is increased from the value of the previous first indicated pacing interval $T_{n-1}$. For example, the new first indicated pacing interval $T_n$ may be increased by an amount that is based at least partially on the duration of the most recent A-A interval $AA_n$ and on the duration of the previous value of the first indicated pacing interval $T_{n-1}$. If no atrial beat is sensed during the new first indicated pacing interval $T_n$, measured as the time from the occurrence of the atrial beat concluding the most recent A-A interval $AA_n$, then pacing control module 3505 may instruct the atrial therapy circuit to deliver an atrial pacing pulse upon the expiration of the new first indicated pacing interval $T_n$.

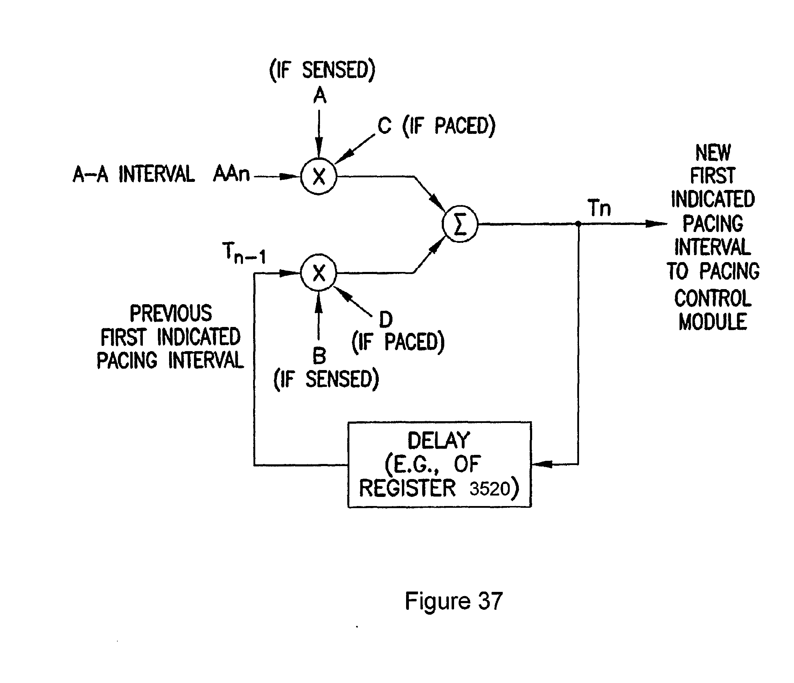

Figure 37 is a signal flow diagram illustrating another conceptualization of operating filter 3515, with certain differences from Figure 36 more particularly described below. In this embodiment, the pacing control module 3505, which controls the timing and delivery of pacing pulses, provides an input to filter 3515 that indicates whether the most recent A-A interval $AA_n$ was concluded by an evoked beat initiated by a pacing stimulus delivered by the CRM device, or was concluded by an intrinsic beat sensed by a trial sensing circuit.

If the most recent A-A interval $AA_n$ was concluded by an intrinsic beat, then the most recent A-A interval, $AA_n$, and the previous value of the first indicated pacing interval, $T_{n-1}$, are each scaled by respective constants A and B, and then summed to obtain the new value of the first indicated pacing interval $T_n$, which is stored in register 3520 and provided to pacing control module 3505. Alternatively, if the most recent A-A interval $AA_n$ was concluded by an evoked/paced beat, then the most recent A-A interval $AA_n$ and the previous value of the first indicated pacing interval $T_{n-1}$ are each scaled by respective constants C and D, and then summed to obtain the new value of the first indicated pacing interval $T_n$, which is stored in register 3520 and provided to pacing control module 3505. In one embodiment, the coefficients C and D are different from each other, and are either programmable, variable, or constant. In a further embodiment, the coefficient C is a different value from the coefficient A, and/or the coefficient D is a different value than the coefficient B, and these coefficients are either programmable, variable, or constant. In another embodiment, the coefficient D is the same value as the coefficient B.

In one embodiment, operation of filter 3515 is described by $T_n = A \bullet AA_n + B \bullet T_{n-1}$, if $AA_n$ is concluded by an intrinsic beat, and is described by $T_n = C \bullet AA_n + D \bullet T_{n-1}$, if $AA_n$ is concluded by a paced beat, where A, B, C and D are coefficients (also referred to as "weights"), $AA_n$ is the most recent A-A interval duration, $T_n$ is the new value of the first indicated pacing interval, and $T_{n-1}$ is the previous value of the first indicated pacing interval. If no atrial beat is sensed during the new first indicated pacing interval $T_n$, which is measured as the time from the occurrence of the atrial beat concluding the most recent A-A interval $AA_n$, then pacing control module 3505 instructs atrial therapy circuit to deliver an atrial pacing pulse upon the expiration of the new first indicated pacing interval $T_n$.

Another approach to operating filter 3515 is illustrated in the signal flow graph of Figure 8. In this embodiment, the coefficients A, B, C, and D can be more particularly described using an intrinsic coefficient (a), a paced coefficient (b), and a weighting coefficient (w). In one such embodiment, $A=a \cdot w$, $B=(1-w)$, $C=b \cdot w$, and $D=(1-w)$. In one example, operation of the filter 3515 is described by $T_n = a \bullet w \bullet AA_n + (1-w) \bullet T_{n-1}$, if $AA_n$ is concluded by an intrinsic beat, otherwise is described by $T_n = b \bullet w \bullet AA_n + (1-w) \bullet T_{n-1}$, if $AA_n$ is concluded by a paced beat.

If no atrial beat is sensed during the new first indicated pacing interval $T_n$, which is measured as the time from the occurrence of the atrial beat concluding the most recent A-A interval $AA_n$, then pacing control module 3505 instructs atrial therapy circuit to deliver an atrial pacing pulse upon the expiration of the new first indicated pacing interval $T_n$. In one embodiment, the coefficients a and b are different from each other, and are either programmable, variable, or constant.

The above-described parameters (e.g., A, B, C, D, a, b, w) are stated in terms of time intervals (e.g., $AA_n$, $T_n$, $T_{n-1}$). However, an alternate system may produce results in terms of rate, rather than time intervals, without departing from the present method and apparatus. In one embodiment, weighting coefficient w, intrinsic coefficient a, and paced coefficient b, are variables. Different selections of w, a, and b, will result in different operation of the present method and apparatus. For example, as w increases the weighting effect of the most recent A-A interval $AA_n$ increases and the weighting effect of the previous first indicated pacing rate $T_{n-1}$ decreases. In one embodiment, w=1/16=0.0625. In another embodiment, w=1/32. Another possible range for w is from w=1/2 to w=1/1024. A further possible range for w is from about 0 to about.1. Other values of w, which need not include division by powers of two, may be substituted without departing from the present method and apparatus.

In one embodiment, intrinsic coefficient a, is selected to be less than (or, alternatively, less than or equal to) 1.0. In one example, the intrinsic coefficient a is selected to be lesser in value than the pacing coefficient b. In one embodiment, a may be about.0.6 and b may be about 1.5. In another embodiment, a=1.0 and b=1.05. One possible range for a is from a=0.6 to a=1.0, and for b is from b=1.05 to b=1.5. The coefficients may vary without departing from the present method and apparatus.

In one embodiment, for a<1.0, filter 3515 provides a new first indicated pacing interval $T_n$ that is at least slightly shorter than the expected intrinsic A-A interval being measured by timer 3515. Thus, filter 3515 operates to promote atrial pacing by increasing the APP-indicated rate until it becomes slightly faster than the intrinsic atrial rate. The APP-indicated rate is then gradually decreased to search for the underlying intrinsic atrial heart rate. After a sensed atrial beat, the APP filter 3515 again increases the APP indicated pacing rate until it becomes faster than the intrinsic atrial rate by a small amount. As a result, most atrial heart beats are paced, rather than sensed.

The overdrive pacing as described above, or as implemented in connection with pacing one or more ventricles may be provided as therapy for disordered breathing. Additionally, such pacing therapy may be activated upon detection or prediction of disordered breathing. For example, pacing may occur at a programmed rate until a disordered breathing episode is detected. After detection of disordered breathing, the CRM device may switch to overdrive pacing to mitigate the disordered breathing.

In another example, the CRM may deliver pacing at a programmed rate until patient conditions indicate that disordered breathing is likely to occur. After disordered breathing is predicted, the CRM may deliver overdrive pacing to prevent or mitigate episodes of disordered breathing.

Although disordered breathing may occur while the patient is awake, it is most likely to occur during sleep. In another example, the CRM may be equipped with a sleep detection system. The CRM may switch from pacing at a programmed rate to overdrive pacing when the CRM detects that the patient is asleep or when the CRM detects a particular sleep state, e.g., non-REM sleep.

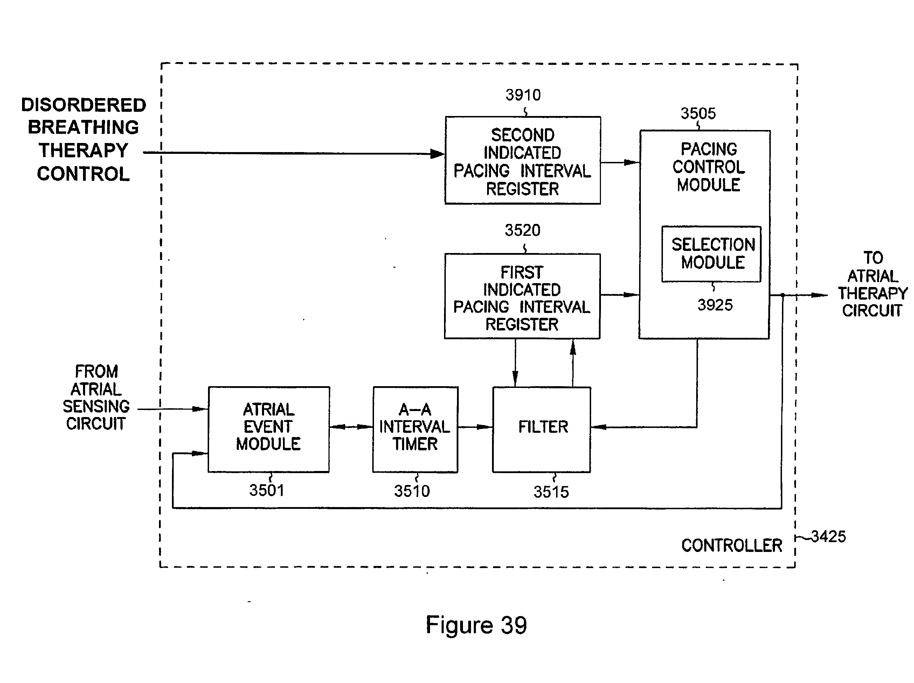

Figure 39 is a block diagram illustrating generally, by way of example, but not by way of limitation, another conceptualization of portions of controller 3425, with certain differences from Figure 35 more particularly described below. In Figure 39, controller 3425 receives from a disordered breathing therapy control circuit a control signal indicating an overdrive pacing rate for disordered breathing therapy. The signal may be based, for example, on the severity, duration, frequency or type of disordered breathing experienced by the patient, or by other factors, such as therapy interaction and/or patient comfort. In one example, the control signal may be based on a disordered breathing index, such as an apnea/hypopnea index. The disordered breathing therapy pacing rate is expressed in terms of a second indicated pacing interval stored in register 3910.

Pacing control module 3505 delivers a control signal, which directs atrial therapy circuit to deliver a pacing pulse, based on either (or both) of the first or second indicated pacing intervals, stored in registers 3520 and 3910, respectively. In one embodiment, pacing control module 3505 includes a selection module 3915 that selects between the new first indicated pacing interval $T_n$ and the second indicated pacing interval that is modulated by disordered breathing conditions.

In one embodiment, selection module 3925 selects the shorter of the first and second indicated pacing intervals as the selected indicated pacing interval $S_n$. If no atrial beat is sensed during the selected indicated pacing interval $S_n$, which is measured as the time from the occurrence of the atrial beat concluding the most recent A-A interval $AA_n$, then pacing control module 3505 instructs atrial therapy circuit to deliver an atrial pacing pulse upon the expiration of the selected indicated pacing interval $S_n$.

In general terms, for this embodiment, the atrium is paced at the higher of the disordered breathing therapy rate and the APP-indicated rate. If, for example, the patient is experiencing no disordered breathing or only mild disordered breathing, the disordered breathing therapy rate is lower than the patient's intrinsic rate, then atrial pacing pulses will be delivered at the APP-indicated rate, which is typically slightly higher than the patient's intrinsic atrial heart rate. But if, for example, the patient is experiencing more significant disordered breathing, so that the disordered breathing therapy rate is higher than the APP-indicated rate, then pacing pulses generally will be delivered at the disordered breathing therapy rate. In an alternative embodiment, the pacing rate is determined by blending the disordered breathing therapy rate and the APP-indicated rate, rather than by selecting the higher of these two indicated rates (i.e., the shorter of the first and second indicated pacing intervals). In one such example, selection module 3925 applies predetermined or other weights to the first and second indicated pacing intervals to compute the selected pacing interval $S_n$.

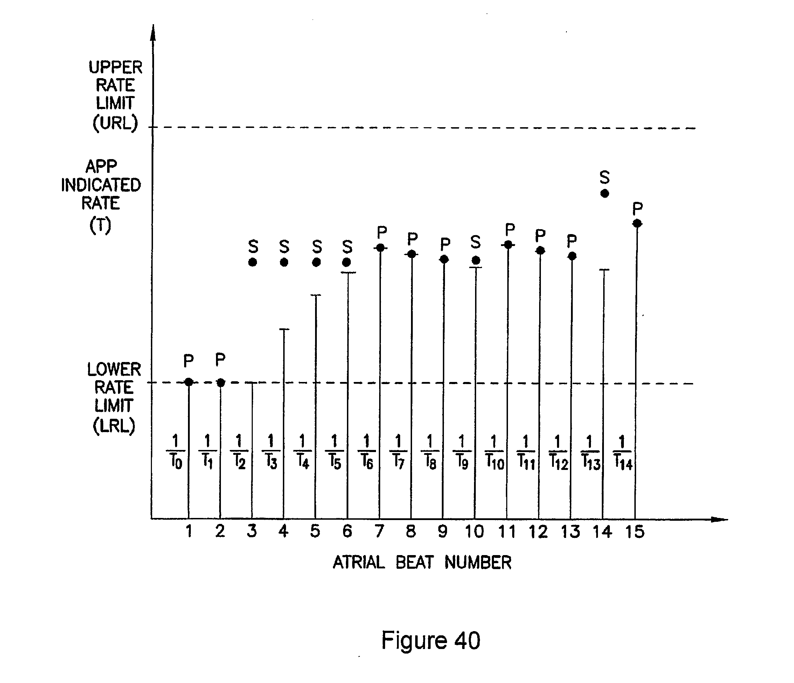

Figure 40 is a graph illustrating generally, by way of example, but not by way of limitation, one embodiment of an APP-indicated rate for successive atrial heart beats for one mode of operating filter 3515. As discussed above, the APP-indicated rate is simply the frequency, between atrial heart beats, associated with the first indicated pacing interval. Stated differently, the APP indicated rate is inversely related to the duration of the first indicated pacing interval. If pacing is based solely on the APP indicated rate, pacing control module 3505 directs atrial therapy circuit to issue a pacing pulse after the time since the last atrial beat equals or exceeds the first indicated pacing interval. However, as described above, in certain embodiments, pacing control module 3505 directs atrial therapy circuit to issue a pacing pulse based on factors other than the APP indicated rate, for example, based on the severity of disordered breathing experienced by the patient.

In the example illustrated in Figure 40, a first paced atrial beat, indicated by a "P" was issued upon expiration of the first indicated pacing interval (i.e., the APP indicated pacing interval) $T_0$, as computed based on a previous atrial beat. In one embodiment, the new APP indicated pacing interval $T_1$ is computed based on the duration of most recent A-A interval $AA_1$ and a previous value of the APP indicated pacing interval $T_0$, as discussed above. In Figure 40, the new APP indicated pacing interval $T_1$ corresponds to a lower rate limit (LRL) time interval. In one embodiment, as illustrated in Figure 40, the allowable range of the APP indicated pacing interval is limited so that the APP indicated pacing interval does not exceed the duration of the LRL time interval, and so that the APP indicated pacing interval is not shorter than the duration of an upper rate limit (URL) time interval.

In the example of Figure 40, the second atrial beat is also paced upon expiration of the APP indicated pacing interval $T_1$. In one embodiment, the new APP indicated pacing interval $T_2$ is computed based on the duration of most recent A-A interval $AA_2$ and a previous value of the APP indicated pacing interval, $T_1$, as discussed above. The first and second atrial beats are paced beats because the APP indicated atrial heart rate is higher than the underlying intrinsic atrial heart rate.

The third atrial beat is sensed well before expiration of the APP indicated pacing interval $T_2$, such that no pacing pulse is issued. For the sensed third atrial beat, filter 3515 computes the new APP indicated pacing interval $T_3$ as being shorter in duration relative to the previous APP indicated pacing interval $T_2$.

The fourth, fifth, and sixth atrial beats are sensed before expiration of the APP indicated pacing interval $T_3$, $T_4$, and $T_5$, respectively. For each of the sensed fourth, fifth, and sixth atrial beats, filter 3515 computes a new APP indicated pacing interval as being shorter in duration relative to the previous APP indicated pacing interval.

At the time of the seventh atrial beat, the APP indicated heart rate has increased above the underlying intrinsic atrial heart rate, such that the seventh atrial beat is paced upon expiration of the APP indicated pacing interval $T_6$. Because the seventh atrial beat is paced, rather than sensed, the new APP indicated pacing interval $T_7$ is computed as being longer than the previous APP indicated pacing interval $T_6$.

Similarly, the eighth and ninth atrial beats are each paced upon expiration of the corresponding APP indicated pacing interval, i.e., $T_7$, and $T_8$, respectively. Each APP indicated pacing interval $T_7$, and $T_8$ is longer than the corresponding previous APP indicated pacing interval, i.e., $T_6$, and $T_7$, respectively. In this way, the APP indicated atrial heart rate is gradually decreased to search for the underlying intrinsic atrial heart rate.

At the time of the tenth atrial beat, the APP indicated heart rate has been lowered sufficiently to allow the sensing of the tenth atrial beat. The tenth atrial beat is sensed before expiration of the APP indicated pacing interval $T_9$, such that no pacing pulse is issued. For the sensed tenth atrial beat, filter 3515 computes the new APP indicated pacing interval $T_{10}$ as being shorter in duration relative to the previous APP indicated pacing interval $T_9$.

The eleventh atrial beat is paced upon expiration of the APP indicated pacing interval $T_{10}$. For the paced eleventh atrial beat, filter 3515 computes the new APP indicated pacing interval $T_{11}$ as being longer in duration relative to the previous APP indicated pacing interval $T_{10}$. Similarly, the twelfth and thirteenth atrial beats are each paced upon expiration of the corresponding APP indicated pacing interval, i.e., $T_{11}$, and $T_{12}$, respectively. Each APP indicated pacing interval $T_{12}$, and $T_{13}$ is longer than the corresponding previous APP indicated pacing interval, i.e., $T_{11}$, and $T_{12}$, respectively. In this way, the APP indicated atrial heart rate is gradually decreased to find the underlying intrinsic atrial heart rate.

The fourteenth atrial beat is sensed before expiration of the APP indicated pacing interval $T_{13}$, such that no pacing pulse is issued. For the sensed fourteenth atrial beat, filter 3515 computes the new APP indicated pacing interval $T_{14}$ as being shorter in duration relative to the previous APP indicated pacing interval $T_{13}$.

The fifteenth atrial beat is paced upon expiration of the APP indicated pacing interval $T_{14}$. For the paced fifteenth atrial beat, filter 3515 computes the new APP indicated pacing interval $T_{15}$ as being longer in duration relative to the previous APP indicated pacing interval $T_{14}$.

The intrinsic coefficient a of filter 3515 controls the "attack slope" of the APP indicated heart rate as the APP indicated heart rate increases because of sensed intrinsic beats. The paced coefficient b of filter 3515 controls the "decay slope" of the APP indicated heart rate as the APP indicated heart rate decreases during periods of paced beats. In one embodiment, in which $a<1.0$ and $b>1.0$, decreasing the value of a further beneath 1.0 increases the attack slope such that the APP indicated rate increases faster in response to sensed intrinsic beats, while decreasing the value of b toward 1.0 decreases the decay slope such that the APP indicated rate decreases more slowly during periods of paced beats. Conversely, for $a<1.0$ and $b>1.0$, increasing the value of a toward 1.0 decreases the attack slope such that the APP indicated rate increases more slowly in response to sensed intrinsic beats, while increasing the value of b from 1.0 increases the decay slope such that the APP indicated rate decreases more quickly during periods of paced beats.

In one embodiment, for $a<1.0$ and $b>1.0$, decreasing both a and b increases the APP indicated rate such that the APP indicated rate is higher above the mean intrinsic rate. Because the APP indicated rate is higher, variability in the intrinsic heart rate is less likely to result in sensed events. On the other hand, for $a<1.0$ and $b>1.0$, increasing both a and b decreases the APP indicated rate such that it is closer to, the mean intrinsic rate. Because the APP indicated rate is closer to the mean intrinsic rate, the same degree of variability in the intrinsic heart rate is more likely to result in sensed events. Thus, by adjusting the coefficients of filter 3515, as discussed above, it is possible to obtain more intrinsic beats than paced beats for a particular degree of variability in the patient's heart rate.

In one embodiment, these coefficients are programmable by the user, such as by using remote programmer. In another embodiment, the user selects a desired performance parameter (e.g., desired degree of overdrive pacing, desired attack slope, desired decay slope, etc.) from a corresponding range of possible values, and CRM device automatically selects the appropriate combination of coefficients of filter 3515 to provide a filter setting that corresponds to the selected user-programmed performance parameter, as illustrated generally by Table 5. Other levels of programmability or different combinations of coefficients may also be used.

TABLE 5

| Example of Automatic Selection of Aspects of Filter Setting Based on a User-Programmable Performance Parameter. | | |
|---|---|---|
| User-Programmable Performance Parameter | Intrinsic Coefficient a | Paced Coefficient b |
| 1 (Less Aggressive Attack/Decay) | 1.0 | 1.05 |
| 2 | 0.9 | 1.2 |
| 3 | 0.8 | 1.3 |
| 4 | 0.7 | 1.4 |
| 5 (More Aggressive Attack/Decay) | 0.6 | 1.5 |

Figure 40 illustrates that sensed atrial beats increase the APP indicated rate by an amount that is based on the sensed atrial heart rate. Thus, for an abrupt and large increase in sensed atrial rate, the APP indicated rate will increase faster than for a slower and smaller increase in sensed atrial heart rate. However, increases in the APP indicated rate do not depend solely on the sensed atrial heart rate. Instead, such increases in the APP indicated heart rate also depend on the previous value of the APP indicated heart rate. This provides a smoothing function so that the APP indicated heart rate is not overly sensitive to a single extremely premature atrial beat, changes in the atrial rate are more gradual, and the degree of such rate changes is programmably adjustable, as described above. Moreover, in one embodiment, filter 3515 operates continuously to provide continuous rate adjustment based on the APP indicated rate.

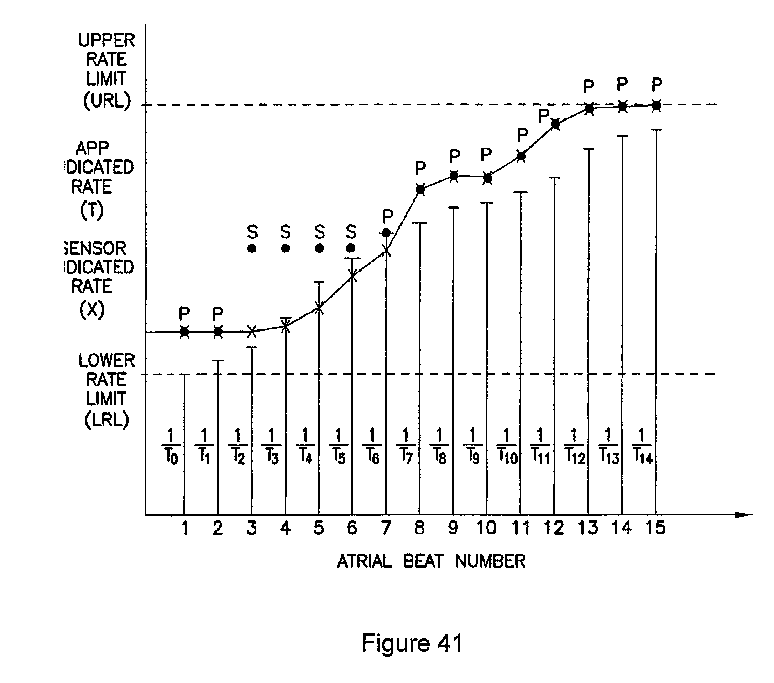

Figure 41 is a graph illustrating generally, by way of example, but not by way of limitation, one embodiment of selecting between more than one indicated pacing interval. Figure 41 is similar to Figure 40 in some respects, but Figure 41 includes a second indicated pacing interval. In one embodiment, the first indicated pacing interval is the APP indicated pacing interval, described above, and the second indicated pacing interval is a disordered breathing therapy pacing interval, based on the severity, frequency, duration, type, or other parameter of disordered breathing experienced by the patient.

In one embodiment, a selected indicated pacing interval is based on the shorter of the first and second indicated pacing intervals. Stated differently, CRM device provides pacing pulses at the higher indicated pacing rate. In the example illustrated in Figure 41, the first and second beats and the eighth through fifteenth beats are paced at the disordered breathing therapy indicated rate, because it is higher than the APP indicated atrial rate and the intrinsic (sensed) atrial rate. The third, fourth, fifth and sixth atrial beats are sensed intrinsic beats that are sensed during the shorter of either of the APP and sensor indicated pacing intervals. The seventh beat is paced at the APP indicated rate, because it is higher than the disordered breathing therapy indicated rate, and because no intrinsic beat is sensed during the APP indicated interval $T_6$. In this embodiment, the ranges of both the sensor indicated rate and the APP indicated rate are limited so that they do not extend to rates higher than the URL or to rates lower than the LRL. In one embodiment, the above-described equations for filter 3515 operate to increase the APP indicated rate toward the disordered breathing therapy indicated rate when the sensor indicated rate is greater than the APP indicated rate, as illustrated by first through third and eighth through fifteenth beats in Figure 41. In an alternate embodiment, however, $T_n = b \bullet w \bullet AA_n + (1-w) \bullet T_{n-1}$, if $AA_n$ is concluded by a APP indicated paced beat, and $T_n = T_{n-1}$ if $AA_n$ is concluded by a disordered breathing therapy indicated paced beat, thereby leaving the APP indicated rate unchanged for disordered breathing therapy indicated paced beats. In one embodiment, the LRL and the URL are programmable by the user, such as by using remote programmer.

In one embodiment, filter 3515 includes variable coefficients such as, for example, coefficients that are a function of heart rate (or its corresponding time interval). In one example, operation of the filter 3515 is described by $T_n = a \bullet w \bullet AA_n + (1-w) \bullet T_{n-1}$, if $AA_n$ is concluded by an intrinsic beat, otherwise is described by $T_n = b \bullet w \bullet AA_n + (1-w) \bullet T_{n-1}$, if $AA_n$ is concluded by a paced beat, where at least one of a and b are linear, piecewise linear, or nonlinear functions of one or more previous A-A intervals such as, for example, the most recent A-A interval, $AA_n$.

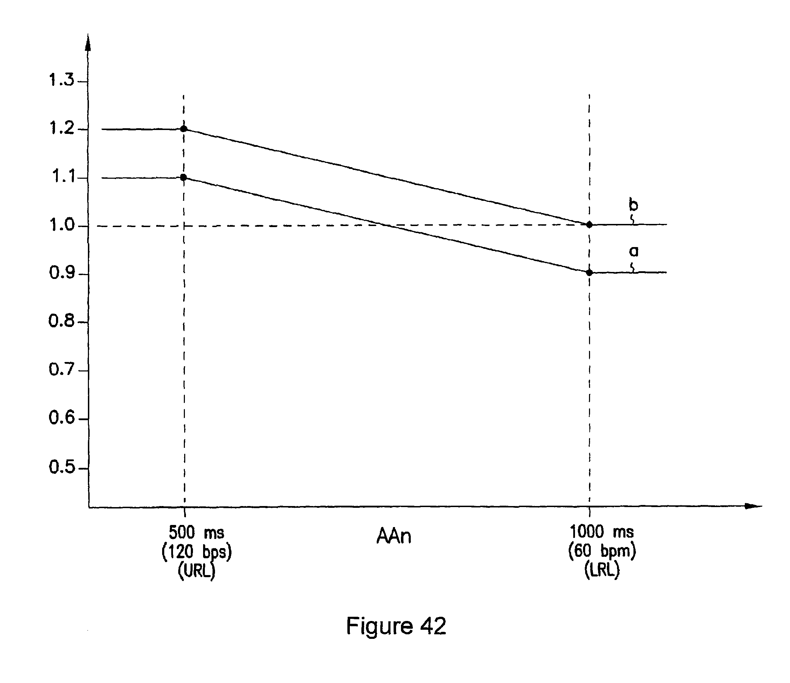

Figure 42 is a graph illustrating generally, by way of example, but not by way of limitation, one embodiment of using at least one of coefficients a and b as a function of one or more previous A-A intervals such as, for example, the most recent A-A interval, $AA_n$. In one such example, a is less than 1.0 when $AA_n$ is at or near the lower rate limit (e.g., 1000 millisecond interval or 60 beats/minute), and a is greater than 1.0 when $AA_n$ is at or near the upper rate limit (e.g., 500 millisecond interval or 120 beats/minute). For a constant b, using a smaller value of a at lower rates will increase the pacing rate more quickly for sensed events; using a larger value of a at higher rates increases the pacing rate more slowly for sensed events. In another example, b is close to 1.0 when $AA_n$ is at or near the lower rate limit, and b is greater than 1.0 when $AA_n$ is at or near the upper rate limit. For a constant a, using a smaller value of b at lower rates will decrease the pacing rate more slowly for paced events; using a larger value of b at higher rates decreases pacing rate more quickly for paced events.

The above-described system provides, among other things, a cardiac rhythm management system including an atrial pacing preference (APP) filter for promoting atrial pacing. The APP filter controls the timing of delivery of atrial pacing pulses. The atrial pacing preference pacing may be initiated upon detection or prediction of disordered breathing, for example, to provide overdrive pacing to terminate or mitigate occurrences of disordered breathing.

The atrial pacing pulses are delivered at a first indicated pacing rate, i.e., the APP-indicated rate, that is typically at a small amount above the intrinsic atrial heart rate. For sensed beats, the APP indicated pacing rate is increased until it becomes slightly faster than the intrinsic atrial heart rate. The APP-indicated pacing rate is then gradually decreased to search for the underlying intrinsic atrial heart rate. Then, after a sensed atrial beat, the APP filter again increases the APP indicated pacing rate until it becomes faster than the intrinsic atrial rate by a small amount. As a result, most atrial heart beats are paced, rather than sensed.

Although the preceding discussion contemplates providing atrial overdrive pacing for disordered breathing therapy, similar processes for providing ventricular overdrive pacing or bi-ventricular overdrive pacing may be implemented. The pacing rate may be adjusted based on characteristics of the disordered breathing experienced by the patient. For example, the overdrive pacing may be modulated by the type, severity, frequency, and/or duration of the disordered breathing.

Further, the smoothed pacing rate may be limited. For example, the pacing rate may be capped or limited before therapy is delivered. In another implementation, the intrinsic input interval may be limited to some predetermined value. The predetermined may be set by the physician or may be determined from other variables. By limiting the input intrinsic interval, the output pacing rate is limited. Limiting the smoothed pacing rate may be useful in managing atrial fibrillation or flutter, for example.

<u>Sleep Quality Monitoring</u>

In accordance with various embodiments of the invention, conditions related to sleep quality, e.g., sleep fragmentation and/or other arousal-based measures, patient-reported restful sleep, and patient-reported discomfort during therapy delivered during sleep, among other sleep quality factors, may be used to assess the impact of the therapy on the patient. Referring again to Figure 2, the therapy assessment processor 260 may assess therapy impact based on sleep quality using information acquired by a sleep quality monitor 254. For example, if a patient is receiving effective disordered breathing therapy and has low sleep fragmentation, reports restful sleep with no discomfort, the therapy effectiveness may be relatively high and the adverse effects of the therapy on the patient may be relatively low. If sleep fragmentation unrelated to disordered breathing episodes is relatively high, or if the patient reports discomfort or feeling tired after sleeping, these conditions may indicate that the disordered breathing therapy is causing sleep disturbances and/or other undesirable effects.

Because disordered breathing generally occurs during sleep, it may be particularly important to assess sleep quality during disordered breathing therapy delivery. It is undesirable to provide therapy that eliminates the disordered breathing but increases sleep fragmentation. In such a situation, the disordered breathing therapy may exacerbate the adverse effects produced by the respiratory disturbances. Thus, it may be preferable to assess the impact of the therapy on the patient and adjust the therapy to improve sleep quality. Sleep fragmentation and sleep disruptions may also occur if disordered breathing therapy is ineffective and disordered breathing occurs during sleep. Therefore, a therapy impact assessment based on detected sleep quality and/or patient-reported restful sleep may preferably take into account an assessment of therapy effectiveness.

Sleep quality assessments depend upon acquiring sleep-related data, including the patient's typical sleep patterns and the physiological, environmental, contextual, emotional, and other conditions affecting the patient during sleep. Diagnosis of sleep disorders and assessment of sleep quality often involves the use of a polysomnographic sleep study at a dedicated sleep facility. However, such studies are costly, inconvenient to the patient, and may not accurately represent the patient's typical sleep behavior. In a polysomnographic sleep study, the patient is instrumented for data acquisition and observed by trained personnel. Sleep assessment in a laboratory setting presents a number of obstacles in acquiring an accurate picture of a patient's typical sleep patterns. For example, spending a night in a sleep laboratory typically causes a patient to experience a condition known as "first night syndrome," involving disrupted sleep during the first few nights in an unfamiliar location. In addition, sleeping while instrumented and observed may not result in a realistic perspective of the patient's normal sleep patterns.

Further, polysomnographic sleep studies provide an incomplete data set for the analysis of some sleep disorders, including, for example, sleep disordered breathing. A number of physiological conditions associated with sleep disordered breathing are detectable during periods of wakefulness, e.g., decreased heart rate variability, elevated sympathetic nerve activity, norepinephrine concentration, and increased blood pressure variability. Collection of data during periods of sleep and/or during periods of wakefulness may provide a more complete picture of the patient's sleep quality.

Various aspects of sleep quality, including number and severity of arousals, sleep disordered breathing episodes, nocturnal limb movements, and cardiac, respiratory, muscle, and nervous system functioning may provide important information for diagnosis and/or therapy delivery. An initial step to sleep quality evaluation is an accurate and reliable method for discriminating between periods of sleep and periods of wakefulness. Further, acquiring data regarding the patient's sleep states or stages, including sleep onset, termination, REM, and NREM sleep states, and arousal events, including autonomic arousals may be used in connection sleep quality assessment. For example, the most restful sleep occurs during stages 3 and 4 non-REM sleep. One indicator of sleep quality is the percentage of time a patient spends in these sleep stages. Knowledge of the patient's sleep patterns may be used to diagnose sleep disorders and/or adjust patient therapy, including, e.g., cardiac or respiratory therapy. Trending disordered breathing episodes, arousal episodes, and other sleep quality aspects may be helpful in determining and maintaining appropriate therapies for patients suffering from disorders ranging from snoring to chronic heart failure.

The present invention involves methods and systems for acquiring sleep quality data and using the sleep quality data to assess the effectiveness and/or impact of disordered breathing therapy delivered to the patient. Methods of the invention involve sensing conditions associated with the sleep quality of the patient including physiological and/or non-physiological conditions. Data related to the patient's sleep quality is collected based on the sensed conditions. Sensing for conditions affecting the patient and related to sleep quality may occur during periods of wakefulness and/or during periods of sleep. Sensing the conditions associated with sleep quality and/or collecting the sleep quality data may be performed using a device having a component that is at least in part implantable.

A representative set of the conditions associated with sleep quality is listed in Table 1. Patient conditions used to evaluate sleep quality may include, for example, both physiological and non-physiological (i.e., contextual) conditions.

Each of the conditions listed in Table 1 may serve a variety of purposes in evaluating sleep quality. For example, a subset of the conditions may be used to detect whether the patient is asleep and to track the various stages of sleep and arousal incidents. Another subset of the conditions may be used to detect disordered breathing episodes. Yet another subset may be used to detect abnormal limb movements. In one implementation, some or all of the listed conditions may be collected over a relatively long period of time and used to analyze long term sleep quality trends. Trending may be used in connection with an overall assessment of sleep quality and diagnosis and treatment of sleep-disordered breathing, movement disorders, and/or other sleep disorders.

Table 6 provides examples of how some physiological and non-physiological conditions may be used in connection with sleep quality assessment.

Table 6

| Condition Type | Condition | Examples of how condition is used in sleep quality assessment |
|---|---|---|
| Physiological | Heart rate | Decrease in heart rate may indicate disordered breathing episode. Decrease in heart rate may indicate the patient is asleep. |
| | Heart rate variability | May be used to determine sleep state. Changes in heart rate variability, detected during periods of sleep or wakefulness, may indicate that the patient suffers from sleep disordered breathing. |
| | QT interval | May be used to detect sleep apnea. |
| | Ventricular filling pressure | May be used to identify/predict pulmonary congestion associated with respiratory disturbance. |
| | Blood pressure | Variation in blood pressure is associated with apnea. |
| | Snoring | Associated with a higher incidence of obstructive sleep apnea and may be used to detect disordered breathing. |
| | Respiration pattern | May be used to detect disordered breathing episodes. May be used to determine the type of disordered breathing. May be used to detect sleep. |
| | Patency of upper airway | Related to obstructive sleep apnea and may be used to detect episodes of obstructive sleep apnea. |
| | Pulmonary congestion | Associated with respiratory disturbances. |
| | Sympathetic nerve activity (SNA) | Apnea termination is associated with a spike in SNA. SNA activity may be elevated during periods of wakefulness if the patient experiences sleep disordered breathing. |

(continued)

| Condition Type | Condition | Examples of how condition is used in sleep quality assessment |
|---|---|---|
| | Electroencephalogram (EEG) | May be used to determine sleep stages, including REM and NREM sleep stages |
| | CO2 saturation | Low CO2 levels may indicate initiation of central apnea. |
| | O2 saturation | O2 desaturation occurs during severe apnea/ hypopnea episodes. |
| | Blood alcohol content | Alcohol tends to increase the incidence of snoring & obstructive apnea. |
| | Adrenalin | End of apnea associated with a spike in blood adrenaline. |
| | Brain Natriuretic Peptide (BNP) | A marker of heart failure status, which is associated with Cheyne-Stokes Respiration. |
| | C-Reactive Protein | A measure of inflammation that may be related to apnea. |
| | Drug/Medication/Tobacco use | These substances may affect incidence of both central & obstructive apnea. |
| | Muscle atonia | Muscle atonia may be used in connection with detection of REM and non-REM sleep. |
| | Eye movement | Eye movement may be used in connection with detection of REM and non-REM sleep. |
| | Activity | May be used to detect sleep and patient well being. |
| | Limb movements | May be used to detect abnormal limb movements during sleep. |

(continued)

| Condition Type | Condition | Examples of how condition is used in sleep quality assessment |
|---|---|---|
| Non-physiological | Ambient Temperature | Ambient temperature may predispose the patient to episodes of disordered breathing during sleep. |
| | Humidity | Humidity may predispose the patient to episodes of disordered breathing during sleep. |
| | Pollution | Pollution may predispose the patient to episodes of disordered breathing during sleep. |
| | Posture | Posture may be used to determine if the patient is asleep. Posture may predispose the patient to disordered breathing. |
| | Time | Used to establish historical sleep time. |
| | Ambient noise level | Noise level may affect sleep quality. |
| | Location | Patient location may used to determine if the patient is in bed as a part of sleep detection. |
| | Altitude | Altitude may predispose the patient to episodes of disordered breathing and may affect sleep quality. |
| | Barometric Pressure | Barometric pressure may predispose the patient to episodes of disordered breathing. |
| | Proximity to bed | May be used to determine if patient is in bed. |
| | Historical sleep time | May be used in connection with sleep detection. |
| | Medical history | History of medical disorders, e.g., CHF, that are associated with disordered breathing such as Cheyne-Stokes respiration. |
| | Age | Age is associated with increased risk of disordered breathing, RLS and other sleep disruptive disorders. |
| | Weight | Associated with sleep disordered breathing, e.g., obstructive sleep apnea. |
| | Gender | |
| | Obesity | |
| | Neck size | |
| | Patient reported drug, alcohol, nicotine use | Patient drug, alcohol and nicotine use may affect sleep quality. |
| | Psychological history | Psychological factors, e.g., clinical depression may be associated with insomnia. |
| | Emotional state | Emotional state, e.g., stress, anxiety, euphoria, may affect sleep quality. |
| | Daytime sleepiness Patient perceptions of sleep quality | May be used to evaluate sleep quality. |

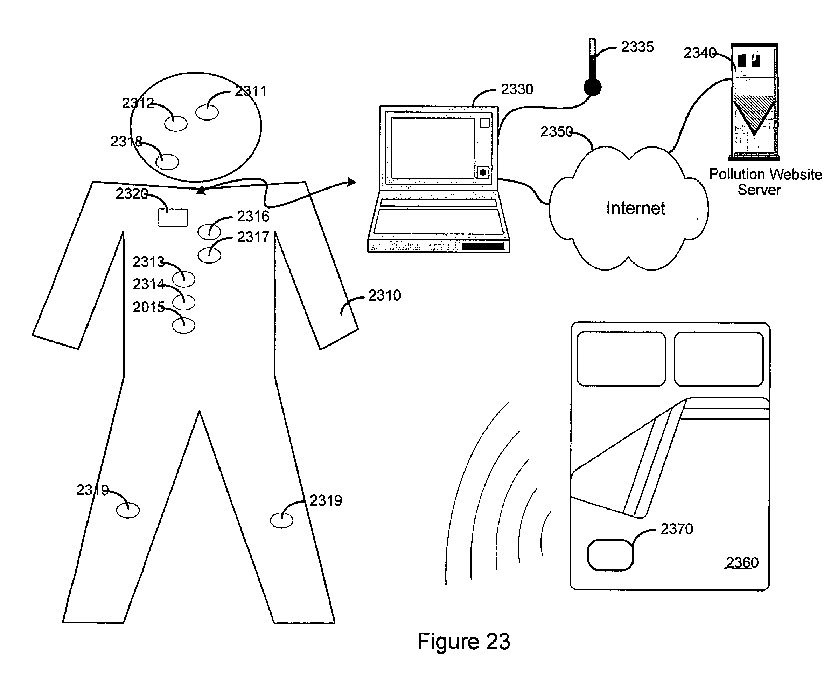

Figure 23 illustrates a patient 2310 instrumented with a sleep quality monitor according to embodiments of the invention. The sleep quality monitor 2320 collects sleep quality data from the patient using a number of sensors 2311-2319. In one configuration, the collected data is analyzed by a therapy assessment processor that may be an integrated component of an implantable disordered breathing therapy system. The collected sleep quality data may be downloaded to a patient-external device 2330 for storage, analysis, or display.

In the implementation illustrated in Figure 23, the implantable sleep quality monitor 2320 is coupled to a number of sensors 2311-2319. In this example, the sensors include an EGM sensor 2316 for detecting heart rate and heart rate variability conditions. A transthoracic impedance sensor 2317 is used to detect the respiration conditions of the patient, including, for example, minute ventilation, respiration rate, and tidal volume. An activity detector, e.g., accelerometer, 2315 may be used to detect patient activity conditions. The sleep quality monitor 2320 senses patient conditions including the patient's posture and location using a posture sensor 2314 and a proximity to bed sensor 2313, respectively. The sleep quality monitor 2320 senses the patient's brain activity using EEG sensors 2311 and the patient's eye movements using EOG sensors 2312. Jaw and limb movements are sensed using accelerometers attached to the patient's jaw 2318 and legs 2319.

In this application, the sleep quality monitor 2320 is configured to track the patient's heart rate, heart rate variability, minute ventilation, respiration rate, tidal volume, posture, proximity to bed, brain activity, eye movements, jaw movements and leg movements. At periodic intervals, the sleep quality monitor 2320 samples signals from the sensors and stores data regarding the detected conditions in memory circuitry within the sleep quality monitor 2320. The sleep quality monitor 2320 may additionally access an external input unit 2330 to detect patient reported conditions, for example, recent tobacco and medication use by the patient. Further, the sleep quality data monitor 2320 may monitor conditions using one or more external sensors. In the illustrated example, a thermometer 2335 is coupled through the external programmer 2330 and a pollution website 2340 is accessible to the sleep quality monitor 2320 through the internet 2350.

The sleep quality monitor 2320 may operate to acquire data during periods of both sleep and wakefulness. It may be beneficial, for example, to track changes in particular conditions measured during periods of wakefulness that are associated with sleep disordered breathing. For example, some patients who suffer from sleep apnea experience changes in heart rate variability, blood pressure variability, and/or sympathetic nerve activity during periods of wakefulness. Detection and analysis of the physiological changes attributable to sleep disorders and measurable during the time the patient is awake provides a more complete picture of sleep quality.

In another example, the patient's sleep quality may be evaluated by determining the patient's activity level while the patient is awake. The activity level of the patient during the day may provide important information regarding the patient's sleep quality. For example, if the patient is very inactive during periods of wakefulness, this may indicate that the patient's sleep is of inadequate quality or duration. Such information may also be used in connection with assessing the efficacy of a particular sleep disorder therapy and/or adjusting the patient's sleep disorder therapy. Patient activity information may be sensed for example, for example, using an accelerometer and/or transthoracic impedance sensor disposed within or on an implantable device. Collection of patient activity information may be performed over a period of time. Assessment of the patient activity data may indicate changes in the patient's well-being as indicated by a drop in activity level.

The sleep quality monitor 2320 may calculate one or more sleep quality metrics quantifying the patient's sleep quality. A representative set of the sleep quality metrics include, for example, sleep efficiency, sleep fragmentation, number of arousals per hour, denoted the arousal index (AI).

The sleep quality monitor 2320 may also compute one or more metrics quantifying the patient's disordered breathing, such as the apnea hypopnea index (AHI) providing the number of apneas and hypopneas per hour, and the percent time in periodic breathing (%PB).

Further, metrics associated with sleep movement disorders may also be determined by the sleep quality monitor 2320. Such metrics may include, for example, a general sleep movement disorder index (MDI) representing the number of abnormal movements arising from movement disorders such as restless leg syndrome, periodic limb movement disorder and bruxism per hour. In addition, specific indices may be calculated for each type of movement disorder, e.g., a bruxism index (BI) characterizing the number of jaw movements per hour, a RLS index (RLSI) characterizing the number of restless leg syndrome episodes per hour, and a PLM index (PLMI) characterizing the number of periodic limb movements experienced by the patient per hour.

In addition, percentage of sleep time during which the patient experiences movement disorders (%MD) may be calculated. Specific metrics relating to the percentage of time during which the patient experiences bruxism (%B), restless leg syndrome (%RLS), and periodic leg movement disorder (%PLMD) may also be determined.

Further, sleep summary metrics may be computed, either directly from the collected patient condition data, or by combining the above-listed sleep quality and sleep disorder metrics. In one embodiment, a composite sleep disordered respiration metric (SDRM) may be computed by combining the apnea hypopnea index AHI and the arousal index AI. The composite sleep disordered respiration metric (SDRM) may be computed as a linear combination of the AHI and AI as follows:

$$SDRM = c_1*AHI + c_2*AI \quad [6]$$

where $c_1$ and $c_2$ are constants chosen to balance the relative contributions of respiratory and arousal effects on sleep disturbance. The AHI may be monitored by performing disordered breathing detection based on transthoracic impedance measurements as previously described. The AI may be estimated, for example, by monitoring the patient activity, minute ventilation, and posture sensors for body motion indicating sleep termination or arousal. A more sensitive measure of arousal may be made using EEG signals. In this implementation, the constant $c_2$ may be adjusted to reflect the increased sensitivity to arousal.

In another embodiment, an undisturbed respiration sleep time (URST) or undisturbed respiration sleep efficiency (URSE) may be computed based on the amount of time the patient spends asleep in bed without respiratory disturbance.

The URST or URSE metrics may be determined using three parameters: total time in bed (TIB), total time asleep (TA), and combined sleep time duration in disturbed respiration (STDR). Time in bed may be determined by a combination of posture sensing and sensing the proximity of the patient to bed. The posture condition of the patient may determined, for example, using an implantable multiaxis accelerometer sensor.

The patient's total time in bed (TIB) may be determined using a proximity to bed sensor. The proximity to bed sensor may use a receiver in the sleep quality monitor 2320 for receiving signals transmitted from a beacon 2370 located at the patient's bed 2360. If the proximity to bed receiver detects a signal of sufficient strength from the proximity to bed beacon 2370, then the receiver detects that the patient is in bed 2360.

Total time asleep (TA) may be determined using the sleep detection method described in more detail above. The total sleep time in disturbed respiration (STDR) may be determined, for example, based on detection of sleep and disordered breathing using the sleep and disordered breathing detection methods described above.

The patient's undisturbed respiration sleep time (URST) is calculated as:

$$URST = TA - STDR \quad [7]$$

where TA = total time asleep and STDR = sleep time in disturbed breathing.

The undisturbed respiration sleep efficiency (URSE) in percent is calculated

$$URSE = 100 * URST/TIB \quad [8]$$

where URST = undisturbed respiration sleep time and TIB = total time in bed.

Similar metrics may be calculated for movement disorders generally, or for specific movement disorders, e.g., RLS, PLMD, or bruxism. For example, the composite RLS, PLMD, and bruxism metrics, RLSM, PLMDM, and BM, respectively, may be calculated using equations similar in form to equation 6 above:

$$RLSM = c_1*RLSI + c_2*AI \quad [9]$$

where RLSI = number of restless leg movement syndrome episodes per hour, AI = number of arousals per hour, and $c_1$ and $c_2$ are constants chosen to balance the relative contributions of abnormal movement and arousal effects on sleep disturbance.

$$PLMDM = c_1*PLMDI + c_2*AI \quad [10]$$

where PLMDI = number of periodic leg movement syndrome episodes per hour, AI = number of arousals per hour, and $c_1$ and $c_2$ are constants chosen to balance the relative contributions of abnormal movement and arousal effects on sleep disturbance.

$$BM = c_1 * BMI + c_2 * AI \qquad [11]$$

where BMI = number of bruxism movement episodes per hour, AI = number of arousals per hour, and $c_1$ and $c_2$ are constants chosen to balance the relative contributions of abnormal movement and arousal effects on sleep disturbance.

The patient's undisturbed movement sleep time (UMST) and undisturbed movement sleep efficiency (UMSE) may be calculated for each movement related disorder separately or in combination using equations similar in form to equations 2 and 3, above.

In addition, a composite sleep disorder index SDI quantifying the combined effect of both respiratory and movement disorders may be computed by combining the apnea hypopnea index (AHI), the movement disorder index (MDI), and the arousal index (AI).

A sleep disturbance index (SDI) may be computed as a linear combination of the AHI, and the combined disorder index $DI_C$. The combined disorder index may include both abnormal breathing and movement components. For example, the sleep disturbance index SDI is characterizable by the equation:

$$SDI = c_4 * DI_C + c_3 * AI, \qquad [12]$$

where $DI_C$ is a combined disorder index of the form:

$$DI_C = c_{41} * DI_1 + c_{42} * DI_2 \qquad [13]$$

In equation 12, $c_4$ and $c_3$ are constants chosen to balance the relative contributions of the combined disorder and arousal effects, respectively. The disorder index, $DI_C$, may be used to characterize the effects of one or more sleep disorders, including, e.g., disorders associated with disturbed respiration and/or abnormal movements. The combined disorder index may represent only one disorder index, or may be a linear combination of two or more sleep disorder indices, e.g., the apnea/hypopnea index (AHI) and the abnormal movement disorder index (MDI). The constants $c_{41}$ and $c_{42}$ may be used as weighting factors associated with particular disorder indices.

The patient's undisturbed sleep time (UST) may be calculated:

$$UST = TA - STSD \qquad [14]$$

where TA = total time asleep and STSD = sleep time spent in sleep disorders.

The undisturbed sleep efficiency (USE) in percent may be calculated:

$$USE = 100 * UST/TIB \qquad [15]$$

where UST = undisturbed sleep time and TIB = total time in bed.

Sleep quality metrics, such as those described above, or other metrics, may be acquired and analyzed using the sleep quality data collection and analysis unit 2320. Sleep quality metrics, in addition to raw or processed data based on physiological and non-physiological conditions may determined periodically, e.g., daily, and stored or transmitted to another device. Such data can be presented to the patient's health care professional on a real-time basis, or as a long-term, e.g., month long or year long, trend of daily measurements.

The health care professional may access the data during clinic visits via programmer interrogation of the implanted device, through occasional or periodic trans-telephonic device interrogations, or through an automatic or "on-demand" basis in the context of an advanced patient management system. The health care professionals may use the sleep quality indicator trends alone or in conjunction with other device-gathered or clinical data to diagnose disorders and/or adjust the patient's device or medical therapy as needed to improve the patient's quality of sleep.

Sleep Detection

Various embodiments of the invention involve the use of sleep information to adapt therapy for disordered breathing. A disordered breathing therapy system, such as the cardiac electrical therapy system illustrated in Figure 2 may include a sleep detector 256. The sleep detector may include circuitry for detecting sleep onset and offset, sleep stages, including REM and non-REM sleep stages, and arousals from sleep, including autonomic arousals.

Collecting information related to sleep involves discriminating between a state of sleep and a state of wakefulness. One method of detecting sleep involves comparing one or more detected physiological conditions to thresholds indicative of sleep. When the detected conditions are consistent with thresholds indicating sleep, then sleep onset is declared. For example, decreased patient activity is a condition associated with sleep. When the patient's activity falls below a predetermined threshold, the system declares the onset of sleep. When the patient's activity rises above the threshold, the system declares the end of sleep. In a similar manner, a number of patient conditions, such as heart rate, respiration rate, brain wave activity, etc., may be compared individually or collectively compared to thresholds or other indices to detect sleep.

A method for detecting sleep in accordance with embodiments of the invention involves adjusting a sleep threshold associated with a first patient condition using a second patient condition. The first patient condition is compared to the adjusted threshold to determine if the patient is asleep or awake.

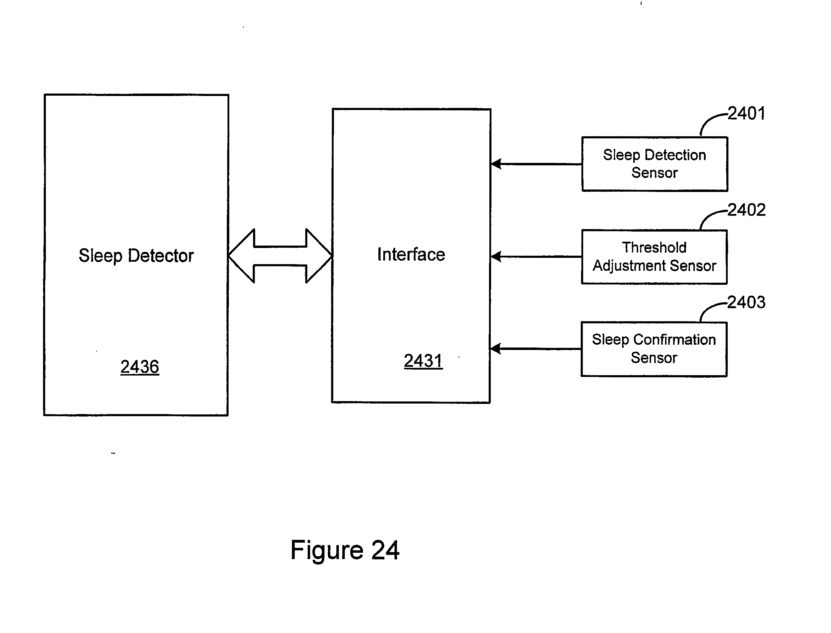

Figure 24 illustrates a sleep detector that may be used in connection with a therapy system for disordered breathing. The sleep detector 2436 uses a number of sensors 2401, 2402, 2403 to sense sleep-related patient conditions. A representative set of sleep-related conditions include, for example, patient activity, patient location, posture, heart rate, QT interval, eye movement, respiration rate, transthoracic impedance, tidal volume, minute ventilation, brain activity, muscle tone, body temperature, time of day, and blood oxygen level.

According to embodiments of the invention, a first sleep-related condition detected using a sleep detection sensor 2401 is compared to a sleep threshold for detecting the onset and termination of sleep. A second sleep-related condition, detected using a threshold adjustment sensor 2402, is used to adjust the sleep threshold. Although the example described herein involves one sleep detection sensor 2401 and one threshold adjustment sensor 2402, any number of thresholds or other indices corresponding to a number of sleep detection sensors may be used. Furthermore, conditions detected using any number of adjustment sensors may be used to adjust the thresholds or indices of a plurality of sleep detection signals. Additional sleep-related signals derived from one or more confirmation sensors 2403 may optionally be used to confirm the onset or termination of the sleep condition.

Signals derived from the sensors 2401, 2402, 2403 are received by interface circuitry 2431 that may include, for example, amplifiers, signal processing circuitry, and/or A/D conversion circuitry for processing each sensor signal. The interface circuitry 2431 may further include sensor drive circuitry required to activate the sensors 2401, 2402, 2403.

The sleep detector 2436 is configured to compare the level of a first sleep-related condition detected using the sleep detection sensor 2401 to a sleep threshold adjusted by a second sleep-related condition detected using the threshold adjustment sensor 2402. A determination of sleep onset or sleep termination may be made by the sleep detector 2436 based on the comparison. The onset or termination of sleep may optionally be confirmed using patient conditions derived using a sleep confirmation sensor 2403.

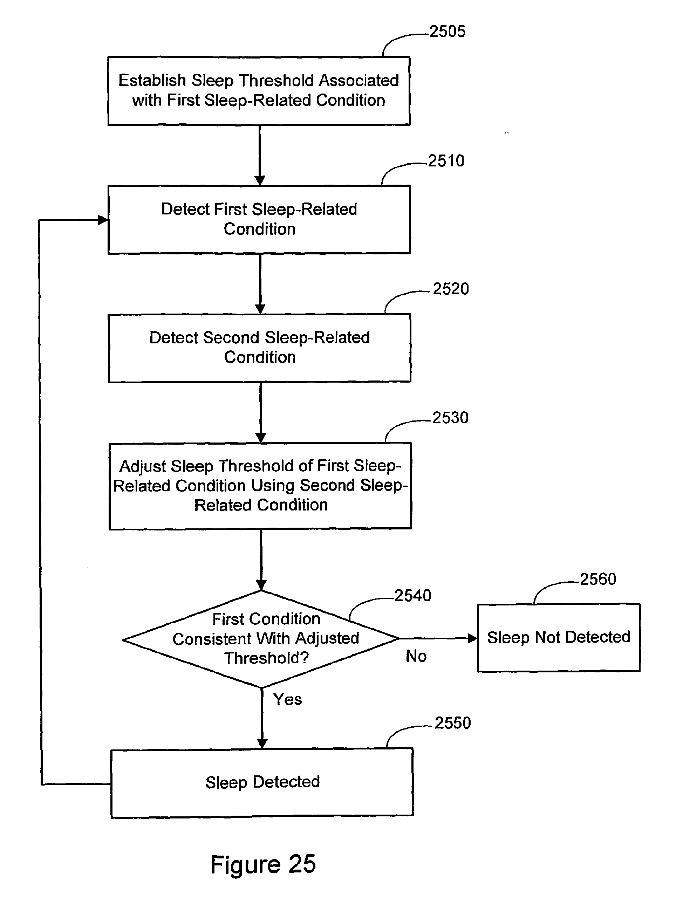

Figure 25 is a flowchart illustrating a method of detecting sleep according to embodiments of the invention. A sleep threshold associated with a first sleep-related patient condition is established 2505. The sleep threshold may be determined from clinical data of a sleep threshold acquired using a group of subjects, for example. The sleep threshold may also be determined using historical data taken from the particular patient for whom the sleep condition is to be detected.

First and second sleep-related conditions are detected 2510, 2520. The first and the second sleep-related conditions may be detected using sensors implanted in the patient, attached externally to the patient or located remote from the patient, for example, as previously described in connection with Figure 3. The first and the second sleep-related conditions may include any condition associated with sleep and are not limited to the representative sleep-related conditions listed above.

The sleep threshold established for the first sleep-related condition is adjusted using the second sleep-related condition 2530. For example, if the second sleep-related condition indicates a high level of activity that is incompatible with a sleep state, the sleep threshold of the first sleep-related condition may be adjusted downward to require sensing a decreased level of the first sleep-related condition before a sleep condition is detected.

If the first sleep-related condition is consistent with sleep according to the adjusted sleep threshold 2540, sleep is detected 2550. If the first sleep-related condition is not consistent with sleep using the adjusted sleep threshold sleep is not detected 2560. After either sleep is detected or not detected, the first and the second sleep-related conditions continue to be detected 2510, 2520 and the threshold adjusted 2530 allowing further evaluation of the sleep state.

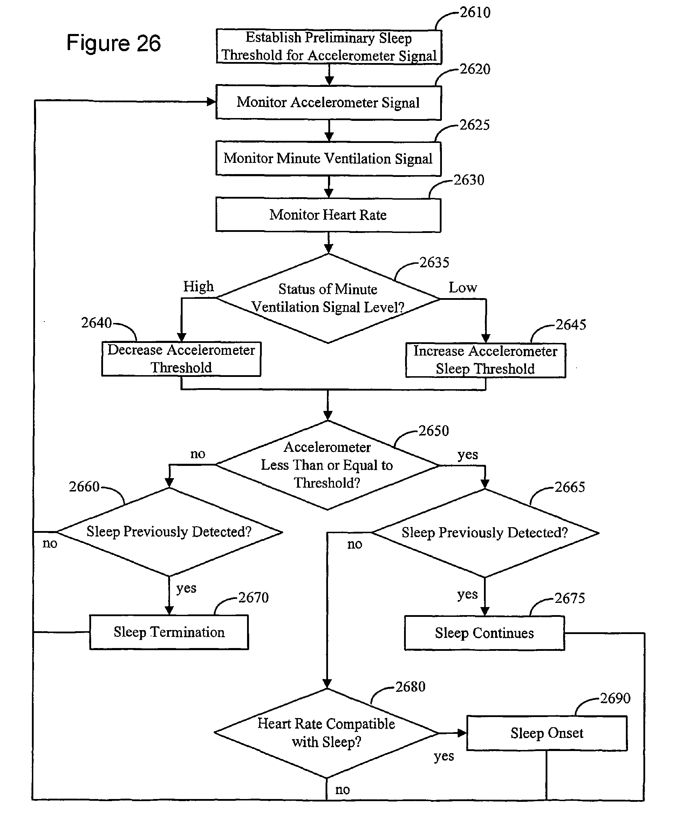

The flow chart of Figure 26 illustrates a method for detecting sleep using accelerometer and minute ventilation (MV) signals according to embodiments of the invention. In the method illustrated in Figure 26, an accelerometer and a minute ventilation sensor are used to detect patient activity and patient respiration conditions, respectively. A preliminary sleep threshold is determined 2610 with respect to the patient activity condition sensed by the accelerometer. The preliminary sleep threshold may be determined from clinical data derived from a group of subjects or from historical data taken from the patient over a period of time.

The activity condition of the patient is monitored 2620 using an accelerometer that may be incorporated in an implantable cardiac rhythm management system as described in connection with Figure 2. Alternatively, the accelerometer may be attached externally to the patient. The patient's MV condition is monitored 2625, for example, using a transthoracic impedance sensor. A transthoracic impedance sensor may be implemented as a component of an implantable CRM device.

In this embodiment, the patient's activity represents the sleep detection condition and is compared to the sleep threshold. The patient's MV is used as the threshold adjustment condition to adjust the sleep threshold. In addition, in this example, the patient's heart rate is monitored 2630 and used to provide a sleep confirmation condition.

The sleep threshold adjustment is accomplished using the patient's MV condition to adjust the activity sleep threshold. If the patient's MV condition is low relative to an expected MV level associated with sleep, the activity sleep threshold is increased. Similarly, if the patient's MV level is high relative to an expected MV level associated with sleep, the activity sleep threshold is decreased. Thus, when the patient's MV level is high, less activity is required to make the determination that the patient is sleeping. Conversely when the patient's MV level is relatively low, a higher activity level may result in detection of sleep. The use of two sleep-related conditions to determine the patient's sleep state enhances the accuracy of sleep detection over previous methods.

Various signal processing techniques may be employed to process the raw sensor signals. For example, a moving average of a plurality of samples of the sensor signals may be calculated. Furthermore, the sensor signals may be amplified, filtered, digitized or otherwise processed.

If the MV level is high 2635 relative to an expected MV level associated with sleep, the activity sleep threshold is decreased 2640. If the MV level is low 2635 relative to an expected MV level associated with sleep, the activity sleep threshold is increased 2645.

If the patient's activity level is less than or equal to the adjusted sleep threshold 2650, and if the patient is currently not in a sleep state 2665, then the patient's heart rate is checked 2680 to confirm that the patient is asleep. If the patient's heart rate is compatible with sleep 2680, then sleep onset is determined 2690. If the patient's heart rate is incompatible with sleep, then the patient's sleep-related conditions continue to be monitored.

If the patient's activity level is less than or equal to the adjusted sleep threshold 2650 and if the patient is currently in a sleep state 2665, then a continuing sleep state is determined 2675 and the patient's sleep-related conditions continue to be monitored for sleep termination to occur.

If the patient's activity level is greater than the adjusted sleep threshold 2650 and the patient is not currently in a sleep state 2660, then the patient's sleep-related conditions continue to be monitored until sleep onset is detected 2690. If the activity level is greater than the adjusted sleep threshold 2650 and the patient is currently in a sleep state 2660, then sleep termination is detected 2670.

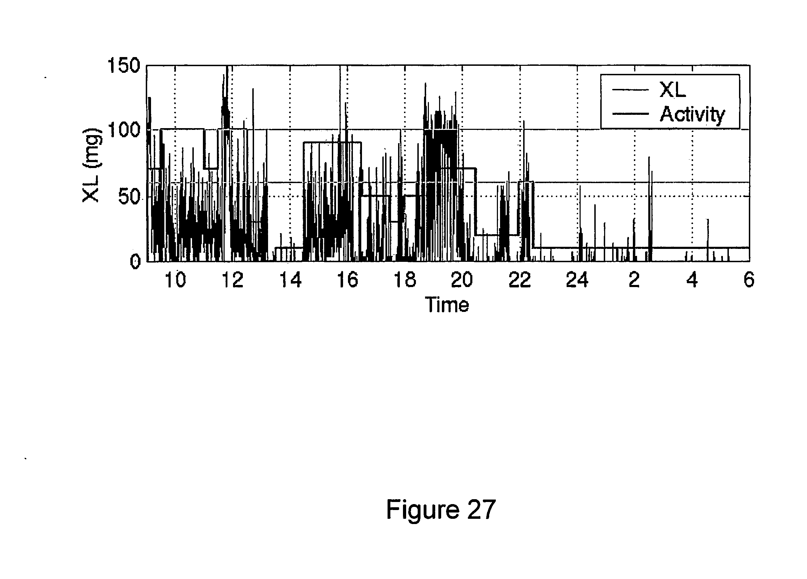

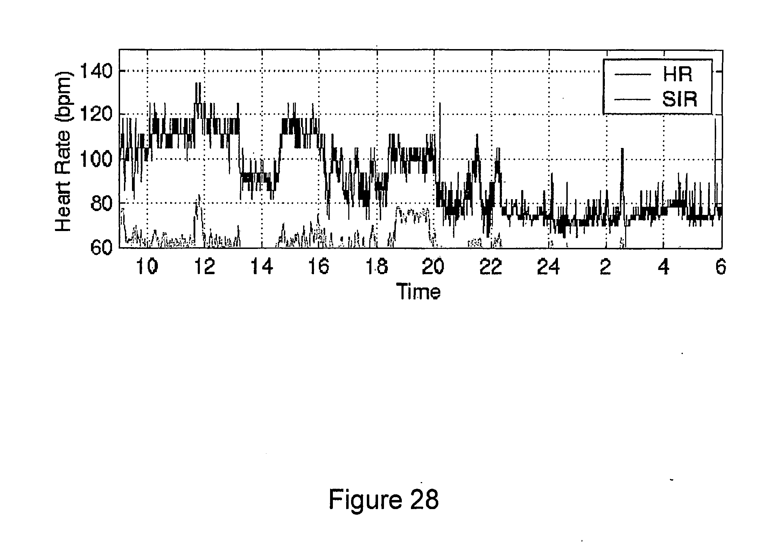

The graphs of Figures 27-30 illustrate the adjustment of the activity sleep threshold. The relationship between patient activity and the accelerometer and MV signals is trended over a period of time to determine relative signal levels associated with sleep. The graph of Figure 27 illustrates the patient's activity as indicated by an accelerometer. The patient's heart rate (HR) and sensor indicated heart rate (SIR) for the same period are shown in the graph of Figure 28. The accelerometer signal indicates a period of sleep associated with a relatively low level of activity beginning slightly before 23:00 and continuing through 6:00. The patient's heart rate appropriately tracks the activity level indicated by the accelerometer indicating a similar period of decreased heart rate corresponding to sleep. The signal level of the accelerometer during known periods of sleep may be used to establish a threshold for sleep detection.

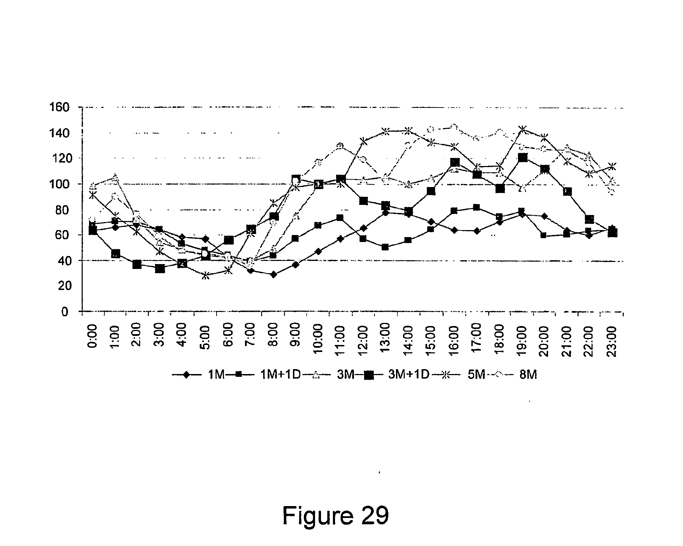

Figure 29 is a graph of the patient's minute ventilation signal over time. Historical data of averaged minute ventilation is graphed to indicate variations over a 24 hour period. MV data is shown for averages of 1 month to 8 months. The minute ventilation data may be used to determine the minute ventilation signal level associated with sleep. In this example, a composite minute ventilation graph using the historical data presents a roughly sinusoidal shape with the relatively low minute ventilation levels occurring during the period approximately from hours 21:00 through 8:00. The decreased minute ventilation level is associated with periods of sleep. The minute ventilation level associated with sleep is used to implement sleep threshold adjustment.

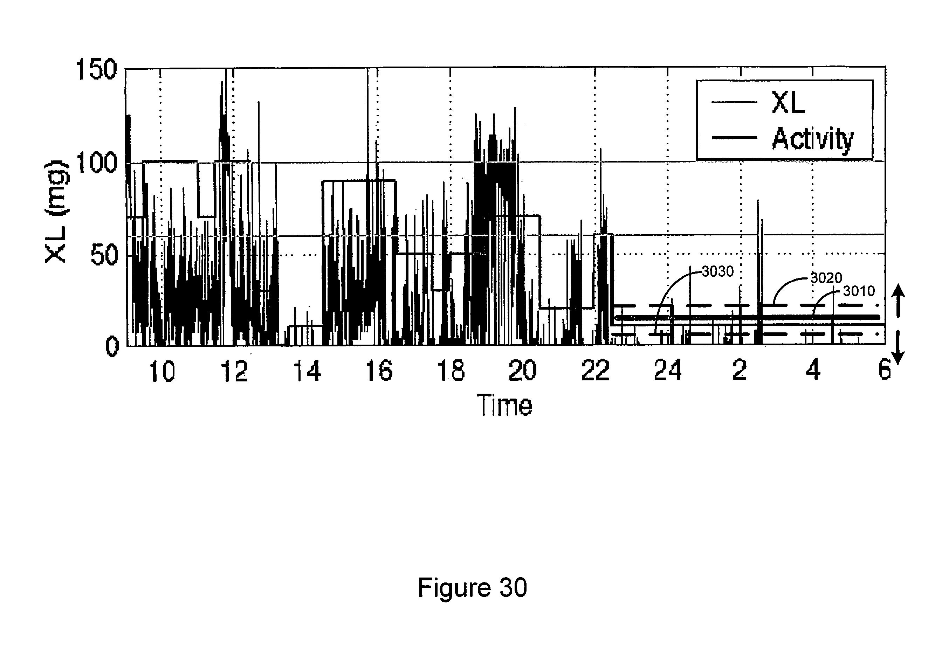

Figure 30 illustrates adjustment of the activity sleep threshold using the MV data. The initial sleep threshold 3010 is established using the baseline activity data acquired as discussed above. If the patient's MV level is low relative to an expected MV level associated with sleep, the activity sleep threshold is increased 3020. If the patient's MV level is high relative to an expected MV level associated with sleep, the activity sleep threshold is decreased 3030. When the patient's MV level is high, less activity detected by the accelerometer is required to make the determination that the patient is sleeping. However, if the patient's MV level is relatively low, a higher activity level may result in detection of sleep. The use of two sleep-related signals to establish and adjust a sleep threshold enhances the accuracy of sleep detection over previous methods.

Additional sleep-related conditions may be sensed and used to improve the sleep detection method described above. For example, a posture sensor may be used to detect the posture of the patient and used to confirm sleep. If the posture sensor signal indicates an upright posture, then the posture sensor signal may be used to override a determination of sleep using the sleep detection and threshold adjustment conditions. Other conditions may also be used in connection with sleep determination or confirmation, including the representative set of sleep-related conditions indicated above. In another example, a proximity to bed sensor may be used alone or in combination with a posture sensor to detect that the patient is in bed and is lying down.

A sleep detection system may detect sleep onset, termination, arousals as well as the sleep stages, including REM and non-REM sleep. REM sleep may be discriminated from NREM sleep, for example, by examining one or more signals indicative of REM, e.g., muscle atonia, rapid eye movements, or EEG signals. Various conditions indicative of sleep state may be detected using sensors, e.g., electroencephalogram (EEG), electrooculogram (EOG), or electromyogram (EMG) sensors, coupled through wired or wireless connections to the sleep detection circuitry. The sleep detection circuitry may analyze the various patient conditions sensed by the sensors to track the patient's sleep through various sleep states, including REM and NREM stages.

<u>Sleep Stage Detection</u>

Sleep and its various states have been linked to an increase in respiratory and cardiac disorders, particularly for patients with cardiopulmonary disorders. For example, some epidemiologic studies note a peak incidence of sudden cardiac death around 5 to 6 am. One explanation for this peak suggests an association between the incidence of sudden death and episodes of rapid eye movement (REM) sleep, morning wakening or arousal. The mechanism eliciting fatal arrhythmia may be related to the intense phasic sympathetic modulation of the cardiovascular system during the REM state or morning wakening.

Non-REM sleep may also be linked to an increased likelihood of cardiac arrhythmia. Some patients are predisposed to nocturnal cardiac paroxysms associated with surges in vagal activity. Because non-REM sleep is associated with conditions of "vagal dominance," characterized by lower heart rate and low-to-high frequency power ratios, non-REM sleep may be implicated in these nocturnal arrhythmias.

Sleep may also be associated with increased respiratory disruptions. Variations in disease, medication, etiology, and phenotype may all contribute to a patient's sleep state propensities to cardiac or respiratory disorders. Sleep stage classification may be used to provide more effective therapy, better diagnostic information, and improved prognostic and preventive therapy capabilities. Sleep stage classification in concert with therapy may result in improved therapy management for both cardiac and respiratory conditions, such as those described above. Tracking physiological changes during sleep may also provide a mechanism for improved diagnosis of sleep-related disorders.

Diagnostic testing or therapeutic device testing may be advantageously performed during sleep or during particular sleep stages. Diagnostic testing may involve, for example, assessing the patient's autonomic integrity during sleep and the possible use of REM episodes as a surrogate for stress testing. Performing diagnostic procedures during sleep recognizes opportunities afforded by sleep or particular sleep stages to routinely perturb the cardiovascular system under controlled conditions to assess the patient's autonomic response.

Therapeutic device testing, such as AVI search, capture threshold, and cardiac template acquisition, may also be performed during sleep. Sleep provides a period of time to perform such therapeutic device tests while the patient's activity is low, resulting in more effective and consistent testing conditions.

Various embodiments of the invention involve sensing a physiological condition associated with REM sleep and using the REM condition to classify the patient's sleep stages. REM-modulated conditions represent a group of physiological conditions that change during REM sleep and may be used to discern REM sleep from non-REM periods. REM sleep, as indicated by its name, is characterized by rapid bursts of eye movements, intense brain activity, and a general state of atonia, or skeletal muscle paralysis.

Various embodiments of the invention exploit the marked loss of skeletal muscle tone during REM to produce a REM-modulated signal. In this implementation, sensing a REM-modulated signal involves sensing the patient's skeletal muscle tone. Other REM-modulated signals may be used to detect REM sleep, including, for example, eye movement and brain wave activity. A representative set of sensors that may be used to sense REM-modulated signals include, for example, electroencephalogram (EEG) electrodes for detecting brain activity, electrooculogram (EOG) sensors for detecting eye movement, sensors for detecting muscle atonia, including electromyogram (EMG) sensors, strain gauge sensors, piezoelectric sensors, mechanical force sensor, or other transducers.

Sensing a condition associated with REM sleep may be used to discern REM sleep periods from non-REM periods. Sleep stage classification may be further enhanced by detecting a condition associated with a sleep-wake status of the patient, the condition associated with the sleep-wake status indicating whether the patient is asleep or awake.

According to embodiments of the invention, a sleep stage classification approach involves sensing sleep-related conditions, including at least one condition modulated by the sleep-wake status of the patient and a REM-modulated condition. The condition modulated by the sleep-wake status of the patient represents a condition that may be used to discriminate between periods of sleep and periods of wakefulness or arousal. Discriminating between periods of sleep and periods of wakefulness may be accomplished, for example, by sensing patient activity. According to this approach, if the patient's activity level is relatively low, e.g., below a sleep threshold, then the patient is determined to be asleep. The level of patient activity may be detected using an accelerometer, heart rate sensor, respiratory minute ventilation (MV) sensor or other types of sensors, for example.

Information derived from the REM-modulated condition may be used in combination with information related to the patient's sleep-wake status. This technique may be used to determine, for example, sleep onset and sleep offset, the duration and degree of arousals from sleep, and to classify sleep stages including REM and non-REM states.

In accordance with embodiments of the invention, a sleep stage classification processor receives the outputs of the one or more sensors configured to sense signals associated with the sleep-related conditions. The sleep stage processor may perform sleep stage classification on a real-time basis, or may process previously acquired and stored sensor data in a batch mode to retrospectively classify the sleep stages of one or more sleep periods.

Sleep stage classification may involve an adaptive approach, wherein the sleep stage processor learns the physiological responses of a patient in various sleep stages. The learned responses may be used to enhance the accuracy and/or sensitivity of the sleep stage classification. Adaptive sleep stage classification may involve monitoring the changes in one or more physiological signals over a period of time and adjusting thresholds used for determining sleep onset, sleep offset, and various sleep stages to accommodate the drift or other changes in the sleep-related signals.

In one configuration, one or more of the sensors used to detect the sleep-related conditions, e.g., the REM-modulated condition and/or the condition associated with the patient's sleep-wake status, may be implantable, or may utilize an implantable component. In another configuration, the sleep stage processor may be partially or fully implantable. In other configurations, both the sensors and the sleep stage processor may be implantable or use implantable components.

As previously discussed, sleep stage classification may be useful in coordinating sleep stage informed therapy delivery to treat various disorders and to perform sleep stage informed testing and monitoring. In one example implementation, cardiac therapy may be triggered during particular sleep stages to reduce the likelihood of cardiac arrhythmia during vulnerable sleep periods. In a similar manner, sleep stage classification may be used to trigger disordered breathing therapy to preclude or reduce episodes of sleep-disordered breathing.

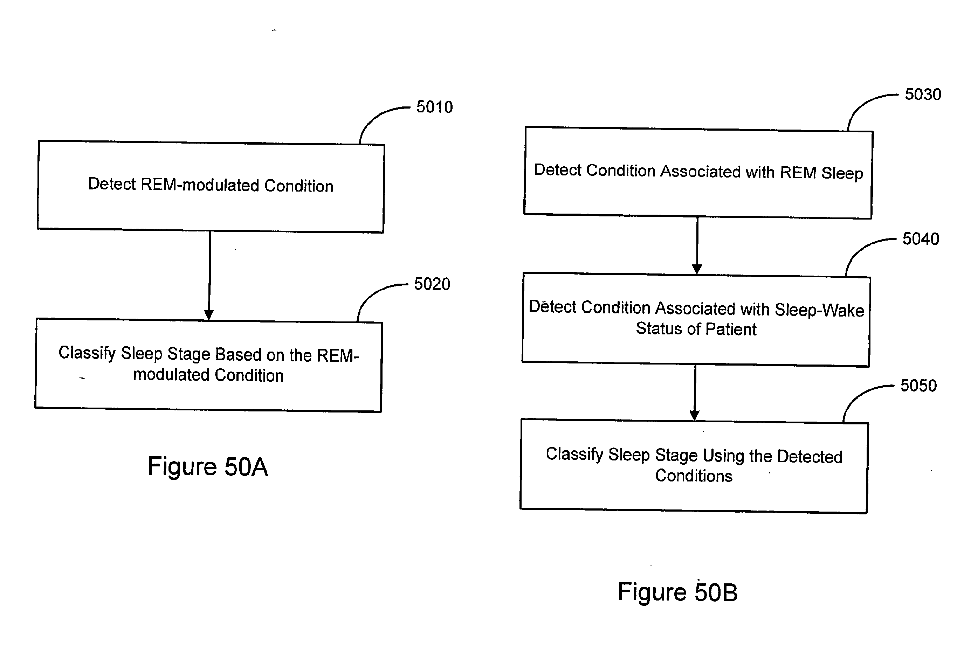

The flow graph of Figure 50A depicts a method of classifying sleep stages according to embodiments of the invention. A REM-modulated condition e.g., brain activity, eye movement, and/or muscle atonia, is detected 5010 and is used to classify 5020 the patient's sleep stage. Using the detected REM-modulated condition, the system may determine that the patient is in a REM sleep stage or in a non-REM period, for example.

Another method for classification of sleep stages in accordance with embodiments of the invention is illustrated in the flow graph of Figure 50B. The method involves detecting sleep-related conditions including at least one REM-modulated condition 5030 and at least one condition 5040 associated with a sleep-wake status of the patient. Sleep stage classification is performed 5050 based on the detected conditions.

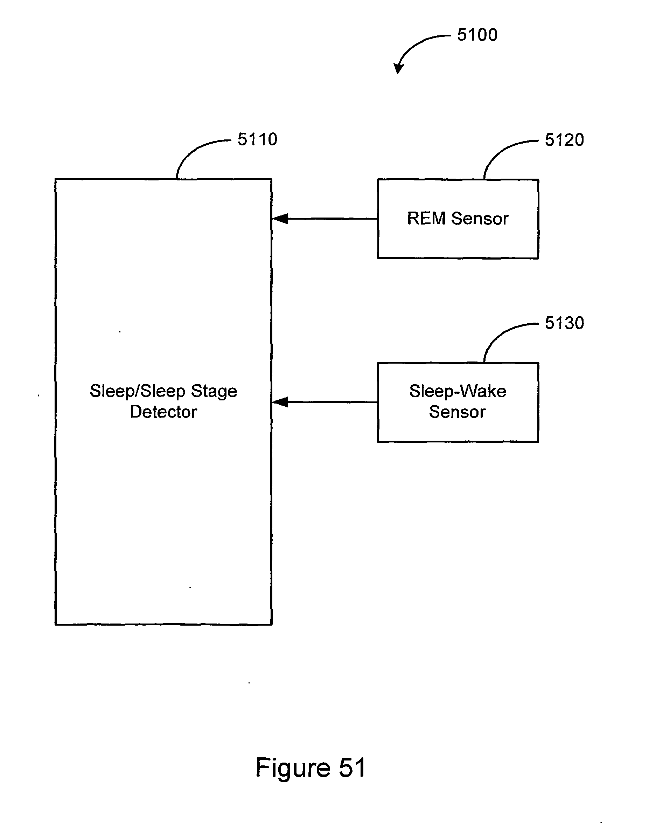

A block diagram of a system 5100 suitable for implementing a sleep stage classification method according to embodiments of the invention is illustrated in Figure 51. The sleep stage classification system 5100 may include one or more sensors 5130 used to sense a physiological signal associated with the sleep-wake status of the patient. In one example implementation, the sleep-wake sensor 5130 may be responsive to patient activity. When the patient's activity falls below a threshold, the patient is considered to be asleep. When the patient's activity rises above the activity threshold, the patient is considered to be awake. Other methods of detecting whether the patient is asleep or awake are also possible.

The system further includes a REM sensor 5120 sensitive to a REM-modulated physiological condition. REM sleep detection may be implemented by comparing the output of a skeletal muscle tone sensor to a threshold, for example. When the REM sensor output indicates loss of muscle tone consistent with a REM sleep threshold, the patient is determined to be in REM sleep. The sleep-wake sensor 5130 and the REM sensor 5120 may optionally be used in cooperation with additional sensors employed to detect additional sleep-related conditions. The additional sleep-related conditions may be used to augment the accuracy, sensitivity, or other functional capabilities, of the sleep stage classification system 5100. For example, a patient posture or torso orientation sensor may be used in combination with a patient activity sensor to provide enhanced detection of the sleep-wake status of the patient. If the patient's activity is low, as indicated by the output of a patient activity sensor, and the patient is lying down, as indicated by the output of a torso orientation sensor, then the combination of the two conditions may allow for more accurate sleep onset detection.

The REM sensor 5120, the sleep-wake status sensor 5130, and any additional sensors are coupled to a sleep/sleep stage detector 5110 that detects and processes the sensor outputs. The sleep/sleep stage detector 5110 may use outputs from the sleep-wake sensor 5130 and the REM sensor 5120 to determine if the patient is awake or asleep, to determine the duration and degree of arousals from sleep, to classify sleep stages including REM and non-REM states, and to determine the duration of various sleep stages, for example.

In one embodiment, one or both the REM sensor 5120 and the sleep-wake sensor 5130 are positioned external to the patient and the sleep/sleep stage detector 5110 is implantable or includes an implantable component. In another embodiment, one or both the REM sensor 5120 and the sleep-wake sensor 5130 are fully or partially implantable and the sleep/sleep stage detector 5110 is positioned externally to the patient. In yet another embodiment, the REM sensor 5120, sleep-wake sensor 5130, and the sleep/sleep stage detector 5110 all include implantable components or are fully implantable.

Components of the sleep stage classification system 5100 may employ wireless communications. For examples, the REM sensor 5120 and sleep-wake sensor 5130 may be coupled to the sleep/sleep stage detector 5110 using a wireless communications link. In one example, some or all of the sensors 5120, 5130 use remote communication capabilities, such as a wireless proprietary or a wireless Bluetooth communications link.

The sleep/sleep stage detector 5110 may adaptively classify sleep stages by learning patient responses in connection with various sleep stages. In one example, the sleep/sleep stage detector 5110 may perform sleep stage classification by comparing sensor signal levels to predetermined thresholds. Initial thresholds may be established using clinical data derived from a group of individuals, or using patient-specific data. After initial thresholds have been established, the sleep/sleep stage detector 5110 may update the thresholds to provide more sensitive and/or more accurate sleep stage classification based on data acquired from the patient over time. A sleep stage threshold may be updated by a recent history of the sensor output level associated with a particular sleep stage. This process may involve collecting data over time to determine the sleep patterns of the patient and adjusting the thresholds based on the sleep patterns. By this process, initially established thresholds, e.g., sleep onset threshold for an accelerometer output, or REM sleep threshold for an EMG sensor output, may be modified as additional data is acquired from the patient regarding the relationship between the sensor output levels and patient's sleep stage.

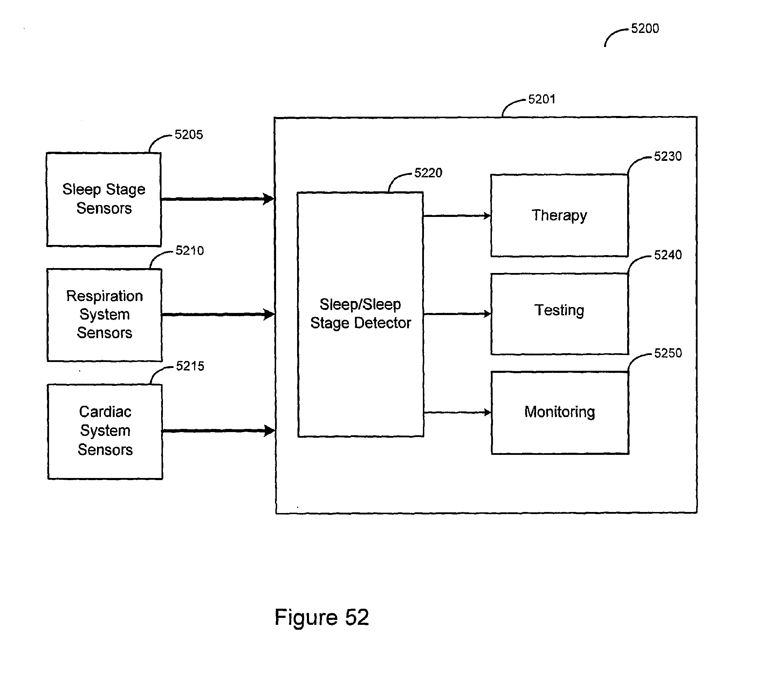

Figure 52 presents a block diagram illustrating sleep stage discrimination circuitry 5200 utilizing a sleep stage classification system implemented in accordance with embodiments of the invention. Sleep stage discrimination circuitry 5200 may be employed, for example, to perform sleep stage informed diagnostic monitoring and/or diagnostic testing to assess the capabilities of the patient's physiological systems. Such diagnostic monitoring or testing may involve one or more physiological systems, including, for example, the cardiac and respiratory systems. Additionally, or alternatively, the sleep stage discrimination circuitry 5200 may be used to provide sleep stage informed therapy to a patient, for example, cardiac rhythm therapy, respiratory therapy, or other types of therapy enhanced by sleep stage classification. Further, the sleep stage discrimination circuitry 5200 may be used to perform sleep stage informed therapeutic device testing. Uses of the sleep stage discrimination circuitry 5200 may be purely or predominantly diagnostic, purely or predominantly therapeutic, or may include a combination of therapeutic and diagnostic operations.

The sleep stage discrimination circuitry 5200 includes a medical device 5201 coupled to a variety of sensors 5205, 5210, 5215. The sensors 5205, 5210, 5215 provide physiological information used in connection with sleep stage classification and the therapeutic and/or diagnostic operations performed by the medical device 5201. The sleep stage sensors 5205 include a sensor capable of detecting a REM-modulated condition, such as skeletal muscle atonia. Additional sleep stage sensors, including one or more sensors indicative of the sleep-wake status of the patient, e.g., a patient activity sensor, may also be used.

The medical device 5201 may also be coupled to sensors 5210, 5215 configured to detect one or more aspects of the patient's physiological systems, including, for example, the cardiac and/or respiratory functions of a patient. In various configurations, the medical system 5200 may monitor, test, or provide therapy to the patient, including cardiac and/or respiratory therapy. In one implementation, cardiac sensors 5215, e.g., cardiac electrodes, may be used to sense the electrical activity of the heart. The cardiac system sensors may comprise patient-internal or patient-external cardiac electrodes electrically coupled to the patient's heart tissue, for example.

The medical device 5201 may be coupled to one or more respiratory system sensors 5210 capable of detecting conditions associated with the respiratory functions of the patient. In one embodiment, the respiratory functions of the patient may be monitored using a transthoracic impedance sensor. Transthoracic impedance tracks the patient's respiratory effort, increasing upon respiratory inspiration and decreasing upon respiratory expiration. The transthoracic impedance signal may be used to determine the patient's respiration tidal volume (TV), minute ventilation (MV), and/or other respiratory parameters, for example. Sensors other than, or in addition to, the cardiac and respiration system sensors described herein may be used to detect cardiac and/or respiration functions of the patient.

The sleep/sleep stage detector 5220 uses information from the sleep stage sensors 5205 to determine the states of the patient's sleep, including, for example, sleep onset, termination, REM and non-REM states. Information generated by the sleep/sleep stage detector 5220 may be used by other components of the medical device 5201 to provide therapy, testing, and/or monitoring coordinated with the patient's sleep stage.

Sleep stage information may be provided to a therapy module 5230 coupled to the sleep/sleep stage detector 5220. The therapy module 5230 controls the delivery of sleep stage informed therapy to the patient. For example, cardiac therapy may be coordinated using sleep stage classification information to provide cardiac arrhythmia therapy during REM or other proarrhythmic sleep periods. Sleep stage classification may also be used, for example, in connection with delivery of sleep informed therapy to preclude or reduce episodes of disordered breathing while the patient is asleep. Other types of therapy may also be enhanced using sleep stage classification.

The sleep/sleep stage detector 5220 may be coupled to a monitoring unit 5250 configured to collect and store historical data acquired from the sleep stage sensors 5205, respiratory system sensors 5210, the cardiac system sensors 5215, and/or other components of the medical device 5201. The monitoring unit 5250 may track one or more patient conditions and provide data used in the analysis of various physiological processes. The monitoring module 5250 may collect data useful in assessing trends of various physiological systems. Trending data may be used in combination with sleep stage classification to identify gradual changes in the patient's physiological conditions, especially those altered by sleep, or by particular sleep stages.

A testing module 5240 may be implemented within the medical device 5201 to control diagnostic tests and/or to control device testing to maintain or improve the operation of the medical device 5201. Information from the sleep/ sleep stage detector 5220 is used by the testing module 5240 to ensure that diagnostic and/or device testing appropriately coincides with a sleep or waking state of the patient, or to a particular state of sleep.

Diagnostic testing may be employed to investigate the functioning of one or more of the patient's physiological systems. Diagnostic testing may include changing one or more parameters of the patient's therapy, e.g., cardiac rhythm therapy, and assessing the impact of the change on the patient. For example, the patient's therapy regime may be altered during sleep, or during a particular sleep stage, to determine the effect of the change on the patient.

A diagnostic testing methodology may use sleep stage classification to determine the general behavior of the patient's physiological responses in connection with various sleep stages. Such a process may involve determining the patient's intrinsic responses to normal variations in physiologic processes. In addition, the patient's evoked physiological responses to device-based stimuli may also be determined.

In one implementation of sleep coordinated diagnostic testing, non-REM sleep may present an opportunity to perform automatic or physician activated diagnostic testing under relatively controlled circumstances. The medical device 5201 may perform diagnostic testing during non-REM sleep when the patient's activity is low. In one configuration, the medical device 5201 may modify or implement a particular cardiac pacing regimen during a non-REM period to determine the effect of such modification on the patient's cardiac system.

In addition to diagnostic testing, various device testing procedures may preferably be conducted while the patient's activity is low, such as during non-REM sleep. For example, a medical device 5201 providing cardiac rhythm management therapy may perform device testing to improve or modify a pacing regimen during the non-REM sleep state. In one implementation, a pacemaker may perform tests during non-REM sleep to optimize a pacing escape interval, such as the AV delay of a dual chamber or bi-ventricular device. In another example, a pacemaker may adjust pacing energy levels based on a capture threshold test performed during non-REM sleep. In yet another embodiment, a cardiac rhythm management system may use non-REM sleep as an opportune period of low patient activity to acquire or update cardiac waveform morphological templates used to identify various cardiac arrhythmias.

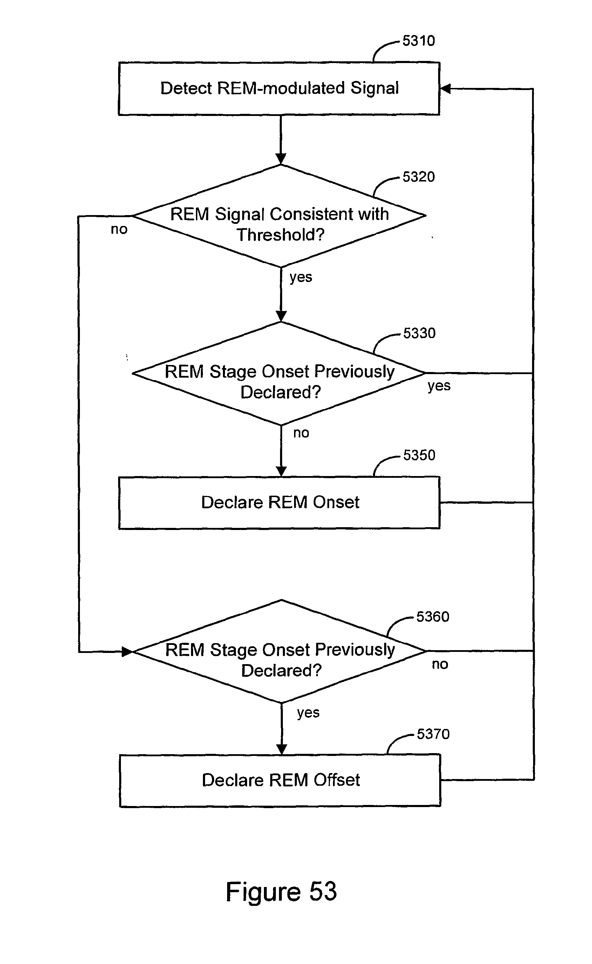

The flow graph of Figure 53 illustrates a method of performing sleep stage classification in accordance with embodiments of the invention. The method involves detecting 5310 at least one REM-modulated signal, e.g., a signal modulated by muscle atonia. If the REM-modulated signal is consistent 5320 with a predetermined REM sleep threshold, then the system determines if REM sleep onset has previously been declared 5330. If REM sleep onset was not previously declared 5330, then the system declares REM sleep onset 5350.

If the REM-modulated signal is not consistent 5320 with the predetermined REM sleep threshold and REM sleep onset was previously declared 5360, then REM sleep offset is declared 5370. If the REM-modulated signal is not consistent 5320 with the REM sleep threshold and REM onset was not previously declared 5360, the system continues to sense 5310 the REM-modulated signal to detect REM sleep onset.

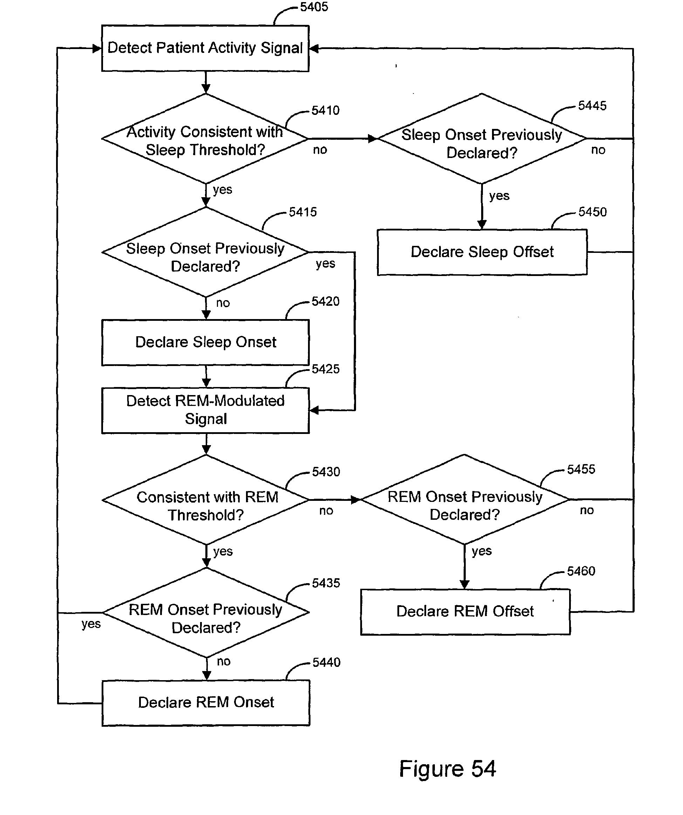

The flow graph of Figure 54 illustrates a method of using a sleep-wake condition in combination with a REM-modulated condition to classify sleep stages according to embodiments of the invention. According to this implementation, the system determines sleep onset and sleep offset by comparing a patient activity signal to a threshold. Various methods of sleep onset and sleep offset detection, for example, the methodologies described herein may be used in connection with the sleep stage classification approaches of the present invention.

The method illustrated in Figure 54 involves determining REM sleep onset and offset using a REM-modulated signal. REM sleep periods may be classified as intervals between REM onset and offset. Non-REM sleep periods may be classified as intervals between sleep onset and offset that are not classified as REM sleep.

A signal related to the activity level of the patient, e.g., accelerometer signal, is detected 5405 and compared 5410 to a predetermined sleep threshold. If the patient's activity level is consistent 5410 with the sleep threshold, and if sleep onset was previously declared 5415, the system detects 5425 a REM-modulated signal. If the patient's activity level is not consistent 5410 with a sleep threshold and if sleep onset was previously declared 5445, then sleep offset is declared 5450. If the patient's activity level is consistent 5410 with the sleep threshold and if sleep onset was not previously declared 5415, the system declares sleep onset 5420 and senses 5425 the REM-modulated signal.

If the REM-modulated signal level is consistent 5430 with a REM sleep threshold, and REM sleep onset 5435 was not previously declared, then REM sleep onset is declared 5440. If the REM-modulated signal level is not consistent 5430 with the REM sleep threshold and REM onset was previously declared 5455, then REM sleep offset is declared 5460.

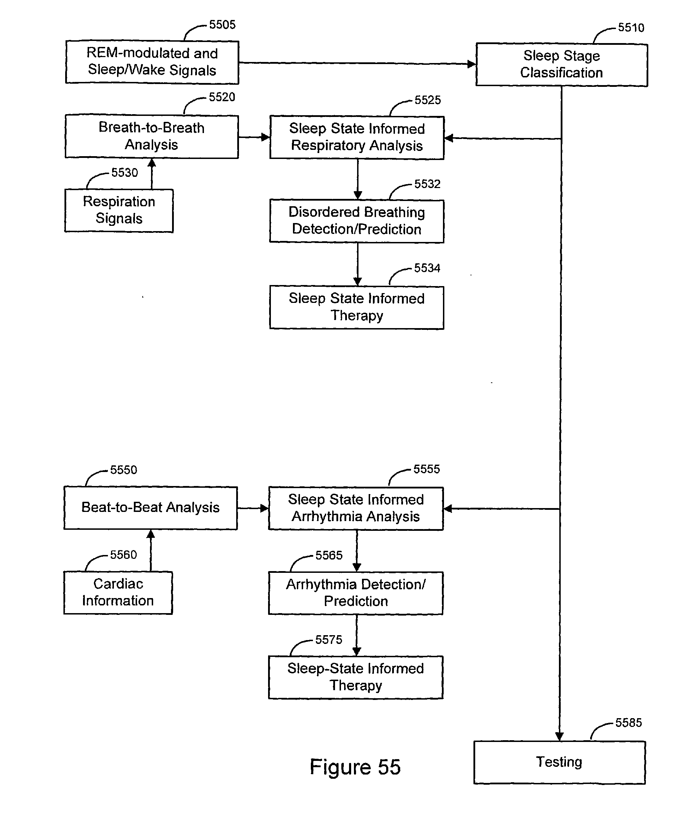

Using the method illustrated in Figure 54, sleep onset, offset, REM, and non-REM sleep may be detected. Periods of REM and/or non-REM sleep may be advantageously used in connection with a number of diagnostic and therapeutic operations, as previously discussed. Figure 55 is a process flow diagram illustrating a process for using sleep stage classification in cooperation with therapy delivery and testing in accordance with embodiments of the invention.

As presented in the process flow diagram of Figure 55, the system detects 5560 cardiac signals and analyzes 5550 the cardiac signals on a beat-to-beat basis. Beat-to-beat cardiac signal analysis 5550 may be used to perform arrhythmia detection 5565 based on rate and/or morphological analysis techniques, for example. Depending on the type of arrhythmia detected, if any, an appropriate therapy 5575 may be delivered to the heart. In one implementation, bradycardia pacing therapy may be delivered to the heart to maintain the patient's rhythm at a hemodynamically sufficient rate. In other examples, a variety of tiered tachyarrhythmia therapies, including, for example, anti-tachycardia pacing, cardioversion, and/or defibrillation may be available to treat detected cardiac tachyarrhythmias.

The illustrative system utilizes REM-modulated and sleep/wake condition signals 5505 for sleep stage classification 5510. Sleep stage classification 5510 may be used in cooperation with the beat-to-beat cardiac signal analysis 5550 to implement sleep stage informed arrhythmia analysis 5555, thus augmenting the delivery of cardiac arrhythmia therapy 5575. In one example, bradycardia pacing therapy may be enhanced by the ability to switch to a lower pacing rate when the patient is determined to be asleep. Such a procedure may be advantageous, for example, both to increase the device lifetime and to reduce stress on the heart. In a further example, preventive arrhythmia therapy 5575 may be delivered during sleep or based on prediction of future arrhythmic events, e.g., upon detection of a proarrhythmic sleep stage 5565. In one example, preventive arrhythmia therapy may be delivered to prevent tachyarrhymias known to occur more frequently during REM sleep or during arousal from sleep.

Sleep stage classification may also be used in connection with therapy to terminate or prevent sleep-disordered breathing. Various therapies may be implemented to treat sleep-disordered breathing, including maintaining continuous positive air pressure to prevent collapse of tissue into the respiratory passage, electrical stimulation of nerves or muscles, and cardiac pacing therapy, for example. Because disordered breathing is more likely to occur when the patient is asleep, disordered breathing detection or prediction 5532 may be augmented by employing sleep stage informed respiratory analysis 5525 in accordance with embodiments of the present invention.

Detection of disordered breathing may be accomplished by detecting 5530 respiration signals representing the patient's breathing cycles and analyzing each breath 5520. In one implementation, disordered breathing, including, e.g., hypopnea and apnea, may be detected 5532 by monitoring the respiratory waveform output produced by a transthoracic impedance sensor.

When the tidal volume (TV) of the patient's respiration, as indicated by the transthoracic impedance signal, falls below a hypopnea threshold, then a hypopnea event is declared. For example, a hypopnea event may be declared if the patient's tidal volume falls below about 50% of a recent average tidal volume or other baseline tidal volume value. If the patient's tidal volume falls further to an apnea threshold, e.g., about 10% of the recent average tidal volume or other baseline value, an apnea event is declared.

Another method of detecting 5532 disordered breathing involves analyzing the patient's respiratory patterns. According to this method, the patient's respiratory cycle is divided into several periods, including, inspiration, expiration, and non-breathing periods. The inspiration, expiration, and non-breathing respiratory periods are analyzed for patterns consistent with various types of disordered breathing.

As described herein, sleep-disordered breathing may be predicted based on a number of patient conditions that increase the likelihood of disordered breathing. Conditions that predispose the patient to disordered breathing include, for example, air pollution, alcohol use, and pulmonary congestion, among other conditions. In addition to predisposing conditions that make disordered breathing more likely, various precursor conditions may be used to determine that a disordered breathing episode is imminent. For example, blood chemistry, hyperventilation, and the regular periodicity of previous disordered breathing episodes may be used to predict an imminent onset of disordered breathing. If disordered breathing is detected or predicted 5532, an appropriate therapy 5534 may be provided to terminate or prevent the disordered breathing.

Sleep stage classification 5510 may also be used to identify preferable periods of time for performing 5585 various testing procedures, including, for example, diagnostic testing and/or testing of therapeutic device parameters. In various implementations, sleep stage informed diagnostic testing may allow testing to assess the patient's autonomic integrity. Sleep stage classification may further allow the use of REM episodes as a surrogate for stress testing, and recognition of opportunities to routinely perturb the cardiovascular system under controlled conditions.

Sleep stage classification also provides an opportunity to test one or more parameters of a therapeutic device while the patient's activity is low. Such testing may involve, for example, capture threshold testing for a cardiac pacing device and cardiac signal morphology template acquisition to be used in connection with cardiac arrhythmia detection.

Thus, sleep stage classification may be used to provide more effective therapy, better diagnostic information, and improved prognostic and predictive capabilities.

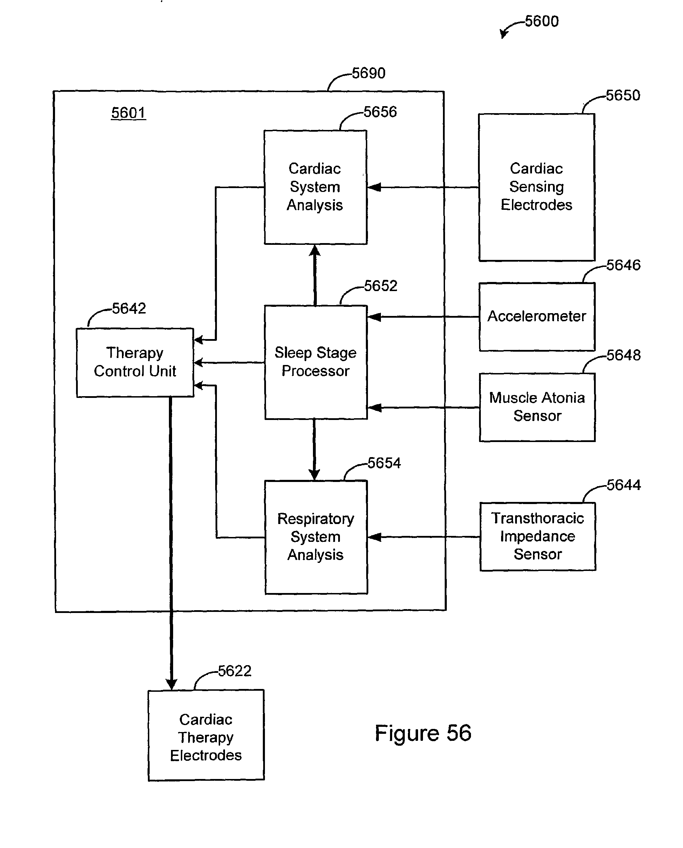

Figure 56 illustrates a medical system that may be used to perform sleep stage informed therapy in accordance with embodiments of the invention. The block diagram of Figure 56 shows the medical system 5600 divided into functional blocks. It will be appreciated by those skilled in the art that there exist many possible configurations in which these functional blocks can be arranged and implemented. The example depicted in Figure 56 is one possible functional arrangement.

Figure 56 illustrates an implantable cardiac pulse generator 5601 enclosed in a housing 5690 and configured to provide therapy for cardiac arrhythmia. Various cardiac electrical therapies, including therapy for disordered breathing and cardiac rhythm therapies, including bradycardia pacing, anti-tachycardia pacing, defibrillation, and cardioversion, may be implemented in cooperation with sleep stage classification in accordance with embodiments of the invention.

Optionally, the medical device 5600 may also be configured to detect respiratory disorders, e.g., sleep-disordered breathing, and to provide therapy to mitigate the respiratory disorders. Disordered breathing therapy, including cardiac pacing and/or other types of disordered breathing therapy, such as continuous positive air pressure (CPAP), nerve stimulation, muscle stimulation or other therapy for treating disordered breathing, may be controlled or provided by components of the cardiac pulse generator 5601.

Although Figure 56 depicts a sleep stage classification system implemented in a cardiac pulse generator 5601, it is understood that configurations, features, and combinations of features described in the disclosure may be implemented in a number of medical devices. Sleep stage classification may be implemented in connection with various diagnostic and therapeutic devices and such embodiments and features are not limited to the particular devices described herein.

Further, although various embodiments involve devices or systems having an implantable control system and implantable sensors, it is understood that therapy or diagnostic systems utilizing the sleep stage classification methodologies of the present invention may be configured so that the control system or components of the control system are arranged externally to the patient. The sensors and the control system, and in particular the patient sleep stage classification system, may involve patient-external components, patient-internal components or a combination of patient-external and patient-internal components.

In the embodiment illustrated in Figure 56, the implantable pulse generator 5601 includes circuitry for providing cardiac rhythm therapy 5642 to treat various arrhythmic conditions. Cardiac arrhythmia therapy is implemented by detecting electrical signals produced by the heart, analyzing the signals for arrhythmia, and providing an appropriate therapy to terminate or reduce the arrhythmia. The pulse generator 5601 is coupled to a cardiac lead system having sensing and therapy electrodes 5650, 5622 electrically coupled to the patient's heart. The cardiac lead system sensing and therapy electrodes 5650, 5622 may include one or more electrodes positioned in or around the heart as well as one or more electrodes positioned on the housing 5690 or header of the pulse generator 5601. In one arrangement, the electrodes used for sensing are also used for therapy delivery. In another arrangement, a set of therapy electrodes different from the sensing electrodes is used.

Cardiac signals sensed by sensing electrodes 5650 of the cardiac lead system are coupled to an arrhythmia analysis unit 5656 configured to identify cardiac arrhythmias. The arrhythmia analysis unit 5656 may use information derived from a sleep stage processor 5652 to provide sleep stage informed arrhythmia detection. If cardiac arrhythmia is detected, the therapy unit 5642 may provide a number of therapies to treat the detected arrhythmia.

The cardiac therapy may include pacing therapy controlled to treat cardiac rhythms that are too slow. In this situation, the therapy unit 5642 controls the delivery of periodic low energy pacing pulses to one or more heart chambers through pacing electrodes of the cardiac lead system 5650. The pacing pulses ensure that the periodic contractions of the heart are maintained at a hemodynamically sufficient rate.

The cardiac therapy may also include therapy to terminate tachyarrhythmia, wherein the heart rhythm is too fast. The arrhythmia analysis unit 5656 detects and treats episodes of tachyarrhythmia, including tachycardia and/or fibrillation. The arrhythmia analysis unit 5656 recognizes cardiac rhythms consistent with various types of tachyarrhythmia. When tachyarrhythmia is identified, the therapy unit 5622 may deliver high energy electrical stimulation to the heart through defibrillation electrodes of the cardiac lead system 5650 to terminate the arrhythmia.

Implementation of an appropriate cardiac therapy may be augmented using sleep stage classification determined by the sleep stage processor 5652 in accordance with embodiments of the invention. As previously discussed, sleep stage classification may be used to determine an optimal arrhythmia therapy. In one example implementation, cardiac therapy may be triggered by signals from the sleep stage processor 5652 to prevent cardiac arrhythmia during REM or other proarrhythmic sleep periods. In another example, the lower rate limit of a bradycardia pacing regimen may be modified when the sleep stage processor 5652 indicates that the patient is asleep.

The sleep stage processor 5652 performs sleep stage classification based on one or more sleep-related signals, including at least one REM-modulated signal. In the illustrative embodiment of Figure 56, a muscle atonia sensor 5648, for example, a EMG sensor, provides a REM-modulated signal to the sleep stage processor 5652. Additionally, a signal responsive to the patient's activity may be used in combination with the REM-modulated signal to augment sleep stage classification. In the example implementation illustrated in Figure 56, the patient activity signal is provided by an accelerometer 5646.

The medical device 5600 may optionally include components for performing respiratory system analysis 5654. In one embodiment, the patient's respiration patterns may be analyzed with knowledge of the patient's sleep stage to determine an appropriate therapy to mitigate detected episodes of disordered breathing or to prevent the occurrence of sleep-disordered breathing.

A transthoracic impedance sensor 5644 may be implemented to produce a signal representing the patient's respiration cycles. A respiration analysis unit 5654 uses sleep stage information provided by the sleep stage processor 5652 in analyzing the patient's respiration patterns to detect episodes of sleep-disordered breathing. Based on sleep stage classification, respiration analysis, and, optionally, cardiac system analysis, cardiac electrical stimulation therapy may be delivered to the patient through the cardiac electrodes 5622 to mitigate or prevent disordered breathing, including sleep apnea, hypopnea, or other forms of disordered breathing. According to one embodiment, preventive therapy for disordered breathing may be initiated if the sleep stage classification processor indicates the patient is asleep, or upon detection of a particular sleep stage.

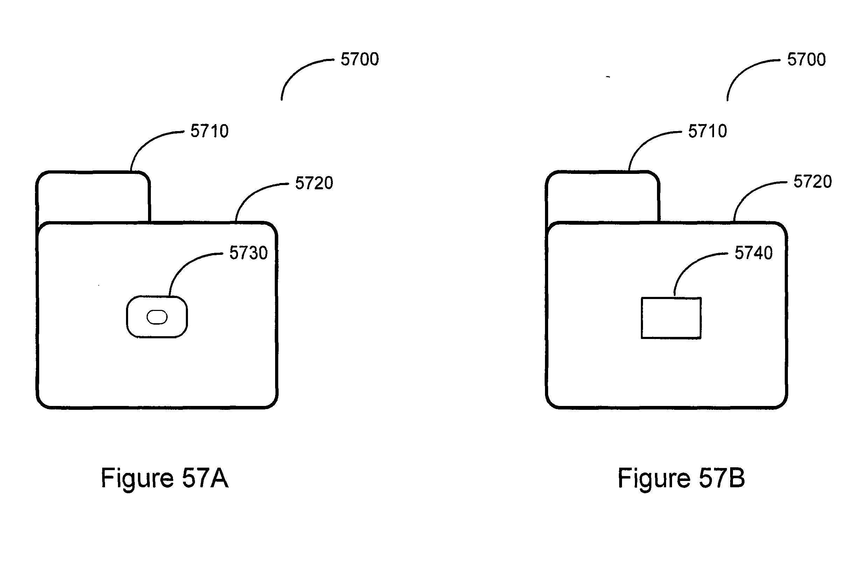

Figures 57A-D illustrate various configurations of a muscle atonia sensor mechanically coupled to an implanted medical device 5700, such as an implantable pacemaker or implantable cardioverter/defibrillator in accordance with embodiments of the invention. The implantable medical device 5700 may include a housing 5720 enclosing the medical device circuitry and a header 5710 for coupling a lead system 5760 to the circuitry of the medical device 5700.

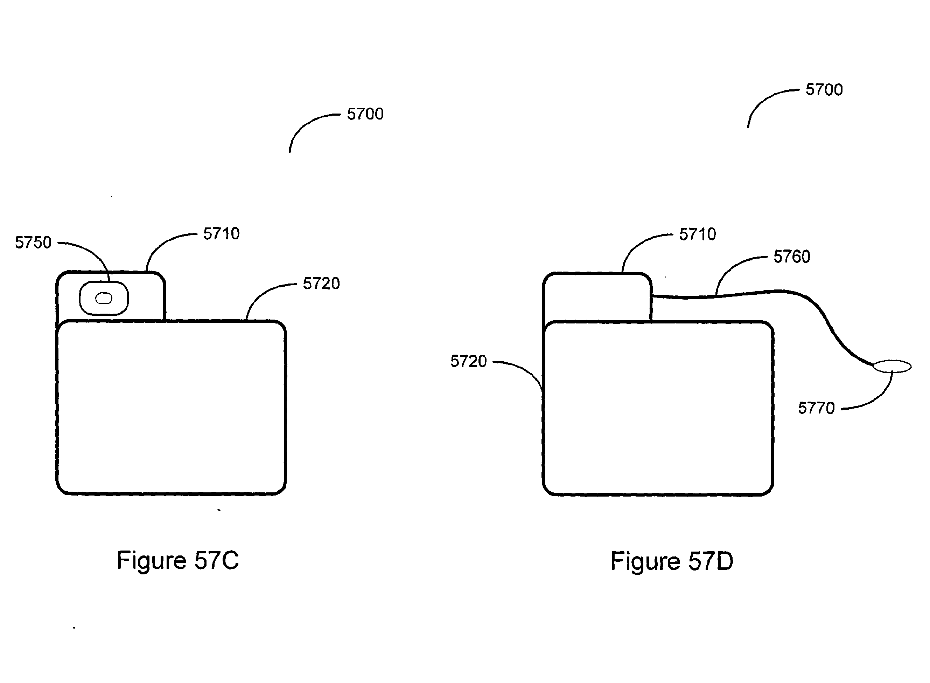

A muscle atonia sensor may be implemented, for example, using an electromyogram (EMG) electrode 5730 or force responsive sensor 5740 positioned on the housing 5720 of the medical device 5700 as illustrated in Figures 57A and 57B, respectively. Figure 57C illustrates a muscle atonia sensor 5750 positioned on the header 5710 of the medical device 5700. Alternatively, a muscle atonia sensor 5770, e.g., EMG electrode or strain gauge, may be positioned on the lead system 5760 or may be coupled to the medical device 5700 through a catheter or lead system 5760, as illustrated in Figure 57D.

Detection of Autonomic Arousal

The cardiac stimulation therapy system of Figure 2 may include a sleep quality monitor 254. Sleep quality assessment may include detecting and assessing the arousals from sleep, sleep disordered breathing episodes, nocturnal limb movements, as well as cardiac, respiratory, muscle, and nervous system functioning. Sleep quality monitoring may provide important information related to various sleep disorders and may be used to adapt therapy used to treat the sleep disorders.

Superficially, sleep may viewed as a monolithic event that is characterized by a period of unconsciousness. If examined in greater detail, sleep periods may be described as involving a series of events or stages. For example, sleep is typically divided into various stages of sleep, including rapid eye movement (REM) sleep and non-REM (NREM) sleep. Non-REM sleep may be further subdivided into stage 1, stage 2 and stage 3 non-REM sleep, for example.

One indicator of sleep quality is the number of arousals experienced during sleep. An arousal is an event that occurs during sleep and may be identified based on changes in EEG signals during non-REM sleep and changes in EEG and EMG signals during REM sleep. Arousal events may or may not culminate in wakefulness. The patient may experience an arousal event during sleep and never wake up.

In one implementation, arousal from sleep has been identified, for example, based on a shift in the patient's EEG signal to a higher frequency for a specified period of time during non-REM sleep assuming sleep has been previously detected. Arousals during REM sleep have been identified by the EEG arousal defined above in addition to changes in an electromyogram (EMG) signal or body movements. Arousals, as identified based on changes in EEG signals, encompass activation of the patient's autonomic nervous system.

Activation of the patient's autonomic nervous system during sleep may be used to identify arousal events referred to herein as an autonomic arousal event. Autonomic arousal events may be identified by an autonomic arousal response involving transient activation of the patient's autonomic nervous system. The autonomic arousal response may or may not result in detectable changes to the patient's EEG signal.

Autonomic arousal events comprise transient changes during sleep that affect autonomic physiological parameters such as heart rate, blood pressure, cardiac output, peripheral vasoconstriction, sympathetic nerve traffic, and arteriole size, among other conditions. For example, an autonomic arousal event may be detected based on a change of about 4 mm Hg increase in systolic blood pressure and/or about a 4 beat per minute increase in heart rate. As previously mentioned, autonomic arousal events begin during sleep and may or may not result in wakefulness. Thus, the patient may experience a number of autonomic arousal events while asleep without achieving a waking state. Nevertheless, these autonomic arousal events disrupt the patient's sleep and degrade sleep quality.

Information about the autonomic arousal events may be stored in memory, and/or transmitted to a separate device for printing or display. Information about the autonomic arousal events may be used to diagnose sleep disorders and/or adjust patient therapy, such as cardiac stimulation therapy, drug therapy, neural stimulation therapy, and/or respiration therapy. Trending sleep information including autonomic arousal events and correlating the sleep information with sleep disorder events may be helpful in determining and maintaining appropriate therapies for patients suffering from a range of sleep disorders.

Many sleep disorder events, e.g., disordered breathing events and movement disorder events, are followed by autonomic arousal events. These autonomic arousals disrupt the normal sleep pattern and may be involved in causing chronic hypertension. The autonomic arousal response may be visible on signals generated by electroencephalogram (EEG) sensors, electromyogram (EMG) sensors, and/or other sensors sensitive to autonomic nervous system changes.

In accordance with embodiments of the present invention, information related to the patient's autonomic arousal response may be collected and/or analyzed. The identification of autonomic arousal events may be used for a variety of purposes, including detecting and/or verifying sleep disorder events, trending the number of arousals per night, and developing various indices such as an arousal index and/or a composite index based on arousals and sleep disorder events. The arousal information may be collected and used in the evaluation of sleep and/or sleep disorders.

Frequent arousals are indicative of a number of medical disorders, including sleep disorders such as sleep disordered breathing. Frequent arousals of the sympathetic nervous system may lead to chronic hypertension or other medical problems. The ability to detect individual and/or aggregate arousals may be used in diagnosing various medical disorders, including disordered breathing. If the patient receives therapy to treat disordered breathing, then the ability to count and trend arousals also provides information regarding therapy efficacy. For example, if arousals decline after therapy is delivered, then it may be assumed that the therapy provides an effective treatment for the disordered breathing. Further, detection of an arousal following delivery of therapy may be used to provide feedback for therapy control.

The methodologies described herein involve using arousal information in combination with disordered breathing information. For example, the system may provide the capability of discriminating between disordered breathing events that cause arousals and disordered breathing events that do not cause arousals. The detection of arousals may allow trending of arousals that occur during sleep. The disordered breathing events that are followed by arousals are considered to be the most disruptive, because repeated arousals prevent the patient from receiving a restful sleep. Some patients continue to experience disordered breathing events during an aroused status. It may be desirable to ignore disordered breathing events that occur during an aroused state. The ability to detect an arousal and ignore subsequently detected disordered breathing events during arousal may improve the accuracy of disordered breathing indices, e.g., apnea/ hypopnea index.

An arousal detection system may comprise, for example, a sensor that generates a signal modulated by changes in muscle tone associated with autonomic arousal. Such a signal may be generated, for example, using an electromyogram sensor or a strain gauge positioned in contact with or near skeletal muscle, such as the pectoral muscle. The sensor may be disposed on an implantable device such as an implantable cardiac rhythm management system, e.g., a pacemaker, defibrillator, cardiac monitor, cardiac resynchronizer, or the like.

Other sensors may be used in connection with arousal detection in addition to or instead of the muscle tone sensor. For example, an accelerometer may be employed to detect patient movement correlated to arousal. An electrogram or other cardiac sensor may be used to detect various cardiac parameters associated with arousal. For example, heart rate increases upon arousal, the AV delay decreases upon arousal, and heart rate variability is modified by autonomic tone changes associated with arousal. Cardiac output increases during arousal, as may be measured via an impedance sensor. Blood pressure, measured, for example, by a lead-based pressure gauge, is modulated by arousal and may be utilized in arousal detection. Peripheral arterial tonography may be used in arousal detection. Arteriole size, which may be measured by photoplethysmography, decreases upon arousal due to sympathetic nervous system activation. Sympathetic nerve traffic modulated by arousal may be sensed using microelectrodes coupled to an implantable device.

In accordance with one embodiment, an arousal detector includes circuitry configured to detect changes in the patient's nervous system. The changes may comprise sympathetic and/or parasympathetic nervous system changes. The arousal detector may be configured to detect the presence of individual arousal events, the presence of aggregate arousals, or the presence of both individual and aggregate arousals.

For example, in one implementation, the sensors may sense conditions that are modulated contemporaneously with an arousal event. In this implementation, the system may detect an individual arousal event during or slightly after the occurrence of the arousal event, for example. In another implementation, the sensors may be sensitive to conditions that are modulated by the aggregate effect of multiple arousal events that occur over a period of time. In such an implementation, detection of individual arousals may or may not occur. The sensors may detect changes in physiological conditions that are caused by the occurrence of multiple arousals. The changes in the physiological conditions are used by the arousal detector to determine that multiple arousal events that have occurred over a period of time. A representative set of conditions indicative of the occurrence of multiple arousal events over a period of time may include, heart rate variability, blood pressure, AV-delay, arteriole size, sympathetic nerve activity, among others. This list is not exhaustive and other conditions may be sensed by the system to determine the occurrence of multiple arousal events.

The therapy assessment processor 260 (Figure 2) may include functionality for evaluating the arousal information and/or determining values or indices using the arousal information. For example, the therapy assessment processor 260 may determine the number of arousals occurring within a sleep period, or other specified time period. The therapy assessment processor 260 may determine an arousal index (arousals detected per unit time), an apnea/hypopnea index (apneas or hypopneas detected per unit time), or other indices. Further, the therapy assessment processor 260 may evaluate the sleep disorder events to determine if arousals are associated with the sleep disorder events. For example, if an arousal is detected within a predetermined time period after a sleep disorder event is detected, the arousal may be associated with the sleep disorder event. Using this process, arousals from sleep that are associated with sleep disorder events can be discriminated from arousals from sleep that are not associated with sleep disorder events.

In one application, for example, the number of arousals may be counted and used to calculate an arousal index to quantify the number of arousals experienced by the patient per unit time. Arousal information may be used to determine a number of sleep quality indices. The arousal information may be used in diagnosing and treating a variety of disorders, including nocturnal sleep disorders, such as sleep disordered breathing, and other conditions. The ability to count and trend these arousals provides diagnostic information regarding patient status with respect to the disorders. For example, autonomic arousals are associated with causing hypertension. A presence of hypertension may be determined or predicted based on arousal information, such as a trend of arousal events over time. Trending arousals may be used to improve therapy used to treat sleep disorders.

Arousals fracture sleep staging, leading to disrupted sleep, and as a consequence, daytime sleepiness. An arousal will bring a patient out of REM sleep or deep sleep (stage 3-4), and bring them temporarily to a waking state. As a consequence, the amount of REM and deep sleep is limited, since the patient has to go back through Stage 1 - 2 sleep before they enter REM or deep sleep.

In one configuration, arousal information may be used by a therapy controller 265 (Figure 2), disposed within an implantable therapy device, for initiating, terminating, or adjusting therapy. Alternatively, the arousal information may be transmitted to the APM system or other remote device for automatic or physician conducted analysis. The APM system may transmit control signals to the implanted device to initiate, terminate or modify therapy delivered by the implanted device. For example, arousal feedback information may be used by an APM system, an implantable cardiac device, to provide closed-loop control of the therapy using arousal information feedback.

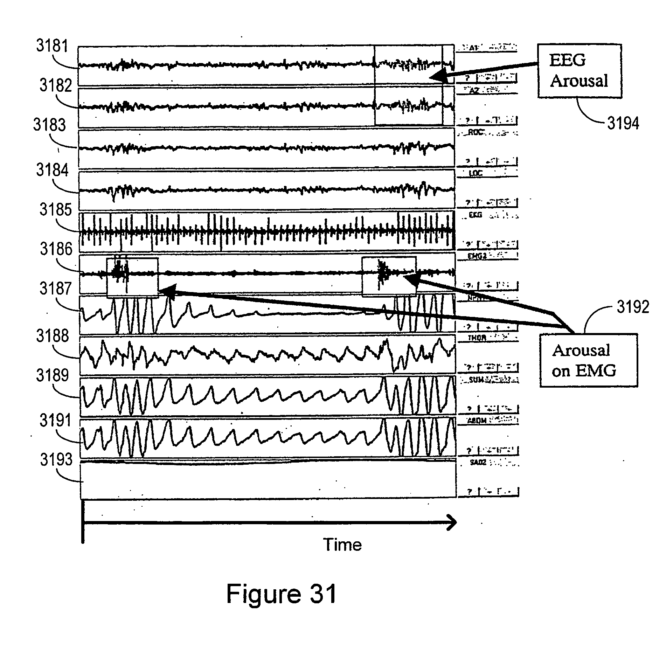

In one implementation, detection of arousals involves evaluating signals generated by sensors for a characteristic signature of autonomic arousal. Autonomic arousal responses, as detected using EEG sensors and EMG sensors, are illustrated in the graph of Figure 31.

Referring now to Figure 31, a sleep study sensor array output is illustrated including an apnea event terminating in an arousal. Arousal detection may be implemented using implantable sensors capable of detecting changes in the sympathetic or parasympathetic nervous system. These changes may be either short-term (i.e., changes associated with individual arousals) or long-term (i.e., aggregate effect of multiple arousals). A short-term effect of arousal includes, for example, the activation of sympathetic nerve activities. Sympathetic or parasympathetic changes, or the changes of autonomic balance can be assessed, for example, by heart rate variability (HRV), which can be detected using a device configured to sense cardiac activity, changes in heart rate, and/or changes in AV conduction.

In the graphs of Figure 31, the abscissa of all the graphs is the same time period during the sleep analysis of a patient. The ordinate of each of the graphs is the signal amplitude of the respective sensor. Traces 3181, 3182, 3183, and 3184 are the top, second, third, and fourth traces respectively, plotted from sensors adapted to produce electroencephalogram (EEG) signals. Evident in all four traces, but particularly pointed out in traces 3181 and 3182 is a characteristic signature of an EEG signal indicative of arousal 3194. A trace 3185 provides an electrocardiogram (EKG) of the heart beats during the time period of the graph. A trace 3186 provides an electromyogram defining muscular movement during the time period of the graph. Particularly evident in the trace 3186 is a characteristic signature of an EMG signal indicative of arousal 3192.

Traces 3187, 3188, 3191, and 3189 illustrate various parameters related to respiration. Trace 3187 is nasal pressure, 3188 is thoracic effort, 3191 is abdominal effort, and 3189 is the sum of the thoracic and abdominal effort. Trace 3193 depicts the blood oxygen saturation level of the patient. Pulmonary activity may be sensed through the use of internal sensors, such as impedance sensors and/or minute ventilation sensors described further below.

In accordance with aspects of the present invention, arousal detection may be used in connection with detection of sleep disorders, such as disordered breathing. Sleep disordered breathing may cause the patient to arouse from sleep frequently during a sleep period. Thus arousals from sleep follow the sleep disorder event. In one configuration, arousal detection may be used as a surrogate for direct detection of the disordered condition. For example, in systems that do not have a respiration sensor capable of detecting disrupted respiration, arousal detection may be used as a surrogate for detecting disrupted respiration. Information from the arousal detector may be used to separate sleep disorder events, e.g., apnea, hypopnea, followed by arousal versus those terminated without arousal. The sleep disorder events that are followed by arousal are considered to be the most disruptive, as these arousals interrupt the normal course of sleep and prevent the patient from receiving a full sleep cycle each night. Detecting these types of sleep disorder events enhances the specificity of sleep disorder event detection and guides diagnosis and/or therapy.

The arousal information may be used to modify therapy for sleep disorder events such as disordered breathing. In various implementations, the arousal information and/or disordered breathing information may be used by a therapy controller 265 (Figure 2) to modify disordered breathing therapy delivered to the patient.

For example, cardiac electrical therapy may be provided by an implanted therapy device. Detection of disordered breathing may be used to initiate the cardiac electrical therapy. Detection of arousal, indicating the end of the disordered breathing event, may be used to terminate the electrical stimulation therapy, for example.

In another example, cardiac electrical therapy may be provided, and the number of arousals monitored. If the cardiac electrical therapy causes an excessive number of arousals, then the cardiac electrical therapy may be adjusted or terminated.

In another example, an APM system may receive information about sleep disorder events from the disordered breathing detector/predictor 258 (Figure 2) and/or arousal information from the arousal detector. The information may be automatically evaluated by the APM system, or may be evaluated by the patient's physician. The APM system may be used to transmit control signals to the therapy controller 265 of an implanted device to initiate, terminate or modify the therapy delivered to the patient.

In various configurations, an EMG sensor may be positioned on a housing or header of an implantable device, such as a cardiac rhythm management device, or may be located on a catheter or lead coupled to the cardiac rhythm management device. An EMG sensor located on a device positioned in the pectoral region provides access to skeletal muscle that may be exploited to detect arousal.

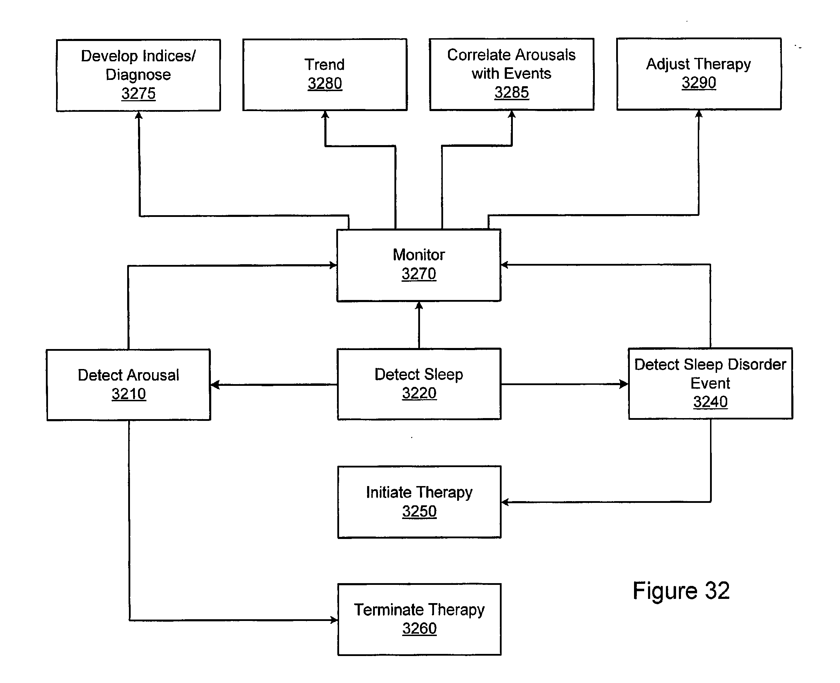

Figure 32 depicts a flow diagram illustrating various optional processes that may be implemented in connection with arousal detection according to embodiments of the invention. Detection of sleep 3220 may be used to inform the arousal detection process 3210 and the sleep disorder event detection process 3240. Information about sleep, sleep disorder events, and arousals from sleep are monitored 3270. The information may be used to diagnose sleep-related disorders and/or other disorders 3275, calculate arousal and sleep disorder indices, develop trend information 3280, correlate arousals with sleep disorder events 385, and/or adjust therapy delivered to the patient 3290. Upon detection of a sleep disorder event 3240, e.g., sleep disordered breathing, therapy to mitigate the sleep disorder event may be initiated 3250. Arousal detection 3210 signals the end of the sleep disorder event, and therapy may be terminated 3260 following detection of arousal from sleep.

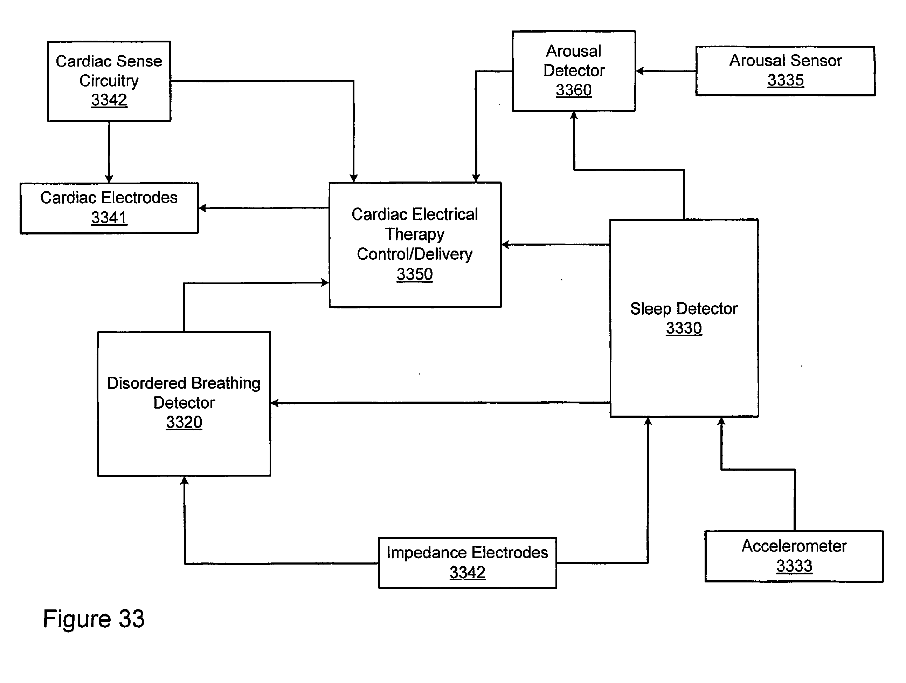

Figure 33 is a block diagram of an arousal detector that is implemented in cooperation with a cardiac rhythm management (CRM) system such as a pacemaker and/or cardioverter/defibrillator with functionality to deliver cardiac electrical stimulation for disordered breathing in accordance with an embodiment of the invention. The system may be partially or completely implantable.

Cardiac sense circuitry 3342, cardiac electrical stimulation control and delivery circuitry 3350, disordered breathing detector 3320, arousal detector 3360, and sleep detector 3330 are arranged within a housing that is hermetically sealed and suitable for implanting within the patient, such as within the pectoral region of the patient's chest. An accelerometer 3333 configured to detect patient activity may also be incorporated within the housing. An EMG sensor is implemented as an arousal sensor 3335 and is disposed on the housing so that the EMG sensor 3335 is positioned in contact with or near skeletal muscle, such as the pectoral muscle. An intracardiac lead system includes cardiac electrodes 3341 for electrically coupling to the patient's heart and one or more transthoracic impedance electrodes 3342 for generating a respiration signal.

The sleep detector 3330 uses the patient activity signal generated by the accelerometer 3333 and the respiration signal generated by the transthoracic impedance electrodes 3342 to determine if the patient is asleep or awake.

The disordered breathing detector 3320 detects disordered breathing events based on the patient's respiration patterns, as described more fully above. The arousal detector 3360 compares the EMG signal to a characteristic arousal signature and detects arousal based on the comparison. Disordered breathing detection and arousal detection may be enhanced using sleep/wake information provided by the sleep detector 3330.

In one embodiment, the CRM provides cardiac electrical stimulation the to one or more heart chambers as therapy for disordered breathing. The cardiac electrical therapy control unit 3350 may utilize signals from the sleep detector 3330, disordered breathing detector 3320, and arousal detector 3360 to initiate, terminate, and/or adjust the cardiac electrical stimulation therapy for disordered breathing. For example, the therapy control unit 3350 may initiate a process for treating disordered breathing episodes when the sleep detector 3330 determines that the patient is asleep.

In one scenario, the therapy control unit 3350 may initiate cardiac electrical stimulation, e.g., cardiac overdrive pacing, to treat disordered breathing upon detection of a disordered breathing event during sleep. In another scenario, the therapy control unit 3350 may initiate cardiac electrical stimulation to treat disordered breathing when sleep is detected. The therapy control unit 3350 may adjust the cardiac electrical stimulation when a disordered breathing event is detected during sleep. If an arousal is detected, then the therapy control unit 3350 may terminate or adjust the cardiac electrical stimulation therapy for disordered breathing.

Respiratory Logbook

Adapting a therapy for disordered breathing may involve monitoring of the symptoms, effects, and/or physiological changes associated with disordered breathing. A respiration monitoring system 254, depicted in Figure 2, may be used to collect information relevant to disordered breathing and/or respiration quality and useful in developing a therapy regimen for improving respiration quality.

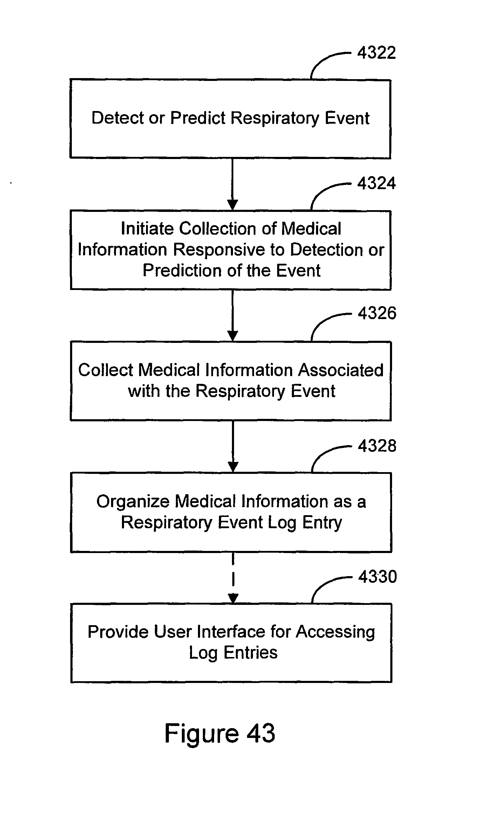

According to one embodiment, the respiration monitor implements an event-based process for collecting and organizing medical information associated with a respiratory event. Figure 43 is a flowchart illustrating a method involving collection of medical information associated with a respiratory event in accordance with embodiments of the invention. The information is stored in a respiratory logbook that is accessible to the user or The respiratory event may be detected and/or predicted 4322. The event may include any detectable or predictable respiratory event, such as disordered breathing (apnea, hypopnea, tachypnea), coughing and/or other breathing rhythm irregularities.

In response to the detection or prediction 4322 of the respiratory event, collection 4324 of medical information for the respiratory event logbook entry is initiated 4324. The medical information may be collected 4326 during the event and/or during a time period proximate to the event. Information may be collected during the event, during a period of time preceding the event, and/or during a period of time following the event. In some embodiments, the information may be collected prior to the prediction or detection of the respiratory event.

To facilitate collection of medical information preceding the prediction or detection of the respiratory event, the medical information may be monitored, e.g., on a continuous or periodic basis, and stored in a temporary buffer. Temporary storage is required to provide information prior to the event prediction or detection, e.g., onset data. The duration of the temporary storage may vary according to the respiratory events for which onset data is desired.

Due to the varied nature of onset data requirements and the reality of limited storage in the system, the system may allow different onset data lengths and different sampling rates for the temporarily stored data. In a preferred embodiment, the system uses a circular buffer to store the temporary data such that the oldest data is replaced by the newest data.

Once initiated, collection of respiratory information, which may involve storage of the information in long term memory, may be performed on a substantially continuous basis, or it may be performed during discrete intervals. Long term collection of data on a periodic basis may be beneficial when the event is relatively prolonged, such an in the case of a disease or disorder that may linger for several days or weeks. Various collection parameters, such as the type of data collected, data collection frequency, and/or data collection intervals may be selectable by the user. Further, the system may be programmable to use different data collection regimens under different conditions over the course of the event. For example, the system may be programmed to collect data more frequently during sleep or during particular stages of the disease progression, for example. The system may be programmed to collect data on a substantially continuous basis during some time intervals, and periodically during other time intervals, for example.

Collecting medical information preceding the respiratory event facilitates enhanced identification of conditions that may be used to detect or predict the occurrence of future events. For example, acquiring information preceding the event affecting patient respiration allows for the identification and assessment of physiological conditions present immediately before and leading up to the event. In one scenario, the patient may experience a period of hyperventilation prior to an apnea event. Collecting respiratory information prior to the apnea event allows the identification of hyperventilation as a precursor condition. The identification of precursor conditions for apnea facilitate increased sensitivity and/or accuracy in detecting or predicting future occurrences of apnea.

Additionally, or alternatively, medical information preceding the respiratory event may provide insight into conditions that predispose the patient to certain respiratory events. Acquiring information preceding the event may allow identification of the triggering or causal factors of the event. For example, a disordered breathing event may follow an episode of snoring, or a change in patient posture. Collection of medical information preceding the disordered breathing event allows the factors that precipitate the disordered breathing event to be identified. Such information may be used to enhance the detection and/or prediction of future events.

Information collected following the event may be used to assess the acute effects of the event. Episodes of disordered breathing, for example, may be associated with acute physiological effects, including negative intrathoracic pressure, hypoxia, and arousal from sleep. Such effects may be detectable for a period of time following the respiratory event.

For example, obstructive sleep apneas are typically terminated by arousal from sleep that occurs several seconds after the apneic peak, allowing the resumption of airflow. Coincident with arousal from sleep, and continuing for some period of time after termination of the event, surges in sympathetic nerve activity, blood pressure, and heart rate occur.

During obstructive apnea events, the effort to generate airflow increases. Attempted inspiration in the presence of an occluded airway results in an abrupt reduction in intrathoracic pressure. The repeated futile inspiratory efforts associated with obstructive sleep apnea may trigger a series of secondary responses, including mechanical, hemodynamic, chemical, neural, and inflammatory responses. Collection of data following obstructive sleep apnea events may be used to determine the presence and/or severity of the secondary responses to obstructive apnea events. The post-event information enhances the ability to evaluate the impact of the secondary responses upon the patient.

As previously described, obstructive sleep apnea events are typically terminated by arousal from sleep. However, arousals are not usually required for the resumption of breathing in central sleep apnea events. In the case of central apnea events, the arousals follow the initiation of breathing. Arousals following central apnea events may facilitate the development of oscillations in ventilation by recurrently stimulating hyperventilation and reducing $PaCO_2$ below the apneic threshold. Once triggered, the pattern of alternating hyperventilation and apnea may be sustained by the combination of increased respiratory drive, pulmonary congestion, arousals, and apnea-induced hypoxia causing $PaCO_2$ oscillations above and below the apneic threshold. Shifts in the patient's state of consciousness, particularly with repeated arousals, may further destabilize breathing. Collecting information during central apnea events and before and/or after the occurrence of the events may allow identification of the oscillations associated with central apnea.

The collected medical information, which may be stored in long term memory, transmitted, printed and/or displayed is organized as a respiratory logbook entry 4328. The medical information may include various physiological and non-physiological data. For example, respiratory system data, cardiovascular system data, nervous system data, posture, activity, medical history data, environmental data (temperature, altitude, air quality) and other types of medical information may be organized as a respiratory logbook entry. The respiratory logbook entry may be stored, transmitted, printed and/or displayed.

A respiratory event logbook may comprise a number of entries, each entry corresponding to a separate respiratory event. The event entries included in medical event log may be organized according to various categories, including for example, event type, event time/date, order of occurrence of the event, therapy provided to treat the event, among other categories. The selection of categories used to organize the information may be programmable by the user. The organized information may be stored in long term memory, displayed, printed, and/or transmitted to a separate device.

The collected information for the events may be optionally accessible 4330 through an interactive user interface. The interactive user interface may provide access to one or more log entries through activation of a selection process, involving a hierarchical selection menu, or other selection method, for example. In one implementation, the user may select a log entry from the menu by activating an input mechanism. Upon selection of the log entry, the user interface may provide graphical or textual depictions of the collected respiratory information associated with the medical event.

The event information of the logbook may be stored in long term memory using various storage methodologies. For example, the logbook may utilize a flat file system, hierarchical database, relational database, or distributed database. Data for a group of events may be analyzed and/or summarized in various formats. Graphical and/or textual summary information may be displayed on the user interface and/or otherwise communicated to the user. For example, histograms, trend graphs, and/or other analytical tools or formats may be generated based on the logbook event entries. A logbook display may have the ability to display trends of the patient's apnea/hypopnea index, histograms of number of apneas/hypopneas and/or obstructive/central events per night, sleep stage diagram (shows the stage of sleep for each night), heart rate trend during the night, oxygen saturation trend during the night.

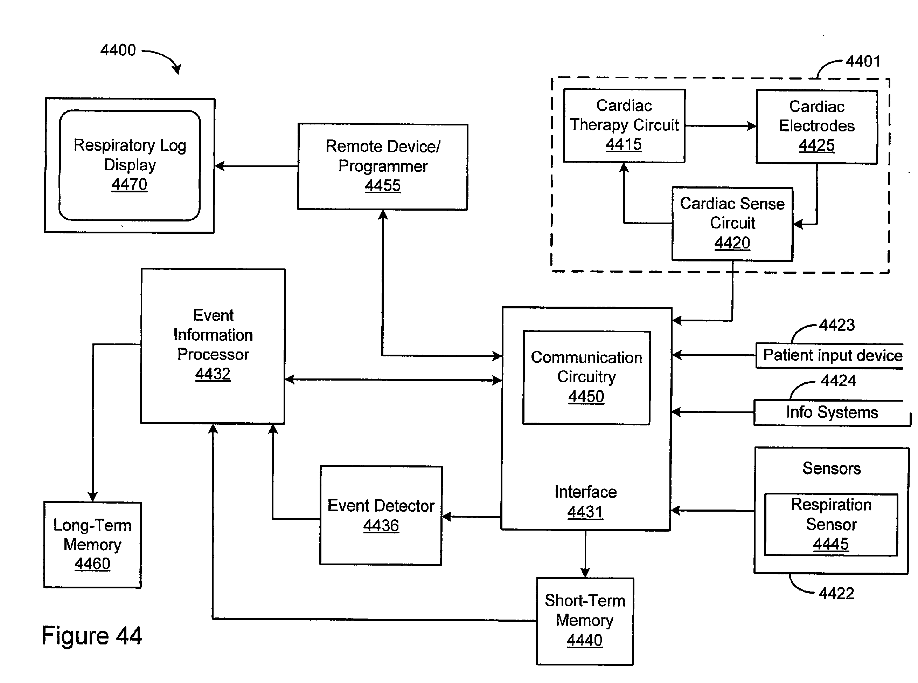

Figure 44 is a block diagram of a logbook system 4400 in accordance with embodiments of the invention. The respiratory logbook system 4400 implements an event-driven method of collecting and organizing data related to events affecting patient respiration.

Various patient conditions may be monitored through sensors 4422, patient input devices 4423, and/or information systems 4424. Data associated with patient conditions may be stored in short term memory 4440. One or more of the patient conditions may be used by event detection circuitry 4436 to detect or predict the occurrence of an event affecting respiration. Detection or prediction of an event affecting respiration initiates the long term storage of information associated with the event by the event information processor 4432 into the long term memory 4460. For example, the event information processor 4432 may collect information supplied by one or more of the sensors 4422, patient input devices 4423, and information systems 4424 before, during, and/or after the detection and/or prediction of the event. The collected information associated with each event is organized as a respiratory logbook entry in the respiratory logbook. The respiratory logbook, or portions thereof, may be stored in long term memory 4460, transmitted to a remote device 4455, and/or displayed on a display device 4470.

The embodiment illustrated in Figure 44 includes a respiration sensor 4445 that senses a physiological condition modulated by patient respiration. In one embodiment, the respiration sensor may comprise a transthoracic impedance sensor. Other methods of sensing respiration are also possible. Such methods may include, for example, the use of patient-external respiratory bands, respiration flowmeter measurements, implantable or patient-external breath sound detection, blood oxygen levels, and/or other processes. The respiration sensor 4445 may be used, for example, to acquire a respiration waveform before, during, and/or after an event affecting the patient respiration. The respiration waveform may be a component of the respiratory log entry for the event.

Information about various conditions affecting the patient and associated with the event may be acquired using sensors 4422, patient input devices 4423 and/or other information systems 4424. The sensors 4422 may comprise patient-internal and/or patient-external sensors coupled through leads or wirelessly to the interface 4431 of the respiratory logbook system 4400. The sensors may sense various physiological and/or non-physiological conditions affecting patient respiration or other physiological systems. The patient input device 4423 allows the patient to input information relevant to conditions affecting the patient that may be useful in generating a respiratory event log. For example, the patient input device 4423 may be particularly useful for acquiring information known to the patient, such as information related to patient smoking, drug use, recent exercise level, and/or other patient activities, perceptions and/or symptoms. The information provided by the patient-input device may include patient-known information relevant to the event affecting respiration that is not automatically sensed or detected by the respiratory logbook system 4400.

The respiratory logbook system 4400 may also include one or more information systems 4424 such as a remote computing device and/or a network-based server. The event information processor 4432 may access the information systems 4424 to acquire information from databases and/or other information sources stored on or generated by the remote computing devices and/or servers. The information acquired from the information system s 4424 may be recorded in the respiratory logbook along with other information relevant to the event affecting respiration. In one exemplary implementation, the respiratory logbook system 4400 may access an internet connected air quality server to collect data related to environmental conditions, such as an ambient pollution index. In another implementation, the respiratory logbook system 4400 may access the patient's medical history through a patient information server.

The sensors 4422, patient input devices 4423, and information systems 4424 are coupled to other components of the respiratory logbook system 4400 through interface circuitry 4431. The interface 4431 may include circuitry for energizing the sensors 4422 and/or for detecting and/or processing signals generated by the sensors. The interface 4431 may include, for example, driver circuitry, amplifiers, filters, sampling circuitry, and/or A/D converter circuitry for conditioning the signals generated by the sensors.

The interface 4431 may also include circuitry 4450 for communicating with the patient input device 4423, information systems 4424, a device programmer 4455, an APM system (not shown), or other remote devices. Communication with the patient input device 4423, information systems 4424 and/or a remote device programmer 4455 and/or other remote devices may be implemented using a wired connection or through a wireless communication link, such as a Bluetooth or other wireless link. The communication circuitry 4450 may also provide the capability to wirelessly communicate with various sensors, including implantable, subcutaneous, cutaneous, and/or non-implanted sensors.

The respiratory logbook system 4400 may optionally be implemented as a component of a medical device that includes a therapy system, such as a cardiac rhythm management system 4401. The cardiac rhythm management system 4401 may include cardiac electrodes 4425 electrically coupled to the patient's heart. Cardiac signals sensed by cardiac sense circuitry 4420 may be used in the detection and treatment of various anomalies of the heart rhythm. Anomalous heart rhythms may include, for example, a rhythm that is too slow (bradycardia), a heart rhythm that is too fast (tachycardia), and/or a heart rhythm that involves insufficiently synchronized contractions of the atria and/or ventricles, a symptom of congestive heart failure.

If an arrhythmia is detected by the cardiac rhythm management system, then a cardiac therapy circuit 4415 may deliver cardiac therapy to the heart in the form of electrical stimulation pulses, such as pacing and/or cardioversion/defibrillation pulses. The cardiac signals and/or cardiac conditions, e.g., arrhythmia conditions, derived or detected through the use of the cardiac signals may be associated with an event affecting respiration. The cardiac information associated with the event may be acquired and organized by the respiratory logbook system 4400.

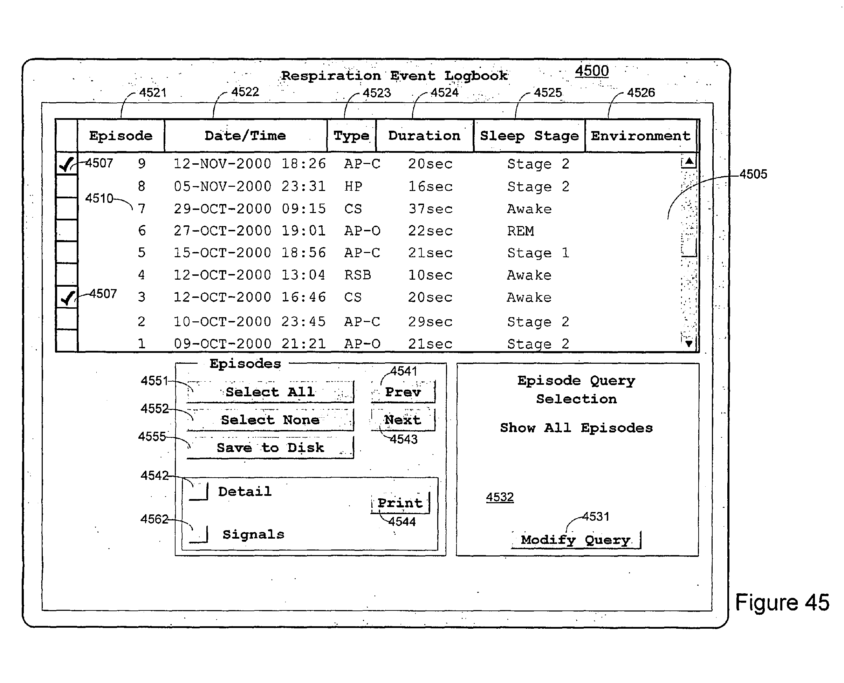

A user interface may be used to view and/or access the respiratory logbook information. Figure 45 illustrates an exemplary depiction of a user interface display 4500. An area 4505 of the display may be used to provide textual or graphical information about respiratory events. As illustrated in Figure 45, a menu 4510 of respiratory events may be presented and may enable the user may access additional information related to the respiratory event. The menu 4510 may provide a summary of parameters associated with the events contained in the respiratory logbook. As illustrated in Figure 45, one or more summary parameter headings, such as episode number 4521, date/time 4522, type 4523, duration 4524, sleep stage 4525, and/or environment 4526, among other parameter headings, may be presented at the top of the menu 4510 or in another convenient location. The summary parameter headings 4521-4526 may be programmable, and additional or alternative parameter headings to those depicted in Figure 45 may be selected, for example.

The type parameter 4523 may contain abbreviations for various respiratory events. For example AP-C and AP-O may abbreviate central and obstructive apneas respectively, HP abbreviates a hypopnea, CS abbreviates Cheyne-Stokes respiration and RSB abbreviates rapid-shallow breathing.

The respiratory events displayed as menu items in the menu 4510 may be selected by a user according to episode number, date/time, duration, type, number, or by other criteria. The menu items may be selected for display based on various criteria ranges and/or thresholds. For example, in the example screen illustrated in Figure 45, different groups of events selected as menu items may be selected by activating the modify query button 4531. The modify query button 4531 and other buttons illustrated on the display may be voice activated, activated through touching the display screen, or by operating a keyboard or pointing device, for example.

In one implementation, activation of the modify query button 4531 initiates a dialog session that allows the user to select respiratory events to be presented in the menu according various criteria such as by date/time, duration, type, number, or by other criteria ranges or thresholds. In one example, the user may select all apnea events to be presented as menu items. In another example, the user may select all events that occurred between a first date and a second date. In yet another example, the user may select all events that occurred while the patient experienced certain environmental conditions, e.g., ambient temperature range and/or humidity range. In yet another example, the user may choose to select all events of the respiratory logbook. The selection criteria may be displayed in an episode query selection area 4532 of the display. The episode query selection area 4532 in the depiction of a respiratory logbook display shown in Figure 45 indicates that all episodes have been selected to be displayed as menu items.

The menu 4510 allows the user to choose respiratory events for which additional textual and/or graphical information is displayed. The additional information provides more detailed information about the selected events beyond the summary information presented in the menu 4510. In the exemplary illustration depicted in Figure 45, the selections are indicated by check marks 4507 beside the selected respiratory events. For convenience, the display may include a select all button 4551 and/or a select none button 4552. Activation of the select all button 4551 causes all events in the menu 4510 to be selected. Activation of the select none button 4552 causes all events in the menu 4510 to be deselected.

Following selection of one or more episodes in the menu, activation of the detail button 4542 causes detailed textual information associated with a selected event to be presented on the display screen. The detail information may be displayed in the area of the screen 4505 previously occupied by the menu 4510, for example. The user may scroll back and forth through the textual information for the one or more selected events using the prev button 4541 and the next button 4543. The textual information may be printed upon activation of the print button 4544, or may be saved to a disk, or other storage medium, through activation of the save to disk button 4555.

Graphical information associated with the selected events may be displayed upon activation of the signals button 4562. In one implementation, a respiration waveform acquired during, before and/or after a selected event may be displayed in the area 4505 of the display previously used for the menu 4510. Waveforms of other parameters, e.g., cardiac rhythm, patient activity, may additionally or alternatively be displayed. In one implementation, a marked waveform may be displayed. For example, a marked respiration waveform may include the respiration waveform acquired before, during, and after the event, along with one or more symbols aligned with the respiration waveform to indicate the occurrence of one or more conditions. The symbol may provide a numerical value or a textual description associated with the respiration characteristic, e.g., average respiration rate, expiratory slope, etc. In one example, various characteristics of disordered breathing events including quantifiable characteristics, such as episode duration, blood oxygen saturation, disordered breathing type, and/or other detected characteristics may also be displayed along with the respiration waveform. A user may scroll through the waveforms associated with the selected events using the prev and next buttons 4541, 4543.

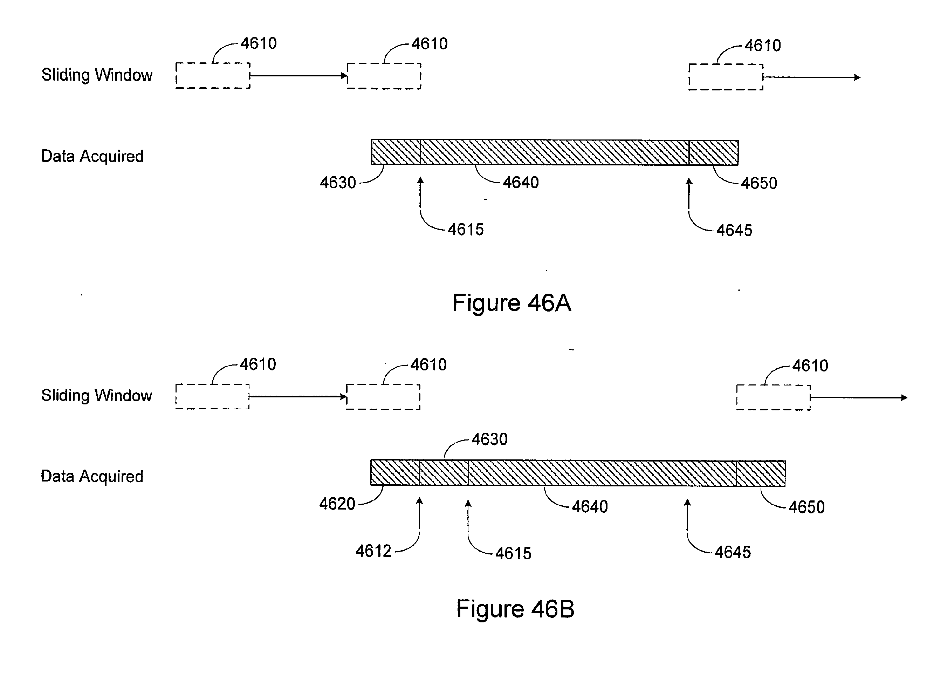

Figure 46A provides a timing diagram illustrating the acquisition of respiratory logbook information for a detected disordered breathing event in accordance with embodiments of the invention. The respiratory logbook system senses and stores in a temporary buffer a sliding scale window 4610 of one or more patient conditions, such as those listed in Table 1. The selection of information that is sensed and stored may be programmable by the physician. The selection of the information to be acquired may be based on the patient's medical history. For example, if the patient suffers from central sleep apnea, the respiratory logbook would preferably be programmed to sense conditions associated with disordered breathing. Conversely, if the patient suffers obstructive sleep apnea, a different set of conditions from those used for central disordered breathing could be sensed.

If an event affecting respiration is detected 4615, then pre-event information 4630 acquired prior to the event is stored. Information is collected and stored during 4640 the event. Upon detection that the event has terminated 4645, post-event information 4650 is collected and stored for a period of time after the termination of the event. The event and post-event information 4640, 4650 may be acquired on a continuous basis, or the information may be acquired during discrete intervals. After the post-event information 4650 is collected, the acquired information 4630, 4640, 4650 is organized as a logbook entry. The respiratory logbook system begins sensing for the next event.

Figure 46B provides a timing diagram illustrating the acquisition of respiratory logbook information for a predicted event affecting respiration in accordance with embodiments of the invention. The respiratory logbook system senses and stores in a temporary buffer a sliding scale window 4610 of one or more patient conditions, such as those listed in Table 1. The conditions that are sensed and stored are programmable and may be selected based on the patient's medical history. For example, the information sensed and stored may include information that has been effectively used to predict the one or more types of events affecting the patient's respiration. If an event affecting respiration is predicted 4612, then pre-prediction information 4620 is acquired and stored. When the event affecting respiration is detected 4615, then pre-event information 4630 acquired prior to the event is stored. Information 4640 is collected and stored during the event. Upon detection that the event has terminated 4645, information 4650 is collected and stored for a period of time after the termination of the event. The pre-event, event and post-event information 4630, 4640, 4650 may be acquired on a continuous basis, or the information may be acquired during discrete intervals. After the post-event information 4640 is collected, the acquired information 4620, 4630, 4640, 4650 is organized as a logbook entry. The respiratory logbook begins sensing for the next event.

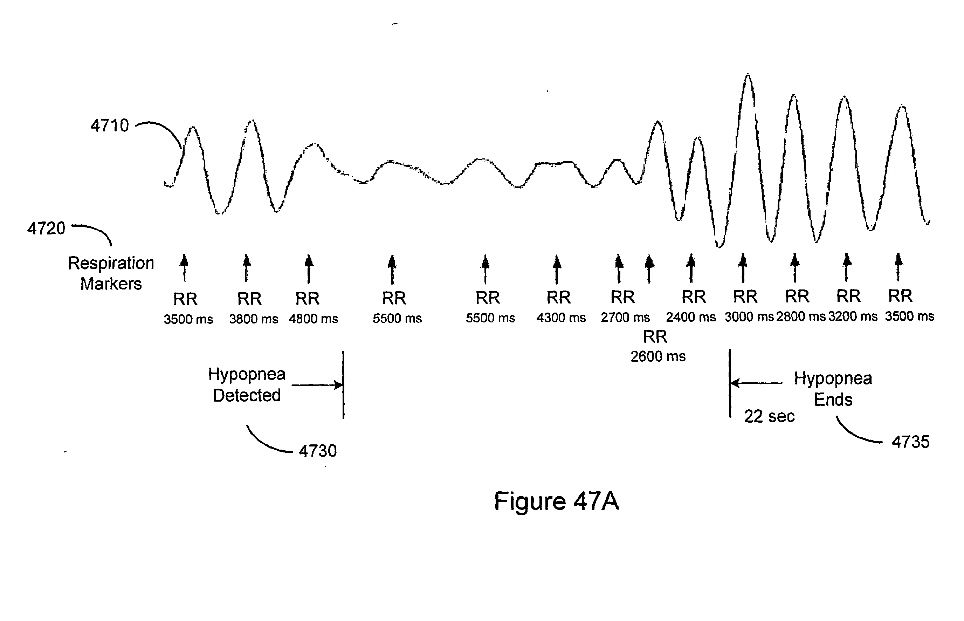

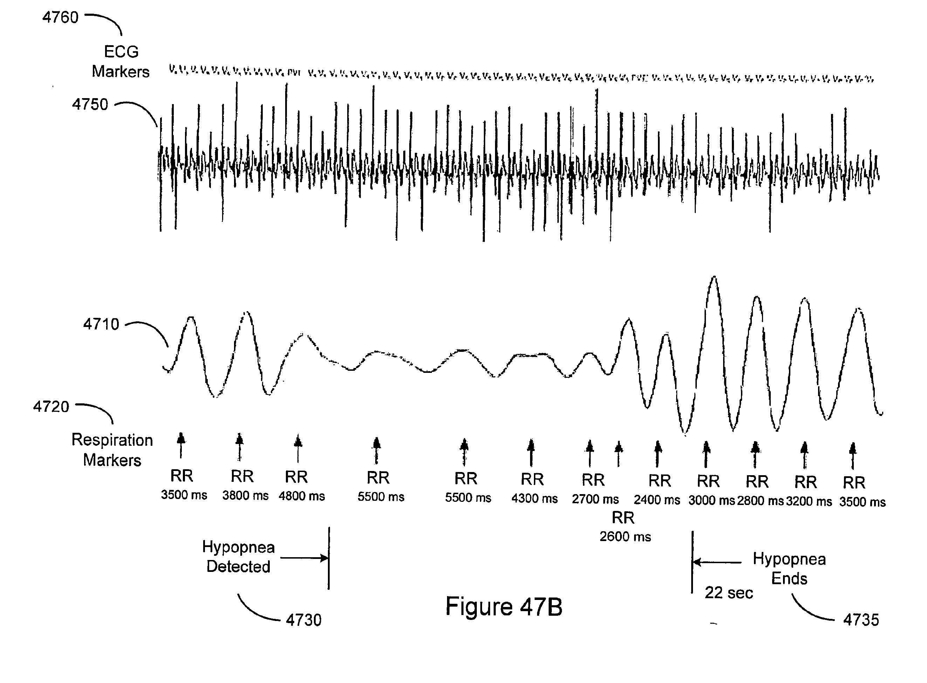

As previously discussed in connection with Figure 45, the respiratory logbook display may include information presented in graphical format. In one embodiment, the user may choose to view a marked respiration waveform, for example. Figures 47A and 47B provide examples of marked respiration waveforms that may be acquired and organized in a respiratory logbook.

Figure 47A illustrates a marked respiration waveform in accordance with embodiments of the invention. In one embodiment, information related to a marked respiration waveform may be acquired continuously as a moving snapshot of respiration-related conditions. In another embodiment, the information related to the marked respiration waveform may be acquired in response to one or more triggering events. For one example, the triggering event may comprise an instruction from a physician or through an advanced patient management system to begin data collection. In another example, the triggering event may comprise detection of various respiration conditions, such as detection of the disordered breathing, or the detection of sleep. In this scenario, the triggering event may initiate the collection of respiration-related data during an interval of time that may include time periods prior to, during, and/or following the disordered breathing event.

As illustrated in Figure 47A, the marked respiration waveform 4710 may comprise respiration symbols positioned at locations relative to the respiration waveform to indicate when respiratory events occur or the time when characteristics are calculated. In this example, the respiration waveform 4710 is marked with respiration symbols 4720 denoting the time between peaks on the waveform and hypopnea symbols denoting when an hypopnea is detected 4730 and when an hypopnea ends 4735 after 22 seconds. In addition, other symbols indicating respiration characteristics and/or disordered breathing characteristics described above may be superimposed on the respiration waveform. The marked respiration waveform may be displayed on a display device to allow the patient's physician to view respiratory disturbances and/or other characteristics.

In addition to displaying the respiration waveform 4710, the display may show other measurements and/or other waveforms. In Figure 47B, an electrocardiogram (ECG) 4750 is shown above respiration waveform 4710. The ECG 4750 is time-aligned with respiration waveform 4710 and can be marked with indicators corresponding to the occurrence of breathing and/or cardiac events, for example. Markers 4760 indicating sensed ventricular events (Vs) and paced ventricular events (Vp) are displayed above the ECG in Figure 47B. Displaying marked respiration waveforms and other waveforms related to patient conditions allows the patient's physician to verify, for example, that a disordered breathing event was properly detected. This confirmation may be used to guide diagnostics and/or therapy. Symbols annotating cardiac and respiratory events provide further diagnostic information for physicians.

Sleep Logbook

A similar technique to the respiratory logbook described above may be employed to generate an event-based sleep logbook. The sleep logbook information may be used to assess the impact of disordered breathing or disordered breathing therapy on the patient's sleep quality. Such an assessment may be performed by the therapy assessment processor 260 of Figure 2. Assessment of therapy impact may be used in turn for developing an appropriate therapy for the patient.

Development of an appropriate therapy may involve automatic access by a device to the sleep logbook, respiratory logbook, and/or other information acquired and/or stored by therapy and/or diagnostic devices. The device may use the information to develop an appropriate therapy regime and may communicate this information to the therapy device. In another example, a physician may access information in the sleep logbook, respiratory logbook, and/or elsewhere and may remotely communicate with a therapy device to initiate, modify, or terminate the patient's therapy.

The sleep logbook represents a system for organizing sleep-related data. According to one embodiment, each sleep logbook entry may include data associated with a particular sleep-related event. An event may comprise various types of events related to sleep. The types of information acquired and the types of sleep-related events represented in the sleep logbook may be programmable by a user.

According to an embodiment of the invention, information may be collected continuously or periodically throughout a sleep period, e.g., throughout a patient's typical sleep time, or during one or more particular sleep stages. The system may initiate acquisition of information before, during and/or after detection of sleep or detection of a particular sleep stage. In this example, each sleep period for which data is collected may be organized as a sleep logbook entry.

The system may initiate acquisition of information responsive to the detection or prediction of an event occurring during sleep. In this example, data associated with each event occurring during sleep may be organized as a sleep logbook entry. The system may collect data during the event and proximate in time to the event. For example, data may be collected before, during, and/or after the detected or predicted event.

The sleep logbook acquires information about one or more conditions related to sleep and/or sleep quality. A representative set of the conditions associated with sleep and/or sleep quality is listed in Table 1. In various embodiments, the acquisition of information may be controlled responsive to triggering events. In this embodiment, the system may start acquiring the information associated with sleep, stop acquiring the information, or continue to acquire the information in response to a triggering event. A triggering event may include, for example, one or more of a physiological event, a non-physiological event, a cardiovascular system event, respiratory system event, nervous system event, muscle system event, sleep-related event, disordered breathing event, sleep stage, or other events.

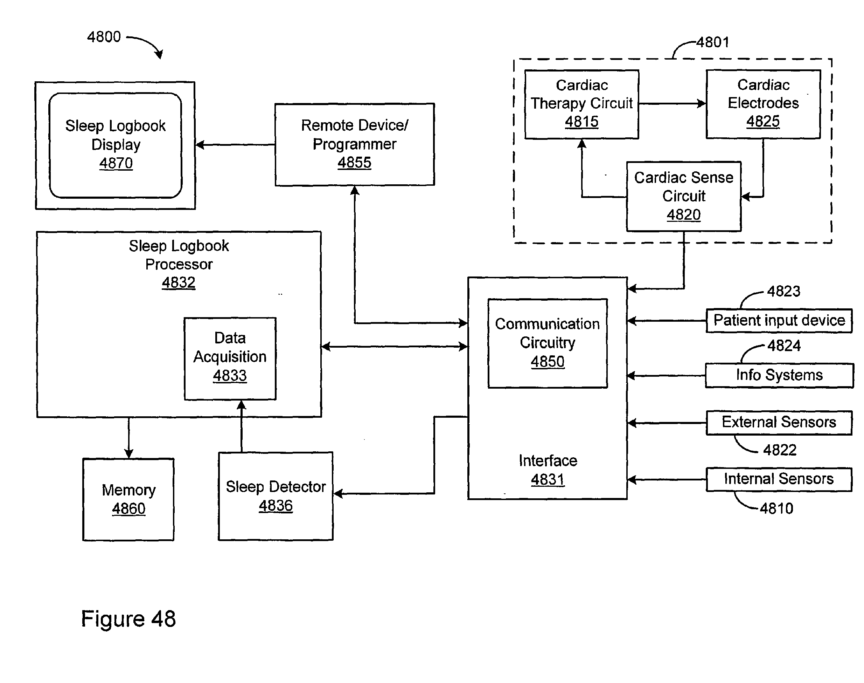

Figure 48 is a block diagram of a sleep logbook system 4800 in accordance with embodiments of the invention. In this exemplary embodiment, the system includes sleep logbook functionality provided along with a cardiac rhythm management. This embodiment is particularly useful for patients benefiting from cardiac pacing and/or defibrillation support through an implantable cardiac pulse generator.

Various patient conditions associated with sleep may be monitored through sensors 4822, patient input devices 4823, and/or information systems 4824. One or more of the patient conditions may be used by sleep detection circuitry 4836 to detect the onset and/or offset of sleep. Detection of sleep onset initiates the collection of information associated with the sleep period by the data acquisition unit 4833 of a sleep logbook processor 4832. For example, the data acquisition unit 4833 may collect information supplied by one or more of the sensors 4822, patient input devices 4823, and information systems 4824 before, during, and/or after the sleep period. The collected information associated with each sleep period is organized as a sleep logbook entry in the sleep logbook. The sleep logbook, or portions thereof, may be stored in memory 4860, transmitted to a remote device 4855, and/or displayed on a display device 4870.

The embodiment illustrated in Figure 48 may include, for example, a respiration sensor that senses a physiological condition modulated by patient respiration. In one embodiment, the respiration sensor may comprise an implantable transthoracic impedance sensor. Other methods of sensing respiration are also possible. Such methods may include, for example, the use of patient-external respiratory bands, respiration flowmeter measurements, implantable or patient-external breath sound detection, blood oxygen levels, and/or other processes. The respiration sensor may acquire information used in the detection of sleep onset and offset, as described in greater detail below. Additionally or alternatively, respiration sensing may be used, for example, to acquire a respiration waveform before, during, and/or after an event affecting the patient respiration. The respiration waveform may be a component of the sleep logbook entry.

Information about various conditions associated with and/or occurring during sleep may be acquired using sensors 4810, 4822, patient input devices 4823 and/or other information systems 4824. The sensors may comprise patient-internal 4810 and/or patient-external sensors 4822 coupled through leads or wirelessly to the interface 4831 of the sleep logbook system 4800. The sensors may sense various physiological and/or non-physiological conditions. The patient input device 4823 allows the patient to input information relevant to conditions affecting the patient that may be useful in generating a sleep log. For example, the patient input device 4823 may be particularly useful for acquiring information known to the patient, such as information related to patient smoking, drug use, recent exercise level, and/or other patient activities, symptoms, or perceptions, including patient perceptions of daytime sleepiness and/or sleep quality. The information provided by the patient-input device may include patient-known information that is not automatically sensed or detected by the sleep logbook system 4800.

The sleep logbook system 4800 may also include one or more information systems 4824 such as a remote computing device and/or a network-based server. The event information processor 4832 may access the information systems 4824 to acquire information from databases and/or other information sources stored on or generated by the remote computing devices and/or servers. The information acquired from the information systems 4824 may be recorded in the sleep logbook along with other information relevant to the event affecting sleep. In one exemplary implementation, the sleep logbook system 4800 may access an internet connected air quality server to collect data related to environmental conditions, such as an ambient pollution index. In another implementation, the sleep logbook system 4800 may access the patient's medical history through a patient information server.

The sensors 4822, patient input devices 4823, and information systems 4824 are coupled to other components of the sleep logbook system 4800 through interface circuitry 4831. The interface 4831 may include circuitry for energizing the sensors 4822 and/or for detecting and/or processing signals generated by the sensors. The interface 4831 may include, for example, driver circuitry, amplifiers, filters, sampling circuitry, and/or A/D converter circuitry for conditioning the signals generated by the sensors.

The interface 4831 may also include circuitry 4850 for communicating with the patient input device 4823, information systems 4824, a device programmer 4855, an APM system (not shown), or other remote devices. Communication with the patient input device 4823, information systems 4824 and/or a remote device programmer 4855 and/or other remote devices may be implemented using a wired connection or through a wireless communication link, such as a Bluetooth or other proprietary wireless link. The communication circuitry 4850 may also provide the capability to wirelessly communicate with various sensors, including implantable, subcutaneous, cutaneous, and/or external sensors.

The sleep logbook functionality may optionally be provided in a medical device that includes a therapy system, such as an implantable cardiac rhythm management system 4801. The cardiac rhythm management system 4801 may include cardiac electrodes 4825 electrically coupled to the patient's heart. Cardiac signals sensed by cardiac sense circuitry 4820 may be used in the detection and treatment of various anomalies of the heart rhythm. Anomalous heart rhythms may include, for example, a rhythm that is too slow (bradycardia), a heart rhythm that is too fast (tachycardia), and/or a heart rhythm that involves insufficiently synchronized contractions of the atria and/or ventricles, a symptom of congestive heart failure.

If an arrhythmia is detected by the cardiac rhythm management system, then a cardiac therapy circuit 4815 may deliver cardiac therapy to the heart in the form of electrical stimulation pulses, such as pacing and/or cardioversion/defibrillation pulses. The cardiac signals and/or cardiac conditions, e.g., arrhythmia conditions, derived or detected through the use of the cardiac signals may be associated with sleep. The cardiac information associated with sleep may be acquired and organized by the sleep logbook system 4800.

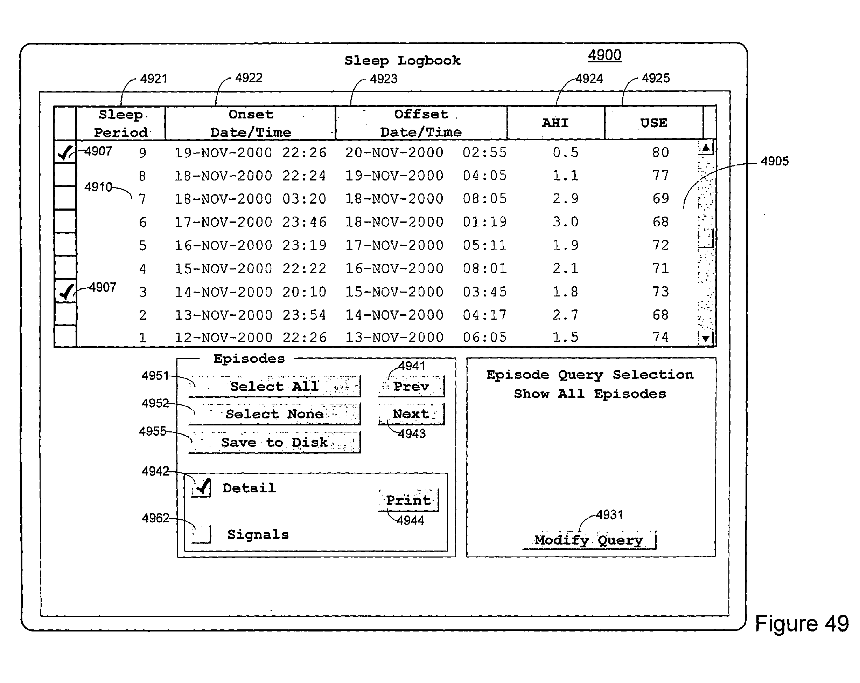

A user interface may be used to view and/or access the sleep logbook information. Figure 49 illustrates an exemplary depiction of a user interface display 4900. An area 4905 of the display may be used to provide textual or graphical information about sleep. As illustrated in Figure 49, a menu 4910 of sleep periods may be presented and may enable the user to access additional information related to the sleep periods and/or to sleep disorder events occurring during the sleep periods. The menu 4910 may provide a summary of parameters associated with sleep periods contained in the sleep logbook. As illustrated in Figure 49, one or more summary parameter headings, such as sleep period 4921, onset date/time 4922, offset date/time 4923, apnea/hypopnea index 4924, uninterrupted sleep efficiency 4925, among other parameter headings, may be presented at the top of the menu 4910 or in another convenient location. The summary parameter headings 4921-4925 may be programmable, and additional or alternative parameter headings to those depicted in Figure 49 may be selected.

The sleep periods displayed as menu items in the menu 4910 may be selected by a user according to episode number, date/time, duration, or by other criteria such as by one or more sleep quality indices. Additionally or alternatively, the menu items may reflect one or more sleep disorder events, e.g., movement disorder events and/or disordered breathing events. The menu items may be selected for display based on various criteria ranges and/or thresholds. For example, in the example screen illustrated in Figure 49, different groups of sleep periods selected as menu items may be selected by activating the modify query button 4931. In an alternate scenario, different groups of sleep disorder events selected as menu items may be selected by activating the modify query button 4931. The modify query button 4931 and other buttons illustrated on the display may be voice activated, activated through touching the display screen, or by operating a keyboard or pointing device, for example.

In one implementation, activation of the modify query button 4931 initiates a dialog session that allows the user to select sleep periods and/or sleep disorder events to be presented in the menu organized with respect to various criteria such as by date/time, duration, type, sleep quality metrics, or by other criteria parameters. In one example, the user may select all sleep periods having an uninterrupted sleep efficiency (USE) metric below a threshold to be presented as menu items. In another example, the user may select all sleep periods between a first date and a second date. In yet another example, the user may select all sleep disorder events of a particular type that occurred while the patient experienced certain environmental conditions, e.g., ambient temperature range and/or humidity range. In yet another example, the user may choose to select all sleep periods or all sleep disorder events represented in the sleep logbook. The selection criteria may be displayed in an episode query selection area 4932 of the display. The episode query selection area 4932 in the depiction of a sleep logbook display shown in Figure 49 indicates that all sleep periods have been selected to be displayed as menu items.

The menu 4910 allows the user to choose sleep periods for which additional textual and/or graphical information is displayed. The additional information provides more detailed information about the selected periods beyond the summary information presented in the menu 4910. In the exemplary illustration depicted in Figure 49, the selections are indicated by check marks 4907 beside the selected sleep periods. For convenience, the display may include a select all button 4951 and/or a select none button 4952. Activation of the select all button 4951 causes all sleep periods in the menu 4910 to be selected. Activation of the select none button 4952 causes all sleep periods in the menu 4910 to be deselected.

Following selection of one or more sleep periods in the menu, activation of the detail button 4942 causes detailed textual information associated with a selected sleep period to be presented on the display screen. The detail information may be displayed in the area of the screen 4905 previously occupied by the menu 4910, for example. The user may scroll back and forth through the textual information for the one or more selected sleep periods using the prev button 4941 and the next button 4943. The textual information may be printed upon activation of the print button 4944, or may be saved to a disk, or other storage medium, through activation of the save to disk button 4955.

Graphical information associated with the selected sleep periods may be displayed upon activation of the signals button 4962. In one implementation, a respiration waveform acquired during all or a portion of a selected sleep period may be displayed in the area 4905 of the display previously used for the menu 4910. In one implementation, a respiration waveform may be acquired before, during and/or after respiration events that occur during sleep. Waveforms of other parameters, e.g., cardiac rhythm, patient activity, may additionally or alternatively be displayed. In one implementation, a marked waveform may be displayed. For example, a marked respiration waveform may include the respiration waveform along with one or more symbols aligned with the respiration waveform to indicate the occurrence of one or more conditions. The symbols may provide a numerical value or a textual description associated with the respiration characteristic, e.g., average respiration rate, expiratory slope, etc. In one example, various characteristics of disordered breathing events including quantifiable characteristics, such as episode duration, blood oxygen saturation, disordered breathing type, and/or other detected characteristics may also be displayed along with the respiration waveform. A user may scroll through the waveforms associated with the selected events using the prev and next buttons 4941, 4943.

Adaptation Based on Therapy Interactions

Some patients may receive cardiac electrical stimulation therapy for both disordered breathing as well as cardiac disorders such as bradycardia and/or CHF. Interactions may occur between cardiac electrical therapy to mitigate disordered breathing and the patient's cardiac pacing regimen, e.g., pacing for bradycardia or cardiac resynchronization. Such interactions may be factored into the assessment of the impact of disordered breathing therapy on the overall therapy delivered to the patient.

Interactions between cardiac therapy and disordered breathing therapy may occur, and detection of the interactions may be used to adjust therapy. In some cases, cardiac electrical therapy to mitigate disordered breathing may enhance cardiac pacing therapy directed to alleviate a cardiac dysfunction, such as bradycardia or CHF. For example, non-excitatory electrical stimulation of the left ventricle during an absolute refractory period may be beneficial to treat both CHF and disordered breathing.

In other examples, cardiac electrical therapy for disordered breathing may work at cross purposes with the patient's cardiac pacing regimen. A pacing therapy delivered for treatment of disordered breathing may increase the percentage of heart beats initiated by atrial pacing. However, a concurrent cardiac resynchronization therapy may be optimal when intrinsic atrial events are allowed to initiate a heart beat. In this situation, the disordered breathing therapy, the cardiac resynchronization therapy, or both therapies, may be adjusted to reduce undesirable therapy interactions.

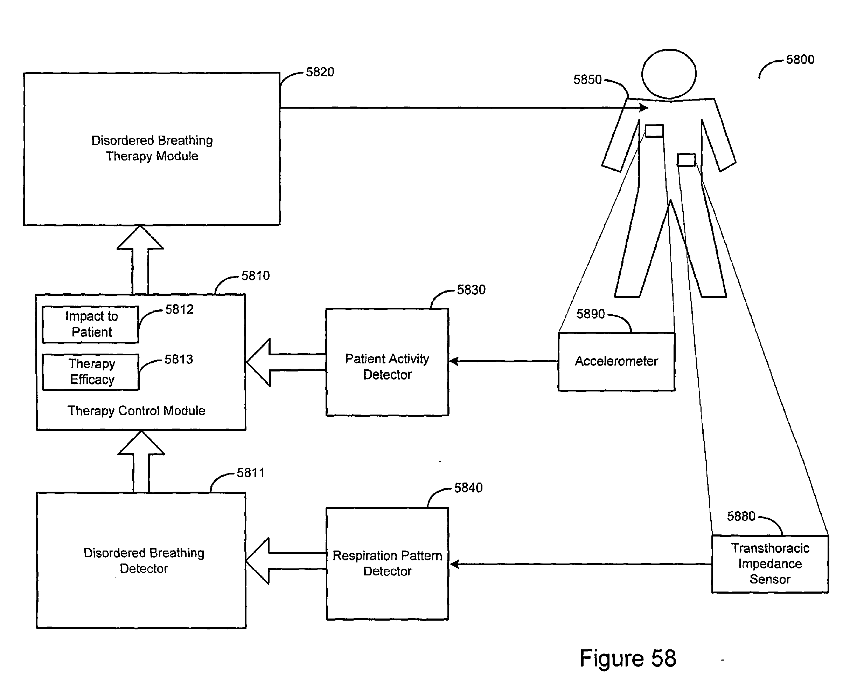

Figure 58 is a block diagram of a sleep-disordered breathing therapy system 5800 arranged in accordance with embodiments of the invention. The therapy system 5800 includes a transthoracic impedance sensor 5880 that provides a signal associated with the respiration of a patient 5850. The output of the transthoracic impedance sensor is coupled to a respiration pattern detector 5840. The patient's respiration patterns are analyzed by a disordered breathing detector 5811 to detect sleep-disordered breathing as described in more detail above.

The sleep-disordered breathing detector 5811 is coupled to a therapy control module 5810. If sleep-disordered breathing is detected by the disordered breathing detector 5811, the therapy control module 5810 signals a therapy module 5820 to deliver disordered breathing therapy to the patient 5850. The therapy control module 5810 performs assessment of the therapy and adapts the therapy to enhance therapy efficacy, to deliver therapy that reduces an impact of the therapy on the patient, or to achieve a combination of these therapeutic goals.

The therapy control module 5820 may include, for example, circuitry 5813 for evaluating therapy efficacy and circuitry 5812 to assess the impact of the therapy on the patient. In one embodiment, the efficacy of the therapy is assessed by analyzing the patient's respiration patterns following therapy delivery to detect and classify further episodes of disordered breathing. If the disordered breathing episode continues, or if the severity of the disordered breathing is not sufficiently mitigated by the therapy, the therapy may be adapted by the therapy control module 5810 to provide a more effective therapy.

In another example implementation, the therapy may be adapted based on one or more of the acute and/or chronic physiological responses to disordered breathing as discussed above. For example, the therapy may be adapted based on a level of hypoxia, intrathoracic pressure, or heart rate surges experienced by the patient during or shortly after a disordered breathing episode. Further, therapy may be adapted based on various chronic conditions, including heart rate variability, or increases in blood pressure or sympathetic nerve activity. A number of chronic physiological responses to disordered breathing may be detected after termination of one or more disordered breathing episodes, e.g., during periods of wakefulness. Further, adaptation of the therapy may be accomplished based on a combination of acute and chronic effects.

One method of evaluating the impact of the therapy on the patient involves determining the number of arousals per hour experienced by the patient. In one example, an accelerometer 5890 coupled to a patient activity detector 5830 may be used to produce a signal indicative of patient activity. If the therapy is effective, but the number of arousals per hour experienced by the patient are unacceptably high, the therapy may be adapted by the therapy control module 5810 to reduce therapy impact.

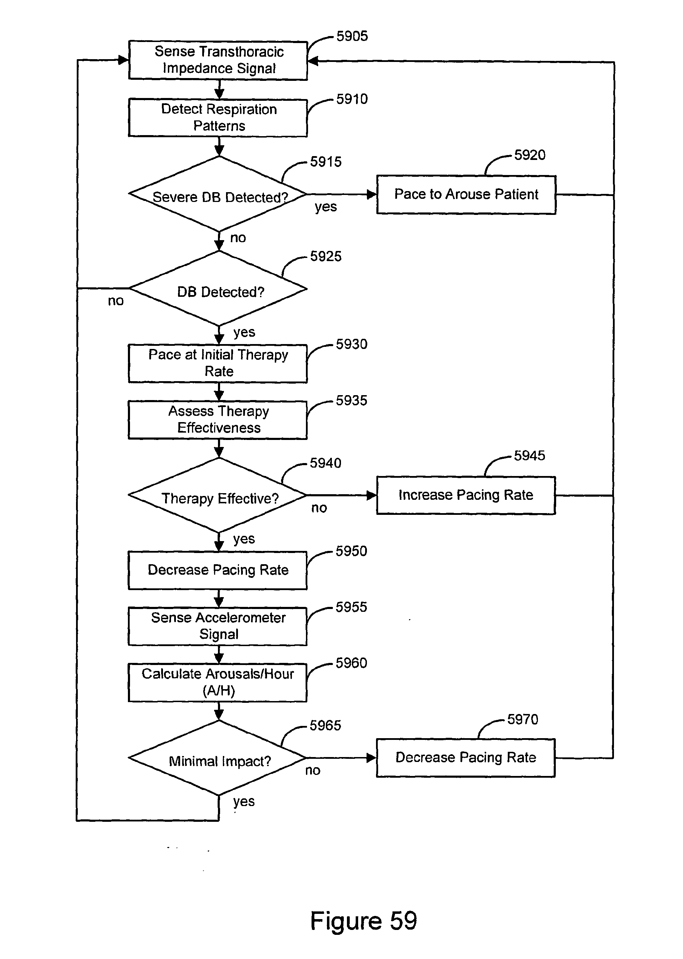

Figure 59 is a flow graph illustrating a method for providing cardiac electrical therapy to mitigate sleep-disordered breathing according to embodiments of the invention. As described above in connection with the block diagram of Figure 58, a transthoracic impedance sensor may be used to provide a signal characterizing the patient's respiration patterns. An accelerometer signal is used to assess therapy impact based on the number of arousals per hour experienced by the patient. More sensitive techniques for detecting arousals, e.g., EEG, may be used place of, or in addition to, the accelerometer method.

The signal from the transthoracic impedance sensor is sensed 5905 and used to detect 5910 the patient's respiration waveform patterns. If the patient's respiration patterns are consistent 5915 with severe disordered breathing (DB), cardiac therapy is delivered 5920 to the patient initially at a relatively aggressive level. In the case of cardiac electrical therapy involving pacing at a relatively high rate, the therapy may cause the patient to arouse from sleep, thereby terminating the severe disordered breathing episode. The cardiac electrical therapy may be modified over the course of the night to reduce the level of impact.

If the patient's respiration patterns do not indicate 5915 severe disordered breathing, but are consistent 5925 with disordered breathing (DB) respiration patterns, cardiac electrical therapy is delivered 5930 at an initial level, for example, at a pacing rate of about 5 to about 15 bpm above the patient's intrinsic rate or the patient's normal sleep rate.

The effectiveness of the therapy is evaluated 5935, for example, by analyzing the patient's respiration patterns to detect and assess further disordered breathing episodes, if any. If the therapy is not effective 5940, e.g., the disordered breathing continues or additional incidents of disordered breathing are detected, the therapy is modified 5945 by increasing the level of therapy by a predetermined amount, e.g., about 5 bpm for cardiac pacing therapy. If the therapy is effective 5940, the therapy level may be decreased 5950 by a predetermined amount, for example, about 5 bpm in the case of cardiac pacing therapy. The level of the adapted therapy may be constrained by upper and lower limits, e.g., upper and lower disordered breathing cardiac pacing therapy limits.

The impact of the therapy on the patient can be determined based on the patient's sleep quality. One measure of sleep quality involves calculating the number of arousals per hour experienced by the patient. During an arousal from sleep, the patient's activity increases. The level of patient activity may be detected by sensing 5955 an accelerometer signal responsive to patient movement, or by analyzing the patient's minute ventilation signal, or using a combination of accelerometer and minute ventilation signals. Based on the detected patient activity as indicated by the activity signals, the number of arousals experienced by the patient per hour (A/H) may be calculated 5960. If the number of arousals per hour experienced by the patient is below a predetermined threshold, then the patient's sleep quality is acceptable, and the impact 5965 of the therapy is determined to be acceptable. If the therapy impact is determined to be acceptable 5965, the therapy level is not modified. If the number of arousals per hour exceeds the threshold, then the impact 5965 of the therapy is not acceptable and the therapy level, for example, the pacing rate, may be decreased 5970.

It is understood that the components and functionality depicted in the figures and described herein can be implemented in hardware, software, or a combination of hardware and software. It is further understood that the components and functionality depicted as separate or discrete blocks/elements in the figures can be implemented in combination with other components and functionality, and that the depiction of such components and functionality in individual or integral form is for purposes of clarity of explanation, and not of limitation.

Various modifications and additions can be made to the various embodiments discussed hereinabove without departing from the scope of the present invention. Accordingly, the scope of the present invention should not be limited by the particular embodiments described above, but should be defined only by the claims set forth below and equivalents thereof.

## Claims

**1.** A medical device, comprising:

a detector system (270, 280, 290) configured to detect patient conditions;
a disordered breathing detection system (258) coupled to the detector system and configured to detect disordered breathing;
a therapy control system (265) coupled to the disordered breathing detection system and the detector system and configured to adapt a cardiac electrical therapy to mitigate the disordered breathing; and
a therapy delivery system (232) coupled to the therapy control system and configured to deliver the adapted therapy to the patient, wherein at least one of the detector system, the disordered breathing detection system, the therapy control system, and the therapy delivery system includes an implantable component.

**2.** The medical device of claim 1, wherein the detector system comprises at least one of a patient-external sensor (290) and a patient input device (270).

**3.** The medical device of claim 1, wherein the therapy control system (265) is configured to adapt the therapy to reduce an impact of the therapy on the patient.

**4.** The medical device of claim 1, wherein the therapy control system (265) is configured to adapt the therapy based on patient comfort.

**5.** The medical device of claim 1, wherein the therapy control system (265) is configured to adapt the therapy based on sleep quality.

**6.** The medical device of claim 1, wherein the therapy control system (265) is configured to adapt the therapy based on respiration quality.

**7.** The medical device of claim 1, wherein the therapy control system (265) is configured to adapt the therapy to enhance therapy efficacy.

**8.** The medical device of claim 1, wherein the therapy control system (265) is configured to adapt the therapy to reduce interaction between a therapy to treat a cardiac disorder and the therapy to mitigate the disordered breathing.

**9.** The medical device of claim 1, wherein the therapy control system (265) is configured to adapt the therapy to a less aggressive therapy if an initial therapy does not reduce an impact of the therapy on the patient.

**10.** The medical device of claim 1, wherein the therapy control system (265) is configured to adapt the therapy by modifying a pacing regimen.

**11.** The medical device of claim 1, wherein the therapy control system (265) is configured to adapt the therapy by adjusting a pacing rate.

**12.** The medical device of claim 1, wherein the therapy control system (265) is configured to adapt the therapy by adjusting a pacing mode.

**13.** The medical device of claim 1, wherein the therapy control system (265) is configured to adapt the therapy by adjusting a pacing site.

**14.** The medical device of claim 1, wherein at least one of the detector system (270, 280, 290), the disordered breathing detection system (258), the therapy control system (265), and the therapy delivery system (232) is disposed within a housing of an implantable transthoracic cardiac stimulation device (402).

**15.** The medical device of claim 1, wherein the patient conditions comprise a non-physiological condition.

**16.** The medical device of claim 1, further comprising a sleep stage detector (256) configured to determine sleep stage, wherein the therapy control system is configured to control sleep stage informed therapy based on the sleep stage.

**17.** The medical device of claim 1, wherein the adapted therapy comprises a biventricular pacing therapy.

**18.** The medical device of claim 1, wherein the adapted therapy comprises a non-excitatory cardiac electrical therapy.

**19.** The medical device of claim 1, wherein the therapy control system (265) is configured to switch from a first therapy to a second therapy based on the patient conditions.

## Patentansprüche

**1.** Medizinische Vorrichtung, umfassend :

ein Detektorsystem (270, 280, 290), das derart ausgelegt ist, dass es Patientenzustände erfasst;
ein System (258) zum Erfassen einer gestörten Atmung, das mit dem Detektorsystem verbunden und derart ausgelegt ist, dass es eine gestörte Atmung erfasst;
ein Therapiesteuerungssystem (265), das mit dem System zum Erfassen einer gestörten Atmung und dem Detektorsystem verbunden und ausgelegt ist, eine elektrische Herztherapie anzupassen, um die gestörte Atmung zu lindern; und
ein Therapieabgabesystem (232), das mit dem Therapiesteuerungssystem verbunden und ausgelegt ist, die angepasste Therapie an den Patienten abzugeben, wobei vom Detektorsystem, dem System zum Erfassen der gestörten Atmung, dem Therapiesteuerungssystem und dem Therapieabgabesystem wenigstens ein System eine implantierbare Komponente enthält.

2. Medizinische Vorrichtung nach Anspruch 1, wobei das Detektorsystem wenigstens einen patientenexternen sensor (290) und/oder eine Patienteneingabevorrichtung (270) umfasst.

3. Medizinische Vorrichtung nach Anspruch 1, wobei das Therapiesteuerungssystem (265) ausgelegt ist, die Therapie derart anzupassen, dass eine Nebenwirkung der Therapie auf den Patienten verringert wird.

4. Medizinische Vorrichtung nach Anspruch 1, wobei das Therapiesteuerungssystem (265) ausgelegt ist, die Therapie auf der Basis des Patientenkomforts anzupassen.

5. Medizinische Vorrichtung nach Anspruch 1, wobei das Therapiesteuerungssystem (265) ausgelegt ist, die Therapie auf der Basis der Schlafqualität anzupassen.

6. Medizinische Vorrichtung nach Anspruch 1, wobei das Therapiesteuerungssystem (265) ausgelegt ist, die Therapie auf der Basis der Atmungsqualität anzupassen.

7. Medizinische Vorrichtung nach Anspruch 1, wobei das Therapiesteuerungssystem (265) ausgelegt ist, die Therapie derart anzupassen, dass die Therapiewirksamkeit erhöht wird.

8. Medizinische Vorrichtung nach Anspruch 1, wobei das Therapiesteuerungssystem (265) ausgelegt ist, die Therapie derart anzupassen, dass eine Interaktion zwischen einer Therapie zum Behandeln einer Herzstörung und der Therapie zum Lindern der gestörten Atmung verringert wird.

9. Medizinische Vorrichtung nach Anspruch 1, wobei das Therapiesteuerungssystem (265) ausgelegt ist, die Therapie an eine weniger aggressive Therapie anzupassen, wenn eine anfängliche Therapie eine Nebenwirkung der Therapie auf dem Patienten nicht verringert.

10. Medizinische Vorrichtung nach Anspruch 1, wobei das Therapiesteuerungssystem (265) ausgelegt ist, die Therapie durch Modifizieren eines Schrittmacherregimes anzupassen.

11. Medizinische Vorrichtung nach Anspruch 1, wobei das Therapiesteuerungssystem (265) ausgelegt ist, die Therapie durch Einstellen einer Schrittmacherrate anzupassen.

12. Medizinische Vorrichtung nach Anspruch 1, wobei das Therapiesteuerungssystem (265) ausgelegt ist, die Therapie durch Einstellen eines Schrittmachermodus anzupassen.

13. Medizinische Vorrichtung nach Anspruch 1, wobei das Theräpiesteuerungssystem (265) ausgelegt ist, die Therapie durch Einstellen einer Schrittmacherstelle anzupassen.

14. Medizinische Vorrichtung nach Anspruch 1, wobei vom Detektorsystem (270, 280, 290), dem system (258) zum Erfassen einer gestörten Atmung, dem Therapiesteuerungssystem (295) und dem Therapieabgabesystem (232) wenigstens ein System innerhalb eines Gehäuses einer implantierbaren transthorakalen Herzstimulationsvorrichtung (402) angeordnet ist.

15. Medizinische Vorrichtung nach Anspruch 1, wobei die Patientenzustände einen nicht-physiologischen zustand umfassen.

16. Medizinische Vorrichtung nach Anspruch 1, ferner umfassend einen Schlafstadiumdetektor (256), der ausgelegt ist, die über das Schlafstadium informierte Therapie auf der Basis des Schlafstadiums zu steuern.

17. Medizinische Vorrichtung nach Anspruch 1, wobei die angepasste Therapie eine biventrikulare Schrittmachertherapie umfasst.

18. Medizinische Vorrichtung nach Anspruch 1, wobei die angepasste Therapie eine nicht-stimulierende, elektrische Herztherapie umfasst.

19. Medizinische Vorrichtung nach Anspruch 1, wobei das Therapiesteuerungssystem (265) ausgelegt ist, von einer ersten Therapie auf eine zweite Therapie auf der Basis der Patientenzustände umzuschalten.

## Revendications

1. Dispositif médical, comprenant :

   un système détecteur (270, 280, 290) configuré pour détecter les états d'un patient :
   un système de détection de trouble respiratoire (258) relié au système détecteur et configuré pour détecter un trouble respiratoire ;
   un système de contrôle de thérapie (265) relié au système de détection de trouble respiratoire et au système détecteur, et configuré pour adapter une thérapie électrique cardiaque afin d'atténuer le trouble respiratoire ; et
   un système d'administration de thérapie (232) relié au système de contrôle de thérapie et configuré pour administrer la thérapie adaptée au patient, dans lequel au moins un parmi le système détecteur, le système de détection de trouble respiratoire, le système de contrôle de thérapie et le système d'administration de thérapie comprend un composant implantable.

2. Dispositif médical selon la revendication 1, dans lequel le système détecteur comprend au moins un parmi un capteur externe au patient (290) et un dispositif d'entrée d'informations sur le patient (270).

3. Dispositif médical selon la revendication 1, dans lequel le système de contrôle de thérapie (265) est configuré pour adapter la thérapie afin de réduire l'impact de la thérapie sur le patient.

4. Dispositif médical selon la revendication 1, dans lequel le système de contrôle de thérapie (265) est configuré pour adapter la thérapie en se basant sur le confort du patient.

5. Dispositif médical selon la revendication 1, dans lequel le système de contrôle de thérapie (265) est configuré pour adapter la thérapie en se basant sur la qualité du sommeil.

6. Dispositif médical selon la revendication 1, dans lequel le système de contrôle de thérapie (265) est configuré pour adapter la thérapie en se basant sur la qualité de la respiration.

7. Dispositif médical selon la revendication 1, dans lequel le système de contrôle de thérapie (265) est configuré pour adapter la thérapie afin d'améliorer l'efficacité de la thérapie.

8. Dispositif médical selon la revendication 1, dans lequel le système de contrôle de thérapie (265) est configuré pour adapter la thérapie afin de réduire l'interaction entre une thérapie pour traiter un trouble cardiaque et la thérapie pour atténuer le trouble respiratoire.

9. Dispositif médical selon la revendication 1, dans lequel le système de contrôle de thérapie (265) est configuré pour adapter la thérapie pour une thérapie moins agressive si la thérapie initiale ne réduit pas l'impact de la thérapie sur le patient.

10. Dispositif médical selon la revendication 1, dans lequel le système de contrôle de thérapie (265) est configuré pour adapter la thérapie en modifiant le régime de stimulation.

11. Dispositif médical selon la revendication 1, dans lequel le système de contrôle de thérapie (265) est configuré pour adapter la thérapie en ajustant la fréquence de stimulation.

12. Dispositif médical selon la revendication 1, dans lequel le système de contrôle de thérapie (265) est configuré pour adapter la thérapie en ajustant le mode de stimulation.

13. Dispositif médical selon la revendication 1, dans lequel le système de contrôle de thérapie (265) est configuré pour adapter la thérapie en ajustant le site de stimulation.

14. Dispositif médical selon la revendication 1, dans lequel au moins un parmi le système détecteur (270, 280,290), le système de détection de trouble respiratoire (258), le système de contrôle de thérapie (265), et le système d'administration de thérapie (232) est disposé à l'intérieur d'un logement d'un dispositif de stimulation cardiaque transthoracique (402) implantable.

15. Dispositif médical selon la revendication 1, dans lequel les états d'un patient comprennent un état non physiologique.

**16.** Dispositif médical selon la revendication 1, comprenant en outre un détecteur d'étape de sommeil (256) configuré pour déterminer l'étape de sommeil, dans lequel le système de contrôle de thérapie est configuré pour contrôler une thérapie informée de l'étape de sommeil en se basant sur l'étape de sommeil.

**17.** Dispositif médical selon la revendication 1, dans lequel la thérapie adaptée comprend une thérapie de stimulation biventriculaire.

**18.** Dispositif médical selon la revendication 1, dans lequel la thérapie adaptée comprend une thérapie électrique cardiaque non excitatoire.

**19.** Dispositif médical selon la revendication 1, dans lequel le système de contrôle de thérapie (265) est configuré pour passer d'une première thérapie à une seconde thérapie en se basant sur les états d'un patient.

110
Sense Conditions Affecting the Patient
120
Adapt Therapy to Mitigate Disordered Breathing Based on Sensed Conditions
130
Deliver the Adapted Therapy

Figure 1

200
Cardiac Electrical Stimulation Generator 232
231
230
Cardiac Arrhythmia Detector 252
Disordered Breathing Therapy Control 267
Therapy Assessment Processor 260
Sleep/Respiration Monitor 254
Therapy Controller 265
Sleep Detector 256
Input Signal Processor 250
Internal Sensors 280
External Sensors 290
Patient Input 270
Cardiac Rhythm Therapy Control 269
Disordered Breathing Detector/ Predictor 258

Figure 2

300
305
301
310
390
341
352
354
351
355
342
363
353

Figure 3

402
406
400
404

Figure 4A

Figure 4B
400
422
Detection Circuitry
403
Noise Reduction Circuitry
424
Sensing Circuitry
Diagnostics Circuitry
410
405
Control System
Micro-Processor
406
Memory
409
420
Power Source
Communications Circuitry
418
Shock Therapy Circuitry
416
Subcutaneous Electrode(s)
414
Can/Indifferent Electrode
407
Pacing Therapy Circuitry
430
461
Non-Electrophysiologic Sensor

Figure 4C

431
CAN
433
437
440
435
COIL

Figure 4D

431
CAN
433
437
440
445
COIL

Figure 4E

431
433
438
437
440
439

462
Can electrode
465
460
464
Coil electrode
462
Can electrode
460
465
Coil electrode
464

Figure 4F

Figure 4G

Figure 4H

461
Pulse
Generator
468
460
Electrode 2
465
469
Electrode 1

Impedance
520
510
Time
Expiration
Inspiration

Figure 5A

530
Sense Conditions Affecting the Patient
540
Detect Disordered Breathing Based on the Sensed Conditions
550
Adapt Therapy to Mitigate the Detected Disordered Breathing
560
Deliver the Adapted Therapy

Figure 5B

Volume (mL)
Impedance (Ω)
Time
640
610
620
630
Inspiration
650
Expiration
660
Non-breathing
670
Next Inspiration

Figure 6

Volume (mL)
Impedance (Ω)
Time
740
710
720
730
Inspiration
750
Expiration
760
Non-breathing
790
Sleep Apnea Interval
795
Severe Sleep Apnea Interval

Figure 7

Impedance
Tidal Volume
Time

Figure 8A

Impedance
Tidal Volume
Time

Figure 8B

901
Establish Sleep Apnea Interval, Severe Sleep Apnea Interval, Hypopnea TV Threshold, Hypopnea Inteval
905
Sense Transthoracic Impedance
910
Inspiration Threshold Reached?
no
yes
915
Inspiration
920
Max Value Reached?
no
yes
935
Expiration
940
Expiration Threshold Reached?
no
yes
955
Non-breathing
960
Inspiration Threshold Reached?
no
965
Time Out?
no
yes
970
Apnea
975
Time Out?
no
yes
980
Severe Apnea
yes
985
Determine Average Tidal Volume
990
Hypopnea TV Reached?
yes
no
992
Time Out?
no
yes
995
Hypopnea

Figure 9

Volume (mL)
Time
1020
Breathing Interval
1030
1010
1040
Duration Threshold
Impedance (Ω)

Figure 10

Volume (mL)
Time
1120
Duration Threshold
Tidal Volume Threshold
1110
Impedance (Ω)

Figure 11

Individual Events
Apnea (absence of breathing)
Hypopnea (intervals of reduced respiration)
Tachypnea (intervals of rapid respiraton)
Other
Central (due to absence of respiratory stimulus from central nervous system)
Obstructive (due to upper airway obstruction)
Central (due to absence of respiratory stimulus from central nervous system)
Obstructive (due to upper airway obstruction)
Hyperpnea (hyper-ventilation)
Rapid, Shallow Breathing (typically of prolonged duration)

Figure 12

Periodic Breathing
Obstructive
(cyclic respiratory patterns with an obstructive apnea or hypopnea event in each cycle)
Central
(cyclic respiratory patterns with a central apnea or hypopnea event in each cycle)
Mixed
(cyclic respiratory patterns with a mixture of obstructive and central apnea or hypopnea events in each cycle)
Cheyne-Stokes
(gradual waxing and waning of tidal volume, with a central apnea and hyperpnea event in each cycle)
Other

Figure 13

Trigger On
Trigger Off
1410
Apnea
Apnea
1420
Apnea
Hypopnea
1430
Hypopnea
Apnea
1450
Hypopnea
Hypopnea
1460
Hypopnea
Hypopnea

Figure 14A

Figure 14B

Figure 14C

Figure 14D

Figure 14E

Time
Voltage

Figure 14F

Time
Voltage

Figure 14G

Figure 15
New Tidal Volume, Breath Interval
1510
Breath Interval > Threshold?
1515
yes
no
Trigger Apnea On
1520
Tidal Volume > Threshold?
1525
yes
no
Declare Apnea
1530
Trigger Apnea Off
1532
Tidal Volume > Threshold?
1535
yes
no
Trigger Hypopnea On
1540
Apnea or Hypopnea Trigger On?
1545
yes
no
Event Duration >Threshold?
1550
yes
no
Not an Event
1555
Trigger Event Off
1560
Previously Triggered?
1565
apnea
hypopnea
no
Declare Apnea
1570
Declare Hypopnea
1575

1601
Detect Disordered Breathing
1602
Sense Motion Associated with Respiratory Effort
1603
Classify Disordered Breathing Event

Figure 16A

Disordered Breathing Event Processor 1637
Respiration Sensor 1635
1641
Disordered Breathing Classification Processor 1651
Motion Sensor 1661

Figure 16B

1671
Voltage
1672
Time
1673
1674
1675
Voltage
Time

Figure 16C

1676
Voltage
1677
Time
1673
1678
1679

Figure 16D

Sense One or More Conditions Associated with Disordered Breathing
1680
Sense Chest Wall Motion
1681
Disordered Breathing Detected?
1682
no
yes
Evaluate Chest Wall Motion
1683
Chest Wall Motion Greater Than Threshold?
1684
yes
no
Motion Associated with Respiratory Effort?
1685
no
yes
Central Disordered Breathing
1686
Obstructive Disordered Breathing
1687
Disordered Breathing Event Continues?
1688
A

Figure 16E

A
1690
Store, Transmit, Analyze Print and/ or Display Disordered Breathing Information (Optional)
1691
Provide Disordered Breathing Therapy Based on Classification of Disordered Breathing (Optional)
1692
Adjust Therapy Based on Classification of Disordered Breathing (Optional)

Figure 16F

1630
Sense Conditions Affecting the Patient
1640
Predict Disordered Breathing Based on the Sensed Conditions
1650
Adapt Therapy to Treat the Predicted Disordered Breathing
1660
Deliver the Adapted Therapy

Figure 16G

1750
Disordered Breathing Therapy Controller
1740
Disordered Breathing Prediction Engine
1710
Internal Sensors
1720
External Sensors
1730
Patient Input

Figure 17

1830
Precursor Conditions
1810
1820
Prediction of DB During Specified Period
1812
Predisposing Conditions
Prediction of Imminent Onset of DB
1814
Disordered Breathing Prediction
1840
Verification Conditions

Figure 18

1911
Criteria 1
1912
Criteria 2
1913
Criteria 3
1914
Criteria N
C1 > Level1
C2 > Level2
P1 > Level3
P2 > Level4
1910
Prediction Criteria Library
1920
Verification Criteria
1900
Disordered Breathing Prediction Engine

Figure 19

Population Data
2002
2004
Patient Specific Data
Establish Prediction Criteria Library
2006
2008
Detect Patient Conditions
no
2055
Disordered Breathing Occurs?
2010
Patient Conditions Consistent with Criteria?
no
2050
Form Possible Prediction Criteria
yes
yes
2015
yes
2045
2060
Consistent with a Possible Criteria?
yes
Predict Disordered Breathing
Accuracy < Selected Number?
no
2020
2040
no
2056
Disordered Breathing Occurs?
Update Prediction Criteria Accuracy
Form Possible Prediction Criteria
no
yes
2025
Update Possible Prediction Criteria Accuracy
Update Prediction Criteria Accuracy
2070
2075
2030
Accuracy > selected number?
no
Accuracy > Selected Number?
no
yes
2080
yes
2035
Add Criteria to Prediction Criteria Library
Form Possible Prediction Critera

Figure 20

2110
Detect Conditions Associated with Disordered Breathing
2120
Disordered Breathing Episode?
no
yes
2130
Deliver Therapy
2140
Detect Conditions Associated with Therapy Effectiveness
2150
Therapy Effective?
yes
no
2160
Adapt Therapy to Provide Less Aggressive Therapy
2170
Adapt Therapy to Provide More Aggressive Therapy

Figure 21

2210
Detect Conditions Associated with Disordered Breathing
2220
Disordered Breathing Episode?
no
yes
2230
Deliver Therapy
2240
Detect Conditions Used to Adapt Therapy
2245
Assess Therapy Efficacy
2250
Therapy Effective?
no
yes
2260
Adapt Therapy
2270
Assess Therapy Impact
2280
Impact Acceptable?
no
yes
2285
Therapy Complete?
no
yes
2290
Terminate Therapy

Figure 22

2340
Pollution Website Server
2335
Internet
2350
2330
2310
2319
2311
2316
2317
2312
2318
2320
2313
2314
2015
2319
2360
2370


Figure 23

2401
Sleep Detection Sensor
2402
Threshold Adjustment Sensor
2403
Sleep Confirmation Sensor
Interface
2431
Sleep Detector
2436

Figure 24

2505
Establish Sleep Threshold Associated with First Sleep-Related Condition
2510
Detect First Sleep-Related Condition
2520
Detect Second Sleep-Related Condition
2530
Adjust Sleep Threshold of First Sleep-Related Condition Using Second Sleep-Related Condition
2540
First Condition Consistent With Adjusted Threshold?
No
2560
Sleep Not Detected
Yes
2550
Sleep Detected

Figure 25

Figure 26

2610
Establish Preliminary Sleep Threshold for Accelerometer Signal
2620
Monitor Accelerometer Signal
2625
Monitor Minute Ventilation Signal
2630
Monitor Heart Rate
2635
Status of Minute Ventilation Signal Level?
High
Low
2640
Decrease Accelerometer Threshold
2645
Increase Accelerometer Sleep Threshold
2650
Accelerometer Less Than or Equal to Threshold?
no
yes
2660
Sleep Previously Detected?
no
yes
2670
Sleep Termination
2665
Sleep Previously Detected?
no
yes
2675
Sleep Continues
2680
Heart Rate Compatible with Sleep?
yes
no
2690
Sleep Onset

XL
Activity
XL (mg)
Time
150
100
50
0
10
12
14
16
18
20
22
24
2
4
6

Figure 27

HR
SIR
Heart Rate (bpm)
140
120
100
80
60
Time
10
12
14
16
18
20
22
24
2
4
6

Figure 28

160
140
120
100
80
60
40
20
0
0:00
1:00
2:00
3:00
4:00
5:00
6:00
7:00
8:00
9:00
10:00
11:00
12:00
13:00
14:00
15:00
16:00
17:00
18:00
19:00
20:00
21:00
22:00
23:00
1M
1M+1D
3M
3M+1D
5M
8M

Figure 29

XL
Activity
3010
3020
3030
XL (mg)
150
100
50
0
10
12
14
16
18
20
22
24
2
4
6
Time

Figure 30

3181
3182
3183
3184
3185
3186
3187
3188
3189
3191
3193
EEG Arousal
3194
3192
Arousal on EMG
Time

Figure 31

Adjust Therapy
3290
Correlate Arousals with Events
3285
Trend
3280
Develop Indices/ Diagnose
3275
Detect Sleep Disorder Event
3240
Monitor
3270
Detect Sleep
3220
Initiate Therapy
3250
Terminate Therapy
3260
Detect Arousal
3210

Figure 32

Arousal Sensor
3335
Arousal Detector
3360
Sleep Detector
3330
Accelerometer
3333
Cardiac Electrical Therapy Control/Delivery
3350
Impedance Electrodes
3342
Cardiac Sense Circuitry
3342
Cardiac Electrodes
3341
Disordered Breathing Detector
3320

Figure 33

3425
INPUT #1
CONTROLLER
PACING
RATE
INPUT #N-1
INPUT #N

Figure 34

TO
ATRIAL
THERAPY
CIRCUIT
3425
CONTROLLER
3505
PACING
CONTROL
MODULE
3520
FIRST
INDICATED
PACING INTERVAL
REGISTER
FILTER
3515
A—A
INTERVAL
TIMER
3510
ATRIAL
EVENT
MODULE
3501
FROM
ATRIAL
SENSING
CIRCUIT

Figure 35

A–A INTERVAL AAn
A
X
B
X
Tn–1
PREVIOUS
FIRST INDICATED
PACING INTERVAL
Σ
Tn
NEW
FIRST
INDICATED
PACING
INTERVAL
TO PACING
CONTROL
MODULE
DELAY
(E.G., OF
REGISTER 3520 )

Figure 36

A–A INTERVAL AAn
(IF SENSED)
A
C (IF PACED)
Tn–1
B
(IF SENSED)
D
(IF PACED)
PREVIOUS
FIRST INDICATED
PACING INTERVAL
Σ
Tn
NEW
FIRST
INDICATED
PACING
INTERVAL
TO PACING
CONTROL
MODULE
DELAY
(E.G., OF
REGISTER 3520)


Figure 37

A–A INTERVAL AAn
(IF SENSED)
a • w
b • w (IF PACED)
Σ
Tn
NEW
FIRST
INDICATED
PACING
INTERVAL
TO PACING
CONTROL
MODULE
$T_{n-1}$
(1–w)
PREVIOUS
FIRST INDICATED
PACING INTERVAL
DELAY
(E.G., OF
REGISTER 3520)

Figure 38

TO ATRIAL THERAPY CIRCUIT
3425
CONTROLLER
PACING CONTROL MODULE
3505
SELECTION MODULE
3925
SECOND INDICATED PACING INTERVAL REGISTER
3910
FIRST INDICATED PACING INTERVAL REGISTER
3520
FILTER
3515
A–A INTERVAL TIMER
3510
ATRIAL EVENT MODULE
3501
DISORDERED BREATHING THERAPY CONTROL
FROM ATRIAL SENSING CIRCUIT

Figure 39

UPPER RATE LIMIT (URL)
APP INDICATED RATE (T)
LOWER RATE LIMIT (LRL)
P
P
S
S
S
S
P
P
P
S
P
P
P
S
P
1/T0
1/T1
1/T2
1/T3
1/T4
1/T5
1/T6
1/T7
1/T8
1/T9
1/T10
1/T11
1/T12
1/T13
1/T14
1 2 3 4 5 6 7 8 9 10 11 12 13 14 15
ATRIAL BEAT NUMBER

Figure 40

UPPER RATE LIMIT (URL)
RATE (T)
RATE (X)
LOWER RATE LIMIT (LRL)
P
S
ATRIAL BEAT NUMBER
1 2 3 4 5 6 7 8 9 10 11 12 13 14 15
$\frac{1}{T_0}$ $\frac{1}{T_1}$ $\frac{1}{T_2}$ $\frac{1}{T_3}$ $\frac{1}{T_4}$ $\frac{1}{T_5}$ $\frac{1}{T_6}$ $\frac{1}{T_7}$ $\frac{1}{T_8}$ $\frac{1}{T_9}$ $\frac{1}{T_{10}}$ $\frac{1}{T_{11}}$ $\frac{1}{T_{12}}$ $\frac{1}{T_{13}}$ $\frac{1}{T_{14}}$

Figure 41

1.3
1.2
1.1
1.0
0.9
0.8
0.7
0.6
0.5
500 ms
(120 bps)
(URL)
1000 ms
(60 bpm)
(LRL)
AAn
b
a

Figure 42

4322
Detect or Predict Respiratory Event
4324
Initiate Collection of Medical Information Responsive to Detection or Prediction of the Event
4326
Collect Medical Information Associated with the Respiratory Event
4328
Organize Medical Information as a Respiratory Event Log Entry
4330
Provide User Interface for Accessing Log Entries

Figure 43

4400
Respiratory Log Display 4470
Remote Device/ Programmer 4455
4401
Cardiac Therapy Circuit 4415
Cardiac Electrodes 4425
Cardiac Sense Circuit 4420
Event Information Processor 4432
Communication Circuitry 4450
4423
Patient input device
4424
Info Systems
Sensors
Respiration Sensor 4445
4422
Interface 4431
Event Detector 4436
Long-Term Memory 4460
Short-Term Memory 4440

Figure 44

Respiration Event Logbook
4500
4521
4522
4523
4524
4525
4526
4505
Episode
Date/Time
Type
Duration
Sleep Stage
Environment
9 12-NOV-2000 18:26 AP-C 20sec Stage 2
8 05-NOV-2000 23:31 HP 16sec Stage 2
7 29-OCT-2000 09:15 CS 37sec Awake
6 27-OCT-2000 19:01 AP-O 22sec REM
5 15-OCT-2000 18:56 AP-C 21sec Stage 1
4 12-OCT-2000 13:04 RSB 10sec Awake
3 12-OCT-2000 16:46 CS 20sec Awake
2 10-OCT-2000 23:45 AP-C 29sec Stage 2
1 09-OCT-2000 21:21 AP-O 21sec Stage 2
4507
4510
4507
Episodes
Select All
Select None
Save to Disk
Prev
Next
4541
4543
4551
4552
4555
Detail
Signals
Print
4542
4562
4544
Episode Query
Selection
Show All Episodes
4532
4531
Modify Query

Figure 45

Sliding Window
Data Acquired
4610
4610
4610
4630
4615
4640
4645
4650
Sliding Window
Data Acquired
4610
4610
4610
4620
4612
4630
4615
4640
4645
4650

Figure 46A

Figure 46B

4710
4720
Respiration Markers
RR 3500 ms
RR 3800 ms
RR 4800 ms
RR 5500 ms
RR 5500 ms
RR 4300 ms
RR 2700 ms
RR 2600 ms
RR 2400 ms
RR 3000 ms
RR 2800 ms
RR 3200 ms
RR 3500 ms
Hypopnea Detected
4730
22 sec
Hypopnea Ends
4735

Figure 47A

4760
ECG Markers
4750
4710
4720
Respiration Markers
RR 3500 ms
RR 3800 ms
RR 4800 ms
RR 5500 ms
RR 5500 ms
RR 4300 ms
RR 2700 ms
RR 2600 ms
RR 2400 ms
RR 3000 ms
RR 2800 ms
RR 3200 ms
RR 3500 ms
Hypopnea Detected
4730
Hypopnea Ends
22 sec
4735


Figure 47B

4801
Cardiac Electrodes 4825
Cardiac Sense Circuit 4820
Cardiac Therapy Circuit 4815
4823
Patient input device
4824
Info Systems
External Sensors
4822
Internal Sensors
4810
Communication Circuitry 4850
Interface 4831
Remote Device/ Programmer 4855
Sleep Logbook Processor 4832
Data Acquisition 4833
Sleep Detector 4836
Memory 4860
4800
Sleep Logbook Display 4870

Figure 48

4900
Sleep Logbook
Sleep Period
Onset Date/Time
Offset Date/Time
AHI
USE
9 19-NOV-2000 22:26 20-NOV-2000 02:55 0.5 80
8 18-NOV-2000 22:24 19-NOV-2000 04:05 1.1 77
7 18-NOV-2000 03:20 18-NOV-2000 08:05 2.9 69
6 17-NOV-2000 23:46 18-NOV-2000 01:19 3.0 68
5 16-NOV-2000 23:19 17-NOV-2000 05:11 1.9 72
4 15-NOV-2000 22:22 16-NOV-2000 08:01 2.1 71
3 14-NOV-2000 20:10 15-NOV-2000 03:45 1.8 73
2 13-NOV-2000 23:54 14-NOV-2000 04:17 2.7 68
1 12-NOV-2000 22:26 13-NOV-2000 06:05 1.5 74
Episodes
Select All
Select None
Save to Disk
Prev
Next
Detail
Signals
Print
Episode Query Selection
Show All Episodes
Modify Query
4905
4907
4910
4921
4922
4923
4924
4925
4931
4941
4942
4943
4944
4951
4952
4955
4962

Figure 49

5010
Detect REM-modulated Condition
5020
Classify Sleep Stage Based on the REM-modulated Condition
Figure 50A
5030
Detect Condition Associated with REM Sleep
5040
Detect Condition Associated with Sleep-Wake Status of Patient
5050
Classify Sleep Stage Using the Detected Conditions
Figure 50B

5100
5110
5120
REM Sensor
5130
Sleep/Sleep Stage Detector
Sleep-Wake Sensor

Figure 51

5200
5201
5230
5240
5250
Therapy
Testing
Monitoring
5220
Sleep/Sleep Stage Detector
5205
5210
5215
Sleep Stage Sensors
Respiration System Sensors
Cardiac System Sensors

Figure 52

5310
Detect REM-modulated Signal
5320
REM Signal Consistent with Threshold?
no
yes
5330
REM Stage Onset Previously Declared?
yes
no
5350
Declare REM Onset
5360
REM Stage Onset Previously Declared?
no
yes
5370
Declare REM Offset

Figure 53

5405
Detect Patient Activity Signal
5410
Activity Consistent with Sleep Threshold?
no
yes
5445
Sleep Onset Previously Declared?
no
yes
5415
Sleep Onset Previously Declared?
yes
no
5450
Declare Sleep Offset
5420
Declare Sleep Onset
5425
Detect REM-Modulated Signal
5430
Consistent with REM Threshold?
no
yes
5455
REM Onset Previously Declared?
no
yes
5435
REM Onset Previously Declared?
yes
no
5460
Declare REM Offset
5440
Declare REM Onset

Figure 54

5505
REM-modulated and Sleep/Wake Signals
5510
Sleep Stage Classification
5520
Breath-to-Breath Analysis
5525
Sleep State Informed Respiratory Analysis
5530
Respiration Signals
5532
Disordered Breathing Detection/Prediction
5534
Sleep State Informed Therapy
5550
Beat-to-Beat Analysis
5555
Sleep State Informed Arrhythmia Analysis
5560
Cardiac Information
5565
Arrhythmia Detection/ Prediction
5575
Sleep-State Informed Therapy
5585
Testing

Figure 55

5600
5690
5601
5656
Cardiac System Analysis
5650
Cardiac Sensing Electrodes
5646
Accelerometer
5652
5642
Therapy Control Unit
Sleep Stage Processor
5648
Muscle Atonia Sensor
5654
5644
Respiratory System Analysis
Transthoracic Impedance Sensor
5622
Cardiac Therapy Electrodes

Figure 56

5700
5710
5720
5740

Figure 57B

5700
5710
5720
5730

Figure 57A

5700
5760
5770
5710
5720
5700
5720
5710
5750

Figure 57D

Figure 57C

5800
5850
5890
Accelerometer
5880
Transthoracic Impedance Sensor
5820
Disordered Breathing Therapy Module
5830
Patient Activity Detector
5840
Respiration Pattern Detector
5810
5812
Impact to Patient
5813
Therapy Efficacy
Therapy Control Module
5811
Disordered Breathing Detector

Figure 58

5905
Sense Transthoracic Impedance Signal
5910
Detect Respiration Patterns
5915
Severe DB Detected?
yes
5920
Pace to Arouse Patient
no
5925
DB Detected?
no
yes
5930
Pace at Initial Therapy Rate
5935
Assess Therapy Effectiveness
5940
Therapy Effective?
no
5945
Increase Pacing Rate
yes
5950
Decrease Pacing Rate
5955
Sense Accelerometer Signal
5960
Calculate Arousals/Hour (A/H)
5965
Minimal Impact?
no
5970
Decrease Pacing Rate
yes

Figure 59

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5645570 A